# EUROPEAN PATENT SPECIFICATION

(11) **EP 4 522 621 B1**
(45) Date of publication and mention of the grant of the patent: **09.12.2026**
(21) Application number: 23731058.6
(22) Date of filing: 09.05.2023
(51) Int. Cl.: C07D 487/14, A61K 31/5025, A61P 35/00, A61P 35/02

(54) **6,6A,7,8,9,10-HEXAHYDRO-5H-PYRAZINO[1',2':4,5]PYRAZINO[2,3-C]PYRIDAZINE DERIVATIVES AS SMARCA4 PROTEIN DEGRADERS FOR THE TREATMENT OF CANCER**
6,6A,7,8,9,10-HEXAHYDRO-5H-PYRAZINO[1',2':4,5]PYRAZINO[2,3-C]PYRIDAZIN-DERIVATE ALS SMARCA4 PROTEINABBAUER ZUR BEHANDLUNG VON KREBS
DÉRIVÉS DE 6,6A,7,8,9,10-HÉXAHYDRO-5H-PYRAZINO[1',2':4,5]PYRAZINO[2,3-C]PYRIDAZINE EN TANT QUE DÉGRADEURS DU PROTÉIN SMARCA4 POUR LE TRAITEMENT DU CANCER

(30) Priority: 10.05.2022 US 202263340185 P
(43) Date of publication of application: 19.03.2025
(73) Proprietor: Prelude Therapeutics Incorporated, Wilmington, DE 19805 (US)
(72) Inventor: ROSE, John A., Newark, Delaware 19702 (US); BASCH, Corey Howard, Delmar, Delaware 19949 (US); MEI, Song, Wilmington, Delaware 19808 (US); BERSH, Klare Lazor, Glen Mills, Pennsylvania 19342 (US); ROTH, Danielle Julie Beam, Wilmington, Delaware 19805 (US); ROHLFING, Katarina, Conshohocken, Pennsylvania 19428 (US); WU, Xiaowei, Wilmington, Delaware 19809 (US); SHVARTSBART, Artem, Kennett Square, Pennsylvania 19348 (US); COMBS, Andrew Paul, Kennett Square, Pennsylvania 19348 (US); LU, Liang, Hockessin, Delaware 19707 (US)
(74) Representative: Carpmaels & Ransford LLP
(86) International application number: PCT/US2023/066755
(87) International publication number: WO 2023/220577

(56) References cited:
- WO-A1-2016/105518
- WO-A1-2021/252666
- WO-A1-2022/099117

## Description

### TECHNICAL FIELD

The description provides bifunctional compounds comprising a target protein binding moiety and a E3 ubiquitin ligase binding moiety, and associated methods of use. The bifunctional compounds are useful as modulators of targeted ubiquitination, especially with respect to Switch/Sucrose Non-Fermentable (SWI/SNF)-Related, Matrix-Associated, Actin-Dependent Regulator of Chromatin, Subfamily A, Member 2 (SMARCA2) (i.e., BRAHMA or BRM), which are degraded and/or otherwise inhibited by bifunctional compounds according to the present disclosure.

### BACKGROUND

The human SWItch/Sucrose Non-Fermentable (SWI/SNF) complexes are ATP-dependent chromatin remodelers. These large complexes play important roles in essential cellular processes, such as transcription, DNA repair and replication by regulating DNA accessibility.

Mutations in the genes encoding up to 20 canonical SWI/SNF subunits are observed in nearly 20% of all human cancers with the highest frequency of mutations observed in rhabdoid tumors, female cancers (including ovarian, uterine, cervical and endometrial), lung adenocarcinoma, gastric adenocarcinoma, melanoma, esophageal, and renal clear cell carcinoma.

SMARCA2 (BRM) and SMARCA4 (BRG1) are the subunits containing catalytic ATPase domains and they are essential for the function of SWI/SNF in perturbation of histone-DNA contacts, thereby providing access points to transcription factors and cognate DNA elements that facilitate gene activation and repression.

SMARCA2 and SMARCA4 shares a high degree of homology (up to 75%). SMARCA4 is frequently mutated in primary tumors (i.e., deleted or inactivated), particularly in lung cancer (12%), melanoma, liver cancer and pancreatic cancer. SMARCA2 is one of the top essential genes in SMARCA4-mutant (deleted) cancer cell line. This is because SMARCA4 deleted cancer cells exclusively rely on SMARCA2 ATPase activity for their chromatin remodeling activity for cellular functions such as cell proliferation, survival and growth. Thus, targeting SMARCA2 may be promising therapeutic approach in SMARCA4-related or deficient cancers (genetic synthetic lethality).

Previous studies have demonstrated the strong synthetic lethality using gene expression manipulation such as RNAi; downregulating SMARCA2 gene expression in SMARCA4 mutated cancer cells results in suppression of cancer cell proliferation. However, SMARCA2/4 bromodomain inhibitors (e.g., PFI-3) exhibit none to minor effects on cell proliferation inhibition [Vangamudi et al. Cancer Res 2015]. This phenotypic discrepancy between gene expression downregulation and small molecule-based approach lead us to investigating protein degradation bispecific molecules in SMARCA4 deficient cancers.

SMARCA2 is also reported to play roles in multiple myeloma expressing t(4;14) chromosomal translocation [Chooi et al. Cancer Res abstract 2018]. SMARCA2 interacts with NSD2 and regulates gene expression such as PRL3 and CCND1. SMARCA2 gene expression downregulation with shRNA reduces cell cycle S phase and suppresses cell proliferation of t(4;14) MM cells.

Therapeutic compounds that inhibit SMARCA2 and/or SMARCA4 are needed.

WO 2021/252666 relates to "bifunctional compounds comprising a target protein binding moiety and an E3 ubiquitin ligase binding moiety, and associated methods of use".

WO 2016/105518 relates to "bifunctional compounds which act as protein degradation inducing moieties".

WO 2022/099117 relates to "bifunctional compounds comprising a target protein binding moiety and an E3 ubiquitin ligase binding moiety, and associated methods of use".

### SUMMARY

The scope of the invention is defined by the appended claims.

The present disclosure is directed to compounds of Formula (I): 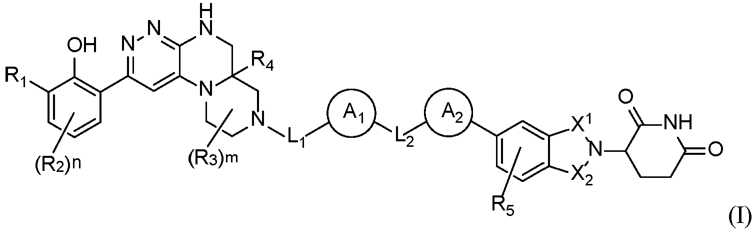 or a pharmaceutically acceptable salt thereof; wherein
R₁ is halo, C₁₋₆ alkyl, or haloalkyl;
each R₂ is independently H, D, or F;
each R₃ is independently H, D, C₁₋₆ alkyl, haloalkyl, or C₃₋₆ cycloalkyl;
n is 1, 2 or 3;
m is 1, 2, 3, 4, 5 or 6;
R₄ is H, D, C₁₋₆ alkyl, C₃₋₆ cycloalkyl, alkoxyalkyl, cyanoalkyl or haloalkyl;
R₅ is H, D, or F;
L₁ is a bond, C(R₃)₂, or CO;
L₂ is a bond, C(R₃)₂, or CO;
ring A₁ is a 3-7 membered cycloalkyl group, a 4-7-membered heterocycloalkyl group, an aryl group, or a heteroaryl group;
ring A₂ is a 3-7 membered cycloalkyl group, a 4-7-membered heterocycloalkyl group, an aryl group, or a heteroaryl group;
X₁ is CH₂, CO, CH=CH (when X₂ = CO), or N=CH (when X₂ = CO); and
X₂ is CH₂, CO, CH=CH (when X₁ = CO), or N=CH (when X₁ = CO).

References in the present description to methods of medical treatment or medical uses are to be understood as references to the compounds of the present invention for use in such methods.

Stereoisomers of the compounds of Formula I, and the pharmaceutical salts and stereoisomers thereof, are also contemplated, described, and encompassed herein. Methods of using compounds of Formula I are described, as well as pharmaceutical compositions including the compounds of Formula I.

### DETAILED DESCRIPTION OF ILLUSTRATIVE EMBODIMENTS

Unless otherwise defined, all technical and scientific terms used herein have the same meaning as commonly understood by one of ordinary skill in the art to which this disclosure pertains..

Where a range of values is provided, it is understood that each intervening value, to the tenth of the unit of the lower limit unless the context clearly dictates otherwise (such as in the case of a group containing a number of carbon atoms in which case each carbon atom number falling within the range is provided), between the upper and lower limit of that range and any other stated or intervening value in that stated range is encompassed within the disclosure. The upper and lower limits of these smaller ranges may independently be included in the smaller ranges is also encompassed within the disclosure, subject to any specifically excluded limit in the stated range. Where the stated range includes one or both of the limits, ranges excluding either both of those included limits are also included in the disclosure.

The following terms are used to describe the present disclosure. In instances where a term is not specifically defined herein, that term is given an art-recognized meaning by those of ordinary skill applying that term in context to its use in describing the present disclosure.

The articles "a" and "an" as used herein and in the appended claims are used herein to refer to one or to more than one (e.g., to at least one) of the grammatical object of the article unless the context clearly indicates otherwise. By way of example, "an element" means one element or more than one element.

The terms "co-administration" and "co-administering" or "combination therapy" refer to both concurrent administration (administration of two or more therapeutic agents at the same time) and time varied administration (administration of one or more therapeutic agents at a time different from that of the administration of an additional therapeutic agent or agents), as long as the therapeutic agents are present in the patient to some extent, preferably at effective amounts, at the same time. In certain preferred aspects, one or more of the present compounds described herein, are co-administered in combination with at least one additional bioactive agent, especially including an anticancer agent. In particularly preferred aspects, the co-administration of compounds results in synergistic activity and/or therapy, including anticancer activity.

The term "compound", as used herein, unless otherwise indicated, refers to any specific chemical compound disclosed herein and includes tautomers, regioisomers, geometric isomers, and where applicable, stereoisomers, including optical isomers (enantiomers) and other stereoisomers (diastereomers) thereof, as well as pharmaceutically acceptable salts and derivatives, including prodrug and/or deuterated forms thereof where applicable, in context. Deuterated small molecules contemplated are those in which one or more of the hydrogen atoms contained in the drug molecule have been replaced by deuterium.

Within its use in context, the term compound generally refers to a single compound, but also may include other compounds such as stereoisomers, regioisomers and/or optical isomers (including racemic mixtures) as well as specific enantiomers or enantiomerically enriched mixtures of disclosed compounds. The term also refers, in context to prodrug forms of compounds which have been modified to facilitate the administration and delivery of compounds to a site of activity. It is noted that in describing the present compounds, numerous substituents and variables associated with same, among others, are described. It is understood by those of ordinary skill that molecules which are described herein are stable compounds as generally described hereunder.

The term "ubiquitin ligase" refers to a family of proteins that facilitate the transfer of ubiquitin to a specific substrate protein, targeting the substrate protein for degradation. For example, an E3 ubiquitin ligase protein that alone or in combination with an E2 ubiquitin-conjugating enzyme causes the attachment of ubiquitin to a lysine on a target protein, and subsequently targets the specific protein substrates for degradation by the proteasome. Thus, E3 ubiquitin ligase alone or in complex with an E2 ubiquitin conjugating enzyme is responsible for the transfer of ubiquitin to targeted proteins. In general, the ubiquitin ligase is involved in polyubiquitination such that a second ubiquitin is attached to the first; a third is attached to the second, and so forth. Polyubiquitination marks proteins for degradation by the proteasome. However, there are some ubiquitination events that are limited to mono-ubiquitination, in which only a single ubiquitin is added by the ubiquitin ligase to a substrate molecule. Mono-ubiquitinated proteins are not targeted to the proteasome for degradation, but may instead be altered in their cellular location or function, for example, via binding other proteins that have domains capable of binding ubiquitin. Further complicating matters, different lysines on ubiquitin can be targeted by an E3 to make chains. The most common lysine is Lys48 on the ubiquitin chain. This is the lysine used to make polyubiquitin, which is recognized by the proteasome.

As used herein, "Cereblon (CRBN) E3 Ubiquitin Ligase" refers to the substrate recognition subunit of the Cullin RING E3 ubiquitin ligase complexes. CRBN are one of the most popular E3 ligases recruited by bifunctional Proteolysis-targeting chimeras (PROTACs) to induce ubiquitination and subsequent proteasomal degradation of a target protein (Maniaci C. et al., Bioorg Med Chem. 2019, 27(12): 2466-2479).

As used herein, the term "alkyl", by itself or as part of another substituent, means, unless otherwise stated, a straight or branched chain hydrocarbon radical having up to twelve carbon atoms. In some embodiments, the number of carbon atoms is designated (i.e., C₁₋C₈ means one to eight carbons). Examples of alkyl groups include methyl, ethyl, n-propyl, iso-propyl, n-butyl, t-butyl, iso-butyl, sec-butyl, n-pentyl, n-hexyl, n-heptyl, n-octyl, and the like. Alkyl groups may be optionally substituted as provided herein. In some embodiments, the alkyl group is a C₁₋C₆ alkyl; in some embodiments, it is a C₁₋C₄ alkyl.

When a range of carbon atoms is used herein, for example, C₁-C₆, all ranges, as well as individual numbers of carbon atoms are encompassed. For example, "C₁-C₃" includes C₁₋C₃, C₁₋C₂, C₂₋C₃, C₁, C₂, and C₃.

The term "optionally substituted", as used in combination with a substituent defined herein, means that the substituent may, but is not required to, have one or more hydrogens replaced with one or more suitable functional groups or other substituents as provided herein. For example, a substituent may be optionally substituted with one or more of: halo, cyano, C₁₋₆ alkyl, C₃₋₆ cycloalkyl, C₂₋₆ alkenyl, C₂₋₆ alkynyl, halo(C₁₋₆)alkyl, C₁₋₆ alkoxy, halo(C₁₋₆ alkoxy), C₁₋₆ alkylthio, C₁₋₆ alkylamino, NH₂, NH(C₁₋₆ alkyl), N(C₁₋₆ alkyl)₂, NH(C₁₋₆alkoxy), N(C₁₋₆ alkoxy)₂, -C(O)NHC₁₋₆ alkyl, -C(O)N(C₁₋₆ alkyl)₂, -C(O)NH₂, -C(O)C₁₋₆ alkyl, -C(O)₂C₁₋₆ alkyl, - NHCO(C₁₋₆ alkyl), -N(C₁₋₆ alkyl)CO(C₁₋₆ alkyl), -S(O)C₁₋₆ alkyl, -S(O)₂C₁₋₆ alkyl, oxo, 6-12 membered aryl, benzyl, pyridinyl, pyrazolyl, thiazolyl, isothiazolyl, or other 5 to 12 membered heteroaryl groups. In some embodiments, each of the above optional substituents are themselves optionally substituted by one or two groups.

The term "optionally substituted -CH₂-," refers to "-CH₂-" or substituted -CH₂-." A substituted -CH₂- may also be referred to as -CH(substituent)- or -C(substituent)(substituent)-, wherein each substituent is independently selected from the optional substituents described herein.

The term "cycloalkyl" as used herein refers to a 3-12 membered cyclic alkyl group, and includes bridged and spirocycles (e.g., adamantine). Cycloalkyl groups may be fully saturated or partially unsaturated. The term "cycloalkyl" also includes multiple condensed ring systems (e.g., ring systems comprising 2, 3 or 4 rings) wherein a single cycloalkyl ring (as defined above) can be condensed with one or more groups selected from heterocycles, carbocycles, aryls, or heteroaryls to form the multiple condensed ring system. Such multiple condensed ring systems may be optionally substituted with one or more (e.g., 1, 2, 3 or 4) oxo groups on the carbocycle or heterocycle portions of the multiple condensed ring. The rings of the multiple condensed ring system can be connected to each other via fused, spiro and bridged bonds when allowed by valency requirements. It is to be understood that the individual rings of the multiple condensed ring system may be connected in any order relative to one another. It is also to be understood that the point of attachment of a multiple condensed ring system (as defined above for a cycloalkyl) can be at any position of the cycloalkylic ring. Examples of cycloalkyl groups include cyclopropyl, cyclobutyl, cyclopentyl, cycloheptyl, cyclohexyl, cycloheptyl, cyclooctyl, indenyl, bicyclo[2.2.1]heptanyl, bicyclo[3.1.1]heptanyl, bicyclo[4.1.0]heptanyl, spiro[3.3] heptanyl, and spiro[3.4]octanyl. In some embodiments, the cycloalkyl group is a 3-7 membered cycloalkyl.

The term "akenyl" as used herein refers to C₂₋C₁₂ alkyl group that contains at least one carbon-carbon double bond. In some embodiments, the alkenyl group is optionally substituted. In some embodiments, the alkenyl group is a C₂₋C₆ alkenyl.

The term "akynyl" as used herein refers to C₂₋C₁₂ alkyl group that contains at least one carbon-carbon triple bond. In some embodiments, the alkenyl group is optionally substituted. In some embodiments, the alkynyl group is a C₂₋C₆ alkynyl.

The terms "alkoxy," "alkylamino" and "alkylthio", are used in their conventional sense, and refer to those alkyl groups attached to the remainder of the molecule via an oxygen atom ("oxy"), an amino group ("amino") or thio group. The term "alkylamino" includes mono- di-alkylamino groups, the alkyl portions can be the same or different.

The terms "halo" or "halogen", by itself or as part of another substituent, means a fluorine, chlorine, bromine, or iodine atom.

The term "heteroalkyl" refers to an alkyl group in which one or more carbon atom has been replaced by a heteroatom selected from S, O, P and N. Exemplary heteroalkyls include alkyl ethers, secondary and tertiary alkyl amines, alkyl amides, alkyl sulfides, and the like. The group may be a terminal group or a bridging group. As used herein reference to the normal chain when used in the context of a bridging group refers to the direct chain of atoms linking the two terminal positions of the bridging group.

The term "aryl" as used herein refers to a single, all carbon aromatic ring or a multiple condensed all carbon ring system wherein at least one of the rings is aromatic. For example, in certain embodiments, an aryl group has 6 to 12 carbon atoms. Aryl includes a phenyl radical. Aryl also includes multiple condensed ring systems (e.g., ring systems comprising 2, 3 or 4 rings) having about 9 to 12 carbon atoms in which at least one ring is aromatic and wherein the other rings may be aromatic or not aromatic. Such multiple condensed ring systems are optionally substituted with one or more (e.g., 1, 2 or 3) oxo groups on any carbocycle portion of the multiple condensed ring system. The rings of the multiple condensed ring system can be connected to each other via fused, spiro and bridged bonds when allowed by valency requirements. It is to be understood that the point of attachment of a multiple condensed ring system, as defined above, can be at any position of the aromatic ring. Non-limiting examples of aryl groups include, but are not limited to, phenyl, indenyl, naphthyl, 1, 2, 3,4-tetrahydronaphthyl, and the like.

The term "heteroaryl" as used herein refers to a single aromatic ring that has at least one atom other than carbon in the ring, wherein the atoms are selected from the group consisting of oxygen, nitrogen and sulfur; "heteroaryl" also includes multiple condensed ring systems that have at least one such aromatic ring, which multiple condensed ring systems are further described below. Thus, "heteroaryl" includes single aromatic rings of from about 1 to 6 carbon atoms and about 1-4 heteroatoms selected from the group consisting of oxygen, nitrogen and sulfur. The sulfur and nitrogen atoms may also be present in an oxidized form provided the ring is aromatic. Exemplary heteroaryl ring systems include but are not limited to pyridyl, pyrimidinyl, oxazolyl or furyl. "Heteroaryl" also includes multiple condensed ring systems (e.g., ring systems comprising 2, 3 or 4 rings) wherein a heteroaryl group, as defined above, is condensed with one or more rings selected from heteroaryls (to form for example a naphthyridinyl such as 1,8-naphthyridinyl), heterocycles, (to form for example a 1, 2, 3, 4-tetra-hydronaphthyridinyl such as 1,2,3,4-tetrahydro-1,8-naphthyridinyl), carbocycles (to form for example 5,6,7,8-tetrahydroquinolyl) and aryls (to form for example indazolyl) to form the multiple condensed ring system. Thus, a heteroaryl (a single aromatic ring or multiple condensed ring system) has about 1-20 carbon atoms and about 1-6 heteroatoms within the heteroaryl ring. A heteroaryl (a single aromatic ring or multiple condensed ring system) can also have about 5 to 12 or about 5 to 10 members within the heteroaryl ring. Multiple condensed ring systems may be optionally substituted with one or more (e.g., 1, 2, 3 or 4) oxo groups on the carbocycle or heterocycle portions of the condensed ring. The rings of a multiple condensed ring system can be connected to each other via fused, spiro and bridged bonds when allowed by valency requirements. It is to be understood that the individual rings of the multiple condensed ring system may be connected in any order relative to one another. It is also to be understood that the point of attachment of a multiple condensed ring system (as defined above for a heteroaryl) can be at any position of the heteroaryl ring. It is also to be understood that the point of attachment for a heteroaryl or heteroaryl multiple condensed ring system can be at any suitable atom of the heteroaryl ring including a carbon atom and a heteroatom (e.g., a nitrogen). Exemplary heteroaryls include but are not limited to pyridyl, pyrrolyl, pyrazinyl, pyrimidinyl, pyridazinyl, pyrazolyl, thienyl, indolyl, imidazolyl, oxazolyl, isoxazolyl, thiazolyl, furyl, oxadiazolyl, thiadiazolyl, quinolyl, isoquinolyl, benzothiazolyl, benzoxazolyl, indazolyl, quinoxalyl, quinazolyl, 5,6,7,8-tetrahydroisoquinolinyl benzofuranyl, benzimidazolyl, thianaphthenyl, pyrrolo[2,3-b]pyridinyl, quinazolinyl-4(3H)-one, triazolyl, 4,5,6,7-tetrahydro-1H-indazole and 3b,4,4a,5-tetrahydro-1H-cyclopropa[3,4]cyclo-penta[1,2-c]pyrazole. In one embodiment the term "heteroaryl" refers to a single aromatic ring containing at least one heteroatom. For example, the term includes 5-membered and 6-membered monocyclic aromatic rings that include one or more heteroatoms. Non-limiting examples of heteroaryl include but are not limited to pyridyl, furyl, thiazole, pyrimidine, oxazole, and thiadiazole.

The term "heterocyclyl" or "heterocycle" as used herein refers to a single saturated or partially unsaturated ring that has at least one atom other than carbon in the ring, wherein the atom is selected from the group consisting of oxygen, nitrogen and sulfur; the term also includes multiple condensed ring systems that have at least one such saturated or partially unsaturated ring, which multiple condensed ring systems are further described below. Thus, the term includes single saturated or partially unsaturated rings (e.g., 3, 4, 5, 6 or 7-membered rings) from about 1 to 6 carbon atoms and from about 1 to 3 heteroatoms selected from the group consisting of oxygen, nitrogen and sulfur in the ring. The ring may be substituted with one or more (e.g., 1, 2 or 3) oxo groups and the sulfur and nitrogen atoms may also be present in their oxidized forms. Exemplary heterocycles include but are not limited to azetidinyl, tetrahydrofuranyl and piperidinyl. The term "heterocycle" also includes multiple condensed ring systems (e.g., ring systems comprising 2, 3 or 4 rings) wherein a single heterocycle ring (as defined above) can be condensed with one or more groups selected from heterocycles (to form for example a 1,8-decahydronapthyridinyl), carbocycles (to form for example a decahydroquinolyl) and aryls to form the multiple condensed ring system. Thus, a heterocycle (a single saturated or single partially unsaturated ring or multiple condensed ring system) has about 2-20 carbon atoms and 1-6 heteroatoms within the heterocycle ring. Such multiple condensed ring systems may be optionally substituted with one or more (e.g., 1, 2, 3 or 4) oxo groups on the carbocycle or heterocycle portions of the multiple condensed ring. The rings of the multiple condensed ring system can be connected to each other via fused, spiro and bridged bonds when allowed by valency requirements. It is to be understood that the individual rings of the multiple condensed ring system may be connected in any order relative to one another. Accordingly, a heterocycle (a single saturated or single partially unsaturated ring or multiple condensed ring system) has about 3-20 atoms including about 1-6 heteroatoms within the heterocycle ring system. It is also to be understood that the point of attachment of a multiple condensed ring system (as defined above for a heterocylyl) can be at any position of the heterocyclic ring. It is also to be understood that the point of attachment for a heterocycle or heterocycle multiple condensed ring system can be at any suitable atom of the heterocyclic ring including a carbon atom and a heteroatom (e.g., a nitrogen). In one embodiment the term heterocycle includes a C₂₋₂₀ heterocycle. In one embodiment the term heterocycle includes a C₂₋₇ heterocycle. In one embodiment the term heterocycle includes a C₂₋₅ heterocycle. In one embodiment the term heterocycle includes a C₂₋₄ heterocycle. Exemplary heterocycles include, but are not limited to aziridinyl, azetidinyl, pyrrolidinyl, piperidinyl, homopiperidinyl, morpholinyl, thiomorpholinyl, piperazinyl, tetrahydrofuranyl, dihydrooxazolyl, tetrahydropyranyl, tetrahydrothiopyranyl, 1,2,3,4-tetrahydroquinolyl, benzoxazinyl, dihydrooxazolyl, chromanyl, 1,2-dihydropyridinyl, 2,3-dihydrobenzo-furanyl, 1,3-benzodioxolyl, 1,4-benzodioxanyl, spiro[cyclopropane-1,1'-isoindolinyl]-3'-one, isoindolinyl-1-one, 2-oxa-6-azaspiro[3.3]heptanyl, imidazolidin-2-one N-methylpiperidine, imidazolidine, pyrazolidine, butyrolactam, valerolactam, imidazolidinone, hydantoin, dioxolane, phthalimide, 1,4-dioxane, thiomorpholine, thiomorpholine-S-oxide, thiomorpholine-S,S-oxide, pyran, 3-pyrroline, thiopyran, pyrone, tetrahydrothiophene, quinuclidine, tropane, 2-azaspiro[3.3]- heptane, (1R,5S)-3-azabicyclo[3.2.1]octane, (1s,4s)-2-azabicyclo[2.2.2]octane, (1R,4R)-2-oxa-5-azabicyclo[2.2.2]octane and pyrrolidin-2-one. In one embodiment the term "heterocycle" refers to a monocyclic, saturated or partially unsaturated, 3-8 membered ring having at least one heteroatom. For example, the term includes a monocyclic, saturated or partially unsaturated, 4, 5, 6, or 7 membered ring having at least one heteroatom. Non-limiting examples of heterocycle include aziridine, azetidine, pyrrolidine, piperidine, piperidine, piperazine, oxirane, morpholine, and thiomorpholine. The term "9- or 10-membered heterobicycle" as used herein refers to a partially unsaturated or aromatic fused bicyclic ring system having at least one heteroatom. For example, the term 9- or 10-membered heterobicycle includes a bicyclic ring system having a benzo ring fused to a 5-membered or 6-membered saturated, partially unsaturated, or aromatic ring that contains one or more heteroatoms.

As used herein, the term "heteroatom" is meant to include oxygen (O), nitrogen (N), sulfur (S) and silicon (Si). The nitrogen and sulfur can be in an oxidized form when feasible.

As used herein, the term "chiral" refers to molecules which have the property of non-superimposability of the mirror image partner, while the term "achiral" refers to molecules which are superimposable on their mirror image partner.

As used herein, the term "stereoisomers" refers to compounds which have identical chemical constitution but differ with regard to the arrangement of the atoms or groups in space, e.g., enantiomers, diastereomers, tautomers.

The term "patient" or "subject" is used throughout the specification to describe an animal, preferably a human or a domesticated animal, to whom treatment, including prophylactic treatment, with the compositions according to the present disclosure is provided. For treatment of those infections, conditions or disease states which are specific for a specific animal such as a human patient, the term patient refers to that specific animal, including a domesticated animal such as a dog or cat or a farm animal such as a horse, cow, sheep, etc. In general, in the present disclosure, the term patient refers to a human patient unless otherwise stated or implied from the context of the use of the term.

The term "effective" is used to describe an amount of a compound, composition or component which, when used within the context of its intended use, effects an intended result. The term effective subsumes all other effective amount or effective concentration terms, which are otherwise described or used in the present application.

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, e.g., in humans.

"Pharmaceutically acceptable salt" refers to a salt of a compound of the disclosure that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like.

A "pharmaceutically acceptable excipient" refers to a substance that is non-toxic, biologically tolerable, and otherwise biologically suitable for administration to a subject, such as an inert substance, added to a pharmacological composition or otherwise used as a vehicle, carrier, or diluent to facilitate administration of an agent and that is compatible therewith. Examples of excipients include calcium carbonate, calcium phosphate, various sugars and types of starch, cellulose derivatives, gelatin, vegetable oils, and polyethylene glycols.

A "solvate" refers to a physical association of a compound of Formula I with one or more solvent molecules.

"Treating" or "treatment" of any disease or disorder refers, in one embodiment, to ameliorating the disease or disorder (e.g., arresting or reducing the development of the disease or at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or disorder, either physically, (e.g., stabilization of a discernible symptom), physiologically, (e.g., stabilization of a physical parameter), or both. In yet another embodiment, "treating" or "treatment" refers to delaying the onset of the disease or disorder.

In one aspect, the disclosure is directed to a compound of Formula (I): 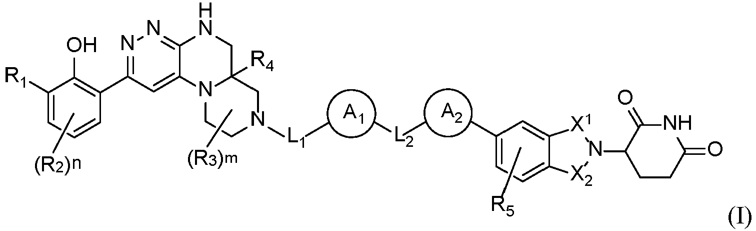 or a pharmaceutically acceptable salt thereof; wherein
R₁ is halo, C₁₋₆ alkyl, or haloalkyl;
each R₂ is independently H, D, or F;
each R₃ is independently H, D, C₁₋₆ alkyl, haloalkyl, or C₃₋₆ cycloalkyl;
n is 1, 2 or 3;
m is 1, 2, 3, 4, 5 or 6;
R₄ is H, D, C₁₋₆ alkyl, C₃₋₆ cycloalkyl, alkoxyalkyl, cyanoalkyl or haloalkyl;
R₅ is H, D, or F;
L₁ is a bond, C(R₃)₂, or CO;
L₂ is a bond, C(R₃)₂, or CO;
ring A₁ is a 3-7 membered cycloalkyl group, a 4-7-membered heterocycloalkyl group, an aryl group, or a heteroaryl group;
ring A₂ is a 3-7 membered cycloalkyl group, a 4-7-membered heterocycloalkyl group, an aryl group, or a heteroaryl group;
X₁ is CH₂, CO, CH=CH (when X₂ = CO), or N=CH (when X₂ = CO); and
X₂ is CH₂, CO, CH=CH (when X₁ = CO), or N=CH (when X₁ = CO).

In some embodiments, R₁ in Formula I is halo, C₁₋₆ alkyl, or haloalkyl. In some embodiments, R₁ in Formula I is halo. In some embodiments, R₁ in Formula I is C₁₋₆ alkyl. In some embodiments, R₁ in Formula I is haloalkyl.

In other embodiments, R₁ in Formula I is F. In other embodiments, R₁ in Formula I is Cl. In other embodiments, R₁ in Formula I is methyl.

In some embodiments, each R₂ in Formula I is independently H, D, or F. In some embodiments, each R₂ in Formula I is H. In some embodiments, each R₂ in Formula I is D. In some embodiments, each R₂ in Formula I is F.

In other embodiments, at least one R₂ in Formula I is H. In other embodiments, at least one R₂ in Formula I is D. In other embodiments, at least one R₂ in Formula I is F.

In some embodiments, n in Formula (I) is 1, 2 or 3. In some embodiments, n in Formula (I) is 1. In other embodiments, n in Formula (I) is 2. In yet other embodiments, n in Formula (I) is 3.

In some embodiments, each R₃ in Formula I is independently H, D, C₁₋₆ alkyl, haloalkyl, or C₃₋₆ cycloalkyl. In some embodiments, each R₃ in Formula I is H. In some embodiments, each R₃ in Formula I is D. In some embodiments, each R₃ in Formula I is C₁₋₆ alkyl. In some embodiments, each R₃ in Formula I is haloalkyl. In some embodiments, each R₃ in Formula I is C₃₋₆ cycloalkyl.

In other embodiments, at least one R₃ in Formula I is H. In other embodiments, at least one R₃ in Formula I is D. In other embodiments, at least one R₃ in Formula I is C₁₋₆ alkyl. In other embodiments, at least one R₃ in Formula I is haloalkyl. In other embodiments, at least one R₃ in Formula I is C₃₋₆ cycloalkyl.

In some embodiments, m in Formula (I) is 1, 2, 3, 4, 5 or 6. In some embodiments, m in Formula (I) is 1. In some embodiments, m in Formula (I) is 2. In other embodiments, m in Formula (I) is 3. In other embodiments, m in Formula (I) is 4. In yet other embodiments, m in Formula (I) is 5. In yet other embodiments, m in Formula (I) is 6.

In some embodiments, R₄ in Formula I is H, D, C₁₋₆ alkyl, C₃₋₆ cycloalkyl, alkoxyalkyl, cyanoalkyl or haloalkyl. In some embodiments, R₄ in Formula I is H. In some embodiments, R₄ in Formula I is D. In some embodiments, R₄ in Formula I is C₁₋₆ alkyl. In other embodiments, R₄ in Formula I is haloalkyl. In other embodiments, R₄ in Formula I is C₃₋₆ cycloalkyl. In yet other embodiments, R₄ in Formula I is alkoxyalkyl. In yet other embodiments, R₄ in Formula I is cyanoalkyl.

In some embodiments, R₅ in Formula I is independently H, D, or F. In some embodiments, R₅ in Formula I is H. In other embodiments, R₅ in Formula I is D. In other embodiments, R₅ in Formula I is F.

In some embodiments, L₁ in Formula I is a bond, C(R₃)₂, or CO. In some embodiments, L₁ in Formula (I) is a bond. In some embodiments, L₁ in Formula (I) is C(R₃)₂. In other embodiments, L₁ in Formula (I) is CO. In other embodiments, L₁ in Formula (I) is methylene.

In some embodiments, L₂ in Formula I is a bond, C(R₃)₂, or CO. In some embodiments, L₂ in Formula (I) is a bond. In some embodiments, L₂ in Formula (I) is C(R₃)₂. In other embodiments, L₂ in Formula (I) is CO. In other embodiments, L₂ in Formula (I) is methylene.

In some embodiments, ring A₁ in Formula (I) is a 3-7 membered cycloalkyl group, a 4-7-membered heterocycloalkyl group, an aryl group, or a heteroaryl group.

In some embodiments, ring A₁ in Formula (I) is a 3-7 membered cycloalkyl group. In some embodiments, ring A₁ is a 4-7-membered heterocycloalkyl group. In other embodiments, ring A₁ is an aryl. In other embodiments, ring A₁ is a heteroaryl group.

In some embodiments, ring A₁ in Formula (I) is a cyclohexyl group. In some embodiments, ring A₁ in Formula (I) is a piperazine group, a morpholine group, a piperidine group, a pyrrolidine group, an azetidine group or an azabicyclo-hexane group.

In some embodiments, ring A₁ in Formula (I) is a piperazine group. In some embodiments, ring A₁ in Formula (I) is a morpholine group. In other embodiments, ring A₁ in Formula (I) is a piperidine group. In other embodiments, ring A₁ in Formula (I) is a pyrrolidine group. In yet other embodiments, ring A₁ in Formula (I) is an azetidine group. In yet other embodiments, ring A₁ in Formula (I) is an azabicyclo-hexane group.

In some embodiments, ring A₂ in Formula (I) is a 3-7 membered cycloalkyl group, a 4-7-membered heterocycloalkyl group, an aryl group, or a heteroaryl group.

In some embodiments, ring A₂ in Formula (I) is a 3-7 membered cycloalkyl group. In some embodiments, ring A₂ is a 4-7-membered heterocycloalkyl group. In other embodiments, ring A₂ is an aryl. In other embodiments, ring A₂ is a heteroaryl group.

In some embodiments, ring A₂ in Formula (I) is a cyclohexyl group. In some embodiments, ring A₂ in Formula (I) is a piperazine group, a morpholine group, a piperidine group, a pyrrolidine group, an azetidine group or an azabicyclo-hexane group.

In some embodiments, ring A₂ in Formula (I) is a piperazine group. In some embodiments, ring A₂ in Formula (I) is a morpholine group. In other embodiments, ring A₂ in Formula (I) is a piperidine group. In other embodiments, ring A₂ in Formula (I) is a pyrrolidine group. In yet other embodiments, ring A₂ in Formula (I) is an azetidine group. In yet other embodiments, ring A₂ in Formula (I) is an azabicyclo-hexane group.

In some embodiments, X₁ in Formula (I) is CH₂, CO, CH=CH (when X₂ = CO), or N=CH (when X₂ = CO).

In some embodiments, X₁ in Formula (I) is CH₂. In some embodiments, X₁ is CO. In other embodiments, X₁ is CH=CH (when X₂ = CO). In other embodiments, X₁ is N=CH (when X₂ = CO).

In some embodiments, X₂ in Formula (I) is CH₂, CO, CH=CH (when X₂ = CO), or N=CH (when X₂ = CO).

In some embodiments, X₂ in Formula (I) is CH₂. In some embodiments, X₂ is CO. In other embodiments, X₂ is CH=CH (when X₁ = CO). In other embodiments, X₂ is N=CH (when X₁ = CO).

In some embodiments, the compounds of Formula (I) are the pharmaceutically acceptable salts. In some embodiments, the compounds of Formula (I) are solvates. In some embodiments, the compounds of Formula (I) are N-oxides. In some embodiments, the compounds of Formula (I) are stereoisomers.

In some embodiments, the compounds of Formula (I) are represented by compounds of Formula II 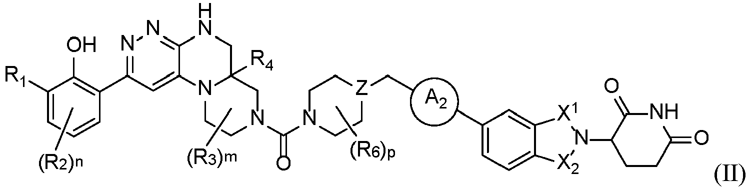 or a pharmaceutically acceptable salt thereof ; wherein
each R₆ is independently H, D, C₁₋₆ alkyl, C₃₋₆ cycloalkyl, or haloalkyl;
p is 1, 2, 3, 4, 5, 6, 7 or 8; and
Z is N or CR₆; and
wherein each R₁, (R₂)ₙ, (R₃)ₘ, R₄, X₁, X₂ and ring A₂ are defined with respect to Formula (I).

In some embodiments, each R₆ in Formula II is independently H, D, C₁₋₆ alkyl, haloalkyl, or C₃₋₆ cycloalkyl. In some embodiments, each R₆ in Formula II is H. In some embodiments, each R₆ in Formula II is D. In some embodiments, each R₆ in Formula II is C₁₋₆ alkyl. In some embodiments, each R₆ in Formula II is haloalkyl. In some embodiments, each R₆ in Formula II is C₃₋₆ cycloalkyl.

In other embodiments, at least one R₆ in Formula II is H. In other embodiments, at least one R₆ in Formula II is D. In other embodiments, at least one R₆ in Formula II is C₁₋₆ alkyl. In other embodiments, at least one R₆ in Formula II is haloalkyl. In other embodiments, at least one R₆ in Formula II is C₃₋₆ cycloalkyl.

In some embodiments, p in Formula II is 1, 2, 3, 4, 5, 6, 7 or 8. In some embodiments, p in Formula II is 1. In some embodiments, p in Formula II is 2. In other embodiments, p in Formula II is 3. In other embodiments, p in Formula II is 4. In other embodiments, p in Formula II is 5. In other embodiments, p in Formula II is 6. In yet other embodiments, p in Formula II is 7. In yet other embodiments, p in Formula II is 8.

In some embodiments, Z in Formula II is N or CR₆. In some embodiments, Z in Formula II is N. In some embodiments, Z in Formula II is CR₆. In some embodiments, Z in Formula II is CH₃.

In some embodiments, the compounds of Formula (I) are represented by compounds of Formula III 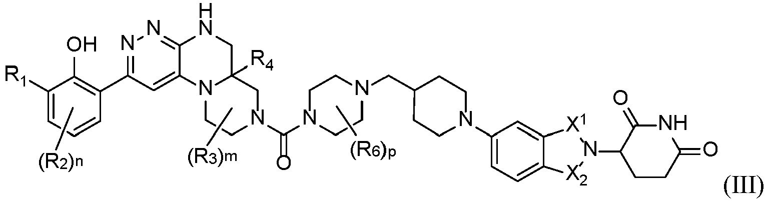 or a pharmaceutically acceptable salt thereof; and
wherein each R₁, (R₂)ₙ, (R₃)ₘ, R₄, (R₆)ₚ, X₁ and X₂ are defined with respect to Formula (I) and Formula (II).

In some embodiments, the compounds of Formula (I) are represented by compounds of Formula IV 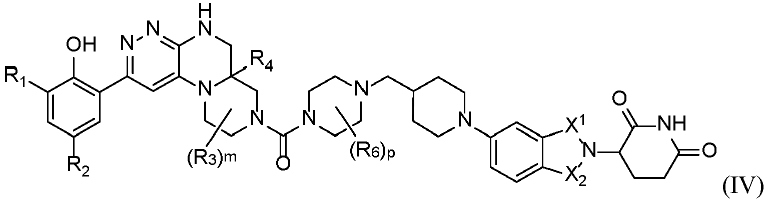 or a pharmaceutically acceptable salt thereof; and
wherein each R₁, R₂, (R₃)ₘ, R₄, (R₆)ₚ, X₁ and X₂ are defined with respect to Formula (I) and Formula (II).

In some embodiments, the compounds of Formula (I) are represented by compounds of Formula V 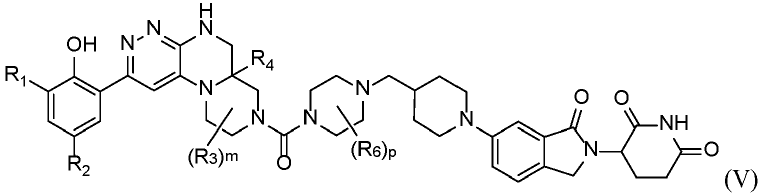 or a pharmaceutically acceptable salt thereof; and
wherein each R₁, R₂, (R₃)ₘ, R₄, and (R₆)ₚ are defined with respect to Formula (I) and Formula (II).

In some embodiments, the compounds of Formula (I) are represented by compounds of Formula VI 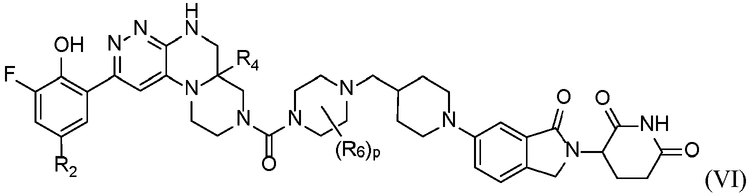 or a pharmaceutically acceptable salt thereof; and
wherein each R₂, R₄, and (R₆)ₚ are defined with respect to Formula (I) and Formula (II).

In yet further embodiments, the compounds of Formula (I) are:
3-(6-(4-(((3R,5S)-4-((S)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(3-(((3R,5S)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) azetidin-1-yl)-1-oxoisoindolin-2-yl)piperidine-2,6-dione;
3-(S-((S)-2-(((3R,SS)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) morpholino)-1-oxoisoindolin-2-yl)piperidine-2,6-dione;
3-(6-(4-((4-((6aS,9S)-2-(3-fluoro-2-hydroxyphenyl)-9-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(4-((4-((S)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
2-(2,6-dioxopiperidin-3-yl)-5-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)isoindoline-1,3-dione;
3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(4-(((3R,5S)-4-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((3R,5S)-4-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((3R,5R)-4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((3R,5R)-4-(2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((3R,5S)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
(R)-3-(6-(4-(((3S,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
(S)-3-(6-(4-(((3S,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
(R)-3-(6-(4-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
(S)-3-(6-(4-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(3-(((3R,5S)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) azetidin-1-yl)-1-oxoisoindolin-2-yl)piperidine-2,6-dione;
3-(5-((S)-2-(((3R,5S)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) morpholino)-1-oxoisoindolin-2-yl)piperidine-2,6-dione;
3-(6-(4-((4-((S)-2-(3,5-difluoro-2-hydroxyphenyl)-6,6a,7,8,9,10-hexahydro-SH-pyrazino [1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(3-((4-((S)-2-(3,5-difluoro-2-hydroxyphenyl)-6,6a,7,8,9,10-hexahydro-5H-pyrazino [1',2':4,5]pyrazino[2,3-c]pyridazine-8-carbonyl)-3,3-dimethylpiperazin-1-yl)methyl)azetidin-1-yl)-1-oxoisoindolin-2-yl)piperidine-2,6-dione;
3-(6-(3-(((3S,5R)-4-((6aS,9S)-2-(3,5-difluoro-2-hydroxyphenyl)-9-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)azetidin-1-yl)-1-oxoisoindolin-2-yl)piperidine-2,6-dione;
3-(6-(4-((4-((6aS,9S)-2-(3,5-difluoro-2-hydroxyphenyl)-9-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-(((3S,5R)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-((4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(4-(((3R,5R)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(3-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3-ethylpiperazin-1-yl)methyl)azetidin-1-yl)-1-oxoisoindolin-2-yl)piperidine-2,6-dione;
3-(6-(4-(((3R,5S)-4-(2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-((4-(2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
2-(2,6-dioxopiperidin-3-yl)-5-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)isoindoline-1,3-dione;
3-(6-(4-(((3R,5R)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-(((3S,5R)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-((4-((S)-2-(3,5-difluoro-2-hydroxyphenyl)-6,6a,7,8,9,10-hexahydro-5H-pyrazino [1',2':4,5]pyrazino[2,3-c]pyridazine-8-carbonyl)-3-ethylpiperazin-1-yl)methyl)piperidin-1-yl)-1-oxoisoindolin-2-yl)piperidine-2,6-dione;
5-(4-(((3S,5R)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-2-(2,6-dioxopiperidin-3-yl)isoindoline-1,3-dione;
3-(6-((3R,4R)-4-(((1R,5S,6R)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0] hexan-3-yl)methyl)-3-fluoropiperidin-1-yl)-1-oxoisoindolin-2-yl)piperidine-2,6-dione;
3-(6-((3R,4R)-4-(((1R,5S,6R)-6-(((6aS,9S)-2-(3,5-difluoro-2-hydroxyphenyl)-9-methyl-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)-3-fluoropiperidin-1-yl)-1-oxoisoindolin-2-yl)piperidine-2,6-dione;
3-(6-(4-(((1R,5S,6r)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl) methyl)-3,3-difluoropiperidin-1-yl)-1-oxoisoindolin-2-yl)piperidine-2,6-dione;
3-(6-(4-(((1R,5S,6r)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl) methyl)-3,3-difluoropiperidin-1-yl)-1-oxoisoindolin-2-yl)piperidine-2,6-dione;
3-(6-((rac-3R,4R)-4-(((3S,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)-3-fluoropiperidin-1-yl)-1-oxoisoindolin-2-yl)piperidine-2,6-dione;
3-(6-((3R,4R)-3-fluoro-4-(((3S,5R)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-(((3R,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(5-((S)-2-(((3R,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) morpholino)-1-oxoisoindolin-2-yl)piperidine-2,6-dione;
3-(6-(4-(((3S,5S)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-((1-((S)-2-(3,5-difluoro-2-hydroxyphenyl)-6,6a,7,8,9,10-hexahydro-5H-pyrazino [1',2':4,5]pyrazino[2,3-c]pyridazine-8-carbonyl)piperidin-4-yl)methyl)piperazin-1-yl)-1-oxoisoindolin-2-yl)piperidine-2,6-dione;
3-(6-(2-(((1R,5S,6s)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl) methyl)morpholino)-1-oxoisoindolin-2-yl)piperidine-2,6-dione;
3-(6-(2-(((1R,5S,6r)-6-(((6aS,9S)-2-(3,5-difluoro-2-hydroxyphenyl)-9-methyl-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)morpholino)-1-oxoisoindolin-2-yl)piperidine-2,6-dione;
3-(5-((S)-2-(((3R,4R)-4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-fluoropiperidin-1-yl)methyl) morpholino)-1-oxoisoindolin-2-yl)piperidine-2,6-dione;
3-(6-((S)-3-(((R)-3-(((S)-2-(3,S-difluoro-2-hydroxyphenyl)-5,6,6a,7,9,10-hexahydro-8H-pyrazino[1',2':4,5]pyrazino[2,3-c]pyridazin-8-yl)methyl)pyrrolidin-1-yl)methyl)pyrrolidin-1-yl)-1-oxoisoindolin-2-yl)piperidine-2,6-dione;
3-(5-((S)-3-((4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)piperidin-1-yl)methyl)pyrrolidin-1-yl)-1-oxoisoindolin-2-yl)piperidine-2,6-dione;
3-(6-(4-(((1S,4r)-4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidine-2,6-dione;
3-(6-(4-((4-((S)-2-(3,5-difluoro-2-hydroxyphenyl)-6,6a,7,8,9,10-hexahydro-5H-pyrazino [1',2':4,5]pyrazino[2,3-c]pyridazine-8-carbonyl)-4-fluoropiperidin-1-yl)methyl)piperidin-1-yl)-1-oxoisoindolin-2-yl)piperidine-2,6-dione;
3-(6-(4-(((1R,4s)-4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidine-2,6-dione;
3-(6-(4-(((2S,5R)-4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,5-dimethyl-piperazin-1-yl) methyl)piperidin-1-yl)-1-oxoisoindolin-2-yl)piperidine-2,6-dione;
3-(6-(4-(((15,4r)-4-(((S)-2-(3-fluoro-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidine-2,6-dione;
3-(6-(4-(((3R,5S)-4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
3-(6-(4-(((3R,5S)-4-(6a-ethyl-2-(3-fluoro-2-hydroxyphenyl)-6,6a,7,8,9,10-hexahydro-5*H-*pyrazino[1',2':4,5]pyrazino[2,3-*c*]pyridazine-8-carbonyl)-3,5-dimethyl-piperazin-1-yl)methyl) piperidin-1-yl)-1-oxoisoindolin-2-yl)piperidine-2,6-dione;
3-(6-(4-(((3*R*,5*S*)-4-(2-(3,5-difluoro-2-hydroxyphenyl)-6a-ethyl-6,6a,7,8,9,10-hexahydro-5*H*-pyrazino[1',2':4,5]pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
3-(6-(4-(((3*R*,5*S*)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethyl-piperazin-1-yl)methyl) piperidin-1-yl)-1-oxoisoindolin-2-yl)piperidine-2,6-dione;
3-(6-(4-(((3*R*,5*R*)-4-(2-(3-chloro-2-hydroxyphenyl)-6a-methyl-6,6a,7,8,9,10-hexahydro-5*H*-pyrazino[1',2':4,5]pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
3-(6-(4-(((3*R*,5*S*)-4-(2-(3,5-difluoro-2-hydroxyphenyl)-6a-methyl-6,6a,7,8,9, 10-hexahydro-5*H*-pyrazino[1',2':4,5]pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
or a pharmaceutically acceptable salt thereof.

In yet further embodiments, the compounds of Formula (I) are:
(S)-3-(6-(4-((4-((R)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-(((3S,5S)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5R)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((2S,5R)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
3-(6-(4-(((3R,5S)-4-((S)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-(((3R,5S)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((2R,4S,6S)-1-((S)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(S)-3-(6-(4-(((2R,4R,6S)-1-((R)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(3S)-3-(6-(4-((1-((S)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-2,2-dimethylpiperidin-4-yl)methyl)piperazin-1-yl)-1-oxoisoindolin-2-yl)piperidine-2,6-dione;
(3S)-3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(3S)-3-(6-(4-(((3R,5R)-4-(2-(3-chloro-2-hydroxyphenyl)-6a-ethyl-6,6a,7,8,9,10-hexahydro-5H-pyrazino[1',2':4,5] pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
(3S)-3-(6-(4-((4-(2-(3-chloro-2-hydroxyphenyl)-6a-ethyl-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
(3S)-3-(6-(4-(((2S,5R)-4-(2-(3-chloro-2-hydroxyphenyl)-6a-ethyl-6,6a,7,8,9,10-hexahydro-5H-pyrazino[1',2':4,5] pyrazino[2,3-c]pyridazine-8-carbonyl)-2,5-dimethyl-piperazin-1-yl)methyl)piperidin-1-yl)-1-oxoisoindolin-2-yl)piperidine-2,6-dione;
(3S)-3-(6-(4-(((2S,5R)-4-(6a-ethyl-2-(3-fluoro-2-hydroxyphenyl)-6,6a,7,8,9,10-hexahydro-5H-pyrazino [1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(4-(((3S,5S)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((3R,5R)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((3R,5R)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((2S,5R)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-2,5-dimethylpiperazin-1-yl)methyl) piperidin-1-yl)-1-oxoisoindolin-2-yl)piperidine-2,6-dione;
3-(6-(4-(((3R,5S)-4-(2-(3,5-difluoro-2-hydroxyphenyl)-6a-ethyl-6,6a,7,8,9,10-hexahydro-5H-pyrazino[1',2':4,5] pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
3-(6-(4-(((3R,5S)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((3R,5S)-4-(2-(3,5-difluoro-2-hydroxyphenyl)-6a-ethyl-6,6a,7,8,9,10-hexahydro-5H-pyrazino[1',2':4,5] pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
3-(6-(4-(((3R,5S)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
(3*S*)-3-(6-(4-((4-(2-(3-fluoro-2-hydroxyphenyl)-6a-(fluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(3*S*)-3-(6-(4-((4-(2-(3-fluoro-2-hydroxyphenyl)-6a-(fluoromethyl)-6,6a,7,8,9,10-hexahydro-5*H*-pyrazino [1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-(((2*S*,5*R*)-4-(2-(3-fluoro-2-hydroxyphenyl)-6a-methyl-6,6a,7,8,9,10-hexahydro-5*H*-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
3-(6-(4-(((2*S*,5*R*)-4-(2-(3-fluoro-2-hydroxyphenyl)-6a-methyl-6,6a,7,8,9,10-hexahydro-5*H*-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
3-(6-(4-(((3R,5*S*)-4-(2-(3-chloro-2-hydroxyphenyl)-6a-methyl-6,6a,7,8,9,10-hexahydro-5*H*-pyrazino[1',2':4,5] pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
((2*S*,6*R*)-2,6-dimethylpiperazin-1-yl)(2-(3-fluoro-2-hydroxyphenyl)-6a-methyl-5,6,6a,7,9,10-hexahydro-8*H*-pyrazino[1',2':4,5]pyrazino[2,3-c]pyridazin-8-yl)methanone;
((2S,6R)-2,6-dimethylpiperazin-1-yl)(2-(3-fluoro-2-hydroxyphenyl)-6a-methyl-5,6,6a,7,9,10-hexahydro-8*H*-pyrazino[1',2':4,5]pyrazino[2,3-c]pyridazin-8-yl)methanone;
(3S)-3-(6-(4-(((3S,5R)-4-(2-(3-fluoro-2-hydroxyphenyl)-6a-(trifluoromethyl)-6,6a,7,8,9,10-hexahydro-5*H*-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(35)-3-(6-(4-(((3R,5R)-4-(2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(35)-3-(6-(4-(((3R,5R)-4-(2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(3S)-3-(6-(4-(((2R,4r,6S)-1-(2-(3-fluoro-2-hydroxyphenyl)-6a-(trifluoromethyl)-6,6a,7,8,9,10-hexahydro-5H-pyrazino[1',2':4,5] pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(3S)-3-(6-(4-(((2R,4r,6S)-1-(2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(3S)-3-(6-(4-((4-(2-(3-fluoro-2-hydroxyphenyl)-6a-(trifluoro-methyl)-6,6a,7,8,9,10-hexahydro-5H-pyrazino[1',2':4,5]pyrazino [2,3-c]pyridazine-8-carbonyl)-3,3-dimethylpiperazin-1-yl)methyl) piperidin-1-yl)-1-oxoisoindolin-2-yl)piperidine-2,6-dione;
(3S)-3-(6-(4-((4-(2-(3-fluoro-2-hydroxyphenyl)-6a-(trifluoro-methyl)-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
(3S)-3-(6-(4-(((1s,4R)-4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluorocyclohexyl) methyl)piperazin-1-yl)-1-oxoisoindolin-2-yl)piperidine-2,6-dione;
(S)-3-(6-(4-(((1R,4R)-4-((S)-2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluorocyclohexyl) methyl)piperazin-1-yl)-1-oxoisoindolin-2-yl)piperidine-2,6-dione;
(S)-3-(6-(4-((4-((R)-2-(3,5-difluoro-2-hydroxyphenyl)-6a-(difluoromethyl)-6,6a,7,8,9,10-hexahydro-5H-pyrazino[1',2':4,5]pyrazino[2,3-c]pyridazine-8-carbonyl)-4-fluoropiperidin-1-yl)methyl)piperidin-1-yl)-1-oxoisoindolin-2-yl)piperidine-2,6-dione;
(3S)-3-(6-(4-((4-(2-(3-chloro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-fluoro-4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)piperidin-1-yl)methyl) piperidin-1-yl)-1-oxoisoindolin-2-yl)piperidine-2,6-dione;
(3S)-3-(6-(4-((4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
3-(6-(4-(((1S,4r)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)cyclohexyl)methyl)piperazin-1-yl)-1-oxoisoindolin-2-yl)piperidine-2,6-dione;
(S)-3-(6-(4-(((1S,4S)-4-(((S)-2-(3-fluoro-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidine-2,6-dione;
3-(6-(4-(((1R,4r)-4-(((R)-2-(3-fluoro-2-hydroxyphenyl)-6a-methyl-5,6,6a,7,9,10-hexahydro-8H-pyrazino[1',2':4,5] pyrazino[2,3-c]pyridazin-8-yl)methyl)cyclohexyl)methyl) piperazin-1-yl)-1-oxoisoindolin-2-yl)piperidine-2,6-dione;
3-(6-(4-(((1R,4r)-4-(((R)-2-(3-fluoro-2-hydroxyphenyl)-6a-methyl-5,6,6a,7,9,10-hexahydro-8H-pyrazino[1',2':4,5]p yrazino[2,3-c]pyridazin-8-yl)methyl)cyclohexyl)methyl) piperazin-1-yl)-1-oxoisoindolin-2-yl)piperidine-2,6-dione;
(S)-3-(6-(4-(((1R,4r)-4-(((R)-2-(3-fluoro-2-hydroxyphenyl)-6a-methyl-5,6,6a,7,9,10-hexahydro-8H-pyrazino[1',2':4,5] pyrazino[2,3-c]pyridazin-8-yl)methyl)cyclohexyl)methyl) piperazin-1-yl)-1-oxoisoindolin-2-yl)piperidine-2,6-dione;
(S)-3-(6-(4-(((1R,4r)-4-(((R)-2-(3-fluoro-2-hydroxyphenyl)-6a-methyl-5,6,6a,7,9,10-hexahydro-8H-pyrazino[1',2':4,5] pyrazino[2,3-c]pyridazin-8-yl)methyl)cyclohexyl)methyl) piperazin-1-yl)-1-oxoisoindolin-2-yl)piperidine-2,6-dione;
3-(6-(4-((4-((*S*)-2-(3,5-difluoro-2-hydroxyphenyl)-6,6a,7,8,9,10-hexahydro-5H-pyrazino[1',2':4,5]pyrazino [2,3-c]pyridazine-8-carbonyl)-3,3-dimethylpiperidin-1-yl)methyl) piperidin-1-yl)-1-oxoisoindolin-2-yl)piperidine-2,6-dione;
(S)-3-(6-(4-(((2R,4R,6S)-1-(2-(3-fluoro-2-hydroxyphenyl)-6a-methyl-6,6a,7,8,9,10-hexahydro-5H-pyrazino[1',2':4,5] pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(S)-3-(6-(4-(((2R,4R,6S)-1-(2-(3-fluoro-2-hydroxyphenyl)-6a-methyl-6,6a,7,8,9,10-hexahydro-5H-pyrazino[1',2':4,5] pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
3-(6-(4-((1-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,2-dimethylpiperidin-4-yl)methyl) piperazin-1-yl)-1-oxoisoindolin-2-yl)piperidine-2,6-dione;
(3S)-3-(6-(4-((1-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,2-dimethylpiperidin-4-yl)methyl) piperazin-1-yl)-1-oxoisoindolin-2-yl)piperidine-2,6-dione;
(3S)-3-(6-(4-((1-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,2-dimethylpiperidin-4-yl)methyl) piperazin-1-yl)-1-oxoisoindolin-2-yl)piperidine-2,6-dione;
or a pharmaceutically acceptable salt thereof.

In yet further embodiments, the compounds of Formula (I) are:
(S)-3-(6-(4-(((3S,5R)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3S,5R)-4-((R)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3S,5R)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3S,5R)-4-((R)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(3-(((3R,5S)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)azetidin-1-yl)-1-oxoisoindolin-2-yl)piperidine-2,6-dione;
(S)-3-(6-(3-(((3R,5S)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)azetidin-1-yl)-1-oxoisoindolin-2-yl)piperidine-2,6-dione;
(R)-3-(6-(3-(((3R,5S)-4-((R)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)azetidin-1-yl)-1-oxoisoindolin-2-yl)piperidine-2,6-dione;
(S)-3-(6-(3-(((3R,5S)-4-((R)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)azetidin-1-yl)-1-oxoisoindolin-2-yl)piperidine-2,6-dione;
(R)-3-(5-((S)-2-(((3R,5S)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)morpholino)-1-oxoisoindolin-2-yl)piperidine-2,6-dione;
(S)-3-(S-((S)-2-(((3R,5S)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)morpholino)-1-oxoisoindolin-2-yl)piperidine-2,6-dione;
(R)-3-(5-((S)-2-(((3R,5S)-4-((R)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)morpholino)-1-oxoisoindolin-2-yl)piperidine-2,6-dione;
(S)-3-(5-((S)-2-(((3R,5S)-4-((R)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)morpholino)-1-oxoisoindolin-2-yl)piperidine-2,6-dione;
(R)-3-(6-(4-((4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((R)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((R)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((R)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((S)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((R)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((S)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((R)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((S)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((R)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((S)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-(((3R,5S)-4-((S)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((R)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((S)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((R)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((R)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((S)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((R)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((S)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5R)-4-((S)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5R)-4-((R)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5R)-4-((S)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5R)-4-((R)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5R)-4-((R)-2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5R)-4-((S)-2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5R)-4-((R)-2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5R)-4-((S)-2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((R)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((R)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((R)-2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((S)-2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((R)-2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((S)-2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-((4-((R)-2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((S)-2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((R)-2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((S)-2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-(((3R,5R)-4-((S)-2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5R)-4-((R)-2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5R)-4-((S)-2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5R)-4-((R)-2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((R)-2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((S)-2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,55)-4-((R)-2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((S)-2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((2S,5R)-4-((S)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
(S)-3-(6-(4-(((2S,5R)-4-((R)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
(R)-3-(6-(4-(((2S,5R)-4-((S)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
(R)-3-(6-(4-(((2S,5R)-4-((R)-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
(S)-3-(6-(4-(((3R,5S)-4-((S)-2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((R)-2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((S)-2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((R)-2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((S)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5S)-4-((R)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((S)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5S)-4-((R)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-((4-((R)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((S)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((S)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((R)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-(((2R,4S,6S)-1-((S)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(S)-3-(6-(4-(((2R,4S,6S)-1-((R)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(R)-3-(6-(4-(((2R,4S,6S)-1-((S)-6a-(difluoromethyl)-2-(3-fluoro-2-hydroxyphenyl)-6,6a,7,8,9,10-hexahydro-5H-pyrazino[1',2":4,5]pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(R)-3-(6-(4-(((2R,4S,6S)-1-((R)-6a-(difluoromethyl)-2-(3-fluoro-2-hydroxyphenyl)-6,6a,7,8,9,10-hexahydro-SH-pyrazino[1',2':4,5]pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(S)-3-(6-(4-((4-((R)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((S)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((S)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((R)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-(((2S,5R)-4-((R)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
(S)-3-(6-(4-(((2S,5R)-4-((S)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
(R)-3-(6-(4-(((2S,5R)-4-((R)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
(R)-3-(6-(4-(((2S,5R)-4-((S)-6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
(S)-3-(6-(4-((4-((R)-2-(3-fluoro-2-hydroxyphenyl)-6a-(fluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((S)-2-(3-fluoro-2-hydroxyphenyl)-6a-(fluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((R)-2-(3-fluoro-2-hydroxyphenyl)-6a-(fluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((S)-2-(3-fluoro-2-hydroxyphenyl)-6a-(fluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((R)-2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((R)-2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((S)-2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(R)-3-(6-(4-((4-((S)-2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-(((3R,5R)-4-((S)-2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5R)-4-((S)-2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5R)-4-((R)-2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3R,5R)-4-((R)-2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
or a pharmaceutically acceptable salt thereof.

It will be apparent that the compounds of Formula I, including all subgenera described herein, may have multiple stereogenic centers. As a result, there exist multiple stereoisomers (enantiomers and diastereomers) of the compounds of Formula I (and subgenera described herein). The present disclosure contemplates and encompasses each stereoisomer of any compound of Formula I (and subgenera described herein), as well as mixtures of said stereoisomers.

Pharmaceutically acceptable salts and solvates of the compounds of Formula I (including all subgenera described herein) are also within the scope of the disclosure.

Isotopic variants of the compounds of Formula I (including all subgenera described herein) are also contemplated by the present disclosure.

### Pharmaceutical Compositions and Methods of Administration

The subject pharmaceutical compositions are typically formulated to provide a therapeutically effective amount of a compound of the present disclosure as the active ingredient, or a pharmaceutically acceptable salt, ester, prodrug, solvate, hydrate or derivative thereof. Where desired, the pharmaceutical compositions contain pharmaceutically acceptable salt and/or coordination complex thereof, and one or more pharmaceutically acceptable excipients, carriers, including inert solid diluents and fillers, diluents, including sterile aqueous solution and various organic solvents, permeation enhancers, solubilizers and adjuvants.

The subject pharmaceutical compositions can be administered alone or in combination with one or more other agents, which are also typically administered in the form of pharmaceutical compositions. Where desired, the one or more compounds of the invention and other agent(s) may be mixed into a preparation or both components may be formulated into separate preparations to use them in combination separately or at the same time.

In some embodiments, the concentration of one or more compounds provided in the pharmaceutical compositions of the present invention is less than 100%, 90%, 80%, 70%, 60%, 50%, 40%, 30%, 20%, 19%, 18%, 17%, 16%, 15%, 14%, 13%, 12%, 11%, 10%, 9%, 8%, 7%, 6%, 5%, 4%, 3%, 2%, 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% (or a number in the range defined by and including any two numbers above) w/w, w/v or v/v.

In some embodiments, the concentration of one or more compounds of the invention is greater than 90%, 80%, 70%, 60%, 50%, 40%, 30%, 20%, 19.75%, 19.50%, 19.25%, 19%, 18.75%, 18.50%, 18.25% 18%, 17.75%, 17.50%, 17.25% 17%, 16.75%, 16.50%, 16.25%, 16%, 15.75%, 15.50%, 15.25% 15%, 14.75%, 14.50%, 14.25% 14%, 13.75%, 13.50%, 13.25%, 13%, 12.75%, 12.50%, 12.25%, 12%, 11.75%, 11.50%, 11.25% 11%, 10.75%, 10.50%, 10.25% 10%, 9.75%, 9.50%, 9.25%, 9%, 8.75%, 8.50%, 8.25% 8%, 7.75%, 7.50%, 7.25%, 7%, 6.75%, 6.50%, 6.25%, 6%, 5.75%, 5.50%, 5.25%, 5%, 4.75%, 4.50%, 4.25%, 4%, 3.75%, 3.50%, 3.25%, 3%, 2.75%, 2.50%, 2.25%, 2%, 1.75%, 1.50%, 1.25% , 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% (or a number in the range defined by and including any two numbers above) w/w, w/v, or v/v.

In some embodiments, the concentration of one or more compounds of the invention is in the range from approximately 0.0001% to approximately 50%, approximately 0.001% to approximately 40%, approximately 0.01% to approximately 30%, approximately 0.02% to approximately 29%, approximately 0.03% to approximately 28%, approximately 0.04% to approximately 27%, approximately 0.05% to approximately 26%, approximately 0.06% to approximately 25%, approximately 0.07% to approximately 24%, approximately 0.08% to approximately 23%, approximately 0.09% to approximately 22%, approximately 0.1% to approximately 21%, approximately 0.2% to approximately 20%, approximately 0.3% to approximately 19%, approximately 0.4% to approximately 18%, approximately 0.5% to approximately 17%, approximately 0.6% to approximately 16%, approximately 0.7% to approximately 15%, approximately 0.8% to approximately 14%, approximately 0.9% to approximately 12%, approximately 1% to approximately 10% w/w, w/v or v/v.

In some embodiments, the concentration of one or more compounds of the invention is in the range from approximately 0.001% to approximately 10%, approximately 0.01% to approximately 5%, approximately 0.02% to approximately 4.5%, approximately 0.03% to approximately 4%, approximately 0.04% to approximately 3.5%, approximately 0.05% to approximately 3%, approximately 0.06% to approximately 2.5%, approximately 0.07% to approximately 2%, approximately 0.08% to approximately 1.5%, approximately 0.09% to approximately 1%, approximately 0.1% to approximately 0.9% w/w, w/v or v/v.

In some embodiments, the amount of one or more compounds of the invention is equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g (or a number in the range defined by and including any two numbers above).

In some embodiments, the amount of one or more compounds of the invention is more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, , 0.15 g, 0.2 g, , 0.25 g, 0.3 g, , 0.35 g, 0.4 g, , 0.45 g, 0.5 g, 0.55 g, 0.6 g, , 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5g, 7 g, 7.5g, 8 g, 8.5 g, 9 g, 9.5 g, or 10 g (or a number in the range defined by and including any two numbers above).

In some embodiments, the amount of one or more compounds of the invention is in the range of 0.0001-10 g, 0.0005-9 g, 0.001-8 g, 0.005-7 g, 0.01-6 g, 0.05-5 g, 0.1-4 g, 0.5-4 g, or 1-3 g.

The compounds according to the invention are effective over a wide dosage range. For example, in the treatment of adult humans, dosages from 0.01 to 1000 mg, from 0.5 to 100 mg, from 1 to 50 mg per day, and from 5 to 40 mg per day are examples of dosages that may be used. An exemplary dosage is 10 to 30 mg per day. The exact dosage will depend upon the route of administration, the form in which the compound is administered, the subject to be treated, the body weight of the subject to be treated, and the preference and experience of the attending physician.

A pharmaceutical composition of the invention typically contains an active ingredient (e.g., a compound of the disclosure) of the present invention or a pharmaceutically acceptable salt and/or coordination complex thereof, and one or more pharmaceutically acceptable excipients, carriers, including but not limited to inert solid diluents and fillers, diluents, sterile aqueous solution and various organic solvents, permeation enhancers, solubilizers and adjuvants.

Described below are exemplary pharmaceutical compositions and methods for preparing the same.

### Pharmaceutical Compositions for Oral Administration

In some embodiments, the invention provides a pharmaceutical composition for oral administration containing a compound of the invention, and a pharmaceutical excipient suitable for oral administration.

In some embodiments, the invention provides a solid pharmaceutical composition for oral administration containing: (i) an effective amount of a compound of the invention; optionally (ii) an effective amount of a second agent; and (iii) a pharmaceutical excipient suitable for oral administration. In some embodiments, the composition further contains: (iv) an effective amount of a third agent.

In some embodiments, the pharmaceutical composition may be a liquid pharmaceutical composition suitable for oral consumption. Pharmaceutical compositions of the invention suitable for oral administration can be presented as discrete dosage forms, such as capsules, cachets, or tablets, or liquids or aerosol sprays each containing a predetermined amount of an active ingredient as a powder or in granules, a solution, or a suspension in an aqueous or nonaqueous liquid, an oil-in- water emulsion, or a water-in-oil liquid emulsion. Such dosage forms can be prepared by any of the methods of pharmacy, but all methods include the step of bringing the active ingredient into association with the carrier, which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet can be prepared by compression or molding, optionally with one or more accessory ingredients. Compressed tablets can be prepared by compressing in a suitable machine the active ingredient in a free- flowing form such as powder or granules, optionally mixed with an excipient such as, but not limited to, a binder, a lubricant, an inert diluent, and/or a surface active or dispersing agent. Molded tablets can be made by molding in a suitable machine a mixture of the powdered compound moistened with an inert liquid diluent.

This invention further encompasses anhydrous pharmaceutical compositions and dosage forms comprising an active ingredient, since water can facilitate the degradation of some compounds. For example, water may be added (e.g., 5%) in the pharmaceutical arts as a means of simulating long-term storage in order to determine characteristics such as shelf- life or the stability of formulations over time. Anhydrous pharmaceutical compositions and dosage forms of the invention can be prepared using anhydrous or low moisture containing ingredients and low moisture or low humidity conditions. Pharmaceutical compositions and dosage forms of the invention which contain lactose can be made anhydrous if substantial contact with moisture and/or humidity during manufacturing, packaging, and/or storage is expected. An anhydrous pharmaceutical composition may be prepared and stored such that its anhydrous nature is maintained. Accordingly, anhydrous compositions may be packaged using materials known to prevent exposure to water such that they can be included in suitable formulary kits. Examples of suitable packaging include, but are not limited to, hermetically sealed foils, plastic or the like, unit dose containers, blister packs, and strip packs.

An active ingredient can be combined in an intimate admixture with a pharmaceutical carrier according to conventional pharmaceutical compounding techniques. The carrier can take a wide variety of forms depending on the form of preparation desired for administration. In preparing the compositions for an oral dosage form, any of the usual pharmaceutical media can be employed as carriers, such as, for example, water, glycols, oils, alcohols, flavoring agents, preservatives, coloring agents, and the like in the case of oral liquid preparations (such as suspensions, solutions, and elixirs) or aerosols; or carriers such as starches, sugars, microcrystalline cellulose, diluents, granulating agents, lubricants, binders, and disintegrating agents can be used in the case of oral solid preparations, in some embodiments without employing the use of lactose. For example, suitable carriers include powders, capsules, and tablets, with the solid oral preparations. If desired, tablets can be coated by standard aqueous or nonaqueous techniques.

Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, microcrystalline cellulose, and mixtures thereof.

Examples of suitable fillers for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof.

Disintegrants may be used in the compositions of the invention to provide tablets that disintegrate when exposed to an aqueous environment. Too much of a disintegrant may produce tablets which may disintegrate in the bottle. Too little may be insufficient for disintegration to occur and may thus alter the rate and extent of release of the active ingredient(s) from the dosage form. Thus, a sufficient amount of disintegrant that is neither too little nor too much to detrimentally alter the release of the active ingredient(s) may be used to form the dosage forms of the compounds disclosed herein. The amount of disintegrant used may vary based upon the type of formulation and mode of administration, and may be readily discernible to those of ordinary skill in the art. About 0.5 to about 15 weight percent of disintegrant, or about 1 to about 5 weight percent of disintegrant, may be used in the pharmaceutical composition. Disintegrants that can be used to form pharmaceutical compositions and dosage forms of the invention include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums or mixtures thereof.

Lubricants which can be used to form pharmaceutical compositions and dosage forms of the invention include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, or mixtures thereof. Additional lubricants include, for example, a syloid silica gel, a coagulated aerosol of synthetic silica, or mixtures thereof. A lubricant can optionally be added, in an amount of less than about 1 weight percent of the pharmaceutical composition.

When aqueous suspensions and/or elixirs are desired for oral administration, the active ingredient therein may be combined with various sweetening or flavoring agents, coloring matter or dyes and, if so desired, emulsifying and/or suspending agents, together with such diluents as water, ethanol, propylene glycol, glycerin and various combinations thereof.

The tablets can be uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Surfactant which can be used to form pharmaceutical compositions and dosage forms of the invention include, but are not limited to, hydrophilic surfactants, lipophilic surfactants, and mixtures thereof. That is, a mixture of hydrophilic surfactants may be employed, a mixture of lipophilic surfactants may be employed, or a mixture of at least one hydrophilic surfactant and at least one lipophilic surfactant may be employed.

A suitable hydrophilic surfactant may generally have an HLB value of at least 10, while suitable lipophilic surfactants may generally have an HLB value of or less than about 10. An empirical parameter used to characterize the relative hydrophilicity and hydrophobicity of non-ionic amphiphilic compounds is the hydrophilic-lipophilic balance (" HLB" value). Surfactants with lower HLB values are more lipophilic or hydrophobic, and have greater solubility in oils, while surfactants with higher HLB values are more hydrophilic, and have greater solubility in aqueous solutions.

Hydrophilic surfactants are generally considered to be those compounds having an HLB value greater than about 10, as well as anionic, cationic, or zwitterionic compounds for which the HLB scale is not generally applicable. Similarly, lipophilic (e.g., hydrophobic) surfactants are compounds having an HLB value equal to or less than about 10. However, HLB value of a surfactant is merely a rough guide generally used to enable formulation of industrial, pharmaceutical and cosmetic emulsions.

Hydrophilic surfactants may be either ionic or non-ionic. Suitable ionic surfactants include, but are not limited to, alkylammonium salts; fusidic acid salts; fatty acid derivatives of amino acids, oligopeptides, and polypeptides; glyceride derivatives of amino acids, oligopeptides, and polypeptides; lecithins and hydrogenated lecithins; lysolecithins and hydrogenated lysolecithins; phospholipids and derivatives thereof; lysophospholipids and derivatives thereof; carnitine fatty acid ester salts; salts of alkylsulfates; fatty acid salts; sodium docusate; acyl lactylates; mono- and di-acetylated tartaric acid esters of mono- and di-glycerides; succinylated mono- and di-glycerides; citric acid esters of mono- and di-glycerides; and mixtures thereof.

Within the aforementioned group, ionic surfactants include, by way of example: lecithins, lysolecithin, phospholipids, lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Ionic surfactants may be the ionized forms of lecithin, lysolecithin, phosphatidylcholine, phosphatidylethanolamine, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG-phosphatidylethanolamine, PVP - phosphatidylethanolamine, lactylic esters of fatty acids, stearoyl-2-lactylate, stearoyl lactylate, succinylated monoglycerides, mono/diacetylated tartaric acid esters of mono/diglycerides, citric acid esters of mono/diglycerides, cholylsarcosine, caproate, caprylate, caprate, laurate, myristate, palmitate, oleate, ricinoleate, linoleate, linolenate, stearate, lauryl sulfate, teracecyl sulfate, docusate, lauroyl carnitines, palmitoyl carnitines, myristoyl carnitines, and salts and mixtures thereof.

Hydrophilic non-ionic surfactants may include, but are not limited to, alkylglucosides; alkylmaltosides; alkylthioglucosides; lauryl macrogolglycerides; polyoxyalkylene alkyl ethers such as polyethylene glycol alkyl ethers; polyoxyalkylene alkylphenols such as polyethylene glycol alkyl phenols; polyoxyalkylene alkyl phenol fatty acid esters such as polyethylene glycol fatty acids monoesters and polyethylene glycol fatty acids diesters; polyethylene glycol glycerol fatty acid esters; polyglycerol fatty acid esters; polyoxyalkylene sorbitan fatty acid esters such as polyethylene glycol sorbitan fatty acid esters; hydrophilic transesterification products of a polyol with at least one member of the group consisting of glycerides, vegetable oils, hydrogenated vegetable oils, fatty acids, and sterols; polyoxyethylene sterols, derivatives, and analogues thereof; polyoxyethylated vitamins and derivatives thereof; polyoxyethylene-polyoxypropylene block copolymers; and mixtures thereof; polyethylene glycol sorbitan fatty acid esters and hydrophilic transesterification products of a polyol with at least one member of the group consisting of triglycerides, vegetable oils, and hydrogenated vegetable oils. The polyol may be glycerol, ethylene glycol, polyethylene glycol, sorbitol, propylene glycol, pentaerythritol, or a saccharide.

Other hydrophilic-non-ionic surfactants include, without limitation, PEG- 10 laurate, PEG- 12 laurate, PEG-20 laurate, PEG-32 laurate, PEG-32 dilaurate, PEG- 12 oleate, PEG- 15 oleate, PEG-20 oleate, PEG-20 dioleate, PEG-32 oleate, PEG-200 oleate, PEG-400 oleate, PEG-15 stearate, PEG-32 distearate, PEG-40 stearate, PEG- 100 stearate, PEG-20 dilaurate, PEG-25 glyceryl trioleate, PEG-32 dioleate, PEG-20 glyceryl laurate, PEG-30 glyceryl laurate, PEG-20 glyceryl stearate, PEG-20 glyceryl oleate, PEG-30 glyceryl oleate, PEG-30 glyceryl laurate, PEG-40 glyceryl laurate, PEG-40 palm kernel oil, PEG-50 hydrogenated castor oil, PEG-40 castor oil, PEG-35 castor oil, PEG-60 castor oil, PEG-40 hydrogenated castor oil, PEG-60 hydrogenated castor oil, PEG-60 corn oil, PEG-6 caprate/caprylate glycerides, PEG-8 caprate/caprylate glycerides, polyglyceryl-10 laurate, PEG-30 cholesterol, PEG-25 phyto sterol, PEG-30 soya sterol, PEG-20 trioleate, PEG-40 sorbitan oleate, PEG-80 sorbitan laurate, polysorbate 20, polysorbate 80, POE-9 lauryl ether, POE-23 lauryl ether, POE-10 oleyl ether, POE-20 oleyl ether, POE-20 stearyl ether, tocopheryl PEG- 100 succinate, PEG-24 cholesterol, polyglyceryl-lOoleate, Tween 40, Tween 60, sucrose monostearate, sucrose mono laurate, sucrose monopalmitate, PEG 10-100 nonyl phenol series, PEG 15-100 octyl phenol series, and poloxamers.

Suitable lipophilic surfactants include, by way of example only: fatty alcohols; glycerol fatty acid esters; acetylated glycerol fatty acid esters; lower alcohol fatty acids esters; propylene glycol fatty acid esters; sorbitan fatty acid esters; polyethylene glycol sorbitan fatty acid esters; sterols and sterol derivatives; polyoxyethylated sterols and sterol derivatives; polyethylene glycol alkyl ethers; sugar esters; sugar ethers; lactic acid derivatives of mono- and di-glycerides; hydrophobic transesterification products of a polyol with at least one member of the group consisting of glycerides, vegetable oils, hydrogenated vegetable oils, fatty acids and sterols; oil-soluble vitamins/vitamin derivatives; and mixtures thereof. Within this group, preferred lipophilic surfactants include glycerol fatty acid esters, propylene glycol fatty acid esters, and mixtures thereof, or are hydrophobic transesterification products of a polyol with at least one member of the group consisting of vegetable oils, hydrogenated vegetable oils, and triglycerides.

In one embodiment, the composition may include a solubilizer to ensure good solubilization and/or dissolution of the compound of the present invention and to minimize precipitation of the compound of the present invention. This can be especially important for compositions for non-oral use, e.g., compositions for injection. A solubilizer may also be added to increase the solubility of the hydrophilic drug and/or other components, such as surfactants, or to maintain the composition as a stable or homogeneous solution or dispersion.

Examples of suitable solubilizers include, but are not limited to, the following: alcohols and polyols, such as ethanol, isopropanol, butanol, benzyl alcohol, ethylene glycol, propylene glycol, butanediols and isomers thereof, glycerol, pentaerythritol, sorbitol, mannitol, transcutol, dimethyl isosorbide, polyethylene glycol, polypropylene glycol, polyvinylalcohol, hydroxypropyl methylcellulose and other cellulose derivatives, cyclodextrins and cyclodextrin derivatives; ethers of polyethylene glycols having an average molecular weight of about 200 to about 6000, such as tetrahydrofurfuryl alcohol PEG ether (glycofurol) or methoxy PEG ; amides and other nitrogen-containing compounds such as 2-pyrrolidone, 2-piperidone, ε-caprolactam, N-alkylpyrrolidone, N-hydroxyalkylpyrrolidone, N-alkylpiperidone, N-alkylcaprolactam, dimethylacetamide and polyvinylpyrrolidone; esters such as ethyl propionate, tributylcitrate, acetyl triethylcitrate, acetyl tributyl citrate, triethylcitrate, ethyl oleate, ethyl caprylate, ethyl butyrate, triacetin, propylene glycol monoacetate, propylene glycol diacetate, ε-caprolactone and isomers thereof, δ-valerolactone and isomers thereof, β-butyrolactone and isomers thereof; and other solubilizers known in the art, such as dimethyl acetamide, dimethyl isosorbide, N-methyl pyrrolidones, monooctanoin, diethylene glycol monoethyl ether, and water.

Mixtures of solubilizers may also be used. Examples include, but not limited to, triacetin, triethylcitrate, ethyl oleate, ethyl caprylate, dimethylacetamide, N-methylpyrrolidone, N-hydroxyethylpyrrolidone, polyvinylpyrrolidone, hydroxypropyl methylcellulose, hydroxypropyl cyclodextrins, ethanol, polyethylene glycol 200-100, glycofurol, transcutol, propylene glycol, and dimethyl isosorbide. Particularly preferred solubilizers include sorbitol, glycerol, triacetin, ethyl alcohol, PEG-400, glycofurol and propylene glycol.

The amount of solubilizer that can be included is not particularly limited. The amount of a given solubilizer may be limited to a bioacceptable amount, which may be readily determined by one of skill in the art. In some circumstances, it may be advantageous to include amounts of solubilizers far in excess of bioacceptable amounts, for example to maximize the concentration of the drug, with excess solubilizer removed prior to providing the composition to a subject using conventional techniques, such as distillation or evaporation. Thus, if present, the solubilizer can be in a weight ratio of 10%, 25%o, 50%), 100%o, or up to about 200%> by weight, based on the combined weight of the drug, and other excipients. If desired, very small amounts of solubilizer may also be used, such as 5%>, 2%>, 1%) or even less. Typically, the solubilizer may be present in an amount of about 1%> to about 100%, more typically about 5%> to about 25%> by weight.

The composition can further include one or more pharmaceutically acceptable additives and excipients. Such additives and excipients include, without limitation, detackifiers, antifoaming agents, buffering agents, polymers, antioxidants, preservatives, chelating agents, viscomodulators, tonicifiers, flavorants, colorants, odorants, opacifiers, suspending agents, binders, fillers, plasticizers, lubricants, and mixtures thereof.

In addition, an acid or a base may be incorporated into the composition to facilitate processing, to enhance stability, or for other reasons. Examples of pharmaceutically acceptable bases include amino acids, amino acid esters, ammonium hydroxide, potassium hydroxide, sodium hydroxide, sodium hydrogen carbonate, aluminum hydroxide, calcium carbonate, magnesium hydroxide, magnesium aluminum silicate, synthetic aluminum silicate, synthetic hydrocalcite, magnesium aluminum hydroxide, diisopropylethylamine, ethanolamine, ethylenediamine, triethanolamine, triethylamine, triisopropanolamine, trimethylamine, tris(hydroxymethyl)aminomethane (TRIS) and the like. Also suitable are bases that are salts of a pharmaceutically acceptable acid, such as acetic acid, acrylic acid, adipic acid, alginic acid, alkanesulfonic acid, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid, uric acid, and the like. Salts of polyprotic acids, such as sodium phosphate, disodium hydrogen phosphate, and sodium dihydrogen phosphate can also be used. When the base is a salt, the cation can be any convenient and pharmaceutically acceptable cation, such as ammonium, alkali metals, alkaline earth metals, and the like. Example may include, but not limited to, sodium, potassium, lithium, magnesium, calcium and ammonium.

Suitable acids are pharmaceutically acceptable organic or inorganic acids. Examples of suitable inorganic acids include hydrochloric acid, hydrobromic acid, hydriodic acid, sulfuric acid, nitric acid, boric acid, phosphoric acid, and the like. Examples of suitable organic acids include acetic acid, acrylic acid, adipic acid, alginic acid, alkanesulfonic acids, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, methanesulfonic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid, uric acid and the like.

### Pharmaceutical compositions for injection

In some embodiments, the invention provides a pharmaceutical composition for injection containing a compound of the present invention and a pharmaceutical excipient suitable for injection. Components and amounts of agents in the compositions are as described herein.

The forms in which the novel compositions of the present invention may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Aqueous solutions in saline are also conventionally used for injection. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, for the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions are prepared by incorporating the compound of the present invention in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, certain desirable methods of preparation are vacuum-drying and freeze- drying techniques which yield a powder of the active ingredient plus any additional desired ingredient from a previously sterile-filtered solution thereof.

### Pharmaceutical Compositions for Topical (e.g., Transdermal) Delivery

In some embodiments, the invention provides a pharmaceutical composition for transdermal delivery containing a compound of the present invention and a pharmaceutical excipient suitable for transdermal delivery.

Compositions of the present invention can be formulated into preparations in solid, semisolid, or liquid forms suitable for local or topical administration, such as gels, water soluble jellies, creams, lotions, suspensions, foams, powders, slurries, ointments, solutions, oils, pastes, suppositories, sprays, emulsions, saline solutions, dimethylsulfoxide (DMSO)-based solutions. In general, carriers with higher densities are capable of providing an area with a prolonged exposure to the active ingredients. In contrast, a solution formulation may provide more immediate exposure of the active ingredient to the chosen area.

The pharmaceutical compositions also may comprise suitable solid or gel phase carriers or excipients, which are compounds that allow increased penetration of, or assist in the delivery of, therapeutic molecules across the stratum corneum permeability barrier of the skin. There are many of these penetration- enhancing molecules known to those trained in the art of topical formulation.

Examples of such carriers and excipients include, but are not limited to, humectants (e.g., urea), glycols (e.g., propylene glycol), alcohols (e.g., ethanol), fatty acids (e.g., oleic acid), surfactants (e.g., isopropyl myristate and sodium lauryl sulfate), pyrrolidones, glycerol monolaurate, sulfoxides, terpenes (e.g., menthol), amines, amides, alkanes, alkanols, water, calcium carbonate, calcium phosphate, various sugars, starches, cellulose derivatives, gelatin, and polymers such as polyethylene glycols.

Another exemplary formulation for use in the methods of the present invention employs transdermal delivery devices ("patches"). Such transdermal patches may be used to provide continuous or discontinuous infusion of a compound of the present invention in controlled amounts, either with or without another agent.

The construction and use of transdermal patches for the delivery of pharmaceutical agents is well known in the art. See, e.g., U.S. Pat. Nos. 5,023,252, 4,992,445 and 5,001,139. Such patches may be constructed for continuous, pulsatile, or on demand delivery of pharmaceutical agents.

### Pharmaceutical Compositions for Inhalation

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices that deliver the formulation in an appropriate manner.

### Other Pharmaceutical Compositions

Pharmaceutical compositions may also be prepared from compositions described herein and one or more pharmaceutically acceptable excipients suitable for sublingual, buccal, rectal, intraosseous, intraocular, intranasal, epidural, or intraspinal administration. Preparations for such pharmaceutical compositions are well-known in the art. See, e.g., Anderson, Philip O.; Knoben, James E.; Troutman, William G, eds., Handbook of Clinical Drug Data, Tenth Edition, McGraw-Hill, 2002; Pratt and Taylor, eds., Principles of Drug Action, Third Edition, Churchill Livingston, New York, 1990; Katzung, ed., Basic and Clinical Pharmacology, Ninth Edition, McGraw Hill, 20037ybg; Goodman and Gilman, eds., The Pharmacological Basis of Therapeutics, Tenth Edition, McGraw Hill, 2001 ; Remingtons Pharmaceutical Sciences, 20th Ed., Lippincott Williams & Wilkins., 2000; Martindale, The Extra Pharmacopoeia, Thirty-Second Edition (The Pharmaceutical Press, London, 1999).

Administration of the compounds or pharmaceutical composition of the present invention can be affected by any method that enables delivery of the compounds to the site of action. These methods include oral routes, intraduodenal routes, parenteral injection (including intravenous, intraarterial, subcutaneous, intramuscular, intravascular, intraperitoneal or infusion), topical (e.g., transdermal application), rectal administration, via local delivery by catheter or stent or through inhalation. Compounds can also be administered intraadiposally or intrathecally.

In some embodiments, the compounds or pharmaceutical composition of the present invention are administered by intravenous injection.

The amount of the compound administered will be dependent on the subject being treated, the severity of the disorder or condition, the rate of administration, the disposition of the compound and the discretion of the prescribing physician. However, an effective dosage is in the range of about 0.001 to about 100 mg per kg body weight per day, preferably about 1 to about 35 mg/kg/day, in single or divided doses. For a 70 kg human, this would amount to about 0.05 to 7 g/day, preferably about 0.05 to about 2.5 g/day. In some instances, dosage levels below the lower limit of the aforesaid range may be more than adequate, while in other cases still larger doses may be employed without causing any harmful side effect, e.g., by dividing such larger doses into several small doses for administration throughout the day.

In some embodiments, a compound of the invention is administered in a single dose.

Typically, such administration will be by injection, e.g., intravenous injection, in order to introduce the agent quickly. However, other routes may be used as appropriate. A single dose of a compound of the invention may also be used for treatment of an acute condition.

In some embodiments, a compound of the invention is administered in multiple doses. Dosing may be about once, twice, three times, four times, five times, six times, or more than six times per day. Dosing may be about once a month, once every two weeks, once a week, or once every other day. In another embodiment a compound of the invention and another agent are administered together about once per day to about 6 times per day. In another embodiment the administration of a compound of the invention and an agent continues for less than about 7 days. In yet another embodiment the administration continues for more than about 6, 10, 14, 28 days, two months, six months, or one year. In some cases, continuous dosing is achieved and maintained as long as necessary.

Administration of the compounds of the invention may continue as long as necessary. In some embodiments, a compound of the invention is administered for more than 1, 2, 3, 4, 5, 6, 7, 14, or 28 days. In some embodiments, a compound of the invention is administered for less than 28, 14, 7, 6, 5, 4, 3, 2, or 1 day. In some embodiments, a compound of the invention is administered chronically on an ongoing basis, e.g., for the treatment of chronic effects.

An effective amount of a compound of the invention may be administered in either single or multiple doses by any of the accepted modes of administration of agents having similar utilities, including rectal, buccal, intranasal and transdermal routes, by intra-arterial injection, intravenously, intraperitoneally, parenterally, intramuscularly, subcutaneously, orally, topically, or as an inhalant.

The compositions of the invention may also be delivered via an impregnated or coated device such as a stent, for example, or an artery-inserted cylindrical polymer. Such a method of administration may, for example, aid in the prevention or amelioration of restenosis following procedures such as balloon angioplasty. Without being bound by theory, compounds of the invention may slow or inhibit the migration and proliferation of smooth muscle cells in the arterial wall which contribute to restenosis. A compound of the invention may be administered, for example, by local delivery from the struts of a stent, from a stent graft, from grafts, or from the cover or sheath of a stent. In some embodiments, a compound of the invention is admixed with a matrix. Such a matrix may be a polymeric matrix and may serve to bond the compound to the stent. Polymeric matrices suitable for such use, include, for example, lactone-based polyesters or copolyesters such as polylactide, polycaprolactonglycolide, polyorthoesters, polyanhydrides, polyaminoacids, polysaccharides, polyphosphazenes, poly (ether-ester) copolymers (e.g. PEO-PLLA); polydimethylsiloxane, poly(ethylene-vinylacetate), acrylate-based polymers or copolymers (e.g. polyhydroxyethyl methylmethacrylate, polyvinyl pyrrolidinone), fluorinated polymers such as polytetrafluoroethylene and cellulose esters. Suitable matrices may be nondegrading or may degrade with time, releasing the compound or compounds. Compounds of the invention may be applied to the surface of the stent by various methods such as dip/spin coating, spray coating, dip-coating, and/or brush-coating. The compounds may be applied in a solvent and the solvent may be allowed to evaporate, thus forming a layer of compound onto the stent. Alternatively, the compound may be located in the body of the stent or graft, for example in microchannels or micropores. When implanted, the compound diffuses out of the body of the stent to contact the arterial wall. Such stents may be prepared by dipping a stent manufactured to contain such micropores or microchannels into a solution of the compound of the invention in a suitable solvent, followed by evaporation of the solvent. Excess drug on the surface of the stent may be removed via an additional brief solvent wash. In yet other embodiments, compounds of the invention may be covalently linked to a stent or graft. A covalent linker may be used which degrades in vivo, leading to the release of the compound of the invention. Any bio-labile linkage may be used for such a purpose, such as ester, amide or anhydride linkages. Compounds of the invention may additionally be administered intravascularly from a balloon used during angioplasty. Extravascular administration of the compounds via the pericard or via advential application of formulations of the invention may also be performed to decrease restenosis.

A variety of stent devices which may be used as described are disclosed, for example, in the following references: U.S. Pat. No. 5451233; U.S. Pat. No. 5040548; U.S. Pat. No. 5061273; U.S. Pat. No. 5496346; U.S. Pat. No. 5292331; U.S. Pat. No. 5674278; U.S. Pat. No. 3657744; U.S. Pat. No. 4739762; U.S. Pat. No. 5195984; U.S. Pat. No. 5292331 ; U.S. Pat. No. 5674278; U.S. Pat. No. 5879382; U.S. Pat. No. 6344053.

The compounds of the invention may be administered in dosages. It is known in the art that due to intersubject variability in compound pharmacokinetics, individualization of dosing regimen is necessary for optimal therapy. Dosing for a compound of the invention may be found by routine experimentation in light of the instant disclosure.

When a compound of the invention is administered in a composition that comprises one or more agents, and the agent has a shorter half- life than the compound of the invention unit dose forms of the agent and the compound of the invention may be adjusted accordingly.

The subject pharmaceutical composition may, for example, be in a form suitable for oral administration as a tablet, capsule, pill, powder, sustained release formulations, solution, suspension, for parenteral injection as a sterile solution, suspension or emulsion, for topical administration as an ointment or cream or for rectal administration as a suppository. The pharmaceutical composition may be in unit dosage forms suitable for single administration of precise dosages. The pharmaceutical composition will include a conventional pharmaceutical carrier or excipient and a compound according to the invention as an active ingredient. In addition, it may include other medicinal or pharmaceutical agents, carriers, adjuvants, etc. Exemplary parenteral administration forms include solutions or suspensions of active compound in sterile aqueous solutions, for example, aqueous propylene glycol or dextrose solutions. Such dosage forms can be suitably buffered, if desired.

### Methods of Use

The method typically comprises administering to a subject a therapeutically effective amount of a compound of the invention. The therapeutically effective amount of the subject combination of compounds may vary depending upon the intended application (in vitro or in vivo), or the subject and disease condition being treated, e.g., the weight and age of the subject, the severity of the disease condition, the manner of administration and the like, which can readily be determined by one of ordinary skill in the art. The term also applies to a dose that will induce a particular response in target cells, e.g., reduction of proliferation or downregulation of activity of a target protein. The specific dose will vary depending on the particular compounds chosen, the dosing regimen to be followed, whether it is administered in combination with other compounds, timing of administration, the tissue to which it is administered, and the physical delivery system in which it is carried.

In certain embodiment, the present invention provides a pharmaceutical composition comprising a compound of bispecific formula, or pharmaceutically acceptable salt thereof.

In certain embodiment, the present invention provides a pharmaceutical composition comprising a compound of bispecific formula for use in degrading a target protein in a cell.

In certain embodiment, a method of degrading a target protein comprising administering to a cell therapeutically effective amount of a bispecific compound, or pharmaceutically acceptable salt, wherein the compound is effective for degrading the target protein.

In certain embodiment, the present invention provides a pharmaceutical composition comprising a compound of bispecific formula, for use in treating or preventing of a disease or disorder in which SMARCA2 and/or SMARCA4 plays a role.

In certain embodiment, the present invention provides a pharmaceutical composition comprising a compound of bispecific formula, for use in treating or preventing of a disease or disorder in which SWI/SNF mutations plays a role.

In certain embodiment, target proteins are SMARCA2, SMARCA4 and/or PB1.

In certain embodiment, target protein complex is SWI/SNF in a cell.

In certain embodiment, diseases or disorders dependent on SMARCA2 or SMARCA4 include cancers.

In certain embodiment, diseases or disorders dependent on SWI/SNF complex include cancers.

Exemplary cancers which may be treated by the present compounds either alone or in combination with at least one additional anti-cancer agent include squamous-cell carcinoma, basal cell carcinoma, adenocarcinoma, hepatocellular carcinomas, and renal cell carcinomas, cancer of the bladder, bowel, breast, cervix, colon, esophagus, head, kidney, liver, lung, neck, ovary, pancreas, prostate, and stomach; leukemias; benign and malignant lymphomas, particularly Burkitt's lymphoma and Non-Hodgkin's lymphoma; benign and malignant melanomas; myeloproliferative diseases; sarcomas, including Ewing's sarcoma, hemangiosarcoma, Kaposi's sarcoma, liposarcoma, myosarcomas, peripheral neuroepithelioma, synovial sarcoma, gliomas, astrocytomas, oligodendrogliomas, ependymomas, gliobastomas, neuroblastomas, ganglioneuromas, gangliogliomas, medulloblastomas, pineal cell tumors, meningiomas, meningeal sarcomas, neurofibromas, and Schwannomas; bowel cancer, breast cancer, prostate cancer, cervical cancer, uterine cancer, lung cancer, ovarian cancer, testicular cancer, thyroid cancer, astrocytoma, esophageal cancer, pancreatic cancer, stomach cancer, liver cancer, colon cancer, melanoma; carcinosarcoma, Hodgkin's disease, Wilms' tumor and teratocarcinomas.

In certain embodiments, the cancers which may be treated using compounds according to the present disclosure include, for example, T-lineage Acute lymphoblastic Leukemia (T-ALL), T-lineage lymphoblastic Lymphoma (T-LL), Peripheral T-cell lymphoma, Adult T-cell Leukemia, Pre-B ALL, Pre-B Lymphomas, Large B-cell Lymphoma, Burkitts Lymphoma, B-cell ALL, Philadelphia chromosome positive ALL and Philadelphia chromosome positive CML.

In certain further embodiment, the cancer is a SMARCA2 and/or SMARAC4-dependent cancer.

In certain embodiment, the present invention provides a pharmaceutical composition comprising a compound of bispecific formula for use in the diseases or disorders dependent upon SMARCA2 and/or SMARCA4 is cancer.

Compounds of the disclosure, as well as pharmaceutical compositions comprising them, can be administered to treat any of the described diseases, alone or in combination with a medical therapy. Medical therapies include, for example, surgery and radiotherapy (e.g., gamma-radiation, neutron beam radiotherapy, electron beam radiotherapy, proton therapy, brachytherapy, systemic radioactive isotopes).

In other aspects, compounds of the disclosure, as well as pharmaceutical compositions comprising them, can be administered to treat any of the described diseases, alone or in combination with one or more other agents.

In other methods, the compounds of the disclosure, as well as pharmaceutical compositions comprising them, can be administered in combination with agonists of nuclear receptors agents.

In other methods, the compounds of the disclosure, as well as pharmaceutical compositions comprising them, can be administered in combination with antagonists of nuclear receptors agents.

In other methods, the compounds of the disclosure, as well as pharmaceutical compositions comprising them, can be administered in combination with an anti-proliferative agent.

### Combination Therapies

For treating cancer and other proliferative diseases, the compounds of the invention can be used in combination with chemotherapeutic agents, agonists or antagonists of nuclear receptors, or other anti-proliferative agents. The compounds of the invention can also be used in combination with a medical therapy such as surgery or radiotherapy, e.g., gamma-radiation, neutron beam radiotherapy, electron beam radiotherapy, proton therapy, brachytherapy, and systemic radioactive isotopes. Examples of suitable chemotherapeutic agents include any of: abarelix, aldesleukin, alemtuzumab, alitretinoin, allopurinol, all-trans retinoic acid, altretamine, anastrozole, arsenic trioxide, asparaginase, azacitidine, bendamustine, bevacizumab, bexarotene, bleomycin, bortezombi, bortezomib, busulfan intravenous, busulfan oral, calusterone, capecitabine, carboplatin, carmustine, cetuximab, chlorambucil, cisplatin, cladribine, clofarabine, cyclophosphamide, cytarabine, dacarbazine, dactinomycin, dalteparin sodium, dasatinib, daunorubicin, decitabine, denileukin, denileukin diftitox, dexrazoxane, docetaxel, doxorubicin, dromostanolone propionate, eculizumab, epirubicin, erlotinib, estramustine, etoposide phosphate, etoposide, exemestane, fentanyl citrate, filgrastim, floxuridine, fludarabine, fluorouracil, fulvestrant, gefitinib, gemcitabine, gemtuzumab ozogamicin, goserelin acetate, histrelin acetate, ibritumomab tiuxetan, idarubicin, ifosfamide, imatinib mesylate, interferon alfa 2a, irinotecan, lapatinib ditosylate, lenalidomide, letrozole, leucovorin, leuprolide acetate, levamisole, lomustine, meclorethamine, megestrol acetate, melphalan, mercaptopurine, methotrexate, methoxsalen, mitomycin C, mitotane, mitoxantrone, nandrolone phenpropionate, nelarabine, nofetumomab, oxaliplatin, paclitaxel, pamidronate, panobinostat, panitumumab, pegaspargase, pegfilgrastim, pemetrexed disodium, pentostatin, pipobroman, plicamycin, procarbazine, quinacrine, rasburicase, rituximab, ruxolitinib, sorafenib, streptozocin, sunitinib, sunitinib maleate, tamoxifen, temozolomide, teniposide, testolactone, thalidomide, thioguanine, thiotepa, topotecan, toremifene, tositumomab, trastuzumab, tretinoin, uracil mustard, valrubicin, vinblastine, vincristine, vinorelbine, vorinstat and zoledronate.

In some embodiments, the compounds of the invention can be used in combination with a therapeutic agent that targets an epigenetic regulator. Examples of epigenetic regulators include bromodomain inhibitors, the histone lysine methyltransferase inhibitors, histone arginine methyl transferase inhibitors, histone demethylase inhibitors, histone deacetylase inhibitors, histone acetylase inhibitors, and DNA methyltransferase inhibitors. Histone deacetylase inhibitors include, e.g., vorinostat. Histone arginine methyl transferase inhibitors include inhibitors of protein arginine methyltransferases (PRMTs) such as PRMT5, PRMT1 and PRMT4. DNA methyltransferase inhibitors include inhibitors of DNMT1 and DNMT3.

For treating cancer and other proliferative diseases, the compounds of the invention can be used in combination with targeted therapies, including JAK kinase inhibitors (e.g. Ruxolitinib), PI3 kinase inhibitors including PI3K-delta selective and broad spectrum PI3K inhibitors, MEK inhibitors, Cyclin Dependent kinase inhibitors, including CDK4/6 inhibitors and CDK9 inhibitors, BRAF inhibitors, mTOR inhibitors, proteasome inhibitors (e.g. Bortezomib, Carfilzomib), HDAC inhibitors (e.g. panobinostat, vorinostat), DNA methyl transferase inhibitors, dexamethasone, bromo and extra terminal family member (BET) inhibitors, BTK inhibitors (e.g. ibrutinib, acalabrutinib), BCL2 inhibitors (e.g. venetoclax), dual BCL2 family inhibitors (e.g. BCL2/BCLxL), PARP inhibitors, FLT3 inhibitors, or LSD1 inhibitors.

In some embodiments, the inhibitor of an immune checkpoint molecule is an inhibitor of PD-1, e.g., an anti-PD-1 monoclonal antibody. In some embodiments, the anti-PD-1 monoclonal antibody is nivolumab, pembrolizumab (also known as MK-3475), or PDR001. In some embodiments, the anti-PD-1 monoclonal antibody is nivolumab or pembrolizumab. In some embodiments, the anti-PD1 antibody is pembrolizumab. In some embodiments, the inhibitor of an immune checkpoint molecule is an inhibitor of PD-L1, e.g., an anti-PD-L1 monoclonal antibody. In some embodiments, the anti-PD-L1 monoclonal antibody is atezolizumab, durvalumab, or BMS-935559. In some embodiments, the inhibitor of an immune checkpoint molecule is an inhibitor of CTLA-4, e.g., an anti-CTLA-4 antibody. In some embodiments, the anti-CTLA-4 antibody is ipilimumab.

In some embodiments, the agent is an alkylating agent, a proteasome inhibitor, a corticosteroid, or an immunomodulatory agent. Examples of an alkylating agent include cyclophosphamide (CY), melphalan (MEL), and bendamustine. In some embodiments, the proteasome inhibitor is carfilzomib. In some embodiments, the corticosteroid is dexamethasone (DEX). In some embodiments, the immunomodulatory agent is lenalidomide (LEN) or pomalidomide (POM).

Compounds of the invention can be prepared using numerous preparatory reactions known in the literature. The Schemes below provide general guidance in connection with preparing the compounds of the invention. One skilled in the art would understand that the preparations shown in the Schemes can be modified or optimized using general knowledge of organic chemistry to prepare various compounds of the invention. Example synthetic methods for preparing compounds of the invention are provided in the Schemes below.

The following Examples are provided to illustrate some of the concepts described within this disclosure. Any example falling outside the scope of the appended claims is provided for reference purposes.

### EXAMPLES

### General Synthetic Procedures

The compounds described herein may be prepared according to the following synthetic schemes and general synthetic procedures.

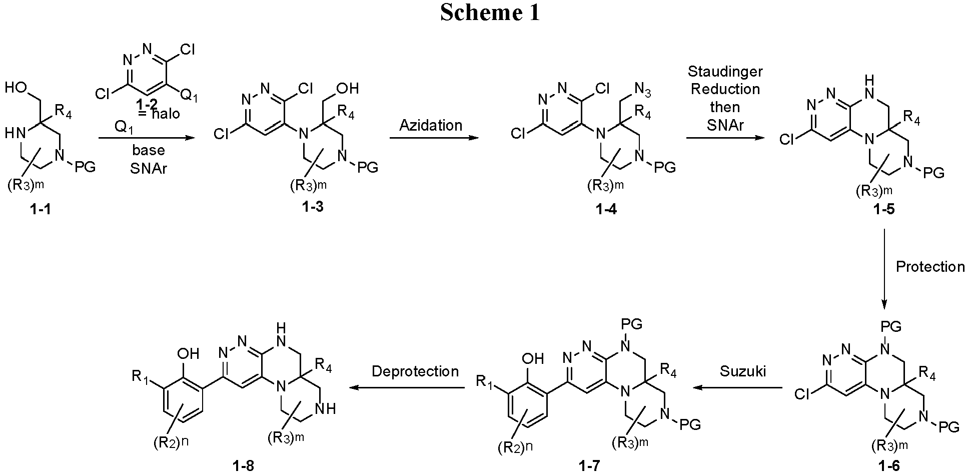

The compounds of Formula (1-8) where R₄ = H, can be made according to the route described in Scheme 1. S_{N}Ar reaction between commercially available starting materials 1-1, where R₄ = H and compounds 1-2, where Q₁ = Cl, Br, in the presence of base (e.g., Cs₂CO₃, NaHCO₃, DIPEA) at elevated room temperatures gives alcohol 1-3. Azidation of compounds 1-3 upon treatment with PPh₃/DIAD/DPPA yields compounds 1-4. Staudinger reduction of compounds 1-4 upon treatment with PPh₃ at elevated temperatures followed by S_{N}Ar in the presence of base (e.g., Cs₂CO₃, NaHCO₃, DIPEA) yields compounds 1-5. Protection of the -NH group with an appropriate group (e.g., Boc, SEM, Bn, etc.) can give compounds 1-6, which can be converted to compounds 1-7 under standard Suzuki conditions (e.g., in the presence of a palladium catalyst, such as but not limited to tetrakis(triphenylphosphine)palladium(0) or [1,1'-bis (diphenylphosphino)ferrocene] dichloropalladium (II), complex with dichloromethane and a base (e.g., a carbonate base)) using the appropriate boronic acid or ester (e.g., 2-hydroxy-phenylboronic acid). Removal of the protecting groups can yield compounds **1-8.**

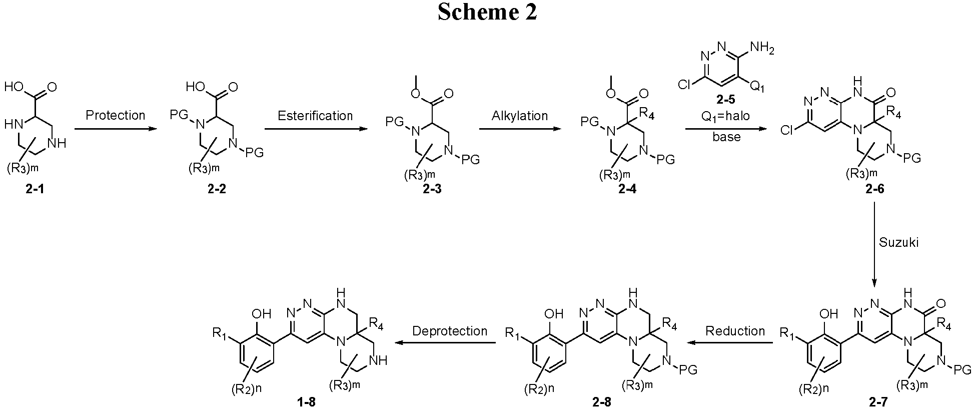

The compounds of **Formula (1-8)** where R₄ ≠ H can be made according to the route described in **Scheme 2.** Protection of the -NH group of commercially available starting materials **2-1** with an appropriate group (e.g., Boc, SEM, Bn, etc.) can give compounds **2-2.** Esterification of compounds **2-2** in the presence of base (e.g., Cs₂CO₃, NaHCO₃, DIPEA) yields compounds **2-3.** Alkylation of compounds **2-3** upon sequential treatment with LiHMDS and an electrophile (e.g. MeI), results in compounds **2-4,** where R₄ ≠ H. One pot amide formation, selective deprotection, and S_{N}Ar between compounds **2-4** and compounds **2-5,** where Q₁ = Cl, Br, in the presence of base (e.g., Cs₂CO₃, NaHCO₃, DIPEA) at elevated temperature gives compounds **2-6.** Compounds **2-6** can be converted to compounds **2-7** under standard Suzuki conditions (e.g., in the presence of a palladium catalyst, such as but not limited to tetrakis-(triphenylphosphine)palladium(0) or [1,1'-bis (diphenylphosphino)ferrocene] dichloropalladium (II), complex with dichloromethane and a base (e.g., a carbonate base)) using the appropriate boronic acid or ester (e.g., 2-hydroxy-phenylboronic acid). Reduction of compounds **2-7** in the presence of BH₃·THF at elevated temperatures yields compounds **2-8.** Removal of the protecting groups can yield compounds **1-8.**

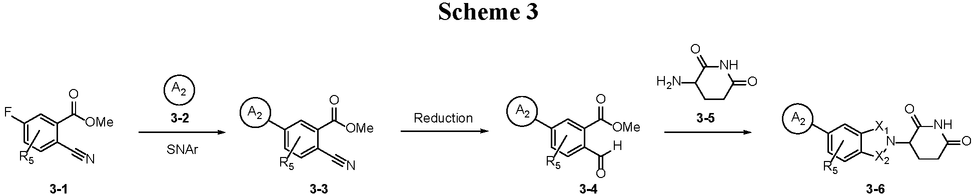

The intermediate of formula **3-6,** where X₁= CO and X₂= CH₂, can be synthesized according to the route described **Scheme 3.** S_{N}Ar between **3-1** and **3-2** (e.g., DIPEA) can afford compound **3-3.** Reduction of the cyano in **3-3,** can be achieved under appropriate conditions (e.g., Raney Nickel) to yield **3-4.** Reductive amination and cyclization **of 3-4** with 3-5 (e.g., DIPEA followed by AcOH and Sodium triacetoxyborohydride) affords **3-6,** wherein A₂ and R₅ are defined above.

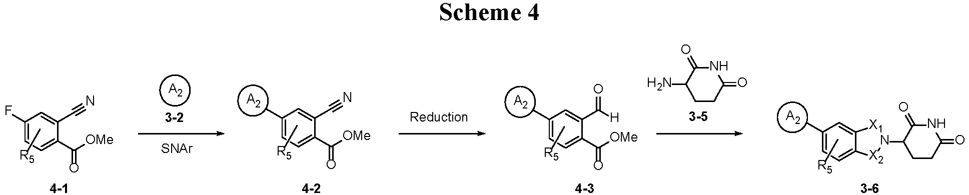

The intermediate of formula **3-6,** where X₁= CH₂ and X₂= CO, can be synthesized according to the route described **Scheme 4.** S_{N}Ar between **4-1** and **3-2** (e.g., DIPEA) can afford compound **4-2.** Reduction of the cyano in **4-2,** can be achieved under appropriate conditions (e.g., Raney Nickel) to yield **4-3.** Reductive amination and cyclization of **4-3** with **3-5** (e.g., DIPEA followed by AcOH and sodium triacetoxyborohydride) affords **3-6,** wherein A₂ and R₅ are defined above.

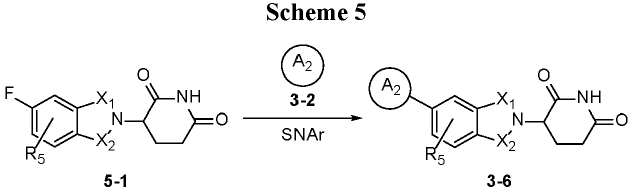

The intermediate of formula **3-6,** where X₁ = CO and X₂ = CO, can be synthesized according to the route described in **Scheme 5.** S_{N}Ar between **5-1** and **3-2** (e.g., DIPEA) can afford compound **3-6,** wherein A₂ and R₅ are defined above.

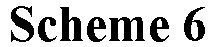

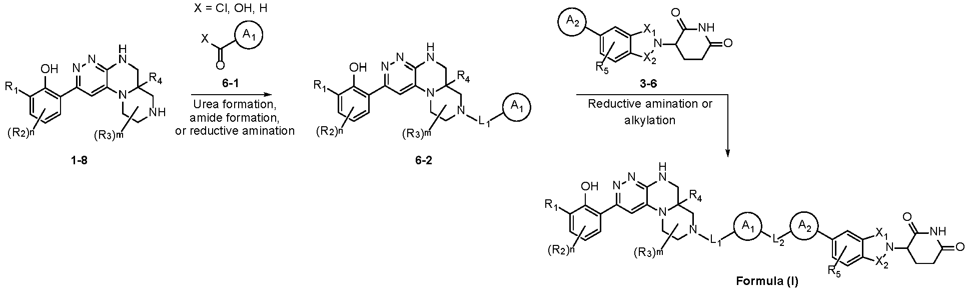

The compounds of the **Formula (I)** can be made according to the route described in **Scheme** 6. The coupling between **1-8** and **6-1** either through urea formation (e.g., DIPEA), amide formation (e.g., HATU) or reductive amination (e.g., sodium triacetoxyborohydride) can afford compounds **6-2.** Subsequent coupling of **6-2** with **3-6** (X₁, X₂ = CH₂ or CO) either through reductive amination (e.g., sodium triacetoxyborohydride) or alkylation (e.g., DIPEA) yields compounds of the **Formula (I).**

### Intermediate 1: (R)-2-fluoro-6-(6,6a,7,8,9,10-hexahydro-5H-pyrazino[1',2':4,5]pyrazino [2,3-c]pyridazin-2-yl)phenol

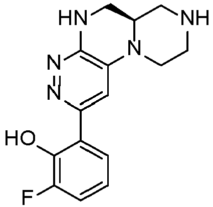

### Step 1: Tert-butyl (R)-4-(3,6-dichloropyridazin-4-yl)-3-(hydroxymethyl)piperazine-1-carboxylate

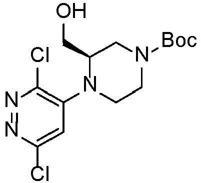

To a solution of 3,4,6-trichloropyridazine (5.70 g, 31.1 mmol) in DMF (24.0 mL) was added *N,N*-diisopropylethylamine (5.95 mL, 34.2 mmol) and *tert-butyl* (*R*)-3-(hydroxymethyl) piperazine-1-carboxylate (7.10 g, 32.8 mmol). The reaction was stirred at 80 °C overnight. The reaction was cooled to 45°C and water (17.0 mL) was slowly added. The resulting clear solution was stirred at 35°C for 30 min until a precipitate formed. Another portion of water (23.0 mL) was charged slowly, and the mixture was stirred at 0°C for an additional 1 h. The mixture was filtered, and the resulting solid was washed with water, collected and dried under vacuum to give tert-butyl (*R*)-4-(3,6-dichloropyridazin-4-yl)-3-(hydroxymethyl)piperazine-1-carboxylate (8.50 g, 75% yield) as an off-white solid. LCMS calcd for C₁₄H₂₁Cl₂N₄O₃ [M + H]⁺: m/z = 363.1; Found: 363.1.

### Step 2: Tert-butyl (R)-3-(azidomethyl)-4-(3,6-dichloropyridazin-4-yl)piperazine-1-carboxylate

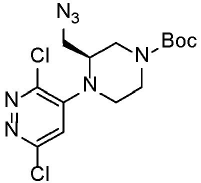

To a solution of *tert-butyl* (*R*)-4-(3,6-dichloropyridazin-4-yl)-3-(hydroxymethyl) piperazine-1-carboxylate (5.45 g, 15.0 mmol) and triphenylphosphine (4.72 g, 18.0 mmol) in THF (150 mL) was added diisopropyl azodicarboxylate (3.54 mL, 18.0 mmol) and diphenylphosphoryl azide (3.90 mL, 18.0 mmol) at 0°C. The reaction was then stirred at room temperature overnight. The reaction mixture was cooled to 0°C, quenched with water and extracted with EtOAc. The combined organic layers were washed with a saturated brine solution and water, dried over Na₂SO₄ and filtered. The filtrate was concentrated under reduced pressure to give crude *tert*-butyl (*R*)-3-(azidomethyl)-4-(3,6-dichloropyridazin-4-yl)piperazine-1-carboxylate (19.4 g), which was used without further purification (30% purity by LCMS). LCMS calcd for C₁₄H₂₀Cl₂N₇O₂ [M+H]⁺: m/z = 388.1; Found: 388.0.

### Step 3: Tert-butyl (S)-2-chloro-5,6,6a,7,9,10-hexahydro-8H-pyrazino[1'2':4,5] pyrazino[2, 3-c]pyridazine-8-carboxylate

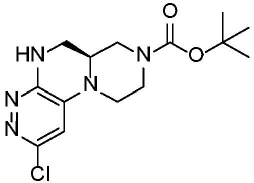

Triphenylphosphine (4.94 g, 18.8 mmol) was added to a stirred solution of crude *tert-*butyl (*R*)-3-(azidomethyl)-4-(3,6-dichloropyridazin-4-yl) piperazine-1-carboxylate (20.3 g, 15.7 mmol, 30% purity) in THF (200 mL). The resulting solution was stirred at 60 °C for 3 hours. Water (20.0 mL) and *N,N*-diisopropylethylamine (8.20 mL, 47.1 mmol) were added sequentially. After 20 hours, the reaction mixture was diluted with EtOAc (100 mL) and water (100 mL). The aqueous layer was separated and extracted with EtOAc. The combined organic layers were washed with a saturated brine solution, dried over Na₂SO₄, filtered and concentrated under reduced pressure. The residue was purified by silica gel flash column chromatography, eluting with a gradient of 0-100% EtOAc/hexanes to give *tert*-butyl (*S*)-2-chloro-5,6,6a,7,9,10-hexahydro-8*H*-pyrazino[1',2':4,5] pyrazino[2,3-c]pyridazine-8-carboxylate (3.10 g, 60% yield) as an off-white solid. LCMS calcd for C₁₄H₂₁ClN₅O₂ [M+H]⁺: m/z = 326.1; Found: 326.2.

### Step 4: Di-tert-butyl (R)-2-chloro-6a, 7,9,10-tetrahydro-5H-pyrazino[1',2':4,5] pyrazino[2, 3-c]pyridazine-5,8(6H)-dicarboxylate

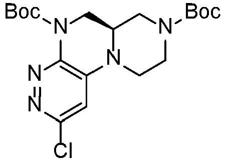

To a stirred solution of tert-butyl (*S*)-2-chloro-5,6,6a,7,9,10-hexahydro-8H-pyrazino [1',2':4,5]pyrazino[2,3-c]pyridazine-8-carboxylate (3.10 g, 9.52 mmol) in DCM (120 mL), *di-tert* butyl dicarbonate (6.23 g, 28.6 mmol) and 4-(dimethylamino)pyridine (1.16 g, 9.52 mmol) were added at room temperature. After 1 hour, the reaction was diluted with DCM (120 mL) and sat. aq. NH₄Cl (50.0 mL). After another 1 hour, the aqueous layer was separated and extracted with DCM. The organic layers were combined, washed with a saturated brine solution, dried over Na₂SO₄ and filtered. The filtrate was concentrated under reduced pressure. The residue was purified by silica gel flash column chromatography, eluting with 50% EtOAc/hexanes to give di-tert-butyl (*R*)-2-chloro-6a,7,9,10-tetrahydro-5*H*-pyrazino[1',2':4,5] pyrazino[2,3-c]pyridazine-5,8(6H)-dicarboxylate (3.90 g, 96% yield). LCMS calcd for C₁₉H₂₉ClN₅O₄ [M+H]⁺: m/z = 426.2; Found: 426.3.

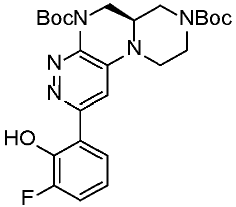

### Step 5: Di-tert-butyl (R)-2-(3-fluoro-2-hydroxyphenyl)-6a,7,9,10-tetrahydro-5H-pyrazino[1',2':4,5]pyrazino[2,3-c]pyridazine-5,8(6H)-dicarboxylate

A mixture of di-*tert*-butyl (R)-2-chloro-6a,7,9,10-tetrahydro-5*H*-pyrazino[1',2':4,5] pyrazino[2,3-*c*]pyridazine-5,8(6*H*)-dicarboxylate (1.20 g, 2.82 mmol), (3-fluoro-2-hydroxyphenyl)boronic acid (659 mg, 4.23 mmol), cesium carbonate (2.02 g, 6.20 mmol) and XPhos Pd G2 (333 mg, 0.420 mmol) was degassed. After addition of 1,4-dioxane (10 mL) and water (1.30 mL), the reaction was stirred at 80 °C for 6 h. The reaction was filtered and condensed. The mixture was purified by silica gel flash column chromatography, eluting with a gradient of 0-45% EtOAc/hexanes to give di-tert-butyl (R)-2-(3-fluoro-2-hydroxyphenyl)-6a,7,9,10-tetrahydro-5*H*-pyrazino[1',2':4,5]pyrazino[2,3-*c*]pyridazine-5,8(6*H*)-dicarboxylate (1.10 g, 78% yield). LCMS calcd for C₂₅H₃₃FN₅O₅ [M+H]+: m/z = 502.3; Found: 502.1.

### Step 6: (R)-2-fluoro-6-(6, 6a,7, 8, 9,10-hexahydro-5H-pyrazino[1',2':4, 5]pyrazino[2,3-c]pyridazin-2-yl)phenol

A 4 N solution of HCl in 1,4-dioxane (4.00 mL) was added dropwise to a solution of di-tert-butyl (R)-2-(3-fluoro-2-hydroxyphenyl)-6a,7,9,10-tetrahydro-5H-pyrazino[1',2':4,5]pyrazino [2,3-c]pyridazine-5,8(6H)-dicarboxylate (1.10 g, 2.12 mmol) in DCM (10.0 mL). The reaction was stirred for 5 hours at room temperature. The reaction was concentrated under reduced pressure to yield (*R*)-2-fluoro-6-(6,6a,7,8,9,10-hexahydro-5*H*-pyrazino[1',2':4,5]pyrazino[2,3-c]pyridazin-2-yl)phenol;dihydrochloride (602 mg, 73% yield) as an off-white solid. LCMS calcd for C₁₅H₁₇FN₅O [M+H]+: m/z = 302.1; Found: 302.0.

### Intermediate 2: (R)-2,4-difluoro-6-(6,6a,7,8,9,10-hexahydro-5H-pyrazino[1',2':4,5]pyrazino [2,3-c]pyridazin-2-yl)phenol

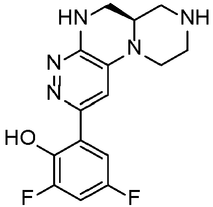

The title compound was prepared using the procedure analogous to that described for Intermediate 1, with (3,5-difluoro-2-hydroxyphenyl)boronic acid replacing (3-fluoro-2-hydroxyphenyl)boronic acid in step 5. LCMS calcd for C₁₅H₁₆F₂N₅O [M+H]+: m/z = 320.1; Found: 320.0

### Intermediate 3: 2,4-difluoro-6-((6aR,9S)-9-methyl-6,6a,7,8,9,10-hexahydro-5H-pyrazino [1',2':4,5]pyrazino[2,3-c]pyridazin-2-yl)phenol

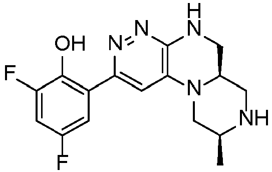

### Step 1: Methyl O-benzyl-N-(tert-butoxycarbonyl)-L-serylL-alaninate

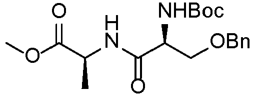

To a stirred suspension of *O*-benzyl-*N*-(*tert*-butoxycarbonyl)-L-serine (20.0 g, 67.7 mmol) and 1-hydroxybenzotriazole hydrate (11.0 g, 81.3 mmol) in DCM (451 mL) was added N,N-diisopropylethylamine (14.2 mL, 81.3 mmol) at 0 °C. 1-Ethyl-3-[3-dimethylaminopropyl] carbodiimide hydrochloride (15.6 g, 81.3 mmol) was added to the reaction mixture and stirred at 0 °C for 15 minutes. A solution of alanine methyl ester hydrochloride (11.3 g, 81.3 mmol) and N,N-diisopropylethylamine (14.2 mL, 81.3 mmol) in DMF (30 mL) was added dropwise at 0 °C over 5 minutes to the reaction mixture. The reaction was warmed to room temperature and stirred for 3 hours. Water (500 mL) was added, and the aqueous phase was extracted with DCM (300 mL x 3). The organic phases were dried over Na₂SO₄, filtered and concentrated under reduced pressure. The residue was purified by silica gel flash column chromatography, eluting with a gradient of 0-100% EtOAC/hexanes to give methyl O-benzyl-N-(tert-butoxycarbonyl)-L-seryl-L-alaninate (26.1 g, 99.0% yield). LCMS calculated for C₁₉H₂₉N₂O₆ (M+H)⁺: m/z = 381.2; Found: 381.0.

### Step 2: Methyl O-benzyl-L-seryl-L-alaninate

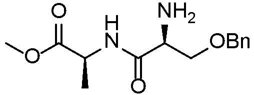

To a solution of methyl *O*-benzyl-*N*-(*tert*-butoxycarbonyl)-*L*-seryl-*L*-alaninate (26.1 g, 68.6 mmol) in DCM (260 mL) was added TFA (51.4 mL, 672 mmol) at room temperature. The reaction was stirred for 3 hours. The reaction mixture was basified to between pH 7 and pH 8 via a saturated aqueous NaHCO₃ solution. The aqueous phase was extracted with DCM (100 mL x 3) and the organics were washed with a saturated brine solution (100 mL x 1). The combined organic phases were dried over Na₂SO₄, filtered, and concentrated under reduced pressure. The residue was used directly in the next step without additional purification to give crude *methyl O-benzyl-L-seryl-L-alaninate* (16.9 g). LCMS calculated for C₁₄H₂₁N₂O₄ (M+H)⁺: m/z = 281.2; Found: 281.0.

### Step 3: (3S,6S)-3-((benzyloxy)methyl)-6-methylpiperazine-2,5-dione

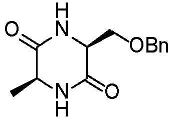

A solution of crude methyl *O*-benzyl-*L*-seryl-*L*-alaninate (16.9 g, 60.3 mmol) in 1,4-dioxane (169 mL) was stirred at 100°C overnight. The reaction was cooled to room temperature, upon which a white solid precipitated out. The white precipitate was filtered, collected, and washed with cold MTBE (100 mL) to give (3*S*,6*S*)-3-((benzyloxy)methyl)-6-methylpiperazine-2,5-dione (11.0 g, yield: 73.0% yield). LCMS calculated for C₁₃H₁₇N₂O₃ (M+H)⁺: m/z = 249.1; Found: 249.2.

### Step 4: (2R,5S)-2-((benzyloxy)methyl)-5-methylpiperazine

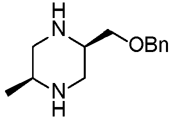

To a solution of (3*S*,6*S*)-3-((benzyloxy)methyl)-6-methylpiperazine-2,5-dione (9.00 g, 36.3 mmol) in THF (201 mL) was added borane dimethyl sulfide complex (27.5 mL, 290 mmol) while on an ice-water bath. The reaction was warmed to room temperature and stirred at 60 °C overnight. The reaction was cooled on an ice-water bath and MeOH was slowly added (200 mL). The reaction mixture was warmed to room temperature and a 1 N HCl aqueous solution was added dropwise to reach a pH ~ 3. The mixture was stirred at 50 °C for 3 hours. The reaction mixture was basified to pH 12 by adding a 1 N NaOH aqueous solution dropwise and the aqueous phase was extracted with CHCl₃ (200 mL x 3). The combined organic phases were dried over Na₂SO₄, filtered, and concentrated under reduced pressure. The residue was directly used in the next step without additional purification to give crude (2R,5S)-2-((benzyloxy)methyl)-5-methylpiperazine (9.80 g). LCMS calculated for C₁₃H₂₁N₂O (M+H)⁺: m/z = 221.2; Found: 221.2.

### Step 5: ((2R,5S)-5-methylpiperazin-2-yl)methanol

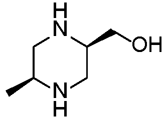

To a solution of crude (2*R*,5*S*)-2-((benzyloxy)methyl)-5-methylpiperazine (9.80, 44.5 mmol) in DCM (445 mL) was added a 1 M solution of BCl₃ (178 mL, 178 mmol) in DCM at -78 °C. The reaction was slowly warmed to room temperature and stirred overnight. The reaction was cooled over an ice-water bath and MeOH (200 mL) was slowly added. The reaction mixture was concentrated to dryness under reduced pressure. The residue was directly used in the next step without additional purification to give crude ((2R,5S)-5-methylpiperazin-2-yl)methanol (9.0 g). LCMS calculated for C₆H₁₅N₂O (M+H)⁺: m/z = 131.1; Found: 131.0.

### Step 6: Di-tert-butyl (2R, 5S)-2-(hydroxymethyl)-5-methylpiperazine-1,4-dicarboxylate

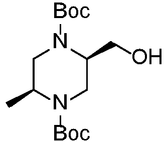

To a solution of crude ((2R,5S)-5-methylpiperazin-2-yl)methanol (9.00 g, 69.1 mmol) in DCM (376 mL) was added triethylamine (120 mL, 864 mmol) and di-tert-butyl dicarbonate (45.3 g, 207 mmol) at 0 °C. The reaction was stirred at room temperature overnight and then concentrated to dryness under reduced pressure. The residue was directly used in the next step without additional purification to give crude di-tert-butyl (2*R*,5*S*)-2-(hydroxymethyl)-5-methylpiperazine-1,4-dicarboxylate (24.0 g). LCMS calculated for C₁₆H₃₁N₂O₅ (M+H)⁺: m/z = 331.2; Found: 331.0.

### Step 7: Tert-butyl (2S,5R)-5-(hydroxymethyl)-2-methylpiperazine-1-carboxylate

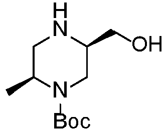

To a solution of crude di-tert-butyl (2R,5S)-2-(hydroxymethyl)-5-methylpiperazine-1,4-dicarboxylate (14.0 g, 42.4 mmol) in EtOH (78.5 mL) was added a solution of NaOH (8.50 g, 212 mmol) in water (78.5 mL). The reaction mixture was stirred at 80 °C overnight. The reaction was cooled to room temperature and pH ~ 9 was reached by adding a 1 N HCl aqueous solution dropwise. The aqueous phase was extracted with CHCl₃ (100 mL x 3) and the combined organic phases were dried over Na₂SO₄, filtered and concentrated under reduced pressure. The crude residue was purified by flash silica gel column chromatography eluting with a gradient of 0-10% DCM/MeOH (containing 0.1% triethylamine) to give tert-butyl (2S,5R)-5-(hydroxymethyl)-2-methylpiperazine-1-carboxylate (2.70 g, 28% yield). LCMS calculated for C₁₁H₂₃N₂O₃ (M+H)⁺: m/z = 231.2; Found: 231.1.

### Step 8: Tert-butyl (2S,5R)-4-(3,6-dichloropyridazin-4-yl)-5-(hydroxymethyl)-2-methylpiperazine-1-carboxylate

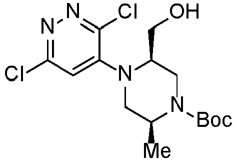

To a solution of 3,4,6-trichloropyridazine (406 mg, 2.21 mmol) in DMF (1.75 mL) was added *N*-*N*-diisopropylethylamine (0.59 mL, 3.39 mmol) and *tert-*butyl (2S,5R)-5-(hydroxymethyl)-2-methylpiperazine-1-carboxylate (300 mg, 1.30 mmol) in DMF (1.75 mL) at room temperature. The reaction was stirred at 80°C overnight. The reaction was cooled to room temperature, diluted with water (15.0 mL) and extracted with EtOAc (15 mL × 3). The combined organic phases were dried over Na₂SO₄, filtered and concentrated under reduced pressure. The crude material was purified by silica gel flash column chromatography eluting with a gradient of 10-100% EtOAc/heptanes to give tert-butyl (2*S*,5*R*)-4-(3,6-dichloropyridazin-4-yl)-5-(hydroxymethyl)-2-methylpiperazine-1-carboxylate (408 mg, 83% yield), as a yellow viscous oil. LCMS calc. for C₁₅H₂₃Cl₂N₄O₃ [M+H]⁺: m/z = 377.1; Found: 377.0.

### Step 9: Tert-butyl (2S, 5R)-5-(azidomethyl)-4-(3,6-dichloropyridazin-4-yl)-2-methylpiperazine-1-carboxylate

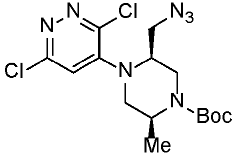

To a solution of *tert-butyl* (2*S*,5*R*)-4-(3,6-dichloropyridazin-4-yl)-5-(hydroxymethyl)-2-methylpiperazine-1-carboxylate (2.70 g, 7.16 mmol) and triphenylphosphine (2.63 g, 10 mmol) in THF (35 mL) was slowly added diisopropyl azodicarboxylate (1.97 mL, 10 mmol) at 0 °C. The reaction mixture was stirred at 0 °C for 10 minutes, followed by the slow addition of diphenylphosphoryl azide (2.76 g, 10 mmol). The reaction mixture was stirred at room temperature overnight. The reaction was concentrated under reduced pressure and a saturated aqueous solution of NaHCO₃ (15.0 mL) was added. The mixture was extracted with EtOAc (15 mL × 3) and the combined organics were dried over Na₂SO₄, filtered and concentrated under reduced pressure. The crude product was purified by silica gel flash column chromatography eluting with a gradient of 10-50% EtOAc/heptanes to give tert-butyl (2S,5R)-5-(azidomethyl)-4-(3,6-dichloropyridazin-4-yl)-2-methylpiperazine-1-carboxylate (2.00 g, 70% yield), as a yellow oil. LCMS calc. for C₁₅H₂₂Cl₂N₇O₂ [M+H]⁺: m/z = 402.1; Found: 401.8.

### Step 10: Tert-butyl (6aS,9S)-2-chloro-9-methyl-5,6,6a,7,9,10-hexahydro-8H-pyrazino[1',2':4, 5] pyrazino[2, 3-c]pyridazine-8-carboxylate

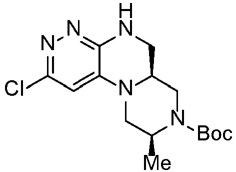

A solution of triphenylphosphine (5.50 g, 20.8 mmol) and tert-butyl (2S,5R)-5-(azidomethyl)-4-(3,6-dichloropyridazin-4-yl)-2-methylpiperazine-1-carboxylate (7.00 g, 17.3 mmol) in THF (45.0 mL) was heated at 60 °C for 3 hours. Water (4.50 mL) and *N*-*N*-diisopropylethylamine (9.00 mL, 51.9 mmol) were added and the reaction mixture was stirred at 60 °C overnight. The reaction was cooled to room temperature and concentrated under reduced pressure. The residual oil was diluted with water (40.0 mL) and extracted with EtOAc (50 mL × 3). The combined organics were dried over Na₂SO₄, filtered and concentrated under reduced pressure. The crude residue was purified by silica gel flash column chromatography, eluting with a gradient of 10-100% EtOAc/hexanes to give tert-butyl (6a*S*,9*S*)-2-chloro-9-methyl-5,6,6a,7,9, 10-hexahydro-8*H*-pyrazino[1',2':4,5]pyrazino[2,3-c]pyridazine-8-carboxylate (1.50 g, 26% yield), as a yellow solid. LCMS calc. for C₁₅H₂₃ClN₅O₂ [M+H]⁺: m/z = 340.2; Found: 340.0.

### Step 11: Di-tert-butyl (6aR,9S)-2-chloro-9-methyl-6a,7,9,10-tetrahydro-5H-pyrazino [1',2':4,5]pyrazino[2,3-c]pyridazine-5,8(6H)-dicarboxylate

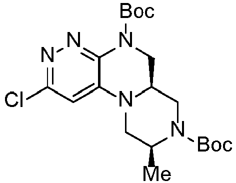

The solution of tert-butyl (6a*S*,9*S*)-2-chloro-9-methyl-5,6,6a,7,9,10-hexahydro-8*H-*pyrazino[1',2':4,5]pyrazino[2,3-c]pyridazine-8-carboxylate (1.50 g, 4.41 mmol) in DCM (24.0 mL) was added triethylamine (1.23 mL, 8.83mmol) and di-*tert*-butyl dicarbonate (1.35 g, 6.18 mmol) at 0 °C. The reaction was stirred at room temperature overnight. The starting material was not completely consumed and 4-dimethylaminopyridine (54.0 mg, 0.440 mmol) and additional di-tert-butyl dicarbonate (675 mg, 3.09 mmol) were added. The reaction was further stirred at room temperature for 3 hours. A saturated aqueous solution of NH₄Cl (30.0 mL) was added and the aqueous phase was extracted with DCM (30 mL x 3). The combined organics were dried over Na₂SO₄, filtered and concentrated under reduced pressure. The crude material was purified by silica gel flash column chromatography, eluting with a gradient of 10-50% EtOAc/hexanes to give di-*tert*-butyl (6a*R*,9*S*)-2-chloro-9-methyl-6a,7,9,10-tetrahydro-5H-pyrazino[1',2':4,5] pyrazino[2,3-*c*]pyridazine-5,8(6H)-dicarboxylate (1.10 g, 57% yield), as white solid. LCMS calc. for C₂₀H₃₁ClN₅O₄ [M+H]⁺: m/z = 440.2; Found: 440.1.

### Step 12: Di-tert-butyl (6aR,9S)-2-(3,5-difluoro-2-hydroxyphenyl)-9-methyl-6a,7,9,10-tetrahydro-5H-pyrazino[1',2':4,5]pyrazino[2,3-c]pyridazine-5,8(6H)-dicarboxylate

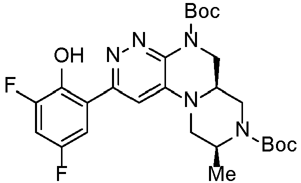

A mixture of di-tert-butyl (6a*R*,9*S*)-2-chloro-9-methyl-6a,7,9,10-tetrahydro-5*H-*pyrazino[1',2':4,5] pyrazino[2,3-*c*]pyridazine-5,8(6*H*)-dicarboxylate (420 mg, 0.950 mmol), (3,5-difluoro-2-hydroxyphenyl)-boronic acid (199 mg, 1.15 mmol), cesium carbonate (622 mg, 1.91 mmol) and XPhos Pd G2 (150 mg, 0.190 mmol) was degassed. After addition of 1,4-Dioxane (4.00 mL) and water (0.500 mL), the reaction was stirred at 80 °C overnight. The mixture was diluted with DCM and washed with water. The combined organics were dried over Na₂SO₄, filtered and concentrated under reduced pressure. The crude residue was purified by silica gel flash column chromatography eluting with a gradient of 0-100% EtOAc/hexanes followed by 15% MeOH/DCM to give di-tert-butyl (6a*R*,9*S*)-2-(3,5-difluoro-2-hydroxyphenyl)-9-methyl-6a,7,9,10-tetrahydro-5*H*-pyrazino[1',2':4,5]pyrazino[2,3-*c*]pyridazine-5,8(6*H*)-dicarboxylate (380 mg, 75% yield). LCMS calc. for C₂₆H₃₄F₂N₅O₅ [M+H]⁺: m/z = 534.3; Found: 534.2.

### Step 13: 2, 4-difluoro-6-((6aR,9S)-9-methyl-6, 6a,7, 8, 9,10-hexahydro-5H-pyrazino[1',2':4,5]pyrazino[2,3-c]pyridazin-2-yl)phenol

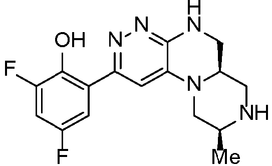

A solution of di-*tert*-butyl (6a*R*,9*S*)-2-(3,5-difluoro-2-hydroxyphenyl)-9-methyl-6a,7,9,10-tetrahydro-5*H*-pyrazino[1',2':4,5]pyrazino[2,3-*c*]pyridazine-5,8(6*H*)-dicarboxylate (200 mg, 0.370 mmol) in DCM (1.00 mL) was treated with a 4 N HCl solution in 1,4-dioxane (2.00 mL, 64.7 mmol) at room temperature for 1 hour. The mixture was concentrated under reduced pressure to give 2,4-difluoro-6-((6a*R*,9*S*)-9-methyl-6,6a,7,8,9,10-hexahydro-5*H*-pyrazino [1',2':4,5]pyrazino[2,3-c]pyridazin-2-yl)phenol (120 mg, 79% yield) as the HCl salt. The material was used without additional purification. LCMS calc. for C₁₆H₁₈F₂N₅O [M+H]⁺: m/z = 334.2; Found: 334.1.

### Intermediate 4: ((2S,6R)-2,6-dimethylpiperazin-1-yl)(2-(3-fluoro-2-hydroxyphenyl)-6a-methyl-5,6,6a,7,9,10-hexahydro-8H-pyrazino[1',2':4,5]pyrazino[2,3-c]pyridazin-8-yl)methanone (Isomer 1)

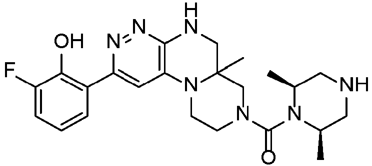

### Step 1: (S)-1,4-bis(tert-butoxycarbonyl)piperazine-2-carboxylic acid

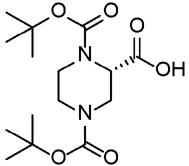

To a solution of (*S*)-piperazine-2-carboxylic acid;dihydrochloride (2.50 g, 12.3 mmol) in a mixture of water (4.92 mL) and 1,4-dioxane (19.7 mL) was added sodium hydroxide (3.64 mL, 47.3 mmol) in water (3.00 mL), followed by *di-tert* butyl dicarbonate (5.64 g, 25.9 mmol) at 0 °C. The reaction was stirred at room temperature overnight. Imidazole (838 mg, 12.3 mmol) was added and the reaction was stirred at room temperature for 20 minutes. The reaction mixture was diluted with DCM and the organics were washed with a 1 N aqueous solution of HCl (4 x 25 mL) and a saturated brine solution (1 x 25 mL). The combined organics were dried over MgSO₄, filtered and concentrated under reduced pressure to give crude (S)-1,4-bis(tert-butoxycarbonyl) piperazine-2-carboxylic acid (4.07 g) as a white solid. The material was used in the following step without additional purification.

### Step 2: 1,4-di-tert-butyl 2-methyl (S)-piperazine-1,2,4-tricarboxylate

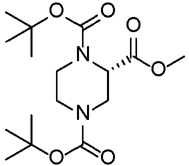

Iodomethane (0.283 mL, 4.55 mmol) was added to a solution of *(S)-1,4-bis(tert-*butoxycarbonyl)-piperazine-2-carboxylic acid (1.00 g, 3.03 mmol) and potassium carbonate (545 mg, 3.94 mmol) in DMF (10.1 mL) at 0 °C. The reaction was warmed to room temperature and stirred for 2 hours. The reaction mixture was quenched with a saturated aqueous solution of NH₄Cl and diluted with water. The aqueous phase was extracted with EtOAc (3 x 25 mL) and the combined organics were washed with a saturated brine solution (1 x 25 mL), dried over MgSO₄, filtered and concentrated under reduced pressure to give crude 1,4-di-tert-butyl 2-methyl (S)-piperazine-1,2,4-tricarboxylate (2.14 g). The material was used in the following step without additional purification. LCMS calcd for C₇H₁₃N₂O₄ [M+H-C₉H₁₆O₂]⁺: m/z = 189.1; Found: 189.0.

### Step 3: 1,4-di-tert-butyl 2-methyl 2-methylpiperazine-1,2,4-tricarboxylate (racemic mixture)

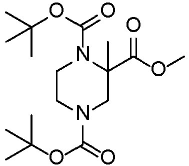

LiHMDS (7.14 mL, 7.14 mmol) was added to a solution of 1,4-di-tert-butyl 2-methyl (S)-piperazine-1,2,4-tricarboxylate (2.14 g) in THF at -78 °C. The reaction was stirred for 2 hours at -78 °C. Iodomethane (741 µL, 11.9 mmol) was added and the reaction was warmed to room temperature and stirred overnight. The reaction mixture was quenched with a saturated aqueous solution of NH₄Cl (25 mL) and extracted with EtOAc (2 x 25 mL). The combined organics were washed with a saturated brine solution (1 x 25 mL), dried over MgSO₄, filtered and concentrated under reduced pressure. The crude material was purified by silica gel flash column chromatography, eluting with a gradient of 0-100% EtOAc/hexanes to give 1,4-di-tert-butyl 2-methyl 2-methylpiperazine-1,2,4-tricarboxylate (2.10 g, 98% yield) as a clear oil. LCMS calcd for C₈H₁₅N₂O₄ [M+H-C₉H₁₆O₂]⁺: m/z = 203.1; Found: 203.1.

### Step 4: tert-butyl 2-chloro-6a-methyl-6-oxo-5,6,6a,7,9,10-hexahydro-8H-pyrazino [1',2': 4, 5]pyrazino[2, 3-c]pyridazine-8-carboxylate (racemic mixture)

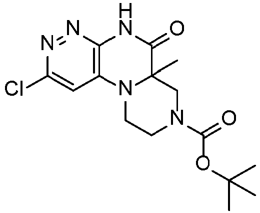

To a solution of 1,4-di-tert-butyl 2-methyl 2-methylpiperazine-1,2,4-tricarboxylate (2.50 g, 7.26 mmol) in DCM (43.6 mL) was carefully added DMF (436 µL) and oxalyl chloride (1.87 mL, 21.8 mmol). The mixture was stirred at room temperature for 30 minutes. The volatiles were removed under reduced pressure and DMF (13.1 mL), N,N-Diisopropylethylamine (6.32 mL, 36.3 mmol) and 4-bromo-6-chloropyridazin-3-amine (3.03 g, 14.5 mmol) were added sequentially. The resulting mixture was stirred at 120 °C overnight. The reaction mixture was diluted with EtOAc (100 mL) and washed with a saturated brine solution (30 mL x 2). The combined organics were dried over MgSO₄, filtered and concentrated under reduced pressure. The crude material was purified by silica gel flash column chromatography, eluting with a gradient of 0-100% EtOAc/hexanes to give tert-butyl 2-chloro-6a-methyl-6-oxo-5,6,6a,7,9,10-hexahydro-8H-pyrazino[1',2':4,5]pyrazino[2,3-c]pyridazine-8-carboxylate (929 mg, 36% yield) as a tan solid. LCMS calcd for C₁₅H₂₁ClN₃O₃ [M+H]⁺: m/z = 354.1; Found: 354.1.

### Step 5: Chiral separation of tert-butyl 2-chloro-6a-methyl-6-oxo-5,6, 6a,7,9,10-hexahydro-8H-pyrazino[1',2':4,5]pyrazino[2,3-c]pyridazine-8-carboxylate (Isomer 1 and 2)

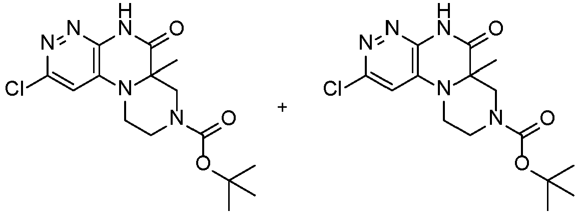

The racemic mixture of tert-butyl 2-chloro-6a-methyl-6-oxo-5,6,6a,7,9,10-hexahydro-8H-pyrazino-[1',2':4,5]pyrazino[2,3-c]pyridazine-8-carboxylate (120 mg, 0.339 mmol) was subjected to chiral separation (Lux Cellulose-2 column, 20 mL/min of 10:90 Hexanes/IPA:MeOH (1:1)) to give *tert-butyl 2-chloro-6a-methyl-6-oxo-5,6, 6a,7,9,10-hexahydro-8H-pyrazino[1',2':4,5] pyrazino[2,3-c]pyridazine-8-carboxylate* (55 mg, 46% yield, peak A, Isomer 1) and *tert-butyl 2-chloro-6a-methyl-6-oxo-5,6,6a,7,9,10-hexahydro-8H-pyrazino[1',2':4,5]pyrazino[2,3-c]pyridazine-8-carboxylate* (45 mg, 38% yield, peak B, Isomer 2). LCMS calcd for C₁₅H₂₁ClN₅O₃ [M+H]⁺: m/z = 354.1; Found: 354.1.

### Step 6: tert-butyl 2-(3-fluoro-2-hydroxyphenyl)-6a-methyl-6-oxo-5,6,6a,7,9,10-hexahydro-8H-pyrazino[1',2':4,5]pyrazino[2,3-c]pyridazine-8-carboxylate (Isomer 1)

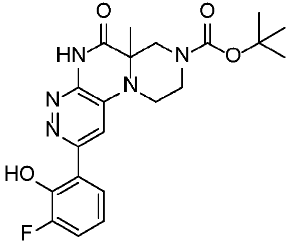

A solution of *tert-butyl* 2-chloro-6a-methyl-6-oxo-5,6,6a,7,9,10-hexahydro-8H-pyrazino[1',2':4,5]-pyrazino[2,3-c]pyridazine-8-carboxylate (peak A, Isomer 1) (55.0 mg, 0.155 mmol), [1,1'-Bis(diphenylphosphino)ferrocene]dichloropalladium(II), complex with dichloromethane (38.1 mg, 46.6 µL), 3-fluoro-2-hydroxyphenylboronic acid (72.7 mg, 0.466 mmol) and potassium carbonate (107 mg, 0.777 mmol) in a mixture of 1,4-dioxane (1.41 mL) and water (0.141 mL) was sparged with nitrogen for 5 minutes. The reaction was stirred at 90 °C for 2 hours. The reaction was cooled to room temperature and concentrated under reduced pressure. The crude material was purified by silica gel flash column chromatography, eluting with a gradient of 0-10% MeOH/DCM to give tert-butyl 2-(3-fluoro-2-hydroxyphenyl)-6a-methyl-6-oxo-5, 6,6a,7,9,10-hexahydro-8*H*-pyrazino[1',2':4,5]pyrazino[2,3-*c*]pyridazine-8-carboxylate (Isomer 1) (47.5 mg, 71% yield). LCMS calcd for C₂₁H₂₅FN₃O₄ [M+H]⁺: m/z = 430.2; Found: 430.1.

### Step 7: tert-butyl 2-(3-fluoro-2-hydroxyphenyl)-6a-methyl-5,6, 6a,7,9,10-hexahydro-8H-pyrazino[1',2':4, 5]pyrazino[2,3-c]pyridazine-8-carboxylate (Isomer 1)

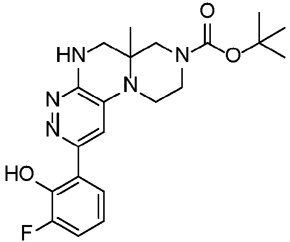

A 1 N solution of BH₃ in THF (559 µL, 559 µmol) was added to a solution of *tert-butyl* 2-(3-fluoro-2-hydroxyphenyl)-6a-methyl-6-oxo-5,6,6a,7,9,10-hexahydro-8*H*-pyrazino[1',2':4,5 pyrazino[2,3-c]pyridazine-8-carboxylate (Isomer 1) (50.0 mg, 112 µmol) in THF (1.12 mL) at room temperature. The reaction was heated to 60 °C and stirred for 24 hours. The reaction was cooled to room temperature and carefully diluted MeOH (10 mL) and then heated at 80 °C for 10 minutes. The reaction was cooled to room temperature, diluted with DCM and the organics were washed with a saturated aqueous solution of NaHCO₃. The combined organics were dried over MgSO₄, filtered and concentrated under reduced pressure. The crude material was purified by silica gel flash column chromatography, eluting with a gradient of 0-20% MeOH/DCM to give tert-butyl 2-(3-fluoro-2-hydroxyphenyl)-6a-methyl-5,6,6a,7,9,10-hexahydro-8H-pyrazino[1',2':4,5]pyrazino[2,3-c]pyridazine-8-carboxylate (Isomer 1) (12.0 mg, 26% yield). LCMS calcd for C₂₁H₂₇FN₅O₃ [M+H]⁺: m/z = 416.2; Found: 416.2.

### Step 8: 2-fluoro-6-(6a-methyl-6,6a,7, 8,9,10-hexahydro-5H-pyrazino[1',2':4, 5]pyrazino [2,3-c]pyridazin-2-yl)phenol (Isomer 1)

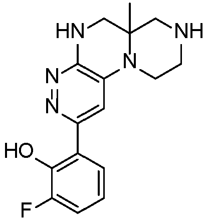

The title compound was prepared using the procedure analogous to those described for Intermediate 3, step 13, with *tert-butyl* 2-(3-fluoro-2-hydroxyphenyl)-6a-methyl-5,6,6a,7,9,10-hexahydro-8*H*-pyrazino-[1',2':4,5]pyrazino[2,3-*c*]pyridazine-8-carboxylate (isomer 1) replacing di-*tert*-butyl (6aR,9S)-2-(3,5-difluoro-2-hydroxyphenyl)-9-methyl-6a, 7,9,1 0-tetrahydro-5*H-*pyrazino[1',2':4,5]pyrazino[2,3-c]pyridazine-5,8(6H)-dicarboxylate. LCMS calcd for C₁₆H₁₉FN₅O [M+H]+: m/z = 316.2; Found: 316.1.

### Step 9: tert-butyl (3R,5S)-4-(chlorocarbonyl)-3,5-dimethylpiperazine-1-carboxylate

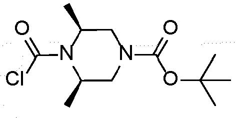

A solution of triphosgene (2.24 g, 7.54 mmol) in DCM (15.0 mL) was carefully added dropwise to a solution of *tert-butyl* (3*R*,5*S*)-3,5-dimethylpiperazine-1-carboxylate (2.15 g, 10.1 mmol) and pyridine (1.22 mL, 15.1 mmol) in DCM (25.0 mL) at 0 °C. The reaction was warmed to room temperature and stirred for 2 hours. Upon completion, the reaction mixture was poured into a 1 N aqueous HCl solution (150 mL), while on ice and stirred for 15 minutes. The organic phase was separated, and the aqueous phase was extracted with DCM (3 x 30 mL). The combined organics were washed with a saturated brine solution (50 mL), dried over MgSO₄, filtered and concentrated under reduced pressure. The crude residue was purified by silica gel flash column chromatography, eluting with a gradient of 0-10% ethyl acetate/hexanes to give *tert-butyl* (3*R*,5*S*)-4-(chlorocarbonyl)-3,5-dimethylpiperazine-1-carboxylate (1.66 g, 60% yield) as a white solid. ¹H NMR (300 MHz, CDCl₃) 4.40 (s, 2H), 4.15 - 3.77 (d, *J =* 18 Hz, 2H), 3.05 (s, 2H), 1.52 (s, 9H), 1.40 - 1.27 (d, *J=* 12 Hz, 6H).

### Step 10: ((2S,6R)-2,6-dimethylpiperazin-1-yl)((R)-2-(3-fluoro-2-hydroxyphenyl)-6a-methyl-5,6,6a,7,9,10-hexahydro-8H-pyrazino[1',2':4,5]pyrazino[2,3-c]pyridazin-8-yl)methanone (Isomer 1)

Triethylamine (19.4 µL, 139 µmol) and tert-butyl (3R,5S)-4-(chlorocarbonyl)-3,5-dimethylpiperazine-1-carboxylate (12.8 mg, 46.4 µmol) in DCM (1.00 mL) was added to a solution of 2-fluoro-6-(6a-methyl-6,6a,7,8,9,10-hexahydro-5H-pyrazino[1',2':4,5]pyrazino[2,3-*c*]pyridazin-2-yl)phenol;dihydrochloride (Isomer 1, 9.00 mg, 20.0 µmol) in DCM (1.15 mL). The reaction was stirred at room temperature overnight. The reaction mixture was quenched with MeOH and a saturated aqueous solution of NaHCO₃ was added. The aqueous phase was extracted with DCM and the combined organics were dried over MgSO4, filtered, and concentrated under reduced pressure. The crude material was dissolved in DCM (1.15 mL), 2,2,2-trifluoroacetic acid (53.2 µL, 0.695 mmol) was added and the reaction was stirred for 1 hour. The reaction mixture was concentrated under reduced pressure and the crude residue was purified by prep-HPLC (Waters CSH-C18, 5 uM, 30x100mm, 6.1-26.1% MeCN/water (containing 0.1%TFA) over 5 min) to give the TFA salt of the title compound (3.00 mg, 19% yield) as an oil. LCMS calcd for C₂₃H₃₁FN₇O₂ [M+H]+: m/z = 456.2; Found: 456.1.

### Intermediate 5: (2-(3-chloro-2-hydroxyphenyl)-6a-methyl-5,6,6a,7,9,10-hexahydro-8H-pyrazino[1',2':4,5]pyrazino[2,3-c]pyridazin-8-yl)((2S,6S)-2,6-dimethylpiperazin-1-yl)methanone (Isomer 1)

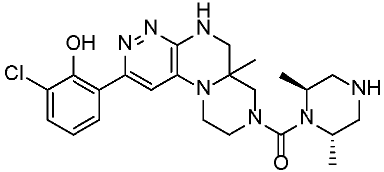

The title compound was prepared using the procedure analogous to those described for Intermediate 4, steps 1-10, with (2-chloro-6-hydroxyphenyl)boronic acid replacing 3-fluoro-2-hydroxyphenylboronic acid in Step 6 and *tert-butyl* (3R,5R)-4-(chlorocarbonyl)-3,5-dimethylpiperazine-1-carboxylate replacing tert-butyl (3*R*,5*S*)-3,5-dimethylpiperazine-1-carboxylate in Step 9. LCMS calcd for C₂₃H₃₁ClN₇O₂ [M+H]+: m/z = 472.2; Found: 472.0.

### Intermediates 6-19:

The intermediates shown below in Table 1 were prepared by methods analogous to that described for the preparing Intermediate 4 using the appropriate starting materials.

**Table 1. Intermediates 6-19**

| **Int.** | **Structure** | **Name** | **Calcd. (M+H)⁺ m/z** | **Found (M+H)⁺ m/z** |
|---|---|---|---|---|
| **Int-6** | 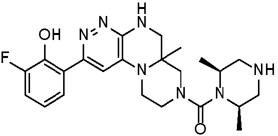 | ((2*S*,6*R*)-2,6-dimethyl-piperazin-1-yl)(2-(3-fluoro-2-hydroxyphenyl)-6a-methyl-5,6,6a, 7,9,10-hexahydro-8H-pyrazino [1',2':4,5]pyrazino[2,3-*c*]pyridazin-8-yl)methanone (Isomer 2) | 456.2 | 456.1 |
| **Int-7** | 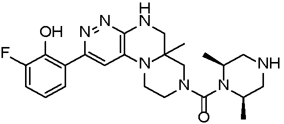 | ((2*S*,6*R)*-2,6-dimethylpiperazin-1-yl)(2-(3-fluoro-2-hydroxyphenyl)-6a-methyl-5,6,6a, 7,9,10-hexahydro-8*H*-pyrazino [1',2':4,5]pyrazino[2,3-*c*]pyridazin-8-yl)methanone (Mixture of Isomers) | 456.2 | 456.1 |
| **Int-8** | 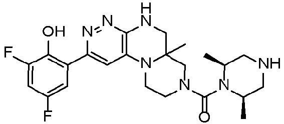 | (2-(3,5-difluoro-2-hydroxyphenyl)-6a-methyl-5,6,6a,7,9,10-hexahydro-8*H-*pyrazino[1',2':4,5]pyrazino[2 ,3-c]pyridazin-8-yl)((2*S*,6*R*)-2,6-dimethylpiperazin-1-yl)methanone (Mixture of Isomers) | 474.2 | 474.0 |
| **Int-9** | 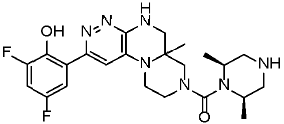 | (2-(3,5-difluoro-2-hydroxyphenyl)-6a-methyl-5,6,6a,7,9,10-hexahydro-8*H-*pyrazino[1',2':4,5]pyrazino[2 ,3-c]pyridazin-8-yl)((2*S*,6*R*)-2,6-dimethylpiperazin-1-yl)methanone (Isomer 1) | 474.2 | 474.0 |
| Int-10 | 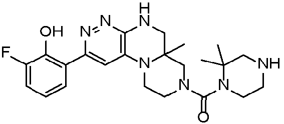 | (2,2-dimethylpiperazin-1-yl)(2-(3-fluoro-2-hydroxyphenyl)-6a-methyl-5,6,6a,7,9,10-hexahydro-8*H-*pyrazino[1',2':4,5]pyrazino [2,3-c]pyridazin-8-yl) methanone (Mixture of Isomers) | 456.2 | 456.2 |
| **Int-11** | 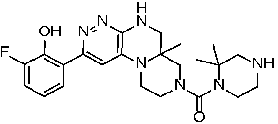 | (2,2-dimethylpiperazin-1-yl)(2-(3-fluoro-2-hydroxyphenyl)-6a-methyl-5,6,6a,7,9,10-hexahydro-8*H-*pyrazino[1',2':4,5]pyrazino [2,3-*c*]pyridazin-8-yl) methanone (Isomer 1) | 456.2 | 456.2 |
| **Int-12** | 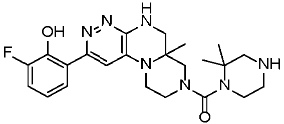 | (2,2-dimethylpiperazin-1-yl)(2-(3-fluoro-2-hydroxyphenyl)-6a-methyl-5,6,6a,7,9,10-hexahydro-8*H-*pyrazino[1',2':4,5]pyrazino [2,3-c]pyridazin-8-yl) methanone (Isomer 2) | 456.2 | 456.2 |
| **Int-13** | 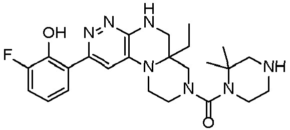 | (2,2-dimethylpiperazin-1-yl)(6a-ethyl-2-(3-fluoro-2-hydroxyphenyl)-5,6,6a,7,9,10-hexahydro-8*H-*pyrazino[1',2':4,5]pyrazino [2,3-*c*]pyridazin-8-yl) methanone (Mixture of Isomers) | 470.3 | 470.1 |
| **Int-14** | 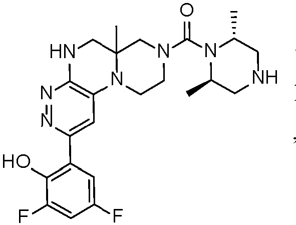 | (2-(3,5-difluoro-2-hydroxyphenyl)-6a-methyl-5,6,6a,7,9,10-hexahydro-8*H-*pyrazino[1',2':4,5]pyrazino[2 3-*c*]pyridazin-8-yl)((2*R*,6*R*)-2,6-dimethylpiperazin-1-yl) methanone (Mixture of Isomers) | 474.2 | 474.0 |
| **Int-15** | 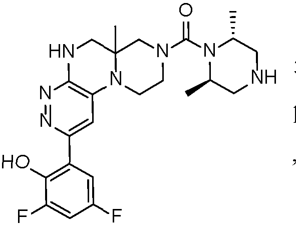 | (2-(3,5-difluoro-2-hydroxyphenyl)-6a-methyl-5,6,6a,7,9,10-hexahydro-8*H-*pyrazino[1',2':4,5]pyrazino[2 3-c]pyridazin-8-yl)((2*R*,6*R)-*2,6-dimethylpiperazin-1-yl)methanone (Isomer 1) | 474.2 | 474.0 |
| **Int -16** | 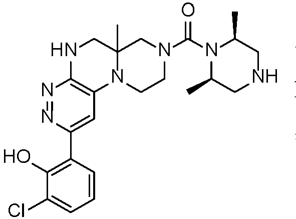 | (2-(3-chloro-2-hydroxyphenyl)-6a-methyl-5,6,6a,7,9,10-hexahydro-8*H-*pyrazino[1',2':4,5]pyrazino[2 ,3-*c*]pyridazin-8-yl)((2*R*,6*S*)-2,6-dimethylpiperazin-1-yl)methanone (Mixture of Isomers) | 472.2 | 472.0 |
| **Int-17** | 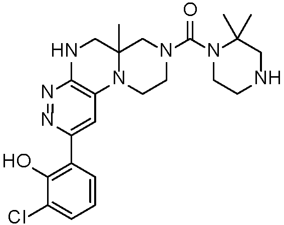 | (2-(3-chloro-2-hydroxyphenyl)-6a-methyl-5,6,6a,7,9,10-hexahydro-8*H-*pyrazino[1',2':4,5]pyrazino [2,3-c]pyridazin-8-yl)(2,2-dimethylpiperazin-1-yl) methanone (Mixture of Isomers) | 472.2 | 472.0 |
| **Int-18** | 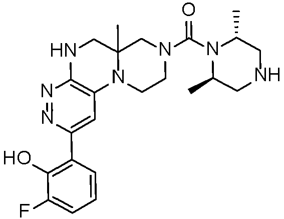 | ((2*R*,6*R*)-2,6-dimethylpiperazin-1-yl)(2-(3-fluoro-2-hydroxyphenyl)-6a-methyl-5,6,6a, 7,9,10-hexahydro-8*H*-pyrazino [1',2':4,5]pyrazino[2,3-*c*] pyridazin-8-yl)methanone (Isomer 1) | 456.3 | 456.1 |
| **Int-19** | 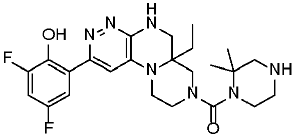 | (2-(3,5-difluoro-2-hydroxyphenyl)-6a-ethyl-5,6,6a,7,9,10-hexahydro-8*H-*pyrazino[1',2':4,5]pyrazino [2,3-*c*]pyridazin-8-yl)(2,2-dimethylpiperazin-1-yl) methanone (Mixture of Isomers) | 488.3 | 488.1 |

### Intermediate 20: 3-(6-(4-(hydroxymethyl)piperidin-1-yl)-1-oxoisoindolin-2-yl)piperidine-2,6-dione

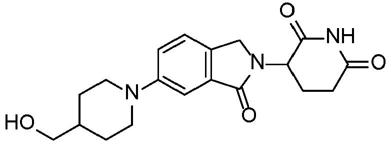

### Step 1: Methyl 2-cyano-5-(4-(hydroxymethyl)piperidin-1-yl)benzoate

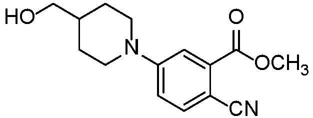

To a solution of methyl 2-cyano-5-fluorobenzoate (2.00 g, 11.2 mmol) and 4-piperidinemethanol (1.67 g, 14.5 mmol) in dimethyl sulfoxide (22.3 mL) was added *N,N-*diisopropylethylamine (5.83 mL, 33.5 mmol). The reaction mixture was heated to 110 °C and stirred for 1.5 hours. The product mixture was diluted with EtOAc (100 mL) and transferred to a separatory funnel. The diluted reaction mixture was washed with a saturated aqueous sodium chloride solution (50 mL × 2). The organic layer was dried over sodium sulfate, filtered and concentrated under reduced pressure. The crude residue was purified by silica gel flash column chromatography, eluting with a gradient 0-100% EtOAc/hexanes to obtain methyl 2-cyano-5-(4-(hydroxymethyl)piperidin-1-yl)benzoate (3.02 g, 98% yield) as a yellow oil. LCMS calcd for C₁₅H₁₈N₂O₃ [M+H]⁺: m/z = 275.1; Found: 275.1.

### Step 2: Methyl 2-formyl-5-(4-(hydroxymethyl)piperidin-1-yl)benzoate

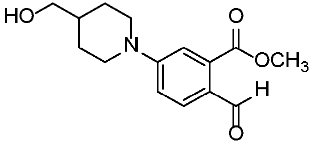

To a solution of methyl 2-cyano-5-(4-(hydroxymethyl)piperidin-1-yl)benzoate (3.00 g, 10.9 mmol), sodium hypophosphite monohydrate (11.7 g, 111 mmol) and acetic acid (12.7 mL, 222 mmol) in pyridine (26.3 mL) was added a slurry of Raney nickel (1.97 g, 33.6 mmol) in water (28.0 mL). The reaction mixture was heated to 70 °C and stirred for 8 hours. The product mixture was filtered through celite and the celite was washed with EtOAc (50 mL × 2). The filtrate was transferred to a separatory funnel and washed with water (150 mL). The aqueous layer was extracted with EtOAc (75 mL × 2). The combined organic layers were dried over sodium sulfate, filtered, and concentrated under reduced pressure. The crude residue was purified by silica gel flash column chromatography eluting with a gradient of 0-100% EtOAc/hexanes to give methyl 2-formyl-5-(4-(hydroxymethyl)piperidin-1-yl)benzoate (2.31 g, 76.0%) as a yellow oil. LCMS calcd for C₁₅H₂₀NO₄ [M+H]⁺: m/z = 278.1; Found: 278.1.

### Step 3: 3-(6-(4-(hydroxymethyl)piperidin-1-yl)-1-oxoisoindolin-2-yl)piperidine-2,6-dione

To a stirring solution of methyl 2-formyl-5-(4-(hydroxymethyl)piperidin-1-yl)benzoate (2.40 g, 8.65 mmol) in DCM (48.8 mL) and DMF (48.8 mL) was added 3-aminopiperidine-2,6-dione hydrochloride (1.85 g, 11.3 mmol) followed by *N*,*N-*diisopropylethylamine (3.77 mL, 21.6 mmol). The reaction mixture was stirred at room temperature for 3 hours. The reaction mixture was cooled to 0 °C and acetic acid (5.94 mL, 104 mmol) followed by sodium triacetoxyborohydride (5.50 g, 26.0 mmol) was added. The reaction mixture was allowed to slowly warm to room temperature and stirred for an additional 3 hours. The reaction mixture was diluted with water (10 mL) and the solution was basified with saturated aqueous NaHCO₃ solution until no further evolution of gas was observed. The basified product mixture was filtered and the solid was washed with water (10 mL × 2). The solid was collected and dried under vacuum to obtain 3-(6-(4-(hydroxymethyl)piperidin-1-yl)-1-oxoisoindolin-2-yl)piperidine-2,6-dione (1.95 g, 63%) as a grey-white solid. LCMS calcd for C₁₉H₂₃N₃O₄ [M+H]⁺: m/z = 358.2; found: 358.1.

### Intermediates 21-26:

The intermediates shown below in Table 2 were prepared by methods analogous to those described for the preparation of Intermediate 20 using the appropriate starting materials.

**Table 2. Intermediates 21-26**

| **Int.** | **Structure** | **Name** | **Calcd. (M+H)⁺ m/z** | **Found (M+H)⁺ m/z** |
|---|---|---|---|---|
| **Int-21** | 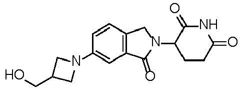 | 3-(6-(3-(hydroxymethyl) azetidin-1-yl)-1-oxoisoindolin-2-yl) piperidine-2,6-dione | 330.1 | 330.2 |
| **Int-22** | 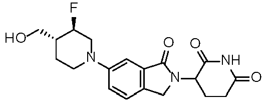 | 3 -(6-((3*R*,4*R*)-3 -fluoro-4-(hydroxymethyl)piperidin-1-yl)-1-oxoisoindolin-2-yl) piperidine-2,6-dione | 376.2 | 376.0 |
| **Int-23** | 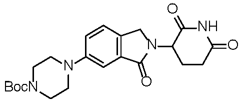 | *tert*-butyl 4-(2-(2,6-dioxopiperidin-3-yl)-3-oxoisoindolin-5-yl) piperazine-1-carboxylate | 329.2 | 329.1 |
| **Int-24** | 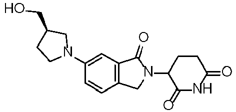 | 3-(6-((*R*)-3-(hydroxymethyl)pyrrolidin-1-yl)-1-oxoisoindolin-2-yl) piperidine-2,6-dione | 344.2 | 344.1 |
| **Int-25** | 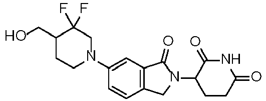 | 3-(6-(3,3-difluoro-4-(hydroxymethyl)piperidin-1-yl)-1-oxoisoindolin-2-yl) piperidine-2,6-dione | 394.2 | 394.0 |
| **Int-26** | 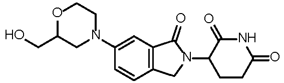 | 3-(6-(2-(hydroxymethyl) morpholino)-1-oxoisoindolin-2-yl) piperidine-2,6-dione | 360.2 | 360.0 |

### Intermediate 27: (S)-3-(6-(4-(hydroxymethyl)piperidin-1-yl)-1-oxoisoindolin-2-yl)piperidine-2,6-dione

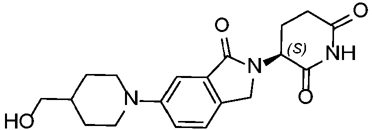

### Step 1: tert-butyl (S)-5-amino-4-(6-(4-(hydroxymethyl)piperidin-1-yl)-1-oxoisoindolin-2-yl)-5-oxopentanoate

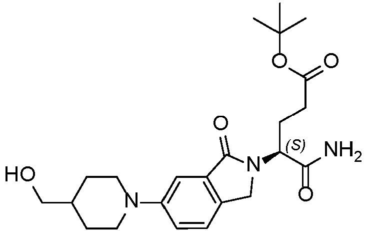

To a solution of methyl 2-formyl-5-[4-(hydroxymethyl)piperidin-1-yl]benzoate (3.0 g, 10.82 mmol) in DCM (45 mL) was charge H-Glu(OtBu)-NH2•HCl (2.84 g, 11.9 mmol), acetic acid (1.24 mL, 21.64 mmol), and N,N-Diisopropylethylamine (2.07 mL, 11.9 mmol). The reaction mixture was stirred at RT for 30 min then cool to 5 °C. Sodium triacetoxyborohydride (0.840 g, 3.97 mmol) was charged portion wise at 5 °C then stirred at room temp overnight. After being diluted with DCM (45 mL), the reaction was quenched with sat. NaHCO₃ aq solution until no more bubbling was observed, while white precipitation formed. Filtered precipitate and washed with water and DCM. Collect solid and dried under vacuum to give a white solid (4.1 g, 88% yield, 98.4% ee). LCMS calcd for C₂₃H₃₄N₃O₅ [M+H]⁺: m/z= 432.1; Found: 432.1

### Step 2: tert-butyl (S)-4-(6-(4-(acetoxymethyl)piperidin-1-yl)-1-oxoisoindolin-2-yl)-5-amino-5-oxopentanoate

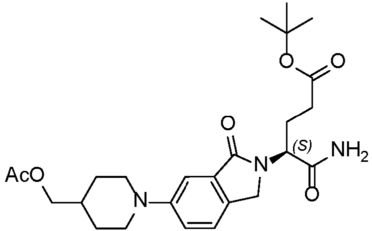

To a stirred solution of *tert-butyl* (*S*)-5-amino-4-(6-(4-(hydroxymethyl)piperidin-1-yl)-1-oxoisoindolin-2-yl)-5-oxopentanoate (3.00 g, 6.95 mmol) in DCM (30.0 mL) was charged *N,N-*Diisopropylethylamine (1.33 mL, 7.65 mmol) followed by acetyl chloride (0.74 mL, 10.4 mmol). The reaction was stirred at RT for 2 hours. The reaction was quenched with sat. NaHCO₃, extracted with DCM, dried over Na₂SO₄, purified by Flash Chromatography with 5% MeOH in DCM to give a pale-yellow solid (3.1 g, 94%). LCMS calcd for C₂₅H₃₆N₃O₆ [M+H]⁺: m/z = 474.1; Found: 474.1.

### Step 3: (S)-3-(6-(4-(hydroxymethyljpiperidin-1-yl)-1-oxoisoindolin-2-yl)piperidine-2,6-dione

To a stirred solution of tert-butyl (*S*)-4-(6-(4-(acetoxymethyl)piperidin-1-yl)-1-oxoisoindolin-2-yl)-5-amino-5-oxopentanoate (1.15 g, 2.43 mmol) in MeCN (12.0 mL) was charged benzenesulfonic acid (768.0 mg, 4.86 mmol). The reaction mixture was heated to 85 °C. After 2 hours, the reaction was cooled to RT and charged with water (12 mL) then stirred at 75 °C for 4 hours until LCMS showed full conversion. The reaction mixture was evaporated to remove volatiles, the residue was cooled to 5 °C and charged with NaHCO₃ aq slowly until pH=6-7 during which white precipitation formed. Filtered and washed with water. Collect solid and dried under vacuum to give an off-white solid (730 mg, 84% yield, 98.2% ee). LCMS calcd for C₁₉H₂₄N₃O₄ [M+H]⁺: m/z = 358.2; Found: 358.1

### Intermediate 28

The intermediate shown below in Table 3 was prepared by the method used in preparing Int-27 using the appropriate starting materials.

**Table 3. Intermediate 28**

| **Int.** | **Structure** | **Name** | **Calcd. (M+H)⁺ m/z** | **Found (M+H)⁺ m/z** |
|---|---|---|---|---|
| **Int-28** | 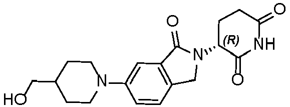 | (*R*)-3-(6-(4-(hydroxymethyl)piperidin-1-yl)-1-oxoisoindolin-2-yl) piperidine-2,6-dione | 358.2 | 358.1 |

### Intermediate 29: 3-(5-((R)-2-(hydroxymethyl)morpholino)-1-oxoisoindolin-2-yl)piperidine-2,6-dione

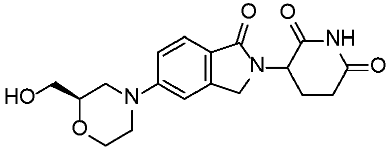

### Step 1: Methyl (R)-2-cyano-4-(2-(hydroxymethyl)morpholino)benzoate

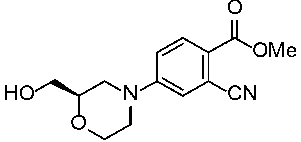

To a stirring solution of (R)-morpholin-2-ylmethanol (255 mg, 2.18 mmol), methyl 2-cyano-4-fluorobenzoate (300 mg, 1.67 mmol) in NMP (6.70 mL) was added N,N-diisopropylethylamine (0.88 mL, 5.02 mmol). The reaction was heated to 120 °C and stirred for 1.5 hours. The reaction mixture was cooled to room temperature and diluted with DCM and a saturated aqueous brine solution. The aqueous layer was extracted with DCM twice. The organic layers were combined, dried over Na₂SO₄, filtered, and concentrated under reduced pressure. The crude residue was purified by silica gel flash column chromatography eluting with a gradient of 0-100% EtOAc/hexane to give methyl (R)-2-cyano-4-(2-(hydroxymethyl)morpholino)benzoate (401 mg, 87% yield) as a yellow oil. LCMS m/z calcd for C₁₄H₁₇N₂O₄ [M+H]⁺: m/z = 277.1; found: 277.0.

### Step 2: Methyl (R)-2-formyl-4-(2-(hydroxymethyl)morpholino)benzoate

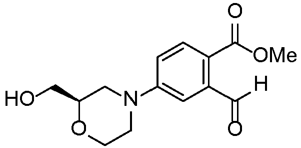

A mixture of methyl (*R*)-2-cyano-4-(2-(hydroxymethyl)morpholino)benzoate (401 mg, 1.45 mmol), pyridine (3.50 mL, 43.2 mmol), acetic acid (1.70 mL, 29.4 mmol) and sodium hypophosphite monohydrate (1.55 g, 14.67 mmol) was treated with a slurry of Raney nickel (262 mg, 4.46 mmol) in water (3.70 mL). The reaction was heated to 70 °C and stirred for 8 hours, while monitored by LCMS. The reaction mixture was filtered through celite and the celite was washed with EtOAc and water. The aqueous layer was extracted with EtOAc and the combined organics were dried over Na₂SO₄, filtered and concentrated under reduced pressure. The crude material was purified by silica gel flash column chromatography eluting with a gradient of 0-100% EtOAc/hexane. The product was obtained as a yellow oil to give methyl (*R*)-2-formyl-4-(2-(hydroxymethyl)morpholino)benzoate (308 mg, 76% yield). LCMS calcd for C₁₄H₁₈NO₅ [M+H]⁺: m/z = 280.1; Found: 280.0.

### Step 3: 3-(5-((R)-2-(hydroxymethyl)morpholino)-1-oxoisoindolin-2-yl)piperidine-2,6-dione

*N,N*-diisopropylethylamine (0.58 mL, 3.31 mmol) was added to a solution of methyl (R)-2-formyl-4-(2-(hydroxymethyl)morpholino)benzoate (308 mg, 1.10 mmol) and 3-amino-piperidine-2,6-dione, HCl (272 mg, 1.65 mmol) in DCM (5.50 mL). The reaction was stirred at room temperature overnight. The reaction was cooled to 0 °C and acetic acid (0.760 mL, 13.2 mmol) and sodium triacetoxyborohydride (701 mg, 3.31 mmol) were added. The reaction was stirred at 0 °C until complete. A saturated aqueous solution of NaHCO₃ was added the crude reaction mixture was concentrated under reduced pressure. The crude material was dissolved in DMSO, filtered and the filtrate was purified by prep-HPLC (Waters CSH-C18 column, 7.2-27.2% MeCN/water (containing 0.1% TFA) over 5 min) to give 3-(5-((R)-2-(hydroxymethyl) morpholino)-1-oxoisoindolin-2-yl)piperidine-2,6-dione;2,2,2-trifluoroacetic acid (259 mg, 50% yield) as a purple oil. LCMS m/z calcd for C₁₈H₂₂N₃O₅ [M+H]⁺: m/z = 360.2; Found: 360.1.

### Intermediate 30: 2-(2,6-dioxopiperidin-3-yl)-5-(4-(hydroxymethyl)piperidin-1-yl)isoindoline-1,3-dione

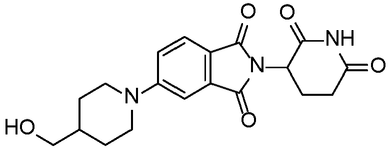

### Step 1: 2-(2,6-dioxopiperidin-3-yl)-5-(4-(hydroxymethyl)piperidin-1-yl)isoindoline-1,3-dione

*N,N*-Diisopropylethylamine (0.380mL, 2.17 mmol) was added to a solution of 2-(2,6-dioxopiperidin-3-yl)-5-fluoroisoindole-1,3-dione (200 mg, 0.720 mmol) and 4-piperidinemethanol (208 mg, 1.81 mmol) in NMP (7.24 mL). The reaction was heated to 100 °C and monitored by LCMS until complete. The reaction was cooled to room temperature and the solution was filtered and purified by prep-HPLC (Waters CSH-C18 column, 18.3-38.3% MeCN/water (containing 0.1% TFA) over 5 min) to give the TFA salt of 2-(2,6-dioxopiperidin-3-yl)-5-(4-(hydroxymethyl)piperidin-1-yl)isoindoline-1,3-dione (185 mg, 53% yield) as a yellow solid. LCMS m/z calcd for C₁₉H₂₂N₃O₃ [M+H]⁺: m/z = 372.2; Found: 372.0.

### Intermediate 31: (R)-(6a-(difluoromethyl)-2-(3-fluoro-2-hydroxyphenyl)-5,6,6a,7,9,10-hexahydro-8H-pyrazino[1',2':4,5]pyrazino[2,3-c]pyridazin-8-yl)(2,2-dimethylpiperazin-1-yl)methanone

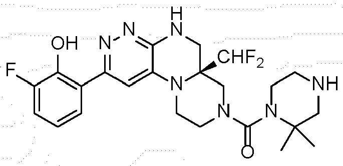

### Step 1. 1,4-di-tert-butyl 2-methyl 2-(difluoromethyl)piperazine-1,2, 4-tricarboxylate

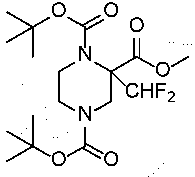

LiHMDS (1 M in THF, 31.9 mL, 31.9 mmol) was added to a solution of *1,4-di-tert-butyl* 2-methyl (S)-piperazine-1,2,4-tricarboxylate (5.5 g, 16 mmol) in THF (80 mL) at -78 °C. The reaction was stirred for 1 hour at -78 °C. Difluoromethyl trifluoromethanesulfonate (6.07 mL, 47.9 mmol) was added to the reaction mixture and the reaction mixture was allowed to slowly warm to room temperature and stirred overnight. The reaction mixture was quenched with a saturated aqueous solution of NH₄Cl (200 mL) and extracted with EtOAc (2 x 200 mL). The combined organics were dried over MgSO₄, filtered, and concentrated under reduced pressure. The crude material was purified by silica gel flash column chromatography with a gradient of 0-30% EtOAc/hexanes to give 1,4-di-tert-butyl 2-methyl 2-(difluoromethyl)piperazine-1,2,4-tricarboxylate as an orange oil (6.0 g, 95%). LCMS calcd for C₈H₁₃F₂N₂O₄ [M+H-C₉H₁₆O₂]⁺: m/z = 239.1; Found: 239.0

### Step 2. 1,4-bis(tert-butoxycarbonyl)-2-(difluoromethyl)piperazine-2-carboxylic acid

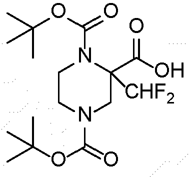

Lithium hydroxide (3.3 g, 0.14 mol) was added to a solution of 1,4-di-tert-butyl 2-methyl 2-(difluoromethyl)piperazine-1,2,4-tricarboxylate (5.5g, 13.9 mmol) in THF (100 mL), methanol (40 mL), and water (40 mL) at room temperature. The reaction mixture was heated to 60 °C and stirred overnight. The reaction mixture was quenched with a 1 M HCl solution (200 mL). The product mixture was transferred to a separatory funnel and extracted with DCM (2 x 200 mL). The combined organic layers were dried over Na₂SO₄, filtered, and concentrated under reduced pressure. The resulting residue was used in the next step without purification. *1,4-bis(tert-*butoxycarbonyl)-2-(difluoromethyl)piperazine-2-carboxylic acid (5.3 g, 100%) was obtained as a white foam. LCMS calcd for C₇H₁₁F₂N₂O₄ [M+H-C₉H₁₆O₂]⁺: m/z = 225.1; Found: 225.0

### Step 3. di-tert-butyl 2-chloro-6a-(difluoromethyl)-6-oxo-6a,7,9,10-tetrahydro-5H-pyrazino[1',2':4,5]pyrazino[2,3-c]pyridazine-5,8(6H)-dicarboxylate

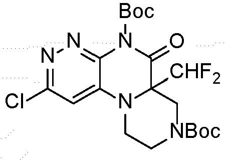

Oxalyl chloride (1.55 mL, 18.1 mmol) was added dropwise to a stirring solution of DMF (1.4 mL, 18.1 mmol) and DCM (35 mL) at 0 °C. The reaction mixture was stirred for 10 minutes at 0 °C. Then a solution of 1,4-bis(*tert*-butoxycarbonyl)-2-(difluoromethyl)piperazine-2-carboxylic acid (5.3 g, 13.9 mmol) and pyridine (1.69 mL, 20.9 mmol) in DCM (10 mL) was added to the reaction mixture at 0 °C. After the addition the reaction mixture was stirred for 30 minutes at 0 °C. Then the reaction mixture was transferred to a separatory funnel and washed with water (2 x 30 mL) and a saturated sodium chloride aqueous solution (30 mL). The organic layer was dried over Na₂SO₄, filtered, and concentrated under reduced pressure. The resulting residue was dissolved in DMF (28 mL), and *N,N*-diisopropylethylamine (7.28 mL, 41.8 mmol) and 4-bromo-6-chloropyridazin-3-amine (2.76 g, 13.2 mmol) were added sequentially. The resulting mixture was stirred at 120 °C overnight. The product mixture was diluted with EtOAc (200 mL) and washed with a saturated sodium chloride aqueous solution (2 x 200 mL). The organic layer was dried over Na₂SO₄, filtered, and concentrated under reduced pressure. The resulting residue was purified by silica gel flash column chromatography with a gradient of 0-100% EtOAc/hexanes to obtain *tert*-butyl 2-chloro-6a-(difluoromethyl)-6-oxo-5,6,6a,7,9,10-hexahydro-8*H*-pyrazino[1',2':4,5]pyrazino[2,3-c]pyridazine-8-carboxylate as a mixture with 4-bromo-6-chloropyridazin-3-amine. The mixture was dissolved in THF (40 mL), and *di-tert-butyl* dicarbonate (4.8 mL, 20.9 mmol) and 4-(dimethylamino)pyridine (223 mg, 1.82 mmol) were added sequentially. The reaction mixture was stirred for 5 hours. The product mixture was concentrated under reduced pressure. The resulting residue was purified by silica gel flash column chromatography with a gradient of 0-100% EtOAc/hexanes to obtain di-tert-butyl 2-chloro-6a-(difluoromethyl)-6-oxo-6a,7,9,10-tetrahydro-5*H*-pyrazino[1',2':4,5]pyrazino[2,3-c]pyridazine-5,8(6H)-dicarboxylate as a pale yellow foamy solid (523 mg, 8%). LCMS calcd for C₂₀H₂₆ClF₂N₃O₃ [M+H]⁺: m/z = 490.2; Found: 489.9

### Step 4. Chiral Separation of di-tert-butyl 2-chloro-6a-(difluoromethyl)-6-oxo-6a,7, 9,10-tetrahydro-5H-pyrazino[1',2':4,5]pyrazino[2,3-c]pyridazine-5,8(6H)-dicarboxylate

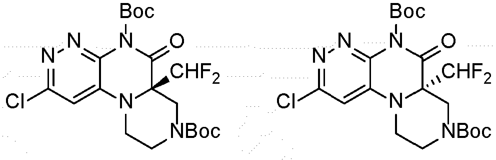

The racemic mixture of di-tert-butyl 2-chloro-6a-(difluoromethyl)-6-oxo-6a,7,9,10-tetrahydro-5*H*-pyrazino[1',2':4,5]pyrazino[2,3-*c*]pyridazine-5,8(6*H*)-dicarboxylate (523 mg) was purified via chiral separation (Lux Cellulose-1, 20 mL/min of 80:10:10 Hexanes/IPA/MeOH) to give di-*tert*-butyl (*S*)-2-chloro-6a-(difluoromethyl)-6-oxo-6a,7,9,10-tetrahydro-5*H-*pyrazino[1',2':4,5]pyrazino[2,3-c]pyridazine-5,8(6*H*)-dicarboxylate (185 mg, Peak A, 35%) and di-*tert-*butyl (R)-2-chloro-6a-(difluoromethyl)-6-oxo-6a,7,9,10-tetrahydro-5*H-*pyrazino[1',2':4,5]pyrazino[2,3-c]pyridazine-5,8(6H)-dicarboxylate (196 mg, Peak B, 37%). LCMS calcd for C₂₀H₂₆ClF₂N₅O₅ [M+H]⁺: m/z = 490.2; Found: 489.9

### Step 5. tert-butyl (S)-6a-(difluoromethyl)-2-(3-fluoro-2-hydroxyphenyl)-6-oxo-5,6,6a,7,9,10-hexahydro-8H-pyrazino[1',2':4,5]pyrazino[2,3-c]pyridazine-8-carboxylate

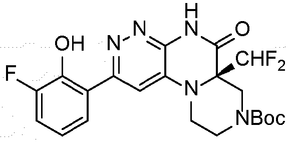

A 20 mL scintillation vial was charged with *tert-butyl* (S)-2-chloro-6a-(difluoromethyl)-6-oxo-5,6,6a,7,9,10-hexahydro-8*H*-pyrazino[1',2':4,5]pyrazino[2,3-*c*]pyridazine-8-carboxylate (326 mg, 0.66 mmol), cesium carbonate (650 mg, 2.0 mmol), XPhos Pd G2 (52.4 mg, 0.066 mmol), and 3-fluoro-2-hydroxyphenylboronic acid (239 mg, 1.5 mmol). The mixture was dissolved in 1,4-dioxane (4 mL) and water (0.5 mL). The reaction mixture was sparged with N₂ gas for 2 minutes, sealed, and heated to 80 °C. The reaction mixture was stirred for 2 hours at 80°C. The product mixture was diluted with EtOAc (50 mL) and washed with water (100 mL). The aqueous layer was extracted with EtOAc (2 x 100 mL). The combined organic layers were dried over Na₂SO₄, filtered, and concentrated under reduced pressure. The resulting residue was purified by silica gel flash column chromatography with a gradient of 0-100% EtOAc/hexanes to obtain *tert-butyl* (*S*)-6a-(difluoromethyl)-2-(3-fluoro-2-hydroxyphenyl)-6-oxo-5,6,6a,7,9,10-hexahydro-8*H*-pyrazino[1',2':4,5]pyrazino[2,3-c]pyridazine-8-carboxylate as a pale oil (205 mg, 66%). LCMS calcd for C₂₁H₂₂F₃N₅O₄ [M+H]⁺: m/z = 466.2; Found: 466.0

### Step 6. tert-butyl (R)-6a-(difluoromethyl)-2-(3-fluoro-2-hydroxyphenyl)-5, 6, 6a,7,9,10-hexahydro-8H-pyrazino[1',2':4,5]pyrazino[2,3-c]pyridazine-8-carboxylate

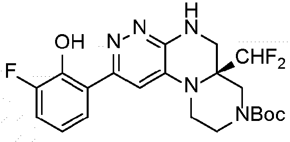

Borane tetrahydrofuran complex (1 M, 7.4 mL, 7.4 mmol) was added to a solution of *tert-*butyl (S)-6a-(difluoromethyl)-2-(3-fluoro-2-hydroxyphenyl)-6-oxo-5,6,6a,7,9,10-hexahydro-8H-pyrazino[1',2':4,5]pyrazino[2,3-c]pyridazine-8-carboxylate (570 mg, 1.22 mmol) in THF (8.5 mL) at room temperature. The reaction mixture was heated to 60 °C for 2 hours. The reaction mixture was cooled to 0 °C and quenched with MeOH (3 mL) slowly. The reaction mixture was diluted with EtOAc (100 mL). The diluted reaction mixture was washed with a saturated sodium bicarbonate aqueous solution (2 x 100 mL). The organic layer was dried over Na₂SO₄, filtered, and concentrated under reduced pressure. The resulting residue was dissolved in THF (8.5 mL) and MeOH (3.7 mL). Acetic acid (2.1 mL, 36.7 mmol) and sodium cyanoborohydride (770 mg, 12.2 mmol) were added sequentially to the reaction mixture at room temperature. The reaction mixture was refluxed at 80 °C for 16 hours. The product mixture was cooled to room temperature and diluted with EtOAc (100 mL). The diluted reaction mixture was washed with a saturated sodium bicarbonate aqueous solution (2 x 100 mL). The organic layer was dried over Na₂SO₄, filtered, and concentrated under reduced pressure. The resulting residue was purified by silica gel flash column chromatography with a gradient of 0-15% MeOH/DCM to obtain tert-butyl (R)-6a-(difluoromethyl)-2-(3-fluoro-2-hydroxyphenyl)-5,6,6a,7,9,10-hexahydro-8H-pyrazino[1',2':4,5]pyrazino[2,3-*c*]pyridazine-8-carboxylate as a dark oil (455 mg, 82%). LCMS calcd for C₂₁H₂₅F₃N₅O₃ [M+H]⁺: m/z = 452.2; Found: 452.1

### Step 7. (S)-2-(6a-(difluoromethyl)-6,6a,7, 8, 9,10-hexahydro-5H-pyrazino[1',2':4,5] pyrazino[2,3-c]pyridazin-2-yl)-6-fluorophenol

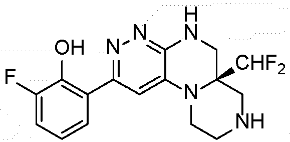

Hydrochloric acid (4 M in 1,4-dioxane, 3.8 mL, 15.2 mmol) was added to a solution of *tert-*butyl (*R*)-6a-(difluoromethyl)-2-(3-fluoro-2-hydroxyphenyl)-5,6,6a,7,9,10-hexahydro-8H-pyrazino[1',2':4,5]pyrazino[2,3-c]pyridazine-8-carboxylate (455 mg, 1.0 mmol) in DCM (10 mL) at room temperature. The reaction was stirred overnight. The product mixture was concentrated under reduced pressure to obtain the HCl salt of (S)-2-(6a-(difluoromethyl)-6,6a,7,8,9,10-hexahydro-5*H*-pyrazino[1',2':4,5]pyrazino[2,3-*c*]pyridazin-2-yl)-6-fluorophenol as a tan solid (427 mg, 100%). LCMS calcd for C₁₆H₁₇F₃N₅O [M+H]⁺: m/z = 352.1; Found: 352.1

### Step 8. tert-butyl 4-(chlorocarbonyl)-3, 3-dimethylpiperazine-1-carboxylate

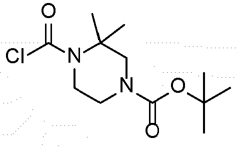

Triphosgene (415 mg, 1.4 mmol) was added portionwise to a stirring solution of *tert-*butyl 3,3-dimethylpiperazine-1-carboxylate (500 mg, 2.33 mmol) and pyridine (570 µL, 7.0 mmol) in DCM (20 mL) at 0 °C. The reaction was warmed to room temperature and stirred for 2 hours. The product mixture was washed with 1 M HCl aqueous solution (50 mL). The aqueous layer was extract with DCM (2 x 50 mL). The combined organic layers were dried over Na₂SO₄, filtered, and concentrated under reduced pressure. The residue obtained was used without further purification. tert-Butyl 4-(chlorocarbonyl)-3,3-dimethylpiperazine-1-carboxylate was obtained as a yellow oil (650 mg, 100%).

### Step 9. tert-butyl (R)-4-(6a-(difluoromethyl)-2-(3-fluoro-2-hydroxyphenyl)-6,6a,7,8,9,10-hexahydro-5H-pyrazino[1',2':4,5]pyrazino[2, 3-c]pyridazine-8-carbonyl)-3,3-dimethylpiperazine-1-carboxylate

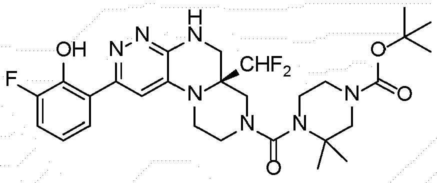

*N,N-*Diisopropylethylamine (701 µL, 4.0 mmol) and 4-(dimethylamino)pyridine (36.9 mg, 0.30 mmol) were added sequentially to a stirring solution of (S)-2-(6a-(difluoromethyl)-6,6a,7,8,9,10-hexahydro-5H-pyrazino[1',2':4,5]pyrazino[2,3-c]pyridazin-2-yl)-6-fluorophenol (427 mg, 1.01 mmol) in dimethylacetamide (10 mL) at room temperature. The reaction mixture was stirred 15 minutes. A solution of tert-butyl 4-(chlorocarbonyl)-3,3-dimethylpiperazine-1-carboxylate (418 mg, 1.51 mmol) in dimethylacetamide (2 mL) was added to the reaction mixture. The reaction mixture was stirred 30 minutes at room temperature. The product mixture was diluted with EtOAc (100 mL) washed with saturated sodium bicarbonate aqueous solution (100 mL). The aqueous layer was extracted with EtOAc (2 x 100 mL). The combined organic layers were dried over Na₂SO₄, filtered and concentrated under reduced pressure. The resulting residue was purified by silica gel flash column chromatography with a gradient of 0-15% MeOH/DCM to obtain tert-butyl (R)-4-(6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazine-1-carboxylate as a yellow oil (502 mg, 84.3%). LCMS calcd for C₂₈H₃₇F₃N₇O₄ [M+H]⁺: m/z = 592.3; Found: 592.2.

### Step 10. (R)-(6a-(difluoromethyl)-2-(3-fluoro-2-hydroxyphenyl)-5,6,6a,7,9,10-hexahydro-8H-pyrazino[1',2':4,5]pyrazino[2,3-c]pyridazin-8-yl)(2,2-dimethylpiperazin-1-yl)methanone

Trifluoroacetic acid (1.54 mL, 20.1 mmol) was added to a stirring solution of *tert-butyl* (R)-4-(6a-(difluoromethyl)-2-(3-fluoro-2-hydroxyphenyl)-6,6a,7,8,9,10-hexahydro-5H-pyrazino[1',2':4,5]pyrazino[2,3-*c*]pyridazine-8-carbonyl)-3,3-dimethylpiperazine-1-carboxylate (502 mg, 0.85 mmol) in DCM (10 mL) at room temperature. The reaction was stirred 1 hour at room temperature. The product mixture was concentrated under reduced pressure to obtain the trifluoroacetic acid salt of (R)-(6a-(difluoromethyl)-2-(3-fluoro-2-hydroxyphenyl)-5,6,6a,7,9,10-hexahydro-8*H*-pyrazino[1',2':4,5]pyrazino[2,3-*c*]pyridazin-8-yl)(2,2-dimethylpiperazin-1-yl)methanone as an oil (610 mg, 100%) LCMS calcd for C₂₃H₂₉F₃N₇O₂ [M+H]⁺: m/z = 492.2; Found: 492.0.

### Intermediate 32: ((R)-6a-(difluoromethyl)-2-(3-fluoro-2-hydroxyphenyl)-5,6,6a,7,9,10-hexahydro-8H-pyrazino[1',2':4,5]pyrazino[2,3-c]pyridazin-8-yl)((2R,6S)-2,6-dimethylpiperazin-1-yl)methanone

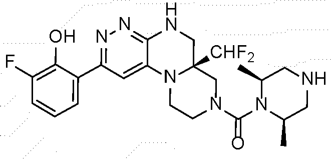

### Step 1. (S)-6a-(difluoromethyl)-2-(3-fluoro-2-hydroxyphenyl)-7,8,9,10-tetrahydro-5H-pyrazino[1',2':4,5]pyrazino[2,3-c]pyridazin-6(6aH)-one

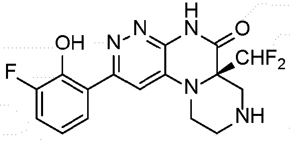

Hydrochloric acid (4 M in dioxane, 0.21 mL, 0.86 mmol) was added to a stirring solution of tert-butyl (*S*)-6a-(difluoromethyl)-2-(3-fluoro-2-hydroxyphenyl)-6-oxo-5,6,6a,7,9,10-hexahydro-8*H*-pyrazino[1',2':4,5]pyrazino[2,3-*c*]pyridazine-8-carboxylate (40 mg, 0.086 mmol) in DCM (2 mL). The reaction mixture was stirred overnight. The product mixture was concentrated under reduced pressure to obtain the HCl salt of (*S*)-6a-(difluoromethyl)-2-(3-fluoro-2-hydroxyphenyl)-7,8,9,10-tetrahydro-5*H*-pyrazino[1',2':4,5]pyrazino[2,3-c]pyridazin-6(6aH)-one (37 mg, 99%). LCMS calcd for C₁₆H₁₄F₃N₅O₂ [M+H]⁺: m/z = 366.1; Found: 366.0

### Step 2. tert-butyl (3R,5S)-4-((S)-6a-(difluoromethyl)-2-(3-fluoro-2-hydroxyphenyl)-6-oxo-6,6a,7,8,9,10-hexahydro-5H-pyrazino[1',2':4,5]pyrazino[2,3-c]pyridazine-8-carbonyl)-3,5-dimethylpiperazine-1-carboxylate

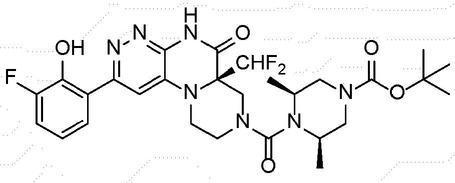

*N,N*-Diisopropylethylamine (30 µL, 0.17 mmol) and 4-(dimethylamino)pyridine (1.6 mg, 0.013 mmol) were added to a stirring solution of (S)-2-(6a-(difluoromethyl)-6,6a,7,8,9,10-hexahydro-5*H*-pyrazino[1',2':4,5]pyrazino[2,3-c]pyridazin-2-yl)-6-fluorophenol (19.2 mg, 0.043 mmol) in dimethylacetamide (1 mL) at room temperature. The reaction mixture was stirred 15 minutes. A solution of *tert*-butyl (3R,5S)-4-(chlorocarbonyl)-3,5-dimethylpiperazine-1-carboxylate (18 mg, 0.065 mmol) in dimethylacetamide (1 mL) was added to the reaction mixture. The reaction mixture was stirred 30 minutes at room temperature. The product mixture was diluted with EtOAc (25 mL) and washed with a saturated sodium bicarbonate aqueous solution (25 mL). The aqueous layer was extract with EtOAc (2 x 25 mL). The combined organic layers were dried over Na₂SO₄, filtered, and concentrated under reduced pressure. The residue obtained was purified by silica gel flash column chromatography with a gradient of 0-10% MeOH/DCM to obtain *tert*-butyl (3*R*,5*S*)-4-((,S)-6a-(difluoromethyl)-2-(3-fluoro-2-hydroxyphenyl)-6-oxo-6,6a,7,8,9,10-hexahydro-5*H*-pyrazino[1',2':4,5]pyrazino[2,3-*c*]pyridazine-8-carbonyl)-3,5-dimethylpiperazine-1-carboxylate as a yellow oil (37 mg, 71%). LCMS calcd for C₂₈H₃₄F₃N₇O₅ [M+H]⁺: m/z = 606.3; Found: 606.3

### Step 3. ((R)-6a-(difluoromethyl)-2-(3-fluoro-2-hydroxyphenyl)-5,6,6a,7,9,10-hexahydro-8H-pyrazino[1',2':4, 5]pyrazino[2,3-c]pyridazin-8-yl)((2R,6S)-2, 6-dimethylpiperazin-1-yl)methanone

Borane tetrahydrofuran complex (1 M in THF, 0.53 mL, 0.53 mmol) was added to a stirring solution of tert-butyl (3*R*,5*S*)-4-((*S*)-6a-(difluoromethyl)-2-(3-fluoro-2-hydroxyphenyl)-6-oxo-6,6a,7,8,9,10-hexahydro-5*H*-pyrazino[1',2':4,5]pyrazino[2,3-*c*]pyridazine-8-carbonyl)-3,5-dimethylpiperazine-1-carboxylate (80 mg, 0.132 mmol) in THF (1 mL) at room temperature. The reaction mixture was heated to 60 °C and stirred overnight. The product mixture was cooled to 0 °C and quenched by slow addition of MeOH (2 mL). The quenched product mixture was heated to 80 °C and stirred for 30 minutes. The product mixture was concentrated under reduced pressure. The residue obtained was dissolved in DCM (2 mL). Trifluoroacetic acid (300 µL, 3.96 mmol) was added to the dissolved residue and stirred at room temperature for 1 hour. The product mixture was concentrated under reduced pressure. The residue obtained was dissolved in acetonitrile and purified by prep-HPLC (Waters CSH-Flouro-Phenyl, 5 uM, 30x100mm, 5-25% MeCN/water (containing 0.1%TFA) over 5 min) to give ((R)-6a-(difluoromethyl)-2-(3-fluoro-2-hydroxyphenyl)-5,6,6a,7,9,10-hexahydro-8*H*-pyrazino[1',2':4,5]pyrazino[2,3-*c*]pyridazin-8-yl)((2R,6S)-2,6-dimethylpiperazin-1-yl)methanone (16 mg, 20%) as its TFA salt. LCMS calcd for C₂₃H₂₈F₃N₇O₂ [M+H]⁺: m/z = 492.2; Found: 492.1

### Intermediates 33 - 36

The intermediates shown below in Table 4 were prepared by methods analogous to that described for preparing Intermediate 31 using the appropriate starting materials.

**Table 4. Intermediates 33 - 36**

| **Int** | **Structure** | **Name** | **Calcd. (M+H)⁺ m/z** | **Found (M+H)⁺ m/z** |
|---|---|---|---|---|
| **33** | 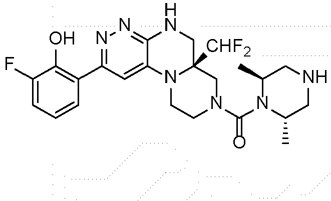 | ((*R*)-6a-(difluoromethyl)-2-(3-fluoro-2-hydroxyphenyl)-5,6,6a,7,9,10-hexahydro-8*H-*pyrazino[1',2':4,5]pyrazino[2,3-*c*]pyridazin-8-yl)((2*S*,6*S*)-2,6-dimethylpiperazin-1-yl) methanone | 492.2 | 492.0 |
| **34** | 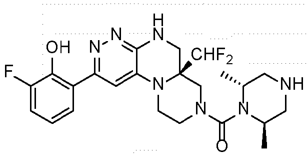 | ((R)-6a-(difluoromethyl)-2-(3-fluoro-2-hydroxyphenyl)-5,6,6a,7,9,10-hexahydro-8*H-*pyrazino[1',2':4,5]pyrazino[2,3-c]pyridazin-8-yl)((2R,6R)-2,6-dimethylpiperazin-1-yl)methanone | 492.2 | 492.0 |
| **35** | 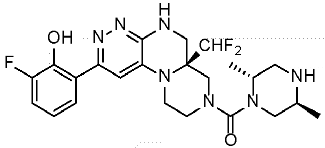 | ((*R*)-6a-(difluoromethyl)-2-(3-fluoro-2-hydroxyphenyl)-5,6,6a,7,9,10-hexahydro-8*H-*pyrazino[1',2':4,5]pyrazino[2,3-*c*]pyridazin-8-yl)((2*R*,5*S*)-2,5-dimethylpiperazin-1-yl)methanone | 492.2 | 492.0 |
| | | | | |
| **36** | 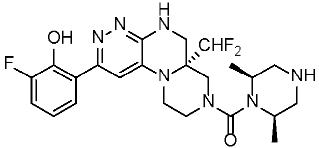 | ((*S*)-6a-(difluoromethyl)-2-(3-fluoro-2-hydroxyphenyl)-5,6,6a,7,9,10-hexahydro-8*H-*pyrazino[1',2':4,5]pyrazino[2,3-*c*]pyridazin-8-yl)((2*R*,6*S*)-2,6-dimethylpiperazin-1-yl) methanone | 492.2 | 492.1 |

### Intermediate 37: (S)-1-(2-(2,6-dioxopiperidin-3-yl)-3-oxoisoindolin-5-yl)piperidine-4-carbaldehyde

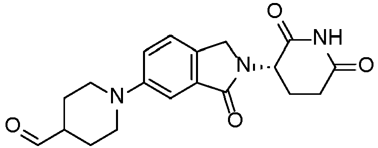

### Step 1. methyl 2-cyano-5-[4-(hydroxymethyl)piperidin-1-yl]benzoate

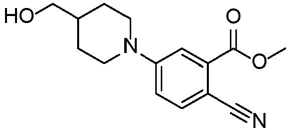

To a solution of methyl 2-cyano-5-fluorobenzoate (5.0 g, 27.9 mmol) and 4-piperidinemethanol (9.64 g, 83.7 mmol) in NMP (20 mL), *N,* N-diisopropylethylamine (7.29 mL, 41.9 mmol) was added and the reaction was heated at 120°C for 4 h. DIPEA was removed using rotary evaporation. The solution was further diluted by ethyl acetate and washed with H₂O. The organic layer was collected and dried over Na₂SO₄, filtered and concentrated. The crude product was purified by FCC (0% to 40% EA/heptanes) to obtain the title compound as a yellowish solid (5.1 g, 18.6 mmol, 67% yield). LCMS: (M+H)⁺ = 275.1.

### Step 2. methyl 2-cyano-5-(4-formylpiperidin-1-yl)benzoate

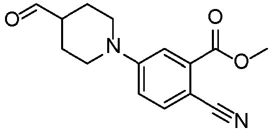

To a solution of methyl 2-cyano-5-[4-(hydroxymethyl)piperidin-1-yl]benzoate (2.5 g, 9.1 mmol) in DCM (30 mL) was added Dess-Martin Periodinane (5.8 g, 13.7 mmol) at 0°C. The reaction was stirred for 15 mins at room temperature, and TLC monitoring showed full consumption of starting material (eluent: 100% EA). The reaction mixture was diluted with DCM and quenched by saturated NaHCO₃ until effervescence stopped. The aqueous phase was extracted with DCM and the organic phase was dried over Na₂SO₄, filtered and concentrated under reduced pressure. The crude was purified by FCC (10% to 85% EA/heptanes) to obtain the title compound that was taken to next step without further purification. ¹H NMR (300 MHz, CDCl₃) δ 9.71 (s, 1H), 7.64 - 7.46 (m, 2H), 6.99 (dt, *J =* 10.4, 5.2 Hz, 1H), 3.98 (dt, *J=* 13.1, 4.1 Hz, 2H), 3.22 - 3.05 (m, 2H), 2.65 - 2.47 (m, 1H), 2.12 - 1.99 (m, 2H), 1.85 - 1.67 (m, 2H).

### Step 3. methyl 2-cyano-5-[4-(dimethoxymethyl)piperidin-1-yl]benzoate

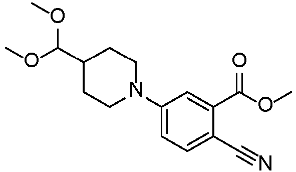

To a solution of methyl 2-cyano-5-(4-formylpiperidin-1-yl)benzoate (2.1 g, 7.71 mmol) in methanol (30 mL) was added trimethyl orthoformate (2.54 mL, 23.1 mmol) at 0°C. The reaction was stirred for 7 h at room temperature. The reaction was quenched by addition of TEA (0.2 eq), solvent was concentrated under reduced pressure and the crude was dissolved in ethyl acetate. This solution was washed with water (30 mL x 2), organic phase was collected, dried over Na₂SO₄, filtered and concentrated under reduced pressure. The crude was purified by FCC (0% to 45% EA/Heptanes) to obtain the title compound. (1.26 g, 3.9 mmol, 52% yield). ¹H NMR (300 MHz, CDCl₃) δ 7.62 - 7.47 (m, 2H), 6.97 (dd, *J =* 8.8, 2.8 Hz, 1H), 4.06 (d, *J =* 6.5 Hz, 1H), 3.98 (s, 3H), 3.93 (d, *J =* 12.9 Hz, 2H), 3.43 - 3.30 (m, 6H), 2.89 (td, *J =* 12.9, 2.4 Hz, 2H), 1.95 - 1.79 (m, 3H), 1.39 (ddd, *J =* 16.0, 13.5, 3.6 Hz, 2H).

### Step 4. methyl 5-[4-(dimethoxymethyl)piperidin-1-yl]-2-formylbenzoate

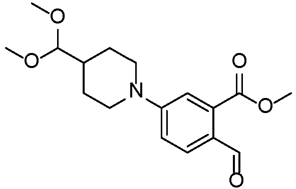

To a solution of methyl 2-cyano-5-[4-(dimethoxymethyl)piperidin-1-yl]benzoate (1.2 g, 3.77 mmol) in pyridine (2.44 mL, 30.2 mmol) and acetic acid (2.16 mL, 37.7 mmol) was added sodium phosphinate hydrate (0.8 g, 7.54 mmol) and water (5 mL). To the stirring solution, Raney nickel slurry in water (663 mg, 11.3 mmol) was added in multiple portions to avoid emulsion formation. The reaction was heated to 75°C overnight. The heating was stopped and the reaction was cooled down, diluted with MeOH, filtered through celite, and washed with MeOH. The filtrate was concentrated to remove pyridine and methanol. The residue was dissolved in ethyl acetate and washed with water. The organic phase was dried over Na₂SO₄, filtered and concentrated. The product was purified by FCC (0% to 35% EA/Heptanes) to obtain the title compound (742 mg, 2.31 mmol, 61% yield).

### Step 5: methyl (S)-5-amino-4-(6-(4-(dimethoxymethyl)piperidin-1-yl)-1-oxoisoindolin-2-yl)-5-oxopentanoate

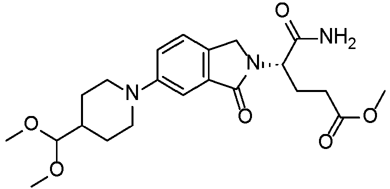

To a solution of methyl 5-[4-(dimethoxymethyl)piperidin-1-yl]-2-formylbenzoate (450.0 mg, 1.4 mmol) in DCM (2 mL) and DMF (2 mL) was added N,N-diisopropylethylamine (0.61 mL, 3.5 mmol) and methyl (*S*)-4,5-diamino-5-oxopentanoate (269 mg, 1.68 mmol). The reaction was stirred at rt for 1h and acetic acid (0.8 mL, 14. mmol) was added to this solution. The reaction was further stirred for another hour and sodium triacetoxyborohydride (890 mg, 4.2 mmol) was added, and the reaction was stirred over the weekend. The reaction was stopped, diluted with DCM (50 mL), and quenched by saturated NaHCO₃ solution dropwise until pH of 8-9 was maintained. The organic phase was collected, dried over Na₂SO₄, filtered and concentrated. The residue was purified by FCC (0% to 100% EA/Heptanes) to obtain the title compound (400 mg, 0.93 mmol, 66% yield).

### Step 6: (S)-3-(6-(4-(dimethoxymethyl)piperidin-1-yl)-1-oxoisoindolin-2-yl)piperidine-2,6-dione

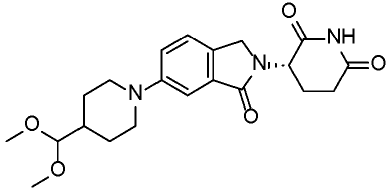

A 1.0 M solution of potassium t-butoxide (8.30 mL, 8.30 mmol) in THF was added to a solution of methyl (*S*)-5-amino-4-(6-(4-(dimethoxymethyl)piperidin-1-yl)-1-oxoisoindolin-2-yl)-5-oxopentanoate (3.00 g, 6.92 mmol) in THF (80.0 mL) at -78 °C and the reaction was stirred for 3 hours. The reaction was warmed to 0 °C and the pH was carefully adjusted to ~ 3 using a 1N aqueous HCl solution. To this solution, a saturated aqueous NaHCO₃ was carefully added dropwise to adjust the pH ~ 6. DCM was added and the phases were separated. The combined organics were washed with water, dried over MgSO₄, filtered and concentrated under reduced pressure. The crude material was triturated with DCM (5.00 mL) followed by MTBE (20.0 mL) upon which a white precipitate crashed out. The solids were filtered and dried via vacuum filtration to obtain 3-(6-(4-(dimethoxymethyl)piperidin-1-yl)-1-oxoisoindolin-2-yl)piperidine-2,6-dione (1.89 g, 68% yield). ¹H NMR (300 MHz, DMSO) δ 10.97 (s, 1H), 7.41 (d, *J =* 8.4 Hz, 1H), 7.25 (dd, *J =* 8.5, 2.3 Hz, 1H), 7.14 (d, *J* = 2.2 Hz, 1H), 5.10 (dd, *J =* 13.3, 5.1 Hz, 1H), 4.33 (d, *J=* 16.8 Hz, 1H), 4.19 (d, *J =* 16.7 Hz, 1H), 4.08 (d, *J =* 6.6 Hz, 1H), 3.77 (d, *J =* 12.3 Hz, 2H), 3.27 (s, 6H), 2.98 - 2.82 (m, 1H), 2.65 (dd, *J=* 29.4, 15.4 Hz, 3H), 2.38 (qd, *J =* 13.3, 4.4 Hz, 1H), 2.04 - 1.92 (m, 1H), 1.72 (d, *J =* 10.8 Hz, 3H), 1.34 (dt, *J =* 21.6, 10.9 Hz, 2H).

### Step 7: (S)-1-(2-(2,6-dioxopiperidin-3-yl)-3-oxoisoindolin-5-yl)piperidine-4-carbaldehyde

2,2,2-Trifluoroacetic acid (2.38 mL, 31.1 mmol) was added to a solution of (S)-3-(6-(4-(dimethoxymethyl)piperidin-1-yl)-1-oxoisoindolin-2-yl)piperidine-2,6-dione (500 mg, 1.25 mmol) in a 3 to 1 mixture of DCM (9.3 mL) and acetone (3.1 mL). The reaction was stirred overnight and concentrated under reduced pressure to obtain the title compound (440 mg, quantitative yield) as the TFA salt. LCMS calcd for C₁₉H₂₂N₃O₄ (M+H)⁺ m/z = 356.2; Found: 356.2.

### Intermediate 38: 1-(2-(2,6-dioxopiperidin-3-yl)-3-oxoisoindolin-5-yl)piperidine-4-carbaldehyde

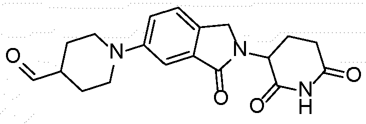

Trifluoroacetic acid (2.38 mL, 31.1 mmol) was added to a stirring solution of 3-(6-(4-(dimethoxymethyl)piperidin-1-yl)-1-oxoisoindolin-2-yl)piperidine-2,6-dione (500 mg, 1.25 mmol) in DCM (9.3 mL) and acetone (3.1 mL) at room temperature. The reaction mixture was stirred 48 hours at room temperature. The product mixture was concentrated. The residue obtained was dissolved in DCM (100 mL) and transferred to a separatory funnel containing saturated sodium carbonate aqueous solution (50 mL) and saturated sodium bicarbonate aqueous solution (50 mL). The aqueous layer was extracted with DCM (3 x 100 mL). The combined organic layers were dried over Na₂SO₄, filtered and concentrated under reduced pressure to obtain 1-(2-(2,6-dioxopiperidin-3-yl)-3-oxoisoindolin-5-yl)piperidine-4-carbaldehyde (440 mg, 99%) as a white solid. LCMS calcd for C₁₉H₂₂N₃O₄ [M+H]⁺: m/z = 356.2; Found: 356.1.

### Intermediate 39: (S)-3-(1-oxo-6-(piperazin-1-yl)isoindolin-2-yl)piperidine-2,6-dione

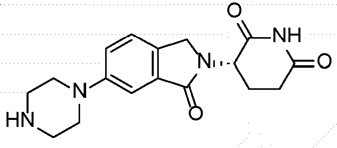

This compound was prepared by a procedure analogous to that described for Intermediate 37, utilizing *tert-butyl* piperazine-1-carboxylate instead of 4-piperidinemethanol in Step 1. LCMS calcd for C₁₇H₂₁N₄O₃ (M+2H-Boc)⁺ m/z = 329.2; Found: 329.1.

### Intermediate 40: (2,2-dimethylpiperazin-1-yl)(6a-ethyl-2-(3-fluoro-2-hydroxyphenyl)-5,6,6a,7,9,10-hexahydro-8H-pyrazino[1',2':4,5]pyrazino[2,3-c]pyridazin-8-yl)methanone (Isomer 1)

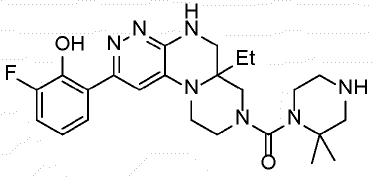

### Step 1. 1,4-di-tert-butyl 2-methyl 2-ethylpiperazine-1,2, 4-tricarboxylate

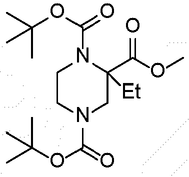

LiHMDS (1 M in THF, 5.3 mL, 5.3 mmol) was added to a solution of 1,4-di-tert-butyl 2-methyl (*S*)-piperazine-1,2,4-tricarboxylate (1.3 g, 3.77 mmol) in THF (19 mL) at -78 °C. The reaction mixture was stirred for 2 hours at -78 °C. Iodoethane (0.91 mL, 11.3 mmol) was added to the reaction mixture at -78 °C. The reaction mixture was stirred overnight and allowed to slowly warm to room temperature. The product mixture was quenched with saturated ammonium chloride aqueous solution (30 mL). The quenched product mixture was diluted with EtOAc (50 mL). The organic layer was washed with water (30 mL) and saturated sodium chloride solution (30 mL). The organic layer was dried over Na₂SO₄, filtered, and concentrated under reduced pressure. The residue obtained was purified by silica gel flash column chromatography with a gradient of 0-60% EtOAc/hexanes to obtain 1,4-di-tert-butyl 2-methyl 2-ethylpiperazine-1,2,4-tricarboxylate (1.2 g, 85%) as a clear oil. LCMS calcd for C₉H₁₇N₂O₄ [M+2H-C₉H₁₆O₂]⁺: m/z = 217.1; Found: 217.1.

### Step 2. 1,4-bis(tert-butoxycarbonyl)-2-ethylpiperazine-2-carboxylic acid

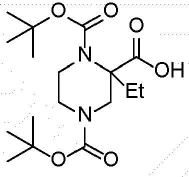

Lithium hydroxide (1.25 g, 52.3 mmol) was added to a stirring solution of *1,4-di-tert-*butyl 2-methyl 2-ethylpiperazine-1,2,4-tricarboxylate (1.3 g, 3.49 mmol) in THF (30 mL), methanol (10 mL), and water (10 mL). The reaction mixture was stirred overnight. The product mixture was diluted with 1 M HCl aqueous solution (70 mL). The diluted product mixture was extracted with DCM (2 x 100 mL). The combined organic layers were dried over Na₂SO₄, filtered, and concentrated under reduced pressure to obtain 1,4-bis(tert-butoxycarbonyl)-2-ethylpiperazine-2-carboxylic acid (1.24 g, 99%) as a clear oil. LCMS calcd for C₈H₁₅N₂O₄ [M+2H-C₉H₁₆O₂]⁺: m/z = 203.1; Found: 203.1.

### Step 3. Synthesis of tert-butyl 2-chloro-6a-ethyl-6-oxo-5,6,6a,7, 9,10-hexahydro-8H-pyrazino[1',2':4, 5]pyrazino[2,3-c]pyridazine-8-carboxylate

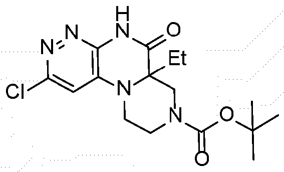

Oxalyl chloride (0.76 mL, 8.86 mmol) was added dropwise to a stirring solution of 1,4-bis(*tert*-butoxycarbonyl)-2-ethylpiperazine-2-carboxylic acid (1.27 g, 3.54 mmol) and dimethylformamide (270 µL, 3.5 mmol) in DCM (27 mL) at 0 °C. The reaction mixture was warmed to room temperature and stirred for 40 minutes. The reaction mixture was concentrated under reduced pressure then redissolved in DMF (8.1 mL). N,N-Diisopropylethylamine (3.1 mL, 17.7 mmol) and 4-bromo-6-chloropyridazin-3-amine (1.48 g, 7.09 mmol) were added in sequence to the stirring reaction mixture at room temperature. The reaction mixture was heated to 120 °C and stirred for 18 hours. The product mixture was diluted with EtOAc (80 mL) and washed with saturated sodium chloride aqueous solution (80 mL). The aqueous layer was extracted with EtOAc (2 x 80 mL). The combined organic layers were dried over Na₂SO₄, filtered, and concentrated under reduced pressure. The residue obtained was purified by silica gel flash column chromatography with a gradient of 0-100% EtOAc/hexanes to obtain *tert-butyl 2-*chloro-6a-ethyl-6-oxo-5,6,6a,7,9,10-hexahydro-8*H*-pyrazino[1',2':4,5]pyrazino[2,3-*c*]pyridazine-8-carboxylate (401 mg, 31%) as a white solid. LCMS calcd for C₁₆H₂₃ClN₅O₃ [M+H]⁺: m/z = 368.2; Found: 368.1.

### Step 4. Synthesis of di-tert-butyl 2-chloro-6a-ethyl-6-oxo-6a,7, 9,10-tetrahydro-5H-pyrazino[1',2':4,5]pyrazino[2,3-c]pyridazine-5,8(6H)-dicarboxylate

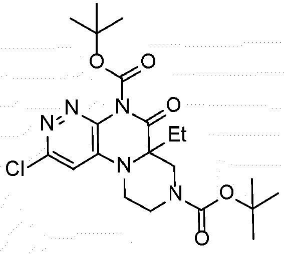

Di-*tert*-butyl decarbonate (470 µL, 2.07 mmol) and 4-(dimethylamino)pyridine (38 mg, 0.31 mmol) were added in sequence to a stirring solution of *tert-butyl* 2-chloro-6a-ethyl-6-oxo-5,6,6a,7,9,10-hexahydro-8*H*-pyrazino[1',2':4,5]pyrazino[2,3-c]pyridazine-8-carboxylate (380 mg, 1.03 mmol) in THF (10 mL) at room temperature. The reaction mixture was stirred for 5 hours. The product mixture was concentrated under reduced pressure. The residue obtained was purified by silica gel flash column chromatography with a gradient of 0-100% EtOAc/hexanes to obtain di-tert-butyl 2-chloro-6a-ethyl-6-oxo-6a,7,9,10-tetrahydro-5H-pyrazino[1',2':4,5]pyrazino[2,3-c]pyridazine-5,8(6H)-dicarboxylate (310 mg, 64%) as a tan foamy solid. LCMS calcd for C₂₁H₃₁ClN₅O₅ [M+H]⁺: m/z = 468.2; Found: 468.0.

### Step 5. Chiral Separation of di-tert-butyl 2-chloro-6a-ethyl-6-oxo-6a,7, 9,10-tetrahydro-5H-pyrazino[1',2':4,5]pyrazino[2,3-c]pyridazine-5,8(6H)-dicarboxylate

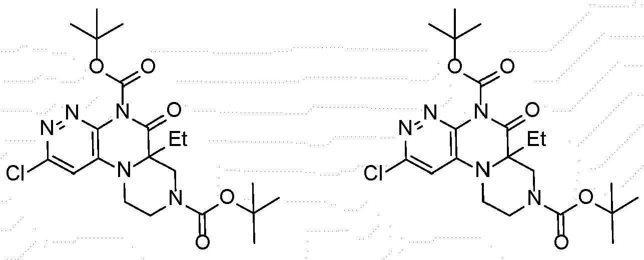

A racemic mixture of di-*tert-*butyl 2-chloro-6a-ethyl-6-oxo-6a,7,9,10-tetrahydro-5*H-*pyrazino[1',2':4,5]pyrazino[2,3-*c*]pyridazine-5,8(6H)-dicarboxylate was purified via chiral separation (310 mg) (Lux Cellulose-4, 25 mL/min of 55:22.5:22.5 Hexanes/IPA/MeOH) to give di-*tert*-butyl 2-chloro-6a-ethyl-6-oxo-6a,7,9,10-tetrahydro-5*H*-pyrazino[1',2':4,5]pyrazino[2,3-c]pyridazine-5,8(6H)-dicarboxylate (118 mg, Peak A, Isomer 1, 38%) and di-tert-butyl 2-chloro-6a-ethyl-6-oxo-6a,7,9,10-tetrahydro-5*H*-pyrazino[1',2':4,5]pyrazino[2,3-*c*]pyridazine-5,8(6H)-dicarboxylate (137 mg, Peak B, Isomer 2, 44%). LCMS calcd for C₂₁H₃₁ClN₅O₅ [M+H]⁺: m/z = 468.2; Found: 468.1.

### Step 6. tert-butyl 6a-ethyl-2-(3-fluoro-2-hydroxyphenyl)-6-oxo-5, 6, 6a,7,9,10-hexahydro-8H-pyrazino[1',2':4, 5]pyrazino[2,3-c]pyridazine-8-carboxylate (Isomer 1)

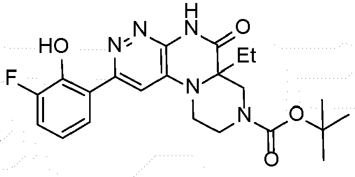

A 20 mL scintillation vial was charged with di-tert-butyl 2-chloro-6a-ethyl-6-oxo-6a,7,9,10-tetrahydro-5*H*-pyrazino[1',2':4,5]pyrazino[2,3-*c*]pyridazine-5,8(6H)-dicarboxylate (Isomer 1) (227 mg, 0.49 mmol), cesium carbonate (474 mg, 1.46 mmol), XPhos Pd G2 (57.3 mg, 0.073 mmol), and 3-fluoro-2-hydroxyphenylboronic acid (151 mg, 0.97 mmol). The mixture was dissolved in 1,4-dioxane (4 mL) and water (0.5 mL). The reaction mixture was sparged with N₂ gas for 2 minutes, sealed, and heated to 80 °C. The reaction mixture was stirred for 2 hours at 80°C. The product mixture was diluted with EtOAc (50 mL) and washed with water (100 mL). The aqueous layer was extracted with EtOAc (2 x 100 mL). The combined organic layers were dried over Na₂SO₄, filtered, and concentrated under reduced pressure. The resulting residue was purified by silica gel flash column chromatography with a gradient of 0-30% acetone/hexanes to obtain *tert-butyl* 6a-ethyl-2-(3-fluoro-2-hydroxyphenyl)-6-oxo-5,6,6a,7,9,10-hexahydro-8H-pyrazino[1',2':4,5]pyrazino[2,3-c]pyridazine-8-carboxylate (Isomer 1) (130 mg, 60%).

### Step 7. 6a-ethyl-2-(3-fluoro-2-hydroxyphenyl)-7,8,9,10-tetrahydro-5H-pyrazino[1',2':4,5] pyrazino[2,3-c]pyridazin-6(6aH)-one (Isomer 1)

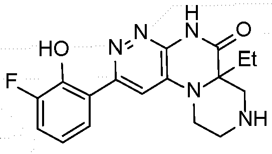

Hydrochloric acid (4 M in dioxane, 789 µL, 3.16 mmol) was added to a stirring solution of *tert*-butyl 6a-ethyl-2-(3-fluoro-2-hydroxyphenyl)-6-oxo-5,6,6a,7,9,10-hexahydro-8H-pyrazino[1',2':4,5]pyrazino[2,3-c]pyridazine-8-carboxylate (Isomer 1) (140 mg, 0.316 mmol) in DCM (8 mL). The reaction mixture was stirred overnight. The product mixture was concentrated under reduced pressure to obtain the HCl salt of 6a-ethyl-2-(3-fluoro-2-hydroxyphenyl)-7,8,9,10-tetrahydro-5*H*-pyrazino[1',2':4,5]pyrazino[2,3-*c*]pyridazin-6(6aH)-one (Isomer 1) (120 mg, 100%). LCMS calcd for C₁₇H₁₉FN₅O₂ [M+H]⁺: m/z = 344.2; Found: 344.1.

### Step 8. tert-butyl 4-(6a-ethyl-2-(3-fluoro-2-hydroxyphenyl)-6-oxo-6,6a,7,8,9,10-hexahydro-5H pyrazino[1',2':4,5]pyrazino[2, 3-c]pyridazine-8-carbonyl)-3,3-dimethylpiperazine-1-carboxylate (Isomer 1)

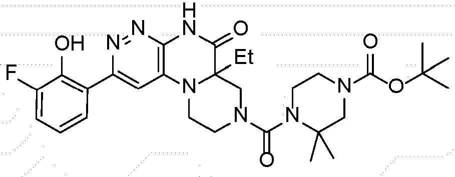

*N,N*-Diisopropylethylamine (220 µL, 1.26 mmol) and 4-(dimethylamino)pyridine (11.6 mg, 0.095 mmol) were added to a stirring solution of 6a-ethyl-2-(3-fluoro-2-hydroxyphenyl)-7,8,9,10-tetrahydro-5*H*-pyrazino[1',2':4,5]pyrazino[2,3-*c*]pyridazin-6(6aH)-one (120 mg, 0.316 mmol) in dimethylacetamide (3 mL) at room temperature. The reaction mixture was stirred 15 minutes. A solution of *tert-*butyl 4-(chlorocarbonyl)-3,3-dimethylpiperazine-1-carboxylate (131 mg, 0.474 mmol) in dimethylacetamide (1 mL) was added to the reaction mixture. The reaction mixture was stirred 30 minutes at room temperature. The product mixture was diluted with EtOAc (50 mL) washed with saturated sodium bicarbonate aqueous solution (50 mL). The aqueous layer was extract with EtOAc (2 x 50 mL). The combined organic layers were dried over Na₂SO₄, filtered, and concentrated under reduced pressure. The residue obtained was purified by silica gel flash column chromatography with a gradient of 0-100% EtOAc/hexanes to obtain *tert-*butyl 4-(6a-ethyl-2-(3-fluoro-2-hydroxyphenyl)-6-oxo-6,6a,7,8,9,10-hexahydro-5*H-*pyrazino[1',2':4,5]pyrazino[2,3-*c*]pyridazine-8-carbonyl)-3,3-dimethylpiperazine-1-carboxylate as a yellow oil (170 mg, 92%). LCMS calcd for C₂₀H₃₀FN₇O₅ [M+H]⁺: m/z = 584.3; Found: 584.2.

### Step 9. tert-butyl 4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazine-1-carboxylate (Isomer 1)

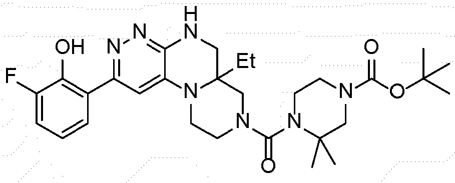

Borane tetrahydrofuran complex (1 M in THF, 2.33 mL, 2.33 mmol) was added to a stirring solution of tert-butyl 4-(6a-ethyl-2-(3-fluoro-2-hydroxyphenyl)-6-oxo-6,6a,7,8,9,10-hexahydro-5*H*-pyrazino[1',2':4,5]pyrazino[2,3-*c*]pyridazine-8-carbonyl)-3,3-dimethylpiperazine-1-carboxylate in THF (9 mL) at room temperature. The reaction mixture was heated to 60 °C and stirred for 5 hours. The reaction mixture was cooled to room temperature and borane tetrahydrofuran complex (1 M in THF, 2.33 mL, 2.33 mmol) was added to the reaction mixture. The reaction mixture was heated to 60 °C and stirred for 3 hours. The product mixture was cooled to 0 °C and quenched by slow addition of MeOH (6 mL). The quenched product mixture was heated to 70 °C and stirred for 3 hours. The product mixture was concentrated under reduced pressure. The residue obtained was purified by silica gel flash column chromatography with a gradient of 0-10% MeOH/DCM to obtain tert-butyl 4-(6a-ethyl-2-(3-fluoro-2-hydroxyphenyl)-6,6a,7,8,9,10-hexahydro-5*H*-pyrazino[1',2':4,5]pyrazino[2,3-*c*]pyridazine-8-carbonyl)-3,3-dimethylpiperazine-1-carboxylate (Isomer 1) (121 mg, 73%). LCMS calcd for C₂₉H₄₁FN₇O₄ [M+H]⁺: m/z = 570.3; Found: 570.3

### Step 10. (2,2-dimethylpiperazin-1-yl)(6a-ethyl-2-(3-fluoro-2-hydroxyphenyl)-5,6,6a,7,9,10-hexahydro-8H-pyrazino[1',2':4,5]pyrazino[2,3-c]pyridazin-8-yl)methanone (Isomer 1)

Trifluoroacetic acid (403 µL, 5.3 mmol) was added to a stirring solution of tert-butyl 4-(6a-ethyl-2-(3-fluoro-2-hydroxyphenyl)-6,6a,7,8,9,10-hexahydro-5*H*-pyrazino[1',2':4,5]pyrazino [2,3-c]pyridazine-8-carbonyl)-3,3-dimethylpiperazine-1-carboxylate (120 mg, 0.211 mmol) in DCM (3 mL) at room temperature. The reaction mixture was stirred 1 hour. The product mixture was concentrated under reduced pressure to obtain the TFA salt of (2,2-dimethylpiperazin-1-yl)(6a-ethyl-2-(3-fluoro-2-hydroxyphenyl)-5,6,6a,7,9,10-hexahydro-8*H*-pyrazino[1',2':4,5] pyrazino[2,3-c]pyridazin-8-yl)methanone (147 mg, 100%). LCMS calcd for C₂₄H₃₃FN₇O₂ [M+H]⁺: m/z = 470.3; Found: 470.1

### Intermediates 41 - 53

The intermediates shown below in Table 5 were prepared by methods analogous to that described for the preparing Intermediate 40 using the appropriate starting materials.

**Table 5. Intermediates 41 - 53**

| **Int.** | **Structure** | **Name** | **Calcd. (M+H)⁺ m/z** | **Found (M+H)⁺ m/z** |
|---|---|---|---|---|
| **41** | 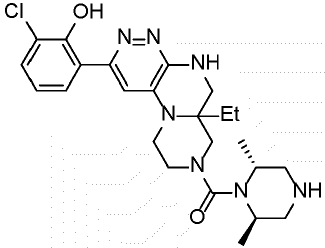 | (2-(3-chloro-2-hydroxyphenyl)-6a-ethyl-5,6,6a,7,9,10-hexahydro-8H-pyrazino [1',2':4,5]pyrazino[2,3-*c*] pyridazin-8-yl)((2*R*,6*R*)-2,6-dimethylpiperazin-1-yl) methanone (Isomer 2) | 486.2 | 486.0 |
| **42** | 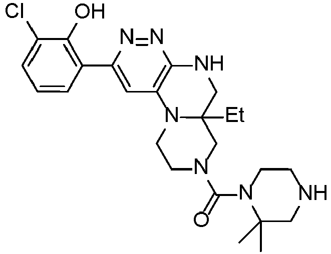 | (2-(3-chloro-2-hydroxyphenyl)-6a-ethyl-5,6,6a,7,9,10-hexahydro-8H-pyrazino [1',2':4,5]pyrazino[2,3-*c*] pyridazin-8-yl)(2,2-dimethyl-piperazin-1-yl)methanone (Isomer 1) | 486.2 | 486.0 |
| **43** | 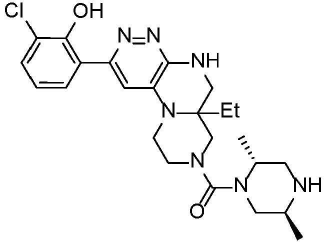 | (2-(3-chloro-2-hydroxyphenyl)-6a-ethyl-5,6,6a,7,9,10-hexahydro-8H-pyrazino [1',2':4,5]pyrazino[2,3-*c*] pyridazin-8-yl)((2*R*,5*S*)-2,5-dimethylpiperazin-1-yl) methanone (Isomer 2) | 486.2 | 486.1 |
| **44** | 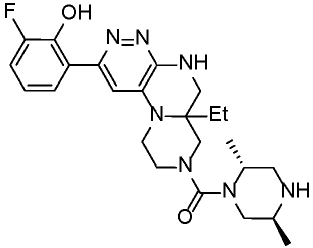 | ((2R,5S)-2,5-dimethyl-piperazin-1-yl)(6a-ethyl-2-(3-fluoro-2-hydroxyphenyl)-5,6,6a,7,9,10-hexahydro-8*H-*pyrazino[1',2':4,5]pyrazino[2,3-c]pyridazin-8-yl)methanone (Isomer 2) | 470.3 | 470.1 |
| **45** | 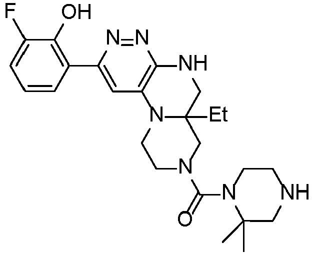 | (2,2-dimethylpiperazin-1-yl)(6a-ethyl-2-(3-fluoro-2-hydroxyphenyl)-5,6,6a, 7,9,10-hexahydro-8H-pyrazino [1',2':4,5]pyrazino[2,3-*c*] pyridazin-8-yl)methanone (Isomer 2) | 470.3 | 470.1 |
| **46** | 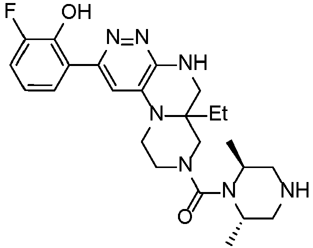 | ((2S,6S)-2,6-dimethyl-piperazin-1-yl)(6a-ethyl-2-(3-fluoro-2-hydroxyphenyl)-5,6,6a,7,9,10-hexahydro-8*H-*pyrazino[1',2':4,5]pyrazino[2,3-c]pyridazin-8-yl)methanone (Isomer 2) | 470.3 | 470.1 |
| **47** | 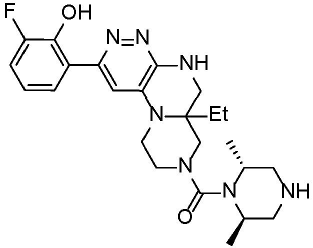 | ((2R,6R)-2,6-dimethyl-piperazin-1-yl)(6a-ethyl-2-(3-fluoro-2-hydroxyphenyl)-5,6,6a,7,9,10-hexahydro-8*H-*pyrazino[1',2':4,5]pyrazino[2,3-c]pyridazin-8-yl)methanone (Isomer 2) | 470.3 | 470.1 |
| **48** | 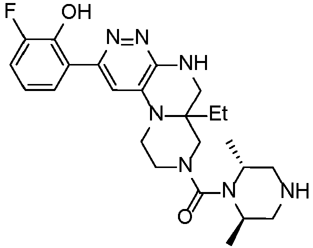 | ((2R,6R)-2,6-dimethyl-piperazin-1-yl)(6a-ethyl-2-(3-fluoro-2-hydroxyphenyl)-5,6,6a,7,9,10-hexahydro-8*H-*pyrazino[1',2':4,5]pyrazino[2,3-*c]-* 103 -yridazine-8-yl)methanone (Isomer 1) | 470.3 | 470.2 |
| **49** | 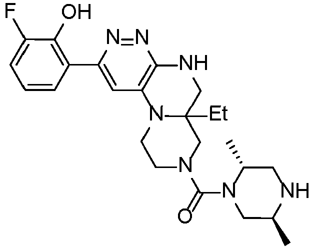 | ((2R,5S)-2,5-dimethyl-piperazin-1-yl)(6a-ethyl-2-(3-fluoro-2-hydroxyphenyl)-5,6,6a,7,9,10-hexahydro-8*H-*pyrazino[1',2':4,5]pyrazino[2,3-*c*]pyridazin-8-yl)methanone (Isomer 1) | 470.3 | 470.1 |
| **50** | 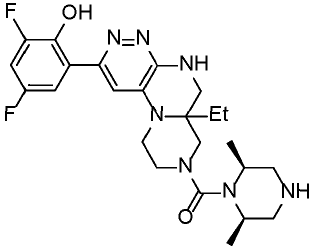 | (2-(3,5-difluoro-2-hydroxyphenyl)-6a-ethyl-5,6,6a,7,9,10-hexahydro-8H-pyrazino[1',2':4,5]pyrazino[2,3-*c*]pyridazin-8-yl)((2*R*,6*S*)-2,6-dimethylpiperazin-1-yl)methanone (Isomer 2) | 488.3 | 488.1 |
| **51** | 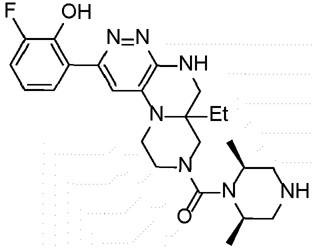 | ((2*R*,6*S*)-2,6-dimethylpiperazin-1-yl)(6a-ethyl-2-(3 -fluoro-2-hydroxyphenyl)-5,6,6a,7,9,10-hexahydro-8*H*-pyrazino [1',2':4,5]pyrazino[2,3-*c*] pyridazin-8-yl)methanone (Isomer 2) | 470.3 | 470.1 |
| **52** | 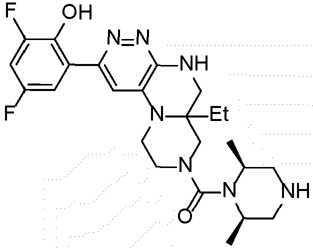 | (2-(3,5-difluoro-2-hydroxyphenyl)-6a-ethyl-5,6,6a, 7,9,10-hexahydro-8H-pyrazino [1',2':4,5]pyrazino[2,3-*c*]pyridazin-8-yl)((2*R*,6*S*)-2,6-dimethylpiperazin-1-yl) methanone (Isomer 1) | 488.3 | 488.1 |
| **53** | 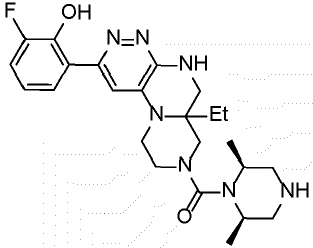 | ((2*R*,6*S*)-2,6-dimethylpiperazin-1-yl)(6a-ethyl-2-(3 -fluoro-2-hydroxyphenyl)-5,6,6a, 7,9,10-hexahydro-8*H*-pyrazino [1',2':4,5]pyrazino[2,3-*c*] pyridazin-8-yl)methanone (Isomer 1) | 470.3 | 470.1 |

### Intermediate 54: (2,2-dimethylpiperazin-1-yl)(2-(3-fluoro-2-hydroxyphenyl)-6a-(fluoromethyl)-5,6,6a,7,9,10-hexahydro-8H-pyrazino[1',2':4,5]pyrazino[2,3-c]pyridazin-8-yl)methanone (Isomer 2)

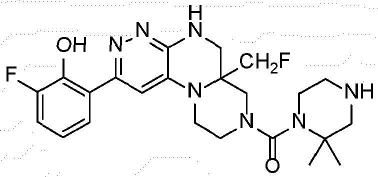

### Step 1. Synthesis of 1,4-di-tert-butyl 2-methyl 2-(fluoromethyl)piperazine-1, 2, 4-tricarboxylate

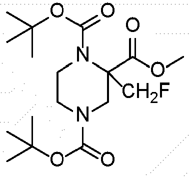

LiHMDS (1 M in THF, 8.97 mL, 8.97 mmol) was added to a solution of 1,4-di*-tert-*butyl 2-methyl (S)-piperazine-1,2,4-tricarboxylate (2.06 g, 5.98 mmol) in THF (24 mL) at -78 °C. The reaction was stirred for 1.5 h at -78 °C. Fluoroiodomethane (1.2 mL, 12 mmol) was added and the reaction was allowed to slowly warm to room temperature and stirred overnight. The reaction mixture was quenched with a saturated ammonium chloride aqueous solution (60 mL) and extracted with EtOAc (2 x 60 mL). The combined organics were dried over Na₂SO₄, filtered, and concentrated under reduced pressure. The crude material was purified by silica gel flash column chromatography with a gradient of 0-30% EtOAc/hexanes to give 1,4-di-*tert*-butyl 2-methyl 2-(fluoromethyl)piperazine-1,2,4-tricarboxylate as an oil (1.98 g, 88%). LCMS calcd for C₈H₁₄FN₂O₄ [M+2H-C₉H₁₆O₂]⁺: m/z = 221.1; Found: 221.1

### Step 2. Synthesis of 1,4-bis(tert-butoxycarbonyl)-2-(fluoromethyl)piperazine-2-carboxylic acid

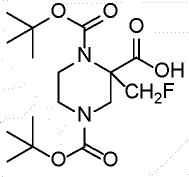

Lithium hydroxide (2.97 g, 70.8 mmol) was added to a solution of 1,4-di-*tert-*butyl 2-methyl 2-(fluoromethyl)piperazine-1,2,4-tricarboxylate (2.8 g, 7.54 mmol) in THF (25 mL), methanol (25 mL), and water (25 mL) at room temperature. The reaction mixture was heated to 55 °C and stirred overnight. The reaction mixture was quenched with a 1 M HCl solution (75 mL). The product mixture was transferred to a separatory funnel and extracted with DCM (2 x 100 mL). The combined organic layers were dried over Na₂SO₄, filtered, and concentrated under reduced pressure. The resulting residue was used in the next step without purification. 1,4-bis(tert-butoxycarbonyl)-2-(fluoromethyl)piperazine-2-carboxylic acid (2.7 g, 99%) was obtained as a yellow oil. LCMS calcd for C₇H₁₂FN₂O₄ [M+2H-C₉H₁₆O₂]⁺: m/z = 207.1 ; Found: 207.0

### Step 3. di-tert-butyl 2-chloro-6a-(fluoromethyl)-6-oxo-6a, 7,9,10-tetrahydro-5H-pyrazino[1',2':4,5]pyrazino[2,3-c]pyridazine-5,8(6H)-dicarboxylate

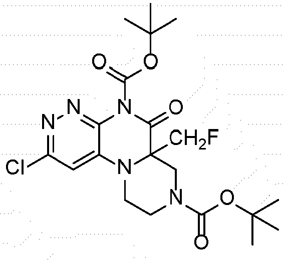

Oxalyl chloride (831 µL, 9.69 mmol) was added dropwise to a stirring solution of DMF (750 µL, 9.69 mmol) and DCM (16 mL) at 0 °C. The reaction mixture was stirred for 10 minutes at 0 °C. Then a solution of 1,4-bis(*tert*-butoxycarbonyl)-2-(fluoromethyl)piperazine-2-carboxylic acid (2.7 g, 7.45 mmol) and pyridine (904 µL, 11.2 mmol) in DCM (5 mL) was added to the reaction mixture at 0 °C. After the addition the reaction mixture was stirred for 30 minutes at 0 °C. Then the reaction mixture was diluted with DCM (30 mL), transferred to a separatory funnel, and washed with water (2 x 30 mL) and a saturated sodium chloride aqueous solution (30 mL). The organic layer was dried over Na₂SO₄, filtered, and concentrated under reduced pressure. The resulting residue was dissolved in DMF (11 mL), and N,N-diisopropylethylamine (3.89 mL, 22.4 mmol) and 4-bromo-6-chloropyridazin-3-amine (1.4 g, 6.71 mmol) were added sequentially. The resulting mixture was stirred at 120 °C overnight. The product mixture was diluted with EtOAc (150 mL) and washed with a saturated sodium chloride aqueous solution (2 x 200 mL). The organic layer was dried over Na₂SO₄, filtered, and concentrated under reduced pressure. The resulting residue was purified by silica gel flash column chromatography with a gradient of 0-100% EtOAc/hexanes to obtain *tert-butyl* 2-chloro-6a-(fluoromethyl)-6-oxo-5,6,6a,7,9,10-hexahydro-8H-pyrazino[1',2':4,5]pyrazino[2,3-c]pyridazine-8-carboxylate as a mixture with 4-bromo-6-chloropyridazin-3-amine. The mixture was dissolved in THF (20 mL), and di*-tert-*butyl decarbonate (1.7 mL, 7.45 mmol) and 4-(dimethylamino)pyridine (120 mg, 0.97 mmol) were added sequentially. The reaction mixture was stirred for 5 hours. The product mixture was concentrated under reduced pressure. The resulting residue was purified by silica gel flash column chromatography with a gradient of 0-100% EtOAc/hexanes to obtain di-tert-butyl 2-chloro-6a-(fluoromethyl)-6-oxo-6a,7,9,10-tetrahydro-5H-pyrazino[1',2':4,5]pyrazino[2,3-c]pyridazine-5,8(6H)-dicarboxylate as a pale yellow foamy solid (465 mg, 13.2%). LCMS calcd for C₂₀H₂₈ClFN₅O₅ [M+H]⁺: m/z = 472.2; Found: 472.0

### Step 4. tert-butyl 2-(3-fluoro-2-methoxyphenyl)-6a-(fluoromethyl)-6-oxo-5,6,6a, 7,9,10-hexahydro-8H-pyrazino[1'2':4,5]pyrazino[2,3-c]pyridazine-8-carboxylate

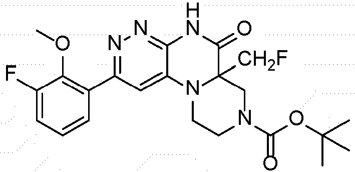

A 20 mL scintillation vial was charged with di-tert-butyl 2-chloro-6a-(fluoromethyl)-6-oxo-6a,7,9,10-tetrahydro-5H-pyrazino[1',2':4,5]pyrazino[2,3-c]pyridazine-5,8(6H)-dicarboxylate (Isomer 1) (465 mg, 0.985 mmol), cesium carbonate (963 mg, 2.96 mmol), XPhos Pd G2 (77.5 mg, 0.01 mmol), and 3-fluoro-2-methoxyphenylboronic acid (385 mg, 2.27 mmol). The mixture was dissolved in 1,4-dioxane (5.8 mL) and water (0.7 mL). The reaction mixture was sparged with N₂ gas for 2 minutes, sealed, and heated to 80°C. The reaction mixture was stirred for 2 hours at 80°C. The product mixture was diluted with EtOAc (50 mL) and washed with water (100 mL). The aqueous layer was extracted with EtOAc (2 x 100 mL). The combined organic layers were dried over Na₂SO₄, filtered, and concentrated under reduced pressure. The resulting residue was purified by silica gel flash column chromatography with a gradient of 0-100% EtOAc/hexanes to obtain *tert*-butyl 2-(3-fluoro-2-methoxyphenyl)-6a-(fluoromethyl)-6-oxo-5,6,6a,7,9,10-hexahydro-8*H*-pyrazino[1',2':4,5]pyrazino[2,3-c]pyridazine-8-carboxylate (332 mg, 73%). LCMS calcd for C₂₂H₂₆F₂N₅O₄ [M+H]⁺: m/z = 462.2; Found: 462.1

### Step 5. di-tert-butyl 2-(3-fluoro-2-methoxyphenyl)-6a-(fluoromethyl)-6a, 7,9,10-tetrahydro-5H-pyrazino[1',2':4,5]pyrazino[2,3-c]pyridazine-5,8(6H)-dicarboxylate

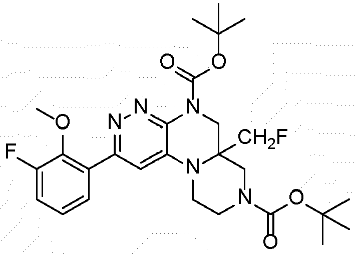

Borane tetrahydrofuran complex (1 M, 2.97 mL, 2.97 mmol) was added to a solution of *tert*-butyl 2-(3-fluoro-2-methoxyphenyl)-6a-(fluoromethyl)-6-oxo-5,6,6a,7,9,10-hexahydro-8H-pyrazino[1',2':4,5]pyrazino[2,3-c]pyridazine-8-carboxylate (342 mg, 0.742 mmol) in THF (5 mL) at room temperature. The reaction mixture was heated to 60 °C. The reaction mixture was stirred at 60 °C for 2 hours. The reaction mixture was cooled to 0 °C and quenched with MeOH (3 mL) slowly. The reaction mixture was diluted with EtOAc (60 mL). The diluted reaction mixture was washed with saturated sodium bicarbonate aqueous solution (2 x 60 mL). The organic layer was dried over Na₂SO₄, filtered, and concentrated under reduced pressure. The resulting residue was dissolved in THF (5 mL) and MeOH (2 mL). Acetic acid (1.27 mL, 22.3 mmol) and sodium cyanoborohydride (466 mg, 7.42 mmol) were added sequentially to the reaction mixture at room temperature. The reaction mixture was refluxed at 80 °C for 16 hours. The product mixture was cooled to room temperature and diluted with EtOAc (100 mL). The diluted reaction mixture was washed with saturated sodium bicarbonate aqueous solution (2 x 100 mL). The organic layer was dried over Na₂SO₄, filtered, and concentrated under reduced pressure. The resulting residue dissolved in THF (5 mL). Di-tert-butyl dicarbonate (340 µL, 1.48 mmol) and 4-(dimethylamino) pyridine (11 mg, 0.09 mmol) were added sequentially. The reaction mixture was stirred 2 hours at room temperature. The product mixture was concentrated under reduced pressure. The resulting residue was purified by silica gel flash column chromatography with a gradient of 0-100% EtOAc/hexanes to obtain di-tert-butyl 2-(3-fluoro-2-methoxyphenyl)-6a-(fluoromethyl)-6a,7,9,10-tetrahydro-5*H*-pyrazino[1',2':4,5]pyrazino[2,3-*c*]pyridazine-5,8(6*H*)-dicarboxylate as a dark oil (270 mg, 66%). LCMS calcd for C₂₇H₃₆F₂N₅O₅ [M+H]⁺: m/z = 548.3; Found: 548.2

### Step 6. Chiral Separation of di-tert-butyl 2-(3-fluoro-2-methoxyphenyl)-6a-(fluoromethyl)-6a,7,9,10-tetrahydro-5H-pyrazino[1',2':4,5]pyrazino[2,3-c]pyridazine-5,8(6H)-dicarboxylate

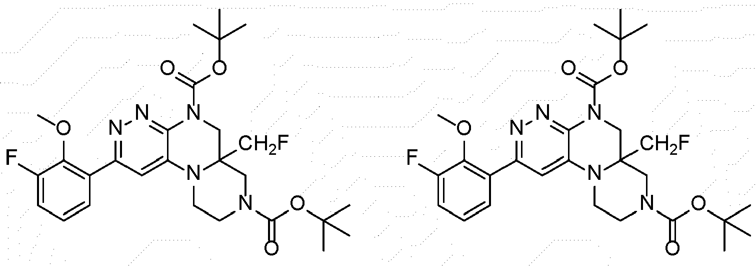

A racemic mixture of di-tert-butyl 2-(3-fluoro-2-methoxyphenyl)-6a-(fluoromethyl)-6a,7,9,10-tetrahydro-5*H*-pyrazino[1',2':4,5]pyrazino[2,3-*c*]pyridazine-5,8(6H)-dicarboxylate (270 mg) was purified via chiral separation (Lux Cellulose-1, 30 mL/min of 65:17.5:17.5 Hexanes/IPA/MeOH) to give di-tert-butyl 2-(3-fluoro-2-methoxyphenyl)-6a-(fluoromethyl)-6a,7,9,10-tetrahydro-5*H*-pyrazino[1',2':4,5]pyrazino[2,3-*c*]pyridazine-5,8(6H)-dicarboxyl (98 mg, Peak A, Isomer 1, 36%) and di-tert-butyl 2-(3-fluoro-2-methoxyphenyl)-6a-(fluoromethyl)-6a,7,9,10-tetrahydro-5H-pyrazino[1',2':4,5]pyrazino[2,3-c]pyridazine-5,8(6H)-dicarboxyl (111 mg, Peak B, Isomer 2, 41%). LCMS calcd for C₂₇H₃₆F₂N₅O₅ [M+H]⁺: m/z = 548.3; Found: 548.2

### Step 7. 2-(3-fluoro-2-methoxyphenyl)-6a-(fluoromethyl)-6,6a, 7,8,9,10-hexahydro-5H-pyrazino[1',2':4,5]pyrazino[2,3-c]pyridazine (Isomer 2)

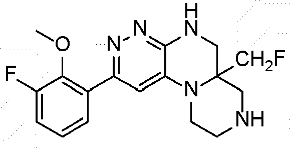

Hydrochloric acid (4 M in 1,4-dioxane, 0.52 mL, 2.09 mmol) was added to a solution of tert-butyl 2-(3-fluoro-2-methoxyphenyl)-6a-(fluoromethyl)-5,6,6a,7,9,10-hexahydro-8H-pyrazino[1',2':4,5]pyrazino[2,3-c]pyridazine-8-carboxylate (Isomer 2) (62.1 mg, 0.14 mmol) in DCM (2.8 mL) at room temperature. The reaction was stirred overnight. The product mixture was concentrated under reduced pressure to obtain the HCl salt of 2-(3-fluoro-2-methoxyphenyl)-6a-(fluoromethyl)-6,6a,7,8,9,10-hexahydro-5*H*-pyrazino[1',2':4,5]pyrazino[2,3-*c*]pyridazine (Isomer 2) as a tan solid (58 mg, 100%). LCMS calcd for C₁₇H₂₀F₂N₅O [M+H]⁺: m/z = 348.2; Found: 348.0

### Step 8. tert-butyl 4-(2-(3-fluoro-2-methoxyphenyl)-6a-(fluoromethyl)-6,6a, 7,8,9,10-hexahydro-5H-pyrazino[1 ', 2': 4, 5]pyrazino[2, 3-c]pyridazine-8-carbonyl)-3, 3-dimethylpiperazine-1-carboxylate (Isomer 2)

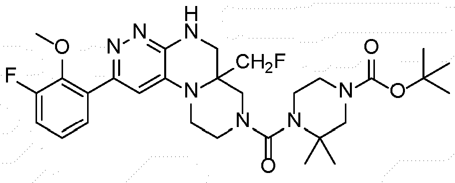

N,N-Diisopropylethylamine (98 µL, 0.56 mmol) and 4-(dimethylamino)pyridine (5.2 mg, 0.04 mmol) was added to a stirring solution of 2-(3-fluoro-2-methoxyphenyl)-6a-(fluoromethyl)-6,6a,7,8,9,10-hexahydro-5H-pyrazino[1',2':4,5]pyrazino[2,3-c]pyridazine (Isomer 2) (59 mg, 0.14 mmol) in dimethylacetamide (2 mL) at room temperature. The reaction mixture was stirred 15 minutes. A solution of *tert-butyl* 4-(chlorocarbonyl)-3,3-dimethylpiperazine-1-carboxylate (58.7 mg, 0.21 mmol) in dimethylacetamide (1 mL) was added to the reaction mixture. The reaction mixture was stirred 30 minutes at room temperature. The product mixture was diluted with EtOAc (30 mL) and washed with saturated sodium bicarbonate aqueous solution (30 mL). The aqueous layer was extract with EtOAc (2 x 30 mL). The combined organic layers were dried over Na₂SO₄, filtered, and concentrated under reduced pressure. The resulting residue was purified by silica gel flash column chromatography with a gradient of 0-15% MeOH/DCM to obtain *tert*-butyl 4-(2-(3-fluoro-2-methoxyphenyl)-6a-(fluoromethyl)-6,6a,7,8,9,10-hexahydro-5*H*-pyrazino[1',2':4,5]pyrazino[2,3-*c*]pyridazine-8-carbonyl)-3,3-dimethylpiperazine-1-carboxylate (Isomer 2) (78 mg, 91%). LCMS calcd for C₂₉H₄₀F₂N₇O₄ [M+H]⁺: m/z = 588.3; Found: 588.2.

### Intermediate 55: (2,2-dimethylpiperazin-1-yl)(2-(3-fluoro-2-hydroxyphenyl)-6a-(fluoromethyl)-5,6,6a,7,9,10-hexahydro-8H-pyrazino[1',2':4,5]pyrazino[2,3-c]pyridazin-8-yl)methanone (Isomer 2)

Boron tribromide (119 µL, 1.25 mmol) was added to a stirring solution of *tert-butyl* 4-(2-(3-fluoro-2-methoxyphenyl)-6a-(fluoromethyl)-6,6a,7,8,9,10-hexahydro-5*H*-pyrazino[1',2':4,5] pyrazino[2,3-c]pyridazine-8-carbonyl)-3,3-dimethylpiperazine-1-carboxylate (Isomer 2) (74 mg, 0.125 mmol) in DCM (4 mL) at 0 °C. The reaction was warmed to room temperature and stirred for 12 hours. The product mixture was cooled to 0 °C and quenched with water (1 mL). The quenched product mixture was transferred to a separatory funnel containing saturated potassium carbonate aqueous solution (30 mL) and extracted with 3:1 CHCl₃:iPrOH (6 x 30 mL). The combined organic layers were dried over MgSO₄, filtered, and concentrated under reduced pressure to obtain (2,2-dimethylpiperazin-1-yl)(2-(3-fluoro-2-hydroxyphenyl)-6a-(fluoromethyl)-5,6,6a,7,9,10-hexahydro-8H-pyrazino[1',2':4,5]pyrazino[2,3-c]pyridazin-8-yl)methanone (Isomer 2) (51 mg, 86%). LCMS calcd for C₂₃H₃₀F₂N₇O₂ [M+H]⁺: m/z = 474.2; Found: 474.2.

### Intermediate 56

The intermediate shown below in Table 6 was prepared by methods analogous to that described for the preparing Intermediate 55 using the appropriate starting materials.

**Table 6. Intermediates 56**

| **Int** | **Structure** | **Name** | **Calcd. (M+H)⁺ m/z** | **Found (M+H)⁺ m/z** |
|---|---|---|---|---|
| **56** | 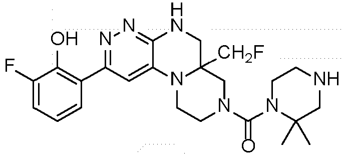 | (2,2-dimethylpiperazin-1-yl)(2-(3 -fluoro-2-hydroxyphenyl)-6a-(fluoromethyl)-5,6,6a,7,9,10-hexahydro-8*H-*pyrazino[1',2':4,5]pyrazino[2,3-*c*]pyridazin-8-yl)methanone (Isomer 1) | 474.2 | 474.2 |

### Example 1: 3-(6-(4-(((3R,5S)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl) methyl)piperidin-1-yl)-1-oxoisoindolin-2-yl)piperidine-2,6-dione

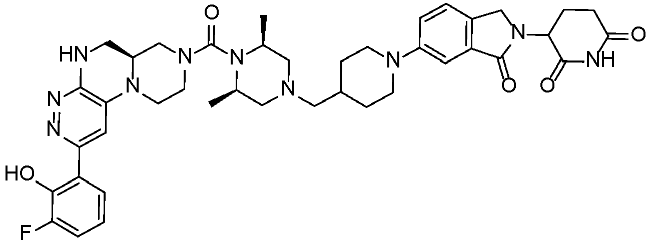

### Step1: ((2R, 6S)-2, 6-dimethylpiperazin-1-yl)((S)-2-(3-fluoro-2-hydroxyphenyl)-5,6,6a,7,9,10-hexahydro-8H-pyrazino[1',2':4,5]pyrazino[2,3-c]pyridazin-8-yl)methanone

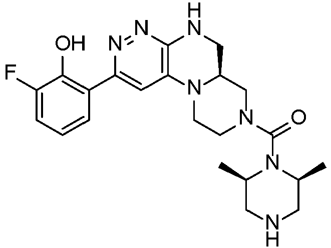

To a vial containing *tert-butyl* cis-3,5-dimethylpiperazine-1-carboxylate (206 mg, 0.96 mmol) was added DCM (4.80 mL) and pyridine (0.190 mL, 2.40 mmol). The reaction was cooled to 0 °C and triphosgene (186 mg, 0.630 mmol) was added. The reaction was left to warm to room temperature and stirred for 1 hour. The reaction was diluted in DCM then washed with a 1 N aqueous solution of HCl. The layers were separated and the aqueous phase was extracted with additional DCM. The organic layers were combined and dried over Na₂SO₄, filtered and concentrated under reduced pressure. The crude material was dissolved in DCM (4.80 mL) and 4-fluoro-2-[(10R)-1,5,6,8,12-pentazatricyclo[8.4.0.02,7]tetradeca-2(7),3,5-trien-4-yl]phenol;dihydrochloride (180 mg, 0.480 mmol) and triethylamine (0.340 mL, 2.40 mmol) were added. The reaction was stirred overnight at room temperature. The crude residue was purified by prep-HPLC (Waters CSH-C18, 23.5-43.5% MeCN/water (containing 0.1%TFA) over 5 min) to give the Boc-intermediate as a brown oil. The residue was dissolved in DCM (4.80 mL) and 2,2,2-trifluoroacetic acid (1.10 mL, 14.4 mmol) was added and the reaction was stirred at room temperature for 1.5 hours. The reaction mixture was concentrated under reduced pressure to obtain ((2*S*,6*R*)-2,6-dimethylpiperazin-1-yl)((*S*)-2-(3-fluoro-2-hydroxyphenyl)-5,6,6a,7,9,10-hexahydro-8H-pyrazino[1',2':4,5]pyrazino[2,3-c]pyridazin-8-yl)methanone as the TFA salt (119.47 mg, 45% yield). The material was used without additional purification. LCMS calcd for C₂₂H₂₀FN₇O₂ [M+H]⁺: m/z = 442.2; found: 442.1.

### Step 2: 3-(6-(4-(((3R,5S)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2 yl)piperidine-2,6-dione

A solution of sulfur trioxide pyridine (94.5 mg, 0.590 mmol) in DMSO (0.500 mL) was added dropwise to a vial containing 3-[5-[4-(hydroxymethyl)piperidin-1-yl]-3-oxo-1*H*-isoindol-2-yl]piperidine-2,6-dione (70.7 mg, 0.200 mmol, Intermediate 20) and triethylamine (0.250 mL, 1.78 mmol) in DMSO (1.50 mL) while the reaction was stirring on ice. After the addition was complete, the reaction was warmed to room temperature and stirred for 1 hour. A solution of ((2*S*,6*R*)-2,6-dimethylpiperazin-1-yl)((*S*)-2-(3-fluoro-2-hydroxyphenyl)-5,6,6a,7,9,10-hexahydro-8*H*-pyrazino[1',2':4,5]pyrazino[2,3-*c*]pyridazin-8-yl)methanone;bis-2,2,2-trifluoroacetic acid (55.0 mg, 0.100 mmol) in DMF (1.50 mL) was added to the reaction mixture and stirred for 30 min. Acetic acid (0.170 mL, 2.97 mmol) was added, the reaction was cooled to 0 °C and sodium triacetoxyborohydride (167.86 mg, 0.790 mmol) was added in one portion. The reaction was stirred at 0 °C until complete. Upon completion, water was added and the reaction was stirred for 1 hour. The reaction mixture was filtered and the filtrate was purified by prep-HPLC (Waters CSH-C18, 5 uM, 30x100mm, 9.5-19.5% MeCN/water (containing 0.1%TFA) over 5 min) to give 3-(6-(4-(((3R,5S)-4-((S)-2-(3-fluoro-2-hydroxyphenyl)-6,6a,7,8,9,10-hexahydro-5H-pyrazino[1',2':4,5]pyrazino[2,3-*c*]pyridazine-8-carbonyl)-3,5-dimethylpiperazin-1-yl)methyl) piperidin-1-yl)-1-oxoisoindolin-2-yl)piperidine-2,6-dione as its TFA salt (36.0 mg, 32% yield). ¹H NMR (400 MHz, MeOD) 7.46 (d, *J =* 8.4 Hz, 1H), 7.38 - 7.28 (m, 4H), 7.20 (s, 1H), 7.02 (td, *J=* 8.1, 4.8 Hz, 1H), 5.14 (dd, *J=* 13.3, 5.2 Hz, 1H), 4.52 - 4.32 (m, 2H), 4.20 (d, *J* = 12.8 Hz, 1H), 3.82 (d, *J =* 12.4 Hz, 2H), 3.79 - 3.70 (m, 2H), 3.62 (d, *J =* 11.8 Hz, 2H), 3.39 (q, *J =* 8.1 Hz, 2H), 3.34 - 3.27 (m, 6H), 3.18 - 3.12 (m, 2H), 3.05 (t, *J=* 12.5 Hz, 1H), 2.98 - 2.84 (m, 5H), 2.79 (dq, *J=* 17.6, 2.3 Hz, 1H), 2.51 (qd, *J=* 13.1, 4.6 Hz, 1H), 2.22 - 2.08 (m, 2H), 1.95 (d, *J* = 12.8 Hz, 2H), 1.52 (q, *J* = 11.2 Hz, 2H), 1.15 (s, 3H), 1.03 (s, 3H). LCMS calcd for C₄₁H₅₀FN₁₀O₅ [M+H]⁺: m/z = 781.4; found: 781.3.

### Examples 2-36:

Examples shown below in Table 7 were prepared as TFA salts by the method used in preparing **Example 1** using the appropriate intermediates and starting materials.

**Table 7. Examples 2-36**

| **Example** | **Structure/Name** | **Calcd. (M+H) ⁺ m/z** | **Found (M+H) ⁺ m/z** |
|---|---|---|---|
| **2** | 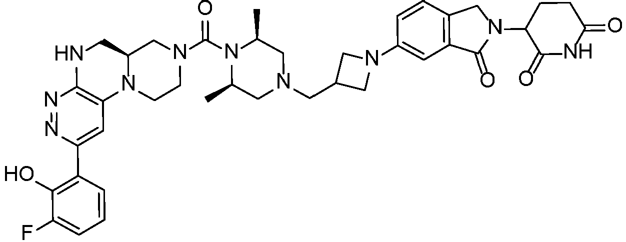 3-(6-(3-(((3*R*,5*S*)-4-((*S*)-2-(3-fluoro-2-hydroxyphenyl)-6,6a,7,8,9,10-hexahydro-5*H*-pyrazino[1',2':4,5]pyrazino[2,3-*c*] pyridazine-8-carbonyl)-3,5-dimethylpiperazin-1-yl)methyl) azetidin-1-yl)-1-oxoisoindolin-2-yl)piperidine-2,6-dione | 753.4 | 753.2 |
| **3** | 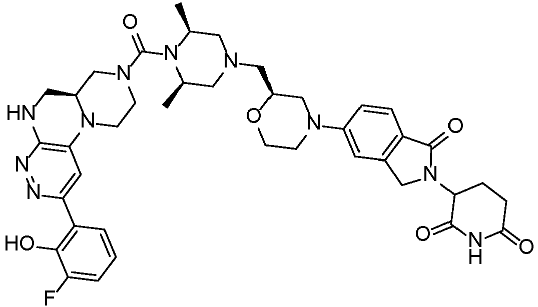 3-(5-((*S*)-2-(((3*R*,5*S*)-4-((*S*)-2-(3-fluoro-2-hydroxyphenyl)-6,6a,7,8,9,10-hexahydro-5*H*-pyrazino[1',2':4,5]pyrazino[2,3-c] pyridazine-8-carbonyl)-3,5-dimethylpiperazin-1-yl)methyl) morpholino)-1-oxoisoindolin-2-yl)piperidine-2,6-dione | 783.4 | 783.3 |
| **4** | 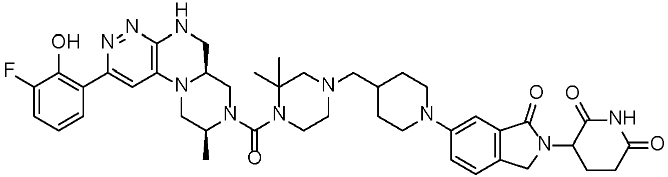 3-(6-(4-((4-((6a*S*,9*S*)-2-(3-fluoro-2-hydroxyphenyl)-9-methyl-6,6a,7,8,9,10-hexahydro-5*H*-pyrazino[1',2':4,5]pyrazino[2,3-*c*] pyridazine-8-carbonyl)-3,3-dimethylpiperazin-1-yl)methyl) piperidin-1-yl)-1-oxoisoindolin-2-yl)piperidine-2,6-dione | 795.4 | 795.1 |
| **5** | 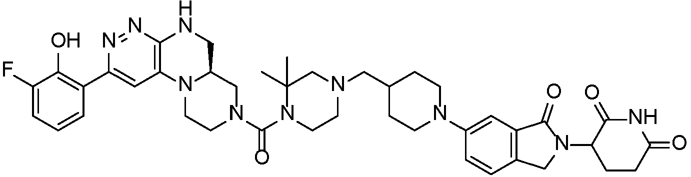 3-(6-(4-((4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione ¹H NMR (300 MHz, DMSO) δ 11.00 (s, 1H), 9.36 (s, 1H), 8.32 (s, 1H), 7.50 - 7.39 (m, 2H), 7.32 (d, *J =* 8.1 Hz, 2H), 7.25 - 7.20 (m, 2H), 7.02 (td, *J=* 8.0, 4.9 Hz, 1H), 5.11 (dd, *J=* 13.2, 5.1 Hz, 1H), 4.37 (d, *J=* 16.9 Hz, 1H), 4.23 (d, *J* = 16.9 Hz, 1H), 4.15 - 4.10 (m, 1H), 3.84 - 3.81 (m, 4H), 3.68 (d, *J=* 10.1 Hz, 2H), 3.52 - 3.19 (m, 6H), 3.15 - 3.04 (m, 3H), 2.93 - 2.79 (m, 5H), 2.69 - 2.55 (m, 1H), 2.40 (ddd, *J=* 26.0, 13.1, 4.4 Hz, 2H), 2.09 - 1.99 (m 2H), 1.83 (d, *J =* 11.9 Hz, 2H), 1.51-1.34 (m, 8H). | 781.4 | 781.1 |
| **6** | 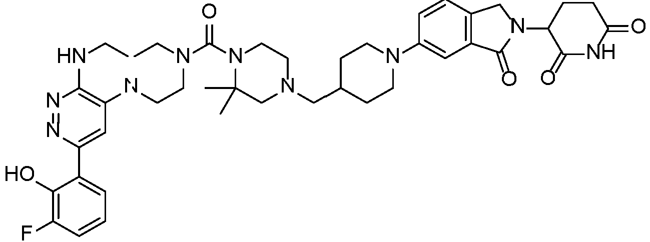 3-(6-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione (Mixture of diastereomers) ¹H NMR (400 MHz, MeOD) δ 7.38 (d, *J=* 8.4 Hz, 1H), 7.30 (d, *J =* 2.2 Hz, 1H), 7.29 - 7.19 (m, 3H), 7.02 (s, 1H), 6.92 (td, *J* = 8.1, 4.9 Hz, 1H), 5.04 (dd, *J=* 13.3, 5.2 Hz, 1H), 4.41 - 4.19 (m, 2H), 3.80 (d, *J=* 12.6 Hz, 1H), 3.72 (d, *J=* 12.3 Hz, 2H), 3.49 - 3.25 (m, 5H), 3.06 (d, *J=* 6.8 Hz, 2H), 2.89 - 2.74 (m, 3H), 2.68 (ddd, *J =* 17.4, 4.6, 2.4 Hz, 1H), 2.40 (qd, *J =* 13.2, 4.7 Hz, 1H), 2.08 (ddd, *J =* 10.0, 5.3, 2.6 Hz, 2H), 1.83 (d, *J =* 12.8 Hz, 2H), 1.56 - 1.34 (m, 7H), 1.31 (s, 3H). | 795.4 | 795.1 |
| **7** | 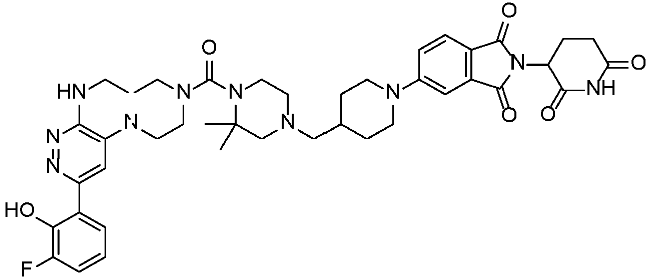 2-(2,6-dioxopiperidin-3-yl)-5-(4-((4-(2-(3-fluoro-2-hydroxyphenyl)-6a-methyl-6,6a,7,8,9,10-hexahydro-5H-pyrazino[1',2':4,5] pyrazino [2,3-c]pyridazine-8-carbonyl)-3,3-dimethylpiperazin-1-yl)methyl) piperidin-1-yl)isoindoline-1,3-dione (Mixture of diastereomers) | 809.4 | 809.1 |
| **8** | 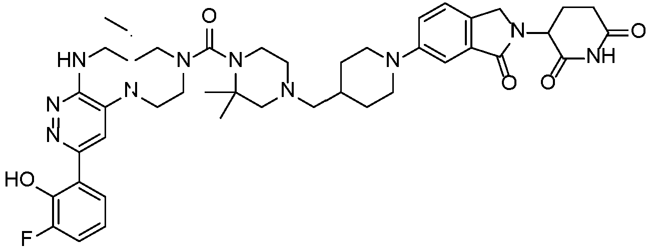 3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione (Mixture of diastereomers) | 809.4 | 809.4 |
| **9** | 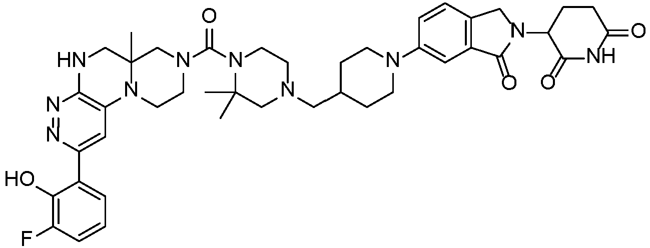 3-(6-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione (Isomer 1) | 794.4 | 795.3 |
| **10** | 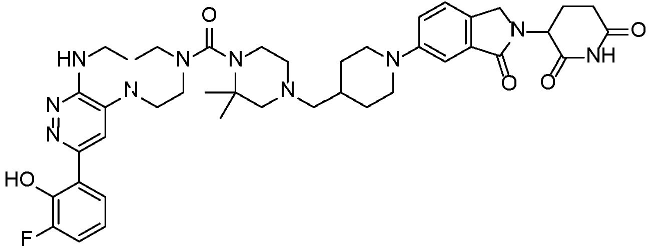 3-(6-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione (Isomer 2) | 794.4 | 795.4 |
| **11** | 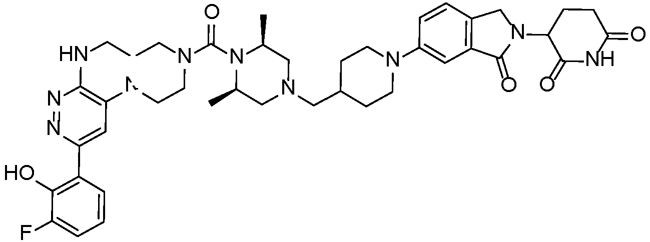 3-(6-(4-(((3*R*,5*S*)-4-2-(3-fluoro-2-hydroxyphenyl)-6a-methyl-6,6a,7,8,9,10-hexahydro-5*H*-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione (Isomer 1) | 795.4 | 795.4 |
| **12** | 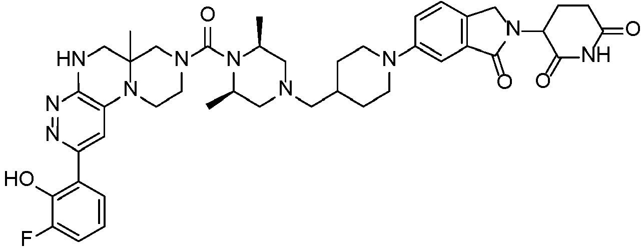 3-(6-(4-(((3*R*,5*S*)-4-2-(3-fluoro-2-hydroxyphenyl)-6a-methyl-6,6a,7,8,9,10-hexahydro-5H-pyrazino[1',2':4,5]pyrazino[2,3-c] pyridazine-8-carbonyl)-3,5-dimethylpiperazin-1-yl)methyl) piperidin-1-yl)-1-oxoisoindolin-2-yl)piperidine-2,6-dione (Isomer 2) | 795.4 | 795.4 |
| **13** | 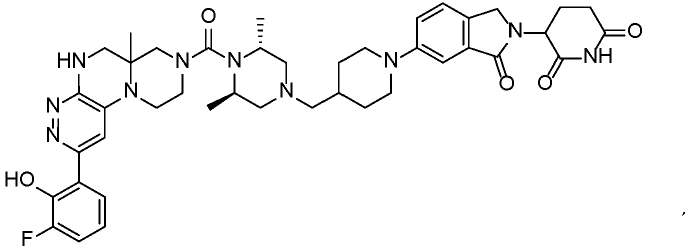 3-(6-(4-(((3R, SR)-4-(2-(3-fluoro-2-hydroxyphenyl)-6a-methyl-6,6a,7,8,9,10-hexahydro-5H-pyrazino[1',2':4,5]pyrazino[2,3-c] pyridazine-8-carbonyl)-3,5-dimethylpiperazin-1-yl)methyl) piperidin-1-yl)-1-oxoisoindolin-2-yl)piperidine-2,6-dione (Isomer 1) | 795.4 | 795.1 |
| **14** | 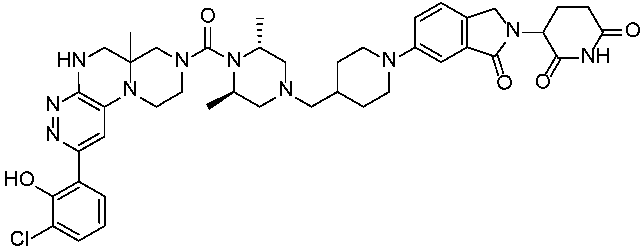 3-(6-(4-(((3*R*,5*R*)-4-(2-(3-chloro-2-hydroxyphenyl)-6a-methyl-6,6a,7,8,9,10-hexahydro-5H-pyrazino[1',2':4,5]pyrazino[2,3-c] pyridazine-8-carbonyl)-3,5-dimethylpiperazin-1-yl)methyl) piperidin-1-yl)-1-oxoisoindolin-2-yl)piperidine-2,6-dione (Isomer 1) | 811.4 | 811.0 |
| **15** | 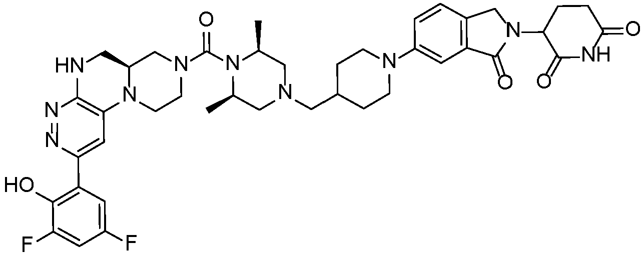 3-(6-(4-(((3*R*,5*S*)-4-((*S*)-2-(3,5-difluoro-2-hydroxyphenyl)-6,6a,7,8,9,10-hexahydro-5*H*-pyrazino[1',2':4,5]pyrazino[2,3-*c*] pyridazine-8-carbonyl)-3,5-dimethylpiperazin-1-yl)methyl) piperidin-1-yl)-1-oxoisoindolin-2-yl)piperidine-2,6-dione ¹H NMR (400 MHz, MeOD) δ 7.36 (d, *J =* 8.3 Hz, 1H), 7.28 - 7.22 (m, 2H), 7.17 (ddd, *J=* 11.0, 8.3, 3.0 Hz, 1H), 7.13 - 7.08 (m, 2H), 5.04 (dd, *J=* 13.3, 5.1 Hz, 1H), 4.60 (s, 1H), 4.37 - 4.23 (m, 2H), 4.10 (d, *J =* 12.5 Hz, 1H), 3.77 - 3.55 (m, 4H), 3.52 (d, *J =* 12.1 Hz, 2H), 3.39 - 3.24 (m, 2H), 3.08 - 3.01 (m, 3H), 2.95 (t, *J =* 12.2 Hz, 1H), 2.89 - 2.73 (m, 5H), 2.72 - 2.63 (m, 1H), 2.39 (qd, *J =* 13.3, 4.8 Hz, 1H), 2.10 - 1.98 (m, 2H), 1.86 (d, *J =* 12.8 Hz, 2H), 1.42 (q, *J= 12.1* Hz, 2H), 1.04 (d, *J=* 6.2 Hz, 6H). | 799.4 | 799.3 |
| **16** | 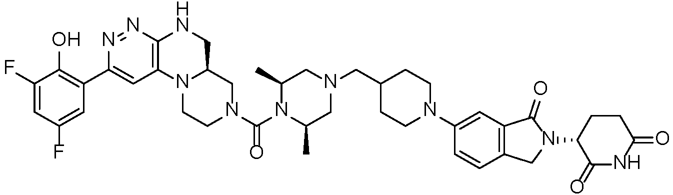 (*R*)-3-(6-(4-(((3*S*,5*R*)-4-((*S*)-2-(3,5-difluoro-2-hydroxyphenyl)-6,6a,7,8,9,10-hexahydro-5*H*-pyrazino[1',2':4,5]pyrazino[2,3-*c*] pyridazine-8-carbonyl)-3,5-dimethylpiperazin-1-yl)methyl) piperidin-1-yl)-1-oxoisoindolin-2-yl)piperidine-2,6-dione | 799.4 | 799.3 |
| **17** | 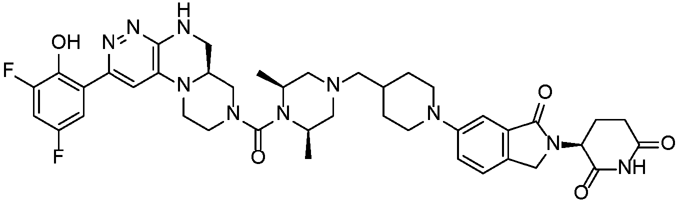 (,S)-3 -(6-(4-(((3S, 5R)-4-((S)-2-(3,5 -difluoro-2-hydroxyphenyl)-6,6a,7,8,9,10-hexahydro-5*H*-pyrazino[1',2':4,5]pyrazino[2,3-*c*] pyridazine-8-carbonyl)-3,5-dimethylpiperazin-1-yl)methyl) piperidin-1-yl)-1-oxoisoindolin-2-yl)piperidine-2,6-dione | 799.4 | 799.2 |
| **18** | 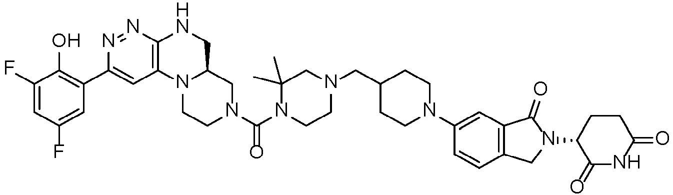 *(R)*-3-(6-(4-((4-((*S*)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione | 799.4 | 799.2 |
| **19** | 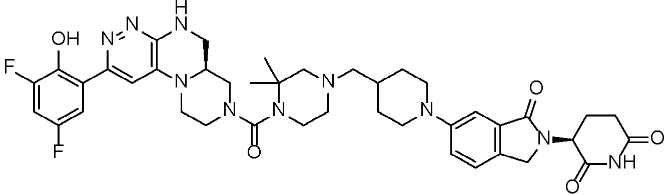 (S)-3-(6-(4-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione | 799.4 | 799.2 |
| **20** | 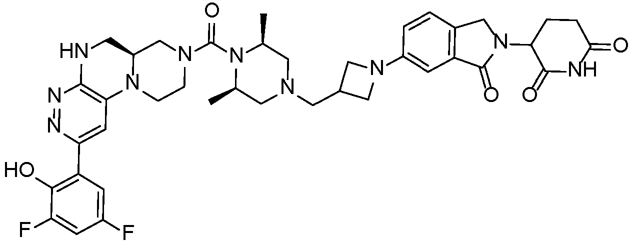 3-(6-(3-(((3*R*,5*S*)-4-((*S*)-2-(3,5-difluoro-2-hydroxyphenyl)-6,6a,7,8,9,10-hexahydro-5*H*-pyrazino[1',2':4,5]pyrazino[2,3-*c*] pyridazine-8-carbonyl)-3,5-dimethylpiperazin-1-yl)methyl)azetidin-1-yl)-1-oxoisoindolin-2-yl)piperidine-2,6-dione | 771.4 | 771.3 |
| **21** | 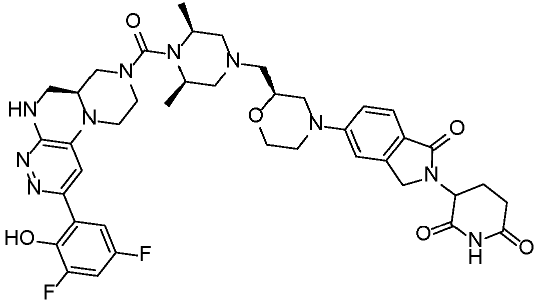 3-(5-((S)-2-(((3R,5S)-4-((S)-2-(3,5-difluoro-2-hydroxyphenyl)-6,6a,7,8,9,10-hexahydro-5*H*-pyrazino[1',2':4,5]pyrazino[2,3-c]pyridazine-8-carbonyl)-3,5-dimethylpiperazin-1-yl)methyl)morpholino)-1-oxoisoindolin-2-yl)piperidine-2,6-dione | 801.4 | 801.3 |
| **22** | 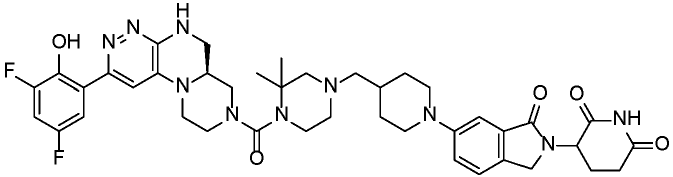 3-(6-(4-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione | 799.4 | 799.3 |
| **23** | 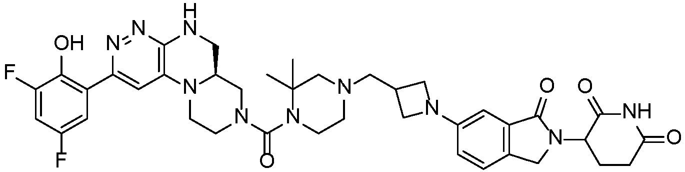 3-(6-(3-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)azetidin-1-yl)-1-oxoisoindolin-2-yl)piperidine-2,6-dione | 771.4 | 771.2 |
| **24** | 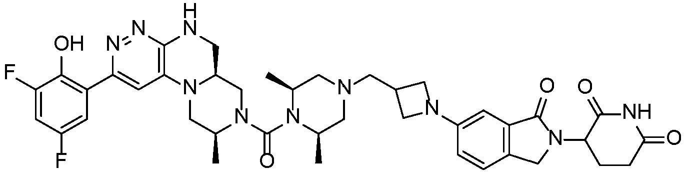 3-(6-(3-(((3*S*,5*R*)-4-((6a*S*,9*S*)-2-(3,5-difluoro-2-hydroxyphenyl)-9-methyl-6,6a,7,8,9,10-hexahydro-5*H*-pyrazino[1',2':4,5]pyrazino[2,3-c]pyridazine-8-carbonyl)-3,5-dimethylpiperazin-1-yl)methyl) azetidin-1-yl)-1-oxoisoindolin-2-yl)piperidine-2,6-dione | 785.4 | 785.3 |
| **25** | 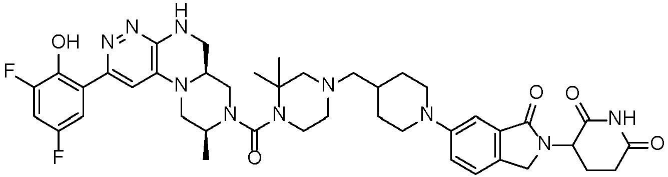 3-(6-(4-((4-((6a*S*,9*S*)-2-(3,5-difluoro-2-hydroxyphenyl)-9-methyl-6,6a,7,8,9,10-hexahydro-5H-pyrazino[1',2':4,5]pyrazino[2,3-c] pyridazine-8-carbonyl)-3,3-dimethylpiperazin-1-yl)methyl) piperidin-1-yl)-1-oxoisoindolin-2-yl)piperidine-2,6-dione | 813.4 | 813.3 |
| **26** | 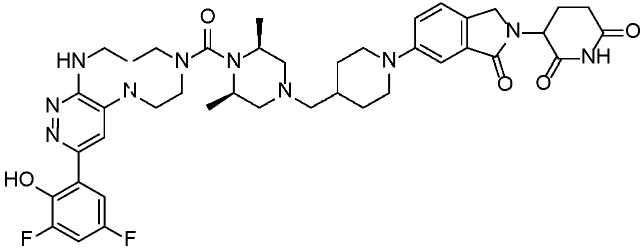 3-(6-(4-(((3*S*,5*R*)-4-(2-(3,5-difluoro-2-hydroxyphenyl)-6a-methyl-6,6a,7,8,9,10-hexahydro-5H-pyrazino[1',2':4,5]pyrazino[2,3-c] pyridazine-8-carbonyl)-3,5-dimethylpiperazin-1-yl)methyl) piperidin-1-yl)-1-oxoisoindolin-2-yl)piperidine-2,6-dione (Mixture of diastereomers) | 813.4 | 813.3 |
| **27** | 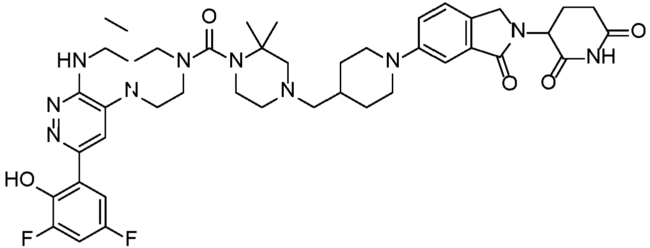 3-(6-(4-((4-(2-(3,5-difluoro-2-hydroxyphenyl)-6a-ethyl-6,6a,7,8,9,10-hexahydro-5*H*-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione (Mixture of diastereomers) | 827.4 | 827.4 |
| **28** | 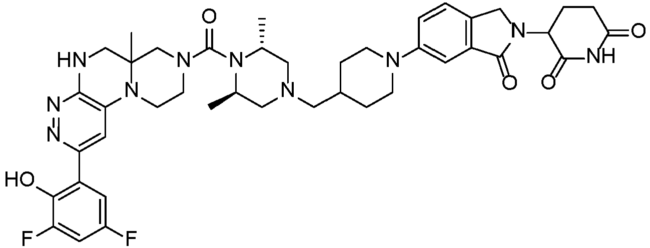 3-(6-(4-(((3R,5R)-4-(2-(3,5-difluoro-2-hydroxyphenyl)-6a-methyl-6,6a,7,8,9,10-hexahydro-5H-pyrazino[1',2':4,5]pyrazino[2,3-c] pyridazine-8-carbonyl)-3,5-dimethylpiperazin-1-yl)methyl) piperidin-1-yl)-1-oxoisoindolin-2-yl)piperidine-2,6-dione (Isomer 1) | 813.4 | 813.1 |
| **29** | 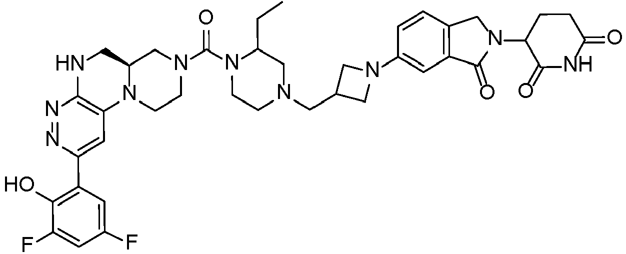 3-(6-(3-((4-((S)-2-(3,5-difluoro-2-hydroxyphenyl)-6,6a,7,8,9,10-hexahydro-5*H*-pyrazino[1',2':4,5]pyrazino[2,3-*c*]pyridazine-8-carbonyl)-3-ethylpiperazin-1-yl)methyl)azetidin-1-yl)-1-oxoisoindolin-2-yl)piperidine-2,6-dione (Mixture of diastereomers) | 771.4 | 771.2 |
| **30** | 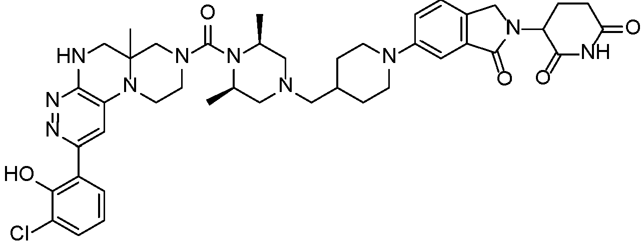 3-(6-(4-(((3*R*,5*S*)-4-(2-(3-chloro-2-hydroxyphenyl)-6a-methyl-6,6a,7,8,9,10-hexahydro-5H-pyrazino[1',2':4,5]pyrazino[2,3-c] pyridazine-8-carbonyl)-3,5-dimethylpiperazin-1-yl)methyl) piperidin-1-yl)-1-oxoisoindolin-2-yl)piperidine-2,6-dione (Mixture of diastereomers) | 811.4 | 811.2 |
| **31** | 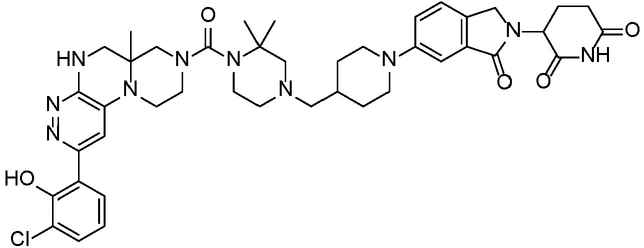 3-(6-(4-((4-(2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione (Mixture of diastereomers) | 811.4 | 811.1 |
| **32** | 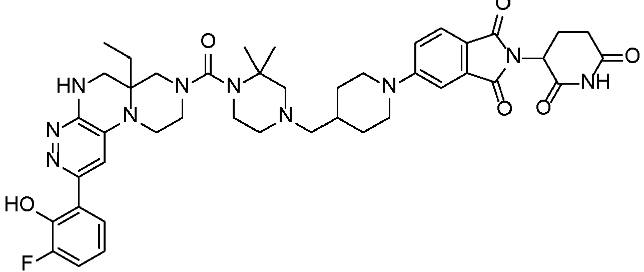 2-(2,6-dioxopiperidin-3-yl)-5-(4-((4-(6a-ethyl-2-(3-fluoro-2-hydroxyphenyl)-6,6a,7,8,9,10-hexahydro-5H-pyrazino [1',2':4,5] pyrazino[2,3-c]pyridazine-8-carbonyl)-3,3-dimethylpiperazin-1-yl)methyl)piperidin-1-yl)isoindoline-1,3-dione (Mixture of diastereomers) | 823.4 | 823.4 |
| **33** | 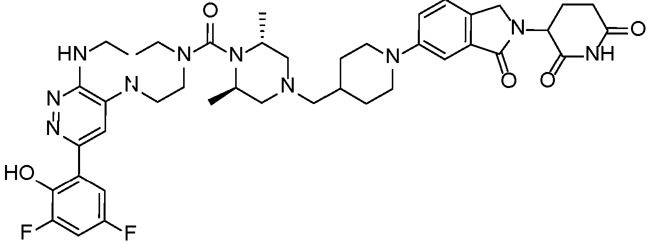 3-(6-(4-(((3R,5R)-4-(2-(3,5-difluoro-2-hydroxyphenyl)-6a-methyl-6,6a,7,8,9,10-hexahydro-5H-pyrazino[1',2':4,5]pyrazino[2,3-c] pyridazine-8-carbonyl)-3,5-dimethylpiperazin-1-yl)methyl) piperidin-1-yl)-1-oxoisoindolin-2-yl)piperidine-2,6-dione (Mixture of diastereomers) | 813.4 | 813.3 |
| **34** | 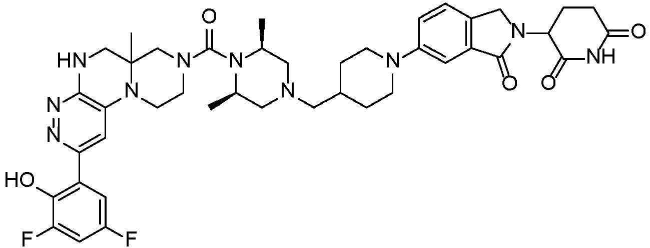 3-(6-(4-(((3*S*,5*R*)-4-(2-(3,5-difluoro-2-hydroxyphenyl)-6a-methyl-6,6a,7,8,9,10-hexahydro-5H-pyrazino[1',2':4,5]pyrazino[2,3-c] pyridazine-8-carbonyl)-3,5-dimethylpiperazin-1-yl)methyl) piperidin-1-yl)-1-oxoisoindolin-2-yl)piperidine-2,6-dione (Isomer 1) | 813.4 | 813.1 |
| **35** | 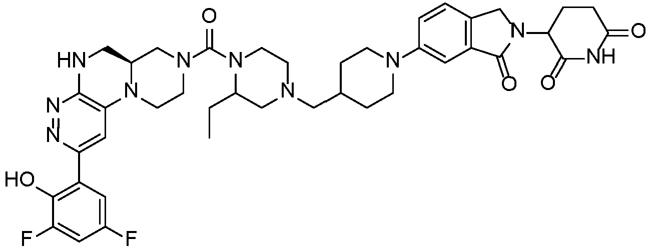 3-(6-(4-((4-((S)-2-(3,5-difluoro-2-hydroxyphenyl)-6,6a,7,8,9,10-hexahydro-5*H*-pyrazino[1',2':4,5]pyrazino[2,3-*c*]pyridazine-8-carbonyl)-3-ethylpiperazin-1-yl)methyl)piperidin-1-yl)-1-oxoisoindolin-2-yl)piperidine-2,6-dione (Mixture of diastereomers) | 799.4 | 799.3 |
| **36** | 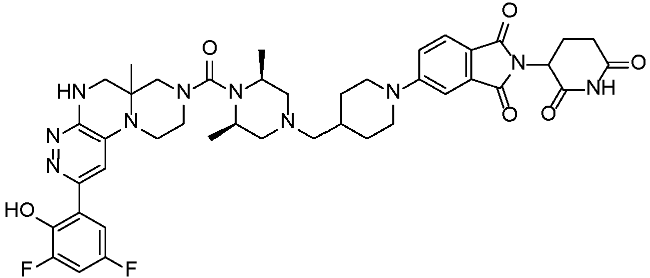 5-(4-(((3S,5R)-4-(2-(3,5-difluoro-2-hydroxyphenyl)-6a-methyl-6,6a,7,8,9,10-hexahydro-5*H*-pyrazino[1',2':4,5]pyrazino[2,3-*c*] pyridazine-8-carbonyl)-3,5-dimethylpiperazin-1-yl)methyl) piperidin-1-yl)-2-(2,6-dioxopiperidin-3-yl)isoindoline-1,3-dione (Mixture of diastereomers) | 827.4 | 827.4 |

### Example 37 and 38: 3-(6-((3R,4R)-4-(((1R,5S,6R)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)-3-fluoropiperidin-1-yl)-1-oxoisoindolin-2-yl)piperidine-2,6-dione (Isomers 1 and 2)

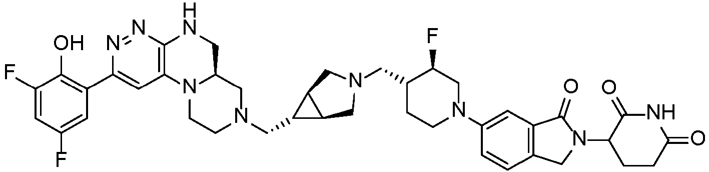

### Step 1. Tert-butyl (1R,5S,6s)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl]methyl]-3-azabicyclo [3.1.0]hexane-3-carboxylate

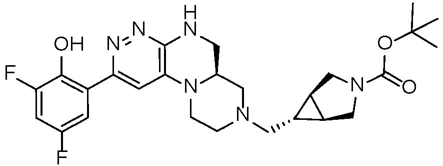

A solution of acetic acid (100 µL, 1.70 mmol), (R)-2,4-difluoro-6-(6,6a,7,8,9,10-hexahydro-5*H*-pyrazino[1',2':4,5]pyrazino[2,3-c]pyridazin-2-yl)phenol (111 mg, 0.283 mmol, Intermediate 2), *tert-butyl* (1*R*,5*S*,6*r*)-6-formyl-3-azabicyclo[3.1.0]hexane-3-carboxylate (90.0 mg, 0.424 mmol) and N,N-diisopropylethylamine (197 µL, 1.13 mmol) in DMF (1.00 mL) was stirred at room temperature for 1 hour. Sodium triacetoxyborohydride (180 mg, 0.849 mmol) was added and the reaction was stirred at room temperature for 2 hours. The reaction mixture was diluted with DCM (3.00 mL) and washed with water (2 x 3 mL). The organics were dried over MgSO₄, filtered and concentrated under reduced pressure. The crude residue was purified by silica gel flash column chromatography eluting with a gradient of 0-10% MeOH/DCM to obtain *tert*-butyl (1*R*,5*S*)-6-[[(10*S*)-4-(3,5-difluoro-2-hydroxyphenyl)-1,5,6,8,12-pentazatricyclo [8.4.0.02,7]tetradeca-2,4,6-trien-12-yl]methyl]-3-azabicyclo[3.1.0]hexane-3-carboxylate (140 mg, 96% yield). LCMS calc. for C₂₆H₃₃F₂N₆O₃ [M+H]⁺: m/z = 515.3; Found: 515.1.

### Step 2. 2-((S)-8-(((1R, 5S, 6r)-3-azabicyclo[3.1.0]hexan-6-yl)methyl)-6,6a,7,8,9,10-hexahydro-5H-pyrazino[1',2':4,5]pyrazino[2,3-c]pyridazin-2-yl)-4,6-difluorophenol

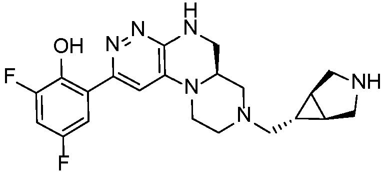

A solution of 4 N HCl in 1,4-dioxane (1.17 mL, 37.9 mmol) was added to a solution of *tert*-butyl (1*R*,5*S*,6*s*)-6-(((*S*)-2-(3,5-difluoro-2-hydroxyphenyl)-5,6,6a,7,9,10-hexahydro-8*H-*pyrazino[1',2':4,5]pyrazino[2,3-c]pyridazin-8-yl)methyl)-3-azabicyclo[3.1.0]hexane-3-carboxylate (140 mg, 0.272 mmol) in DCM (1.00 mL). The reaction was stirred at room temperature for 1 hour. The mixture was concentrated to give the title compound (120 mg, 91% yield) as its HCl salt. The material was used in the following step without additional purification. LCMS calc. for C₂₁H₂₅F₂N₆O [M+H]⁺: m/z = 415.2; Found: 415.1

### Step 3. ((3R,4R)-1-(2-(2,6-dioxopiperidin-3-yl)-3-oxoisoindolin-5-yl)-3-fluoropiperidin-4-yl)methyl 4-methylbenzenesulfonate (Isomers 1 and 2)

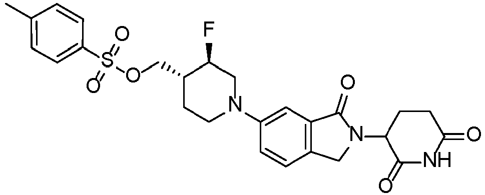

To a mixture of ((3R,4R)-1-(2-(2,6-dioxopiperidin-3-yl)-3-oxoisoindolin-5-yl)-3-fluoropiperidin-4-yl)methyl 4-methylbenzenesulfonate (82.0 mg, 0.218 mmol, Intermediate 22) in pyridine (1.07 mL, 13.3 mmol) was added tosyl chloride (83.3 mg, 0.440 mmol). The mixture was stirred at room temperature for 1 hour. Additional tosyl chloride was added every 2 hours until the starting material was consumed. The mixture was diluted with DCM and the organic phase was washed with water. The combined organics were dried over Na₂SO₄, filtered, and concentrated under reduced pressure. The crude residue was purified by silica gel flash column chromatography, eluting with a gradient of 0-100% EtOAC/DCM to give rac-((3R,4R)-1-(2-(2,6-dioxopiperidin-3-yl)-3-oxoisoindolin-5-yl)-3-fluoropiperidin-4-yl)methyl 4-methyl-benzenesulfonate (110 mg, 95% yield). LCMS calcd for C₂₆H₂₉FN₃O₆S [M+H]⁺: m/z = 530.2; found: 530.2.

### Step 4. 3-[5-[(3R, 4R)-4-[[(1S, 5R)-6-[[(10S)-4-(3,5-difluoro-2-hydroxyphenyl)-1, 5, 6, 8,12-pentazatricyclo[8.4.0.02,7]tetradeca-2(7),3,5-trien-12-yl]methyl]-3-azabicyclo[3.1.0]hexan-3-yl]methyl]-3-fluoropiperidin-1-yl]-3-oxo-1H-isoindol-2-yl]piperidine-2,6-dione (Isomers 1 and 2)

A solution of 2-[(10*S*)-12-[[(1*S*,5*R*)-3-azabicyclo[3.1.0]hexan-6-yl]methyl]-1,5,6,8,12-pentazatricyclo-[8.4.0.02,7]tetradeca-2,4,6-trien-4-yl]-4,6-difluorophenol;dihydrochloride (20.0 mg, 41.0 µmol), ((rac-3R,4R)-1-(2-(2,6-dioxopiperidin-3-yl)-3-oxoisoindolin-5-yl)-3-fluoro-piperidin-4-yl)methyl 4-methyl-benzenesulfonate (24.0 mg, 45.1 µmol), N,N-diisopropylethylamine (42.9 µL, 0.246 mmol) and sodium iodide (12.3 mg, 82.1 µmol) in DMF (1.00 mL) was stirred at 130 °C for 1 hour. The crude mixture reaction mixture was diluted with DMSO (15.0 mL), filtered and the filtrate was purified by prep-HPLC on a C18 column (7.6-27.6% MeCN in 0.1% TFA (aq.), pH = 2) to give the TFA salt of the title compounds (2.50 mg, 7.6% yield, peak 2, Isomer 2, Example 38 5790) and (0.6 mg, 1.9%, peak 1, isomer 1, Example 37 5789) as a white solid. LCMS calc. for C₄₀H₄₅F₃N₉O₄ [M+H]⁺: m/z = 772.4; Found: 772.1.

### Examples 39-41:

Examples shown below in Table 8 were prepared as TFA salts by the method used in preparing Examples 37 & 38 using the appropriate intermediates and starting materials.

**Table 8. Examples 39-41**

| **Example** | **Structure/Name** | **Calcd. (M+H) ⁺ m/z** | **Found (M+H) ⁺ m/z** |
|---|---|---|---|
| **39** | 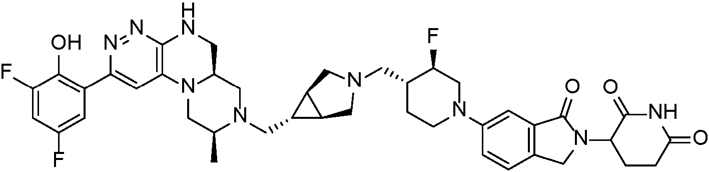 3-(6-((3*R*,4*R*)-4-(((1*R*,5*S*,6*R*)-6-(((6a*S*,9*S*)-2-(3,5-difluoro-2-hydroxyphenyl)-9-methyl-5,6,6a,7,9,10-hexahydro-8*H*-pyrazino [1',2':4,5]pyrazino[2,3-*c*]pyridazin-8-yl)methyl)-3-azabicyclo [3.1.0]hexan-3-yl)methyl)-3-fluoropiperidin-1-yl)-1-oxoisoindolin-2-yl)piperidine-2,6-dione (Isomer 1) | 786.4 | 786.2 |
| **40** | 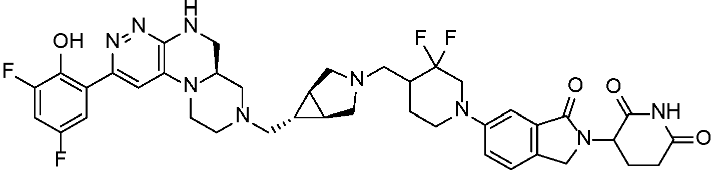 3-(6-(4-(((1*R*,5*S*,6*r*)-6-(((*S*)-2-(3,5-difluoro-2-hydroxyphenyl)-5,6,6a,7,9,10-hexahydro-8*H*-pyrazino[1',2':4,5]pyrazino[2,3-c]pyridazin-8-yl)methyl)-3-azabicyclo[3.1.0]hexan-3-yl)methyl)-3,3-difluoropiperidin-1-yl)-1-oxoisoindolin-2-yl)piperidine-2,6-dione (Isomer 1) | 790.4 | 790.3 |
| **41** | 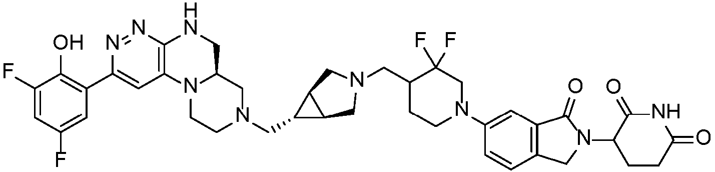 3-(6-(4-(((1*R*,5*S*,6*r*)-6-(((*S*)-2-(3,5-difluoro-2-hydroxyphenyl)-5,6,6a,7,9,10-hexahydro-8*H*-pyrazino[1',2':4,5]pyrazino[2,3-c]pyridazin-8-yl)methyl)-3-azabicyclo[3.1.0]hexan-3-yl)methyl)-3,3-difluoropiperidin-1-yl)-1-oxoisoindolin-2-yl)piperidine-2,6-dione (Isomer 2) | 790.4 | 790.3 |

### Example 42 and 43: 3-(6-((rac-3R,4R)-4-(((3S,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)-3-fluoropiperidin-1-yl)-1-oxoisoindolin-2-yl)piperidine-2,6-dione (Isomers 1 and 2)

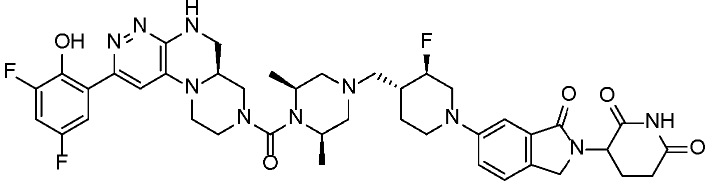

A mixture of [(10*S*)-4-(3,5-difluoro-2-hydroxyphenyl)-1,5,6,8,12-pentazatricyclo [8.4.0.02,7]tetradeca-2(7),3,5-trien-12-yl]-[(2*R*,6*S*)-2,6-dimethylpiperazin-1-yl]methanone;2,2,2-trifluoroacetic acid (14.8 mg, 20.0 µmol, prepared analogously to Example 1, Step 1 with appropriate starting materials), ((rac-3R,4R)-1-(2-(2,6-dioxopiperidin-3-yl)-3-oxoisoindolin-5-yl)-3-fluoropiperidin-4-yl)methyl 4-methylbenzene-sulfonate (14.8 mg, 30.0 µmol, Example 37 and 38, Step 3), *N,N-diisopropylethylamine* (10 µL, 90.0 µmol) and sodium iodide (6.45 mg, 40.0 µmol) in NMP (0.200 mL) was stirred at 120 °C for 1 hour. The mixture was purified by prep-HPLC (Waters CSH-C18, 5 uM, 30x100mm, 6.1-26.1% MeCN/water (containing 0.1%TFA) over 5 min) to give 3-(6-((rac-3R,4R)-4-(((3S,5R)-4-((,S)-2-(3,5-difluoro-2-hydroxyphenyl)-6,6a,7,8,9,10-hexahydro-5*H*-pyrazino[1',2':4,5]pyrazino[2,3-*c*]pyridazine-8-carbonyl)-3, 5-dimethylpiperazin-1-yl)methyl)-3-fluoropiperidin-1-yl)-1-oxoisoindolin-2-yl)piperidine-2,6-dione;bistrifluoroacetic acid (0.600 mg, 3.40% yield) (peak1, Isomer 1, Example 42, 5567) and 3-(6-((3R,4R)-4-(((3S,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)-3-fluoropiperidin-1-yl)-1-oxoisoindolin-2-yl)piperidine-2,6-dione;bistrifluoroacetic acid (5.40 mg, 30.7% yield) (peak2, Isomer 2, Example 43, 5568). LCMS calcd for C₄₁H₄₈F₃N₁₀O₅ [M+H]⁺: m/z = 817.4; found: 817.2.

### Example 44

Examples shown below in Table 9 were prepared as TFA salts by the method used in preparing Example 42 and 43 using the appropriate intermediates and starting materials.

**Table 9. Example 44**

| **Example** | **Structure/Name** | **Calcd. (M+H) ⁺ m/z** | **Found (M+H) ⁺ m/z** |
|---|---|---|---|
| **44** | 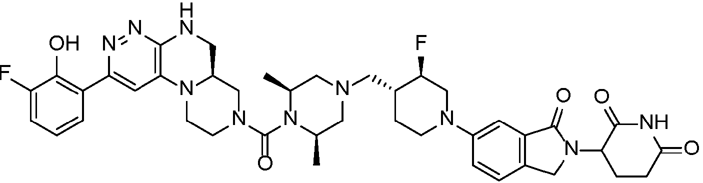 3-(6-((3*R*,4*R*)-3-fluoro-4-(((3*S*,5*R*)-4-((*S*)-2-(3-fluoro-2-hydroxyphenyl)-6,6a,7,8,9,10-hexahydro-5*H*-pyrazino[1',2':4,5] pyrazino[2,3-*c*]pyridazine-8-carbonyl)-3,5-dimethylpiperazin-1-yl)methyl)piperidin-1-yl)-1-oxoisoindolin-2-yl)piperidine-2,6-dione (Isomer 1) | 799.4 | 799.2 |

### Example 45: 3-(6-(4-(((3R,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione

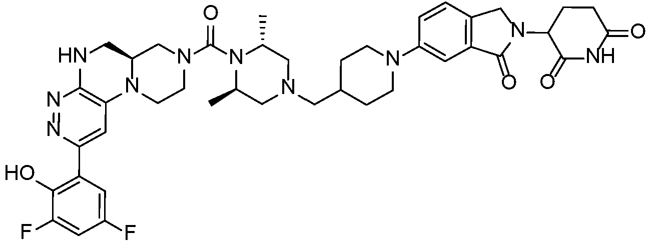

### Step 1: 5-((9H-fluoren-9-yl)methyl) 8-(tert-butyl) (R)-2-(2-((((9H-fluoren-9-yl)methoxy)carbonyl)oxy)-3,5-difluorophenyl)-6a,7,9,10-tetrahydro-5H-pyrazino[1',2':4,5] pyrazino[2,3-c]pyridazine-5,8(6H)-dicarboxylate

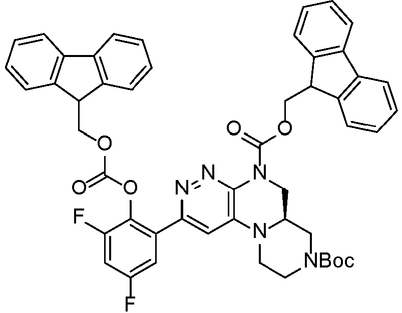

A mixture of di-tert-butyl (10*R*)-4-chloro-1,5,6,8,12-pentazatricyclo[8.4.0.02,7]tetradeca-2(7),3,5-triene-8,12-dicarboxylate (300 mg, 0.700 mmol, Intermediate 1, Step 4), (3,5-difluoro-2-hydroxyphenyl)boronic acid (135 mg, 0.770 mmol), cesium carbonate (504.5 mg, 1.55 mmol) and XPhos Pd G2 (83.1 mg, 0.110 mmol) in 1,4-dioxane (4.00 mL) and water (0.500 mL) was degassed with N₂, and the reaction mixture was stirred at 120 °C for 2 hours. The mixture was diluted with DCM and washed with a saturated brine solution. The aqueous phase was extracted with DCM and the combined organics were dried over Na₂SO₄, filtered, and concentrated under reduced pressure. The crude residue was suspended in a mixture of 1,4-dioxane (4.00 mL) and water (0.500 mL) and 9-fluorenylmethoxycarbonyl chloride (401 mg, 1.55 mmol) was added, followed by sodium bicarbonate (355 mg, 4.23 mmol). After stirring at room temperature for 4 hours, an additional portion of 9-fluorenylmethoxycarbonyl chloride (401 mg, 1.55 mmol) and sodium bicarbonate (355 mg, 4.23 mmol) were added. The reaction was stirred at room temperature overnight. The reaction mixture was diluted with DCM and water. The layers were separated and the combined organics were dried over Na₂SO₄, filtered and concentrated under reduced pressure. The crude material was purified by silica gel flash column chromatography, eluting with a gradient of 0-100% EtOAc/hexanes to give 5-((9H-fluoren-9-yl)methyl) *8-(tert-*butyl) (R)-2-(2-((((9H-fluoren-9-yl)methoxy)carbonyl)oxy)-3,5-difluorophenyl)-6a,7,9,10-tetrahydro-5*H*-pyrazino[1',2':4,5]pyrazino[2,3*-c*]pyridazine-5,8(6*H*)-dicarboxylate (492 mg, 81% yield). LCMS calcd for C₅₀H₄₄F₂N₅O₇ [M+H]⁺: m/z = 864.3; Found: 864.3.

### Step 2: (9H-fluoren-9-yl)methyl (R)-2-(2-((((9H-fluoren-9-yl)methoxy)carbonyl)oxy)-3,5-difluorophenyl)-6,6a,7,8,9,10-hexahydro-5H-pyrazino[1',2':4,5]pyrazino[2,3-c]pyridazine-5-carboxylate

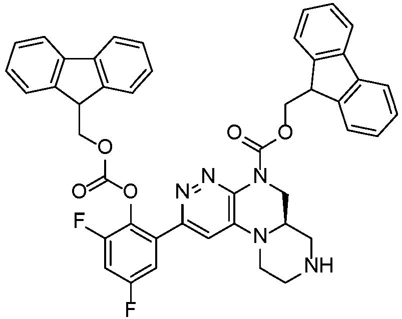

5-((9H-fluoren-9-yl)methyl) 8-(tert-butyl) (R)-2-(2-((((9H-fluoren-9-yl)methoxy) carbonyl)oxy)-3,5-difluorophenyl)-6a,7,9,10-tetrahydro-5*H*-pyrazino[1',2':4,5]pyrazino[2,3-c]pyridazine-5,8(6H)-dicarboxylate (421 mg, 0.487 mmol) was dissolved in DCM (4.87 mL) and treated with a 4 N solution of HCl in 1,4-dioxane (0.610 mL, 2.44 mmol). The reaction was stirred at room temperature for 2 hours. The reaction mixture was concentrated under reduced pressure to give crude (9H-fluoren-9-yl)methyl (R)-2-(2-((((9H-fluoren-9-yl)methoxy) carbonyl)oxy)-3,5-difluorophenyl)-6,6a,7,8,9,10-hexahydro-5*H*-pyrazino[1',2':4,5]pyrazino[2,3-c]pyridazine-5-carboxylate as an HCl salt (407 mg). The material was used without additional purification. LCMS calcd for C₄₅H₃₆F₂N₅O₅ [M+H]⁺: m/z = 764.3; found: 764.2.

### Step 3: ((S)-2-(3,5-difluoro-2-hydroxyphenyl)-5, 6, 6a,7,9,10-hexahydro-8H-pyrazino [1',2':4,5]pyrazino[2,3-c]pyridazin-8-yl)((2R,6R)-2,6-dimethylpiperazin-1-yl)methanone

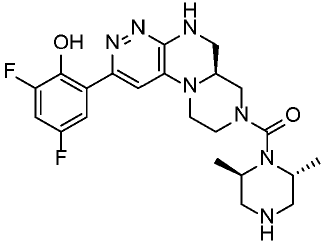

To a vial containing tert-butyl (3R,5R)-3,5-dimethylpiperazine-1-carboxylate (82.0 mg, 0.380 mmol) was added DCM (1.90 mL) and pyridine (0.08 mL, 0.96 mmol), followed by triphosgene (68.1 mg, 0.23 mmol) at 0 °C. The reaction was left to stir at room temperature for 1 hour, upon which the reaction was then was diluted in DCM and a 1 N aqueous solution of HCl. The layers were separated, and the combined organics were dried over Na₂SO₄, filtered and concentrated under reduced pressure. The residue was suspended in DCM (1.90 mL) and crude (9H-fluoren-9-yl)methyl (R)-2-(2-((((9H-fluoren-9-yl)methoxy)carbonyl)oxy)-3,5-difluorophenyl)-6,6a,7,8,9,10-hexahydro-5*H*-pyrazino[1',2':4,5]pyrazino[2,3-*c*]pyridazine-5-carboxylate;dihydrochloride (160 mg, 0.190 mmol) and triethylamine (0.130 mL, 0.960 mmol) were added. The reaction was stirred at room temperature for 48 hours. The crude reaction mixture was diluted in EtOAc and the organic phase was washed with a saturated brine solution. The combined organics were dried over Na₂SO₄, filtered, and concentrated under reduced pressure. The crude residue was dissolved in MeOH (3.00 mL), and water (1.00 mL) and lithium hydroxide (20 eq) were added. The reaction was stirred at 85 °C for 12 hours. The reaction mixture was cooled to room temperature, filtered and the filtrate was purified by prep-HPLC (Waters CSH-C18, 5 uM, 30x100mm, 25.6-45.6% MeCN/water (containing 0.1% TFA) over 5 min) to give boc-intermediate. 2,2,2-trifluoroacetic acid (0.439 mL) was added to a solution of Boc-intermediate in DCM (1.90 mL). The reaction was stirred at room temperature. Upon completion, the reaction mixture was concentrated under reduced pressure to give ((S)-2-(3,5-difluoro-2-hydroxyphenyl)-5,6,6a,7,9,10-hexahydro-8*H*-pyrazino[1',2':4,5]pyrazino[2,3-c]pyridazin-8-yl)((2R,6R)-2,6-dimethylpiperazin-1-yl)methanone as a TFA salt (50.0 mg, 46% yield). The material was used without additional purification. LCMS calcd for C₂₂H₂₈F₂N₇O₂ [M+H]⁺: m/z = 460.2; Found: 460.1.

### Step 4: 3-(6-(4-(((3R, 5R)-4-((S)-2-(3, 5-difluoro-2-hydroxyphenyl)-6, 6a,7,8, 9,10-hexahydro-5H-pyrazino[1',2':4, 5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione

The title compound was prepared using the procedure analogous to those described for preparing Example 1, Step 2 with ((*S*)-2-(3,5-difluoro-2-hydroxyphenyl)-5,6,6a,7,9,10-hexahydro-8*H*-pyrazino[1',2':4,5]pyrazino[2,3-*c*]pyridazin-8-yl)((2*R*,6*R*)-2,6-dimethylpiperazin-1-yl)methanone;2,2,2-trifluoroacetic acid replacing ((2S,6R)-2,6-dimethylpiperazin-1-yl)((S)-2-(3-fluoro-2-hydroxyphenyl)-5,6,6a,7,9,10-hexahydro-8*H*-pyrazino[1',2':4,5]pyrazino[2,3-*c*]pyridazin-8-yl)methanone;2,2,2-trifluoroacetic acid. LCMS calcd for C₄₁H₄₉F₂N₁₀O₅ [M+H]⁺: m/z = 799.4; found: 799.2.

### Examples 46-47:

Examples shown below in Table 10 were prepared as TFA salts by the method used in preparing Example 45 using the appropriate intermediates and starting materials.

**Table 10. Examples 46-47**

| **Example** | **Structure/Name** | **Calcd. (M+H) ⁺ m/z** | **Found (M+H) ⁺ m/z** |
|---|---|---|---|
| **46** | 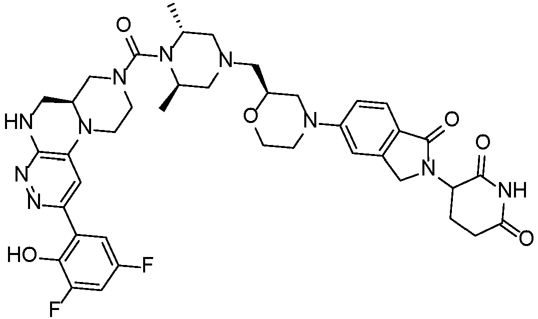 3-(5-((*S*)-2-(((3*R*,5*R*)-4-((*S*)-2-(3,5-difluoro-2-hydroxyphenyl)-6,6a,7,8,9,10-hexahydro-5*H*-pyrazino[1',2':4,5]pyrazino[2,3-*c*] pyridazine-8-carbonyl)-3,5-dimethylpiperazin-1-yl)methyl) morpholino)-1-oxoisoindolin-2-yl)piperidine-2,6-dione | 799.4 | 799.3 |
| **47** | 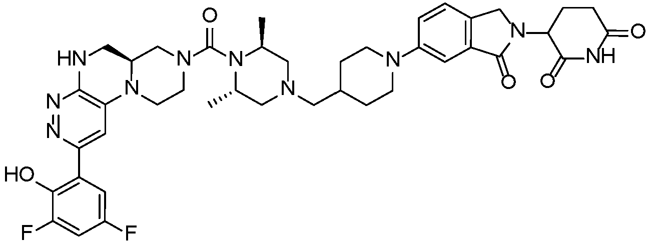 3-(6-(4-(((3*S*,5*S*)-4-((*S*)-2-(3,5-difluoro-2-hydroxyphenyl)-6,6a,7,8,9,10-hexahydro-5*H*-pyrazino[1',2':4,5]pyrazino[2,3-*c*] pyridazine-8-carbonyl)-3,5-dimethylpiperazin-1-yl)methyl) piperidin-1-yl)-1-oxoisoindolin-2-yl)piperidine-2,6-dione | 798.4 | 799.3 |

### Example 48: 3-(6-(4-((1-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)piperidin-4-yl)methyl)piperazin-1-yl)-1-oxoisoindolin-2-yl)piperidine-2,6-dione

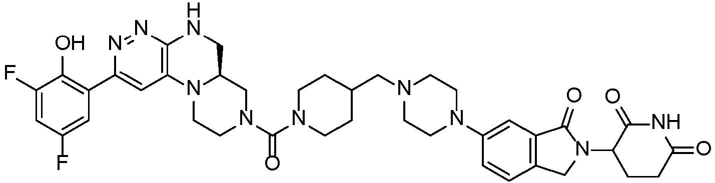

### Step 1. Piperidine-4-carbaldehyde

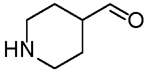

A solution of 4 N HCl in 1,4-dioxane (20 mL, 80 mmol) was added dropwise to a solution of 1-Boc-piperidine-4-carboxaldehyde (601 mg, 2.82 mmol) in 1,4-dioxane (20 mL). The reaction was stirred at room temperature for 2 hours. The reaction mixture was concentrated under reduced pressure to afford the HCl salt of the title compound hydrochloric acid salt (398 mg, 94% yield) as a white solid.

### Step 2. (S)-1-(2-(3,5-difluoro-2-hydroxyphenyl)-6,6a,7,8,9,10-hexahydro-5H-pyrazino[1',2':4,5]pyrazino[2,3-c]pyridazine-8-carbonyl)piperidine-4-carbaldehyde

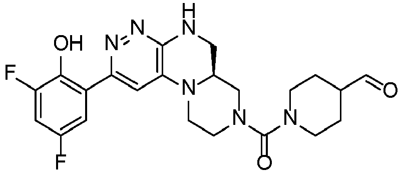

A solution of triphosgene (82.0 mg, 0.28 mmol) in DCM (0.5 mL) was added to a solution of piperidine-4-carbaldehyde;hydrochloride (111 mg, 0.740 mmol) and pyridine (0.120 mL, 1.48 mmol) in DCM (2.50 mL) at 0 °C. The reaction was stirred at 0 °C for 1 hour, then warmed to room temperature and stirred for 3 hours. The reaction was poured into a 1 N aqueous HCl solution (5.00 mL) and the aqueous phase was extracted with DCM (2 x 3 mL). The combined organics were dried over MgSO₄, filtered and concentrated under reduced pressure. The crude residue was dissolved in DCM (3 mL) and 2,4-difluoro-6-[(10R)-1,5,6,8,12-pentazatricyclo[8.4.0.02,7]tetradeca-2(7),3,5-trien-4-yl]phenol;dihydrochloride (73.0 mg, 0.180 mmol, Intermediate 2) and triethylamine (0.10 mL, 0.74 mmol) were added. The reaction was stirred at room temperature for 1 hour. The reaction mixture was diluted in methanol (15 mL), filtered, and the filtrate was purified by prep-HPLC on a C18 column (11.5-31.5% MeCN in 0.1% TFA (aq.), pH = 2) to afford the TFA salt of the title compound (27.0 mg, 26% yield) as a light yellow oil. LCMS calc. for C₂₂H₂₅F₂N₆O₃ [M+H]⁺: m/z = 459.2; Found: 459.0.

### Step 3: 3-(1-oxo-6-(piperazin-1-yl)isoindolin-2-yl)piperidine-2,6-dione

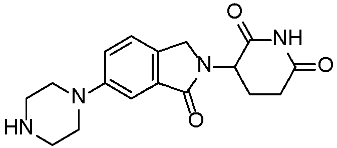

To a vial containing tert-butyl 4-[2-(2,6-dioxopiperidin-3-yl)-3-oxo-1H-isoindol-5-yl]piperazine-1-carboxylate (136.0 mg, 0.320 mmol, Intermediate 23) was added methanol (1.50 mL) and 4N HCl dioxane (1.50 mL, 6.00 mmol). Reaction stirred for 1.5 hours. Reaction was condensed to yield 3-(1-oxo-6-(piperazin-1-yl)isoindolin-2-yl)piperidine-2,6-dione;hydrochloride (115.0 mg, 99% yield) as an off white solid. LCMS calc. for C₁₇H₂₁N₄O₃ [M+H]⁺: m/z = 329.2; Found: 329.1.

### Step 4: 3-(6-(4-((1-((S)-2-(3, 5-difluoro-2-hydroxyphenyl)-6, 6a,7, 8,9,10-hexahydro-5H-pyrazino[1',2':4,5]pyrazino[2,3-c]pyridazine-8-carbonyl)piperidin-4-yl)methyl)piperazin-1-yl)-1-oxoisoindolin-2-yl)piperidine-2,6-dione

A solution of (*S*)-1-(2-(3,5-difluoro-2-hydroxyphenyl)-6,6a,7,8,9,10-hexahydro-5*H-*pyrazino[1',2':4,5]-pyrazino[2,3-*c*]pyridazine-8-carbonyl)piperidine-4-carbaldehyde;2,2,2-trifluoroacetic acid (34.0 mg, 0.060 mmol), 3-(1-oxo-6-(piperazin-1-yl)isoindolin-2-yl)piperidine-2,6-dione;hydrochloride) (33.0 mg, 90.0 µmol), magnesium sulfate (29.0 mg, 0.240 mmol) and sodium acetate (28.0 mg, 0.340 mmol) in DMSO (1.00 mL) was stirred at 40°C for 45 minutes. Sodium triacetoxyborohydride (55.0 mg, 0.26 mmol) was added and the reaction was stirred at room temperature for 6 hours. The reaction was quenched with water (1.0 mL) and diluted with DMSO (15.0 mL). The mixture was filtered and the filtrate was purified by prep-HPLC on a C18 column (7.6-27.6% MeCN in 0.1% TFA (aq.), pH = 2) to afford the TFA salt of the title compound (32.0 mg, 30% yield) as a white solid. LCMS calc. for C₃₉H₄₅F₂N₁₀O₅ [M+H]⁺: m/z = 771.4; Found: 771.2.

### Example 49: 3-(6-(2-(((1R,5S,6s)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-azabicyclo [3.1.0]hexan-3-yl)methyl)morpholino)-1-oxoisoindolin-2-yl)piperidine-2,6-dione

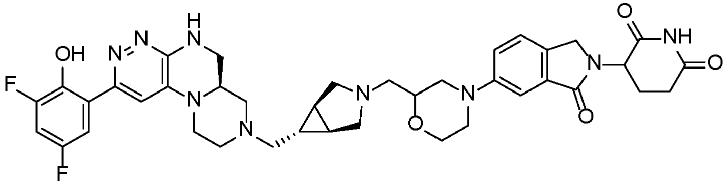

To a solution of 3-[5-[2-(hydroxymethyl)morpholin-4-yl]-3-oxo-1H-isoindol-2-yl]piperidine-2,6-dione (86.3 mg, 0.240 mmol, Intermediate 26) in DMSO (1.50 mL) was added triethylamine (0.33 mL, 2.40 mmol). The reaction was cooled to 0°C and a solution of sulfur trioxide-pyridine (232.0 mg, 1.46 mmol) in DMSO (1.0 mL) was added dropwise. After warming to room temperature and stirring for 3 hours, a solution of 2-[(10*S*)-12-[[(1*S*,5*R*)-3-azabicyclo [3.1.0]hexan-6-yl]methyl]-1,5,6,8,12-pentazatricyclo-[8.4.0.02,7]tetradeca-2(7),3,5-trien-4-yl]-4,6-difluorophenol;2,2,2-trifluoroacetic acid (77.1 mg, 0.120 mmol, Example 37 and 38, Step 1) in MeCN (2.50 mL) was added. At 0°C was added acetic acid (0.55 mL, 9.61 mmol). After 10 minutes on ice, sodium triacetoxyborohydride (256.0 mg, 1.21 mmol) was added and the reaction mixture was left to warm to room temperature and stirred overnight. Upon completion, water was added and diluted in DMSO. The reaction mixture was filtered and the filtrate was purified by prep-HPLC (Waters CSH Phenyl-hexyl, 5 uM, 30x100mm, 8.0-20.0 % MeCN/water (containing 0.1%TFA) over 5 min) to yield 3-(6-(2-(((1*R*,5*S*,6*s*)-6-(((*S*)-2-(3,5-difluoro-2-hydroxyphenyl)-5,6,6a,7,9,10-hexahydro-8H-pyrazino[1',2':4,5]pyrazino[2,3-*c*]pyridazin-8-yl)methyl)-3-azabicyclo[3.1.0]hexan-3-yl)methyl)morpholino)-1-oxoisoindolin-2-yl)piperidine-2,6-dione as its TFA salt (35.2 mg, 13%). ¹H NMR (400 MHz, DMSO) δ 10.99 (s, 1H), 9.76 (s, 1H), 7.95 (s, 1H), 7.62 (s, 1H), 7.49 (d, *J= 8.4* Hz, 1H), 7.39 (d, *J =* 15.7 Hz, 2H), 7.30 - 7.25 (m, 1H), 7.23 (d, *J* = 2.3 Hz, 1H), 5.09 (dd, *J=* 13.3, 5.1 Hz, 1H), 4.46 (s, 2H), 4.36 (d, *J=* 16.9 Hz, 2H), 4.23 (d, *J =* 16.8 Hz, 2H), 4.05 - 3.94 (m, 3H), 3.80 - 3.60 (m, 9H), 3.44 (d, *J =* 36.4 Hz, 4H), 3.30 (d, *J =* 11.3 Hz, 3H), 3.18 - 2.84 (m, 5H), 2.77 (t, *J =* 10.9 Hz, 1H), 2.70 - 2.53 (m, 2H), 2.42 - 2.31 (m, 1H), 2.04 - 1.87 (m, 3H), 1.52 (s, 1H). LCMS calcd for C₃₉H₄₄F₂N₉O₅ [M+H]⁺: m/z= 756.3; found: 756.2.

### Examples 50-53:

Examples shown below in Table 11 were prepared as TFA salts by the method described in the preparation of Example 49 using the appropriate intermediates and starting materials.

**Table 11. Examples 50-53**

| **Example** | **Structure/Name** | **Calcd. (M+H) ⁺ m/z** | **Found (M+H) ⁺ m/z** |
|---|---|---|---|
| **50** | 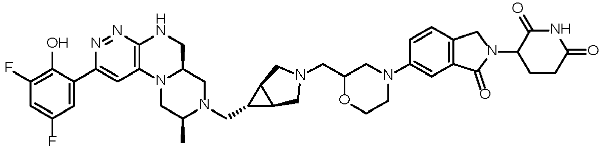 3-(6-(2-(((1*R*,5*S*,6*r*)-6-(((6a*S*,9*S*)-2-(3,5-difluoro-2-hydroxyphenyl)-9-methyl-5,6,6a,7,9,10-hexahydro-8*H*-pyrazino[1',2':4,5]pyrazino[2,3-c]pyridazin-8-yl)methyl)-3-azabicyclo[3.1.0]hexan-3-yl)methyl) morpholino)-1-oxoisoindolin-2-yl)piperidine-2,6-dione | 770.4 | 770.2 |
| **51** | 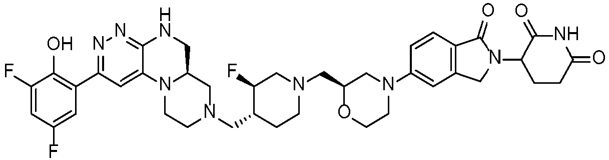 3-(5-((S)-2-(((3R,4R)-4-(((S)-2-(3,5-difluoro-2-hydroxyphenyl)-5,6,6a,7,9,10-hexahydro-8*H*-pyrazino[1',2':4,5]pyrazino[2,3-c]pyridazin-8-yl)methyl)-3-fluoropiperidin-1-yl)methyl)morpholino)-1-oxoisoindolin-2-yl)piperidine-2,6-dione | 776.4 | 776.2 |
| **52** | 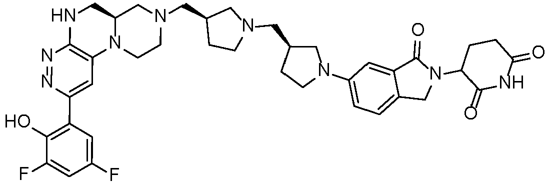 3-(6-((*S*)-3-(((*R*)-3-(((*S*)-2-(3,5-difluoro-2-hydroxyphenyl)-5,6,6a,7,9,10-hexahydro-8*H*-pyrazino[1',2':4,5]pyrazino[2,3-*c*] pyridazin-8-yl)methyl)pyrrolidin-1-yl)methyl)pyrrolidin-1-yl)-1-oxoisoindolin-2-yl)piperidine-2,6-dione | 728.4 | 728.2 |
| **53** | 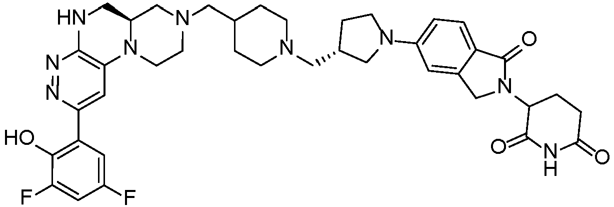 3-(5-((S)-3-((4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)piperidin-1-yl)methyl)pyrrolidin-1-yl)-1-oxoisoindolin-2-yl)piperidine-2,6-dione | 742.4 | 742.2 |

### Example 54: 3-(6-(4-(((1S,4r)-4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)cyclohexyl) methyl)piperazin-1-yl)-1-oxoisoindolin-2-yl)piperidine-2,6-dione

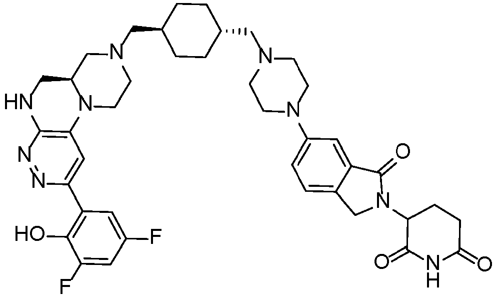

### Step 1: ((1r,4r)-4-(((tetrahydro-2H-pyran-2-yl)oxy)methyl)cyclohexyl)methanol

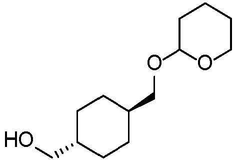

p-Toluenesulfonic acid (36.0 mg, 0.209 mmol) followed by 3,4-dihydro-2H-pyran (1.38 mL, 15.1 mmol) was added to a solution of ((1*r*,4*r*)-cyclohexane-1,4-diyl)dimethanol (2.00 g, 13.9 mmol) in DCM (30 mL) at 0 °C. The reaction was stirred at 0 °C for 30 min, then warmed to room temperature and stirred overnight. The crude reaction mixture was poured into a saturated aqueous solution of sodium bicarbonate and the layers were separated. The organics were washed with water, dried over magnesium sulfate, filtered, and concentrated under reduced pressure. The crude material was purified by silica gel flash column chromatography, eluting with a gradient of 0-100% EtOAc/hexane to give ((1*r*,4*r*)-4-(((tetrahydro-2*H*-pyran-2-yl)oxy)methyl)cyclohexyl)methanol (850 mg, 27% yield) as a clear oil. *Note: Product does not ionize on LCMS. Intermediate was used directly in next step.*

### Step 2: (1r,4r)-4-(((tetrahydro-2H-pyran-2-yl)oxy)methyl)cyclohexane-1-carbaldehyde

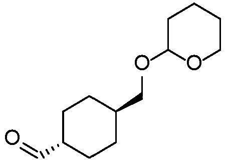

A solution of ((1*r*,4*r*)-4-(((tetrahydro-2*H*-pyran-2-yl)oxy)methyl)cyclohexyl)methanol (286 mg, 1.25 mmol) and Dess-Martin periodinane (706 mg, 1.66 mmol) in DCM (12.5 mL) was stirred at room temperature for 2.5 hours. The crude reaction mixture was diluted with DCM and a saturated aqueous solution of sodium bicarbonate was added. The layers were separated and the organics were dried over magnesium sulfate, filtered and concentrated under reduced pressure to give crude (1*r*,4*r*)-4-(((tetrahydro-2*H*-pyran-2-yl)oxy)methyl)cyclohexane-1-carbaldehyde as a white solid. The material was used in the following step without additional purification. *Note: Product does not ionize. Was used directly in next step.*

### Step 3: 3-(1-oxo-6-(4-(((1r,4r)-4-(((tetrahydro-2H-pyran-2-yl)oxy)methyl)cyclohexyl) methyl)piperazin-1-yl)isoindolin-2-yl)piperidine-2,6-dione

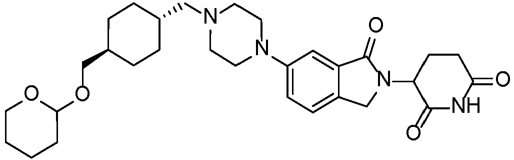

The title compound was prepared using the procedure analogous to that described for preparing Example 48, Step 4 with crude (1*r*,4*r*)-4-(((tetrahydro-2*H*-pyran-2-yl)oxy)methyl) cyclohexane-1-carbaldehyde replacing (*S*)-1-(2-(3,5-difluoro-2-hydroxyphenyl)-6,6a,7,8,9,10-hexahydro-5*H*-pyrazino[1',2':4,5]pyra-zino[2,3-*c*]pyridazine-8-carbonyl)piperidine-4-carbaldehyde;2,2,2-trifluoroacetic acid. LCMS calcd for C₃₀H₄₃N₄O₅ [M+H]⁺: m/z = 539.3; found: 539.3.

### Step 4: 3-(6-(4-(((1r,4r)-4-(hydroxymethyl)cyclohexyl)methyl)piperazin-1-yl)-1-oxoisoindolin-2-yl)piperidine-2,6-dione

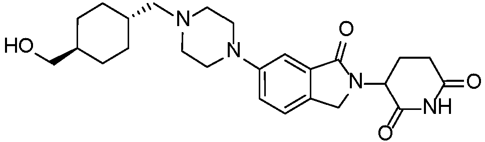

To a 20 mL vial containing a solution of 3-(1-oxo-6-(4-(((1r,4r)-4-(((tetrahydro-2H-pyran-2-yl)oxy)methyl)cyclohexyl)methyl)piperazin-1-yl)isoindolin-2-yl)piperidine-2,6-dione (30.0 mg, 60.0 µmol) in DCM (0.25 mL) and methanol (0.25 mL) was added *p*-TSOH H2O (1.50 mg, 0.01 mmol) in a 1 to 1 mixture of DCM (0.250 mL) and methanol (0.250 mL). The reaction mixture was stirred at 35 °C for 72 hours. The crude reaction mixture was diluted with methanol (5 mL) and the product was purified by prep-HPLC (Waters CSH Phenyl-hexyl, 5 uM, 30x100mm, 5.8-25.8% MeCN/water (containing 0.1%TFA) over 5 min) to yield the TFA salt of 3-(6-(4-(((1*r*,4*r*)-4-(hydroxymethyl)cyclohexyl)methyl)piperazin-1-yl)-1-oxoisoindolin-2-yl)piperidine-2,6-dione (13 mg, 51% yield) as a white solid. LCMS calcd for C₂₅H₃₅N₄O₄ [M+H]⁺: m/z = 455.3; found: 455.2

### Step 5: 3-(6-(4-(((1S,4r)-4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)cyclohexyl) methyl)piperazin-1-yl)-1-oxoisoindolin-2-yl)piperidine-2, 6-dione

The title compound was prepared using the procedure analogous to those described for preparing Example 49 with *3-(6-(4-(((1r,4r)-4-(hydroxymethyl)cyclohexyl)methyl)piperazin-1-yl)-1-oxoisoindolin-2-yl)piperidine-2,6-dione* replacing 3-[5-[2-(hydroxymethyl)morpholin-4-yl]-3-oxo-1*H*-isoindol-2-yl]-piperidine-2,6-dione and Intermediate 2 replacing 2-[(10*S*)-12-[[(1*S*,5*R*)-3-azabicyclo[3.1.0]hexan-6-yl]methyl]-1,5,6,8,12-pentazatricyclo[8.4.0.02,7]tetradeca-2(7),3,5-trien-4-yl]-4,6-difluorophenol;2,2,2-trifluoroacetic acid. LCMS calcd for C₄₀H₄₈F₂N₉O₄ [M+H]⁺: m/z = 756.4; found: 756.3.

### Example 55: 3-(6-(4-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione

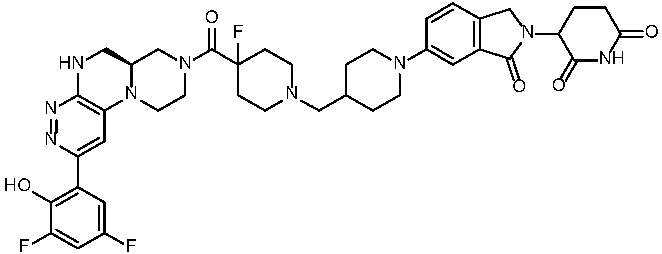

### Step 1: tert-butyl (S)-4-(2-(3, 5-difluoro-2-hydroxyphenyl)-6, 6a,7, 8,9,10-hexahydro-5H-pyrazino[1',2':4,5]pyrazino[2,3-c]pyridazine-8-carbonyl)-4-fluoropiperidine-1-carboxylate

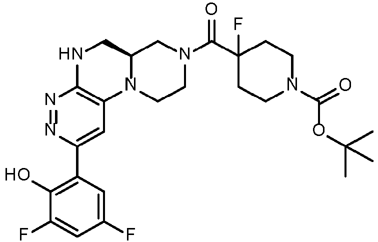

A mixture of N,N-Diisopropylethylamine (50.0 µL, 0.310 mmol), 1-[Bis(dimethylamino)-methylene]-1H-1,2,3-triazolo[4,5-b]pyridinium 3-oxid hexafluorophosphate (35.1 mg, 90.0 µmol), 2,4-difluoro-6-[(10*R*)-1,5,6,8,12-pentazatricyclo[8.4.0.02,7]tetradeca-2,4,6-trien-4-yl]phenol;dihydrochloride (33.2 mg, 80.0 µmol, Intermediate 2) and 4-fluoro-1-[(2-methylpropan-2-yl)oxycarbonyl]piperidine-4-carboxylic acid (19.0 mg, 80.0 µmol) in DMF (200 µL) was stirred at room temperature for 2h. The mixture was diluted with DCM, washed with water, concentrated and purified by silica gel flash column chromatography (0-10% MeOH/DCM) to give *tert*-butyl (S)-4-(2-(3,5-difluoro-2-hydroxyphenyl)-6,6a, 7,8,9,10-hexahydro-5*H*-pyrazino[1',2':4,5]pyrazino[2,3-*c*]pyridazine-8-carbonyl)-4-fluoropiperidine-1-carboxylate (35.0 mg, 83% yield). LCMS calcd for C₂₆H₃₂F₃N₆O₄ [M+H]⁺: m/z= 549.2; found: 549.1.

### Step 2: (S)-(2-(3,5-difluoro-2-hydroxyphenyl)-5, 6, 6a,7,9,10-hexahydro-8H-pyrazino [1',2':4,5]pyrazino[2,3-c]pyridazin-8-yl)(4-fluoropiperidin-4-yl)methanone

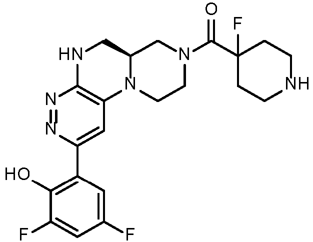

*tert-Butyl* (*S*)-4-(2-(3,5-difluoro-2-hydroxyphenyl)-6,6a,7,8,9,10-hexahydro-5*H-*pyrazino[1',2':4,5]-pyrazino[2,3-*c*]pyridazine-8-carbonyl)-4-fluoropiperidine-1-carboxylate (35.0 mg, 60.0 µmol) was treated with 4 N hydrochloric acid in 1,4-dioxane (0.56 mL, 18.2 mmol) in DCM (1.00 mL) at room temperature for 1 h. The mixture was concentrated under reduced pressure to give (*S*)-(2-(3,5-difluoro-2-hydroxyphenyl)-5,6,6a,7,9,10-hexahydro-8*H-*pyrazino[1',2':4,5]pyrazino[2,3-*c*]pyridazin-8-yl)(4-fluoropiperidin-4-yl)methanone;dihydrochloride (16.0 mg, 48% yield). LCMS calcd for C₂₁H₂₄F₃N₆O₂ [M+H]⁺: m/z= 449.2; found: 449.0.

### Step 3: 3-(6-(4-((4-((S)-2-(3,5-difluoro-2-hydroxyphenyl)-6,6a,7,8,9, 10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione

3-[5-[4-(hydroxymethyl)piperidin-1-yl]-3-oxo-1H-isoindol-2-yl]piperidine-2,6-dione (22.0 mg, 60.0 µmol, Intermediate 20) was dissolved in DMSO (1.00 mL) and then triethylamine (80.0 µL, 0.550 mmol) was added. Sulfurtrioxide pyridine (29.3 mg, 0.180 mmol) in DMSO (500 µL) was added dropwise and the reaction was stirred for 1 hour. After 1 hour, [(10*S*)-4-(3,5-difluoro-2-hydroxyphenyl)-1,5,6,8,12-pentazatricyclo[8.4.0.02,7]tetradeca-2(7),3,5-trien-12-yl]-(4-fluoropiperidin-4-yl)methanone;dihydrochloride (16.0 mg, 30.0 µmol) and acetic acid (50 µL, 0.920 mmol) was added in ACN (1.00 mL). The mixture was stirred for 30 minutes. Sodium triacetoxyborohydride (52.0 mg, 0.250 mmol) was added. The mixture was stirred at room temperature for 1 hour. The mixture was purified by prep-HPLC (Waters CSH C18, 5 uM, 30x100mm, 7.7-27.7% MeCN/water (containing 0.1%TFA) over 5 min) to obtain 3-(6-(4-((4-((*S*)-2-(3,5-difluoro-2-hydroxyphenyl)-6,6a,7,8,9,10-hexahydro-5*H*-pyrazino[1',2':4,5] pyrazino[2,3-c]pyridazine-8-carbonyl)-4-fluoropiperidin-1-yl)methyl)piperidin-1-yl)-1-oxoisoindolin-2-yl)piperidine-2,6-dione as a TFA salt (6.20 mg, 26% yield). LCMS calcd for C₄₀H₄₅F₃N₉O₅ [M+H]⁺: m/z= 788.4; found: 788.1.

### Example 56: 3-(6-(4-(((1R,4s)-4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)cyclohexyl) methyl)piperazin-1-yl)-1-oxoisoindolin-2-yl)piperidine-2,6-dione

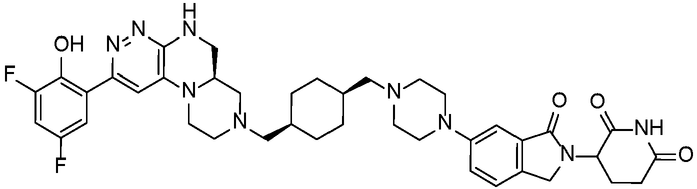

### Step 1: (1s,4s)-4-(((tetrahydro-2H-pyran-2-yl)oxy)methyl)cyclohexane-1-carboxylic acid

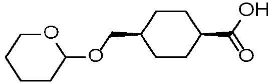

To a 100 mL round bottom flask was added cis-4-(hydroxymethyl)cyclohexanecarboxylic acid (2.24 g, 14.2 mmol), DCM (33.0 mL) and *p*-TSOH·H₂O (49.0 mg, 0.260 mmol). Reaction was heated to 40°C to help partially dissolve the mixture. The reaction was cooled to -78 °C and a solution of 3,4-Dihydro-2H-pyran (1.41 mL, 15.5 mmol) in DCM (33.0 mL) was added dropwise via an addition funnel over 35 minutes. The reaction warmed to room temperature. After 4.5 hours, the reaction was quenched with 30.0 mL sat. sodium bicarbonate and stirred vigorously for 10 minutes. The solution was poured into a separatory funnel with more sat. sodium bicarbonate and DCM. The organic layer was separated, dried and concentrated under reduced pressure. The residue was purified by silica gel flash column (120 g) chromatography eluting with a gradient of 0-100% EtOAC/Hexanes to yield (1s,4s)-4-(((tetrahydro-2H-pyran-2-yl)oxy)methyl)cyclohexane-1-carboxylic acid (1.00 g, 29% yield) as a clear oil. *Note: product did not ionize and was used as is directly in next step without additional purification*

### Step 2: ((1s,4s)-4-(((tetrahydro-2H-pyran-2-yl)oxy)methyl)cyclohexyl)methanol

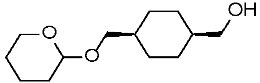

(1s,4s)-4-(((Tetrahydro-2H-pyran-2-yl)oxy)methyl)cyclohexane-1-carboxylic acid (135 mg, 0.560 mmol) was dissolved in THF (1.11 mL) then cooled to 0°C. Borane tetrahydrofuran (0.560 mL, 0.560 mmol) was added dropwise. The reaction was stirred at 0°C for 10 minutes, then slowly allowed to warm to room temperature. After 5 hours, the reaction was quenched with MeOH, concentrated under reduced pressure, then taken up in EtOAc and poured into water. The aqueous phase was extracted with EtOAc (2x 20 mL). The combined organic layers were washed with water followed by brine, dried over MgSO₄, filtered and concentrated under reduced pressure to yield ((1s,4s)-4-(((tetrahydro-2*H*-pyran-2-yl)oxy)methyl)cyclohexyl)methanol (75.5 mg, 59% yield) as a clear oil. *Note: product did not ionize and was used as is directly in next step without additional purification*

### Step 3: 3-(1-oxo-6-(4-(((1s,4s)-4-(((tetrahydro-2H-pyran-2-yl)oxy)methyl) cyclohexyl)methyl)piperazin-1-yl)isoindolin-2-yl)piperidine-2,6-dione

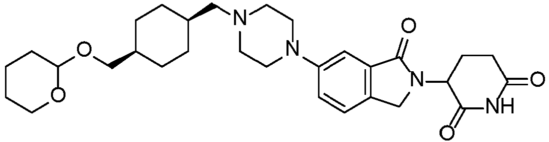

((1*s*,4*s*)-4-(((tetrahydro-2*H*-pyran-2-yl)oxy)methyl)cyclohexyl)methanol (28.7 mg, 0.13 mmol) was dissolved in DMSO (1.2 mL) and triethylamine (0.14 mL, 1.01 mmol). Reaction was cooled to 0°C and a solution of sulfur trioxide pyridine (107 mg, 0.670 mmol) in DMSO (1.20 mL) was added dropwise. After 3.5 hours at RT, 3-(3-oxo-5-piperazin-1-yl-1H-isoindol-2-yl)piperidine-2,6-dione (41.3 mg, 0.130 mmol, Example 48, Step 3), MeCN (1.20 mL) and acetic acid (0.25 mL, 4.4 mmol) was added. The reaction mixture was stirred at room temp for 15 minutes, then sodium triacetoxyborohydride (92.9 mg, 0.44 mmol) was added. After 3 hours, the reaction was quenched with water, diluted in 20 mL of DMSO and loaded onto LCMS auto purification system (17.6-37.6% ACN in water 0.1%TFA over 5 min, Waters CSH-C18 column) to yield 3-(1-oxo-6-(4-(((1s,4s)-4-(((tetrahydro-2H-pyran-2-yl)oxy)methyl)cyclohexyl)-methyl)piperazin-1-yl)isoindolin-2-yl)piperidine-2,6-dione (33 mg, 50%) as its TFA salt (white powder). LCMS calcd for C₃₀H₄₃N₄O₅ [M+H]⁺: m/z = 539.3; Found: 539.2.

### Step 4: 3-(6-(4-(((1s,4s)-4-(hydroxymethyl)cyclohexyl)methyl)piperazin-1-yl)-1-oxoisoindolin-2-yl)piperidine-2,6-dione

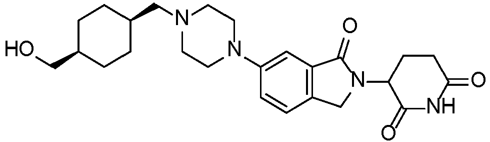

To 3-(1-oxo-6-(4-(((1s,4s)-4-(((tetrahydro-2H-pyran-2-yl)oxy)methyl)cyclohexyl)methyl) piperazin-1-yl)-isoindolin-2-yl)piperidine-2,6-dione (33.0 mg, 0.060 mmol) was added 1,4-dioxane (800 µL) and 4N HCl in dioxane (420 µL, 1.68 mmol). After 1.5 hours, the volatiles were removed *in vacuo* to yield 3-(6-(4-(((1s,4s)-4-(hydroxymethyl)cyclohexyl)methyl) piperazin-1-yl)-1-oxoisoindolin-2-yl)piperidine-2,6-dione (44.0 mg, quantitative yield) as the HCl salt as a white solid. LCMS calcd for C₂₅H₃₅N₄O₄ [M+H]⁺: m/z = 455.3; Found: 455.1.3

### Step 5: 3-(6-(4-(((1R,4s)-4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)cyclohexyl)methyl) piperazin-1-yl)-1-oxoisoindolin-2-yl)piperidine-2,6-dione

3-(6-(4-(((1s,4s)-4-(hydroxymethyl)cyclohexyl)methyl)piperazin-1-yl)-1-oxoisoindolin-2-yl)piperidine-2,6-dione (21.0 mg, 50.0 µmol) was dissolved in DMSO (500 µL), Triethylamine;TEA (50.0 µL, 0.390 mmol). Reaction was cooled to 0 °C and sulfur trioxide pyridine (42.0 mg, 0.26 mmol) in DMSO (0.5 mL) was added dropwise. After 3 hours at room temp, (R)-2,4-difluoro-6-(6,6a,7,8,9,10-hexahydro-5H-pyrazino[1',2':4,5]pyrazino[2,3-c]pyridazin-2-yl)phenol (14.7 mg, 50.0 µmol, Intermediate 2), MeCN (1.00 mL), and acetic acid (110 µL, 1.85 mmol) was added and stirred for 80 minutes. Sodium triacetoxyborohydride (48.96 mg, 0.23 mmol) was then added and reaction was left to stir at RT. After stirring overnight, the reaction was quenched with water, diluted in 15 mL of DMSO and directly purified using a LCMS auto-purification system (10.4-23.4% ACN in water 0.1%TFA over 5 min, Waters CSH-C18 column) to yield 3-(6-(4-(((1R,4s)-4-(((S)-2-(3,5-difluoro-2-hydroxyphenyl)-5,6,6a,7,9,10-hexahydro-8*H*-pyrazino[1',2':4,5]pyrazino[2,3-*c*]pyridazin-8-yl)methyl)cyclohexyl)methyl)piperazin-1-yl)-1-oxoisoindolin-2-yl)piperidine-2,6-dione (9.80 mg, 19%) as a TFA salt as a white solid. LCMS calcd for C₄₀H₄₈F₂N₉O₄ [M+H]⁺: m/z = 756.4; Found: 756.2.

### Example 57: 3-(6-(4-(((2S,5R)-4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,5-dimethyl-piperazin-1-yl)methyl)piperidin-1-yl)-1-oxoisoindolin-2-yl)piperidine-2,6-dione

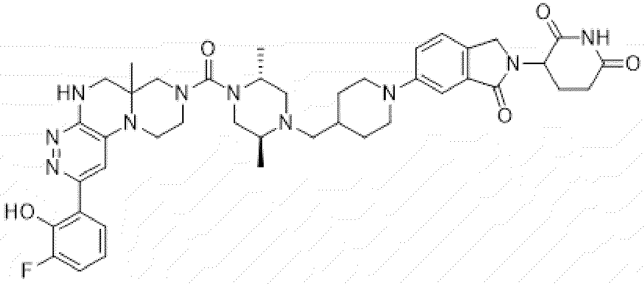

The title compound was prepared as a TFA salt using the procedure analogous to those described for Intermediate 4, steps 1-10, with *(2S,5R)-tert-*butyl 2,5-dimethylpiperazine-1-carboxylate replacing *tert-*butyl (3*R*,5*S*)-3,5-dimethylpiperazine-1-carboxylate in Step 9 and Example 1 using the appropriate starting materials and intermediates. LCMS calcd for C₄₂H₅₂FN₁₀O₅ [M+H]⁺: m/z = 795.4; Found: 795.1.

### Example 58: 3-(6-(4-(((1S,4r)-4-(((S)-2-(3-fluoro-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidine-2,6-dione

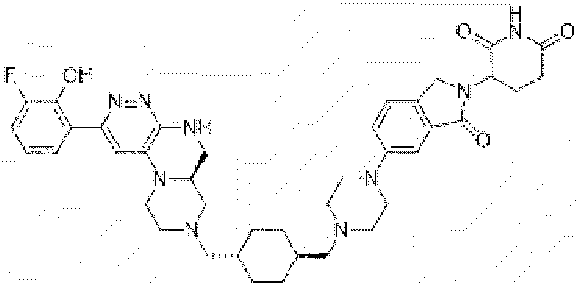

The title compound was prepared as a TFA salt using the procedure analogous to those described for Example 54, steps 1-5, with Intermediate 1 replacing Intermediate 2 in Step 5. LCMS calcd for C₄₀H₄₉FN₉O₄ [M+H]⁺: m/z = 738.4; Found: 738.2.

### Example 59: 3-(6-(4-(((3R,5S)-4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,5-dimethyl-piperazin-1-yl)methyl)piperidin-1-yl)-1-oxoisoindolin-2-yl)piperidine-2,6-dione (Isomer 1)

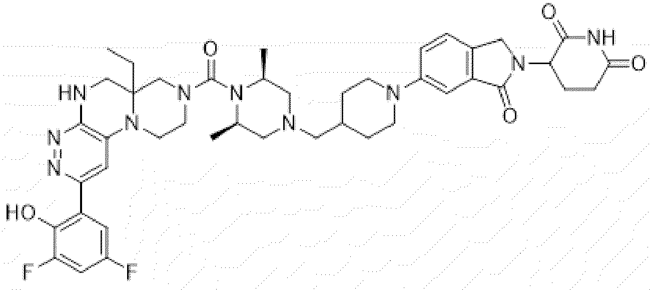

The title compound was prepared as the TFA salt using the procedure analogous to those described for Example 1 using the appropriate starting materials and intermediates. LCMS calcd for C₄₃H₅₃F₂N₁₀O₅ [M+H]⁺: m/z = 827.4; Found: 827.2.

### Example 60: 3-(6-(4-(((3R,5S)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethyl-piperazin-1-yl)methyl)piperidin-1-yl)-1-oxoisoindolin-2-yl)piperidine-2,6-dione (Isomer 1)

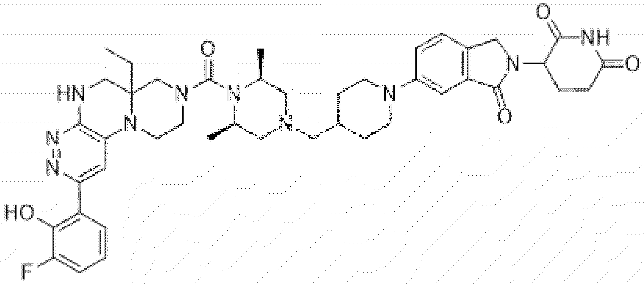

The title compound was prepared as the TFA salt using the procedure analogous to those described for Example 1 using the appropriate starting materials and intermediates. LCMS calcd for C₄₃H₅₄FN₁₀O₅ [M+H]⁺: m/z = 809.4; Found: 809.2.

### Example 61: 3-(6-(4-(((3R,5S)-4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,5-dimethyl-piperazin-1-yl)methyl)piperidin-1-yl)-1-oxoisoindolin-2-yl)piperidine-2,6-dione (Isomer 2)

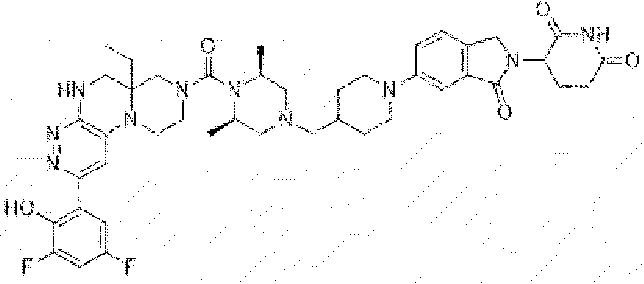

The title compound was prepared as the TFA salt using the procedure analogous to those described for Example 1 using the appropriate starting materials and intermediates. LCMS calcd for C₄₃H₅₃F₂N₁₀O₅ [M+H]⁺: m/z = 827.4; Found: 827.1.

### Example 62: 3-(6-(4-(((3R,5S)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethyl-piperazin-1-yl)methyl)piperidin-1-yl)-1-oxoisoindolin-2-yl)piperidine-2,6-dione (Isomer 2)

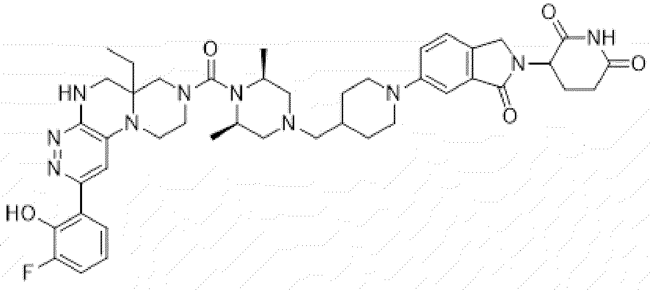

The title compound was prepared as the TFA salt using the procedure analogous to those described for Example 1 using the appropriate starting materials and intermediates. LCMS calcd for C₄₃H₅₄FN₁₀O₅ [M+H]⁺: m/z = 809.4; Found: 809.4.

### Example 63: 3-(6-(4-(((3R,5R)-4-(2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethyl-piperazin-1-yl)methyl)piperidin-1-yl)-1-oxoisoindolin-2-yl)piperidine-2,6-dione (Isomer 2)

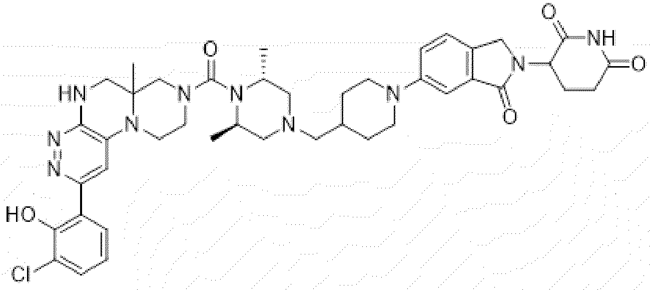

The title compound was prepared as the TFA salt using the procedure analogous to those described for Example 1 using the appropriate starting materials and intermediates. LCMS calcd for C₄₂H₅₂ClN₁₀O₅ [M+H]⁺: m/z = 811.4; Found: 811.0.

### Example 64: 3-(6-(4-(((3R,5S)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethyl-piperazin-1-yl)methyl)piperidin-1-yl)-1-oxoisoindolin-2-yl)piperidine-2,6-dione (Isomer 2)

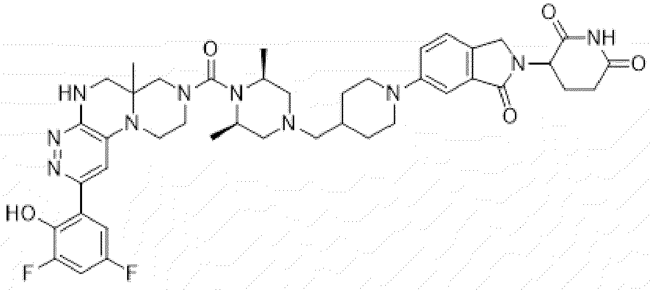

The title compound was prepared as the TFA salt using the procedure analogous to those described for Example 1 using the appropriate starting materials and intermediates. LCMS calcd for C₄₂H₅₁F₂N₁₀O₅ [M+H]⁺: m/z = 813.4; Found: 813.1.

### Example 65: (S)-3-(6-(4-((4-((R)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione

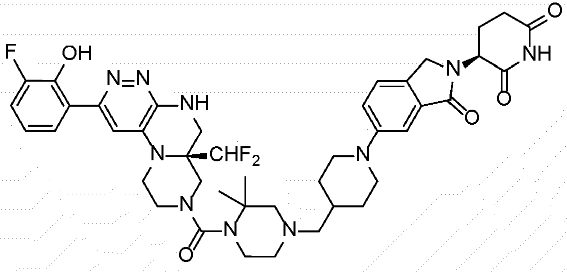

Sodium triacetoxyborohydride (719 mg, 3.4mmol) was added to a stirring solution of acetic acid (0.48 mL, 4.24 mmol), (R)-(6a-(difluoromethyl)-2-(3-fluoro-2-hydroxyphenyl)-5,6,6a,7,9,10-hexahydro-8*H*-pyrazino[1',2':4,5]pyrazino[2,3-*c*]pyridazin-8-yl)(2,2-dimethylpiperazin-1-yl)methanone (610 mg, 0.85 mmol), and Intermediate 38 (445 mg, 1.28 mmol) in DMF (10 mL) at 0 °C. The reaction mixture was stirred 1 hour at 0 °C. The product mixture was diluted with DMSO and purified by prep-HPLC (Waters CSH-C18, 5 uM, 30x100mm, 8.1-28.1% MeCN/water (containing 0.1%TFA) over 5 min) to give (S)-3-(6-(4-((4-((R)-6a-(difluoromethyl)-2-(3-fluoro-2-hydroxyphenyl)-6,6a,7,8,9,10-hexahydro-5H-pyrazino [1',2':4,5]pyrazino[2,3-*c*]pyridazine-8-carbonyl)-3,3-dimethylpiperazin-1-yl)methyl)piperidin-1-yl)-1-oxoisoindolin-2-yl)piperidine-2,6-dione as its TFA salt (235 mg, 24%). ¹H NMR (400 MHz, MeOD) δ 7.46 (s, 1H), 7.39 - 7.28 (m, 5H), 7.03 (s, 1H), 6.27 (t, *J =* 54.6 Hz, 1H), 5.13 (d, *J=* 13.4 Hz, 1H), 4.38 (s, 3H), 4.18 (s, 1H), 4.01 (d, *J* = 9.9 Hz, 1H), 3.90 - 3.76 (m, 3H), 3.65 - 3.35 (m, 7H), 3.15 (s, 4H), 2.97 - 2.84 (m, 4H), 2.78 (d, *J =* 17.6 Hz, 1H), 2.49 (q, *J =* 13.8 Hz, 1H), 2.16 (s, 2H), 1.91 (d, *J=* 12.6 Hz, 2H), 1.66 - 1.37 (m, 9H). LCMS calcd for C₄₂H₅₀F₃N₁₀O₅ [M+H]⁺: m/z = 831.4; Found: 831.3.

### Examples 66 - 70

Examples 66-70 shown below in Table 12 were prepared as TFA salts by the method used in preparing Example 65 using the appropriate intermediates and starting materials.

**Table 12. Examples 66-70**

| **Ex.** | **Structure** | **Calcd. (M+H)⁺ m/z** | **Found (M+H)⁺ m/z** |
|---|---|---|---|
| | | | |
| **66** | 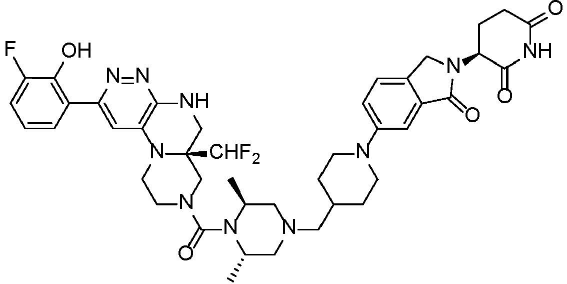 (*S*)-3-(6-(4-(((3*S*,5*S*)-4-((*R*)-6a-(difluoromethyl)-2-(3-fluoro-2-hydroxyphenyl)-6,6a,7,8,9,10-hexahydro-5H-pyrazino [1',2':4,5]pyrazino[2,3-*c*]pyridazine-8-carbonyl)-3,5-dimethylpiperazin-1-yl)methyl)piperidin-1-yl)-1-oxoisoindolin-2-yl)piperidine-2,6-dione | 831.4 | 831.1 |
| **67** | 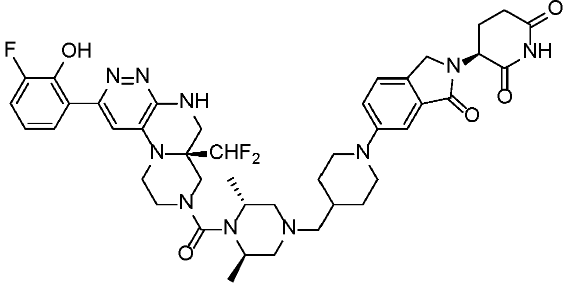 (,S)-3-(6-(4-(((3R,5R)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-*c*]pyridazine-8-carbonyl)-3,5-dimethylpiperazin-1-yl)methyl)piperidin-1-yl)-1-oxoisoindolin-2-yl)piperidine-2,6-dione | 831.4 | 831.1 |
| **68** | 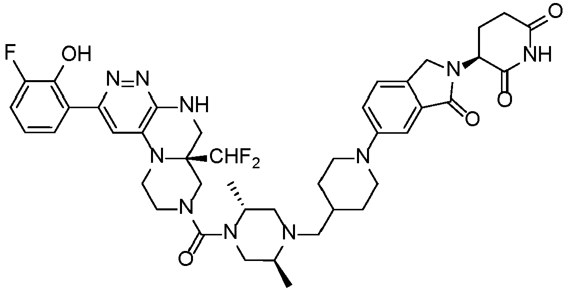 (,S)-3-(6-(4-(((2S,5R)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-*c*]pyridazine-8-carbonyl)-2,5-dimethylpiperazin-1-yl)methyl)piperidin-1-yl)-1-oxoisoindolin-2-yl)piperidine-2,6-dione | 831.4 | 831.1 |
| **69** | 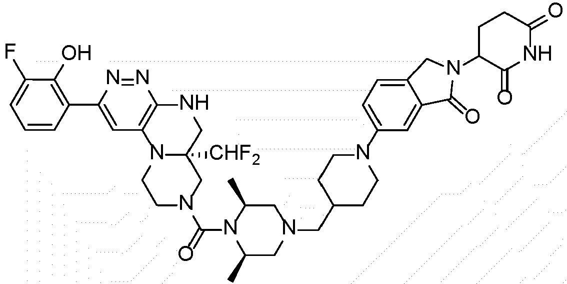 3-(6-(4-(((3*R*,5*S*)-4-((*S*)-6a-(difluoromethyl)-2-(3-fluoro-2-hydroxyphenyl)-6,6a,7,8,9,10-hexahydro-5H-pyrazino [1',2':4,5]pyrazino[2,3-*c*]pyridazine-8-carbonyl)-3,5-dimethylpiperazin-1-yl)methyl)piperidin-1-yl)-1-oxoisoindolin-2-yl)piperidine-2,6-dione | 831.4 | 831.1 |
| **70** | 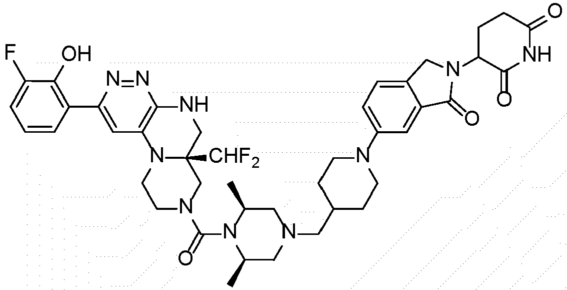 3-(6-(4-(((3R,5S)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-*c*]pyridazine-8-carbonyl)-3,5-dimethylpiperazin-1-yl)methyl)piperidin-1-yl)-1-oxoisoindolin-2-yl)piperidine-2,6-dione | 831.4 | 831.1 |

### Example 71. (S)-3-(6-(4-(((2R,4S,6S)-1-((S)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,6-dimethylpiperidin-4-yl)methyl)piperazin-1-yl)-1-oxoisoindolin-2-yl)piperidine-2,6-dione

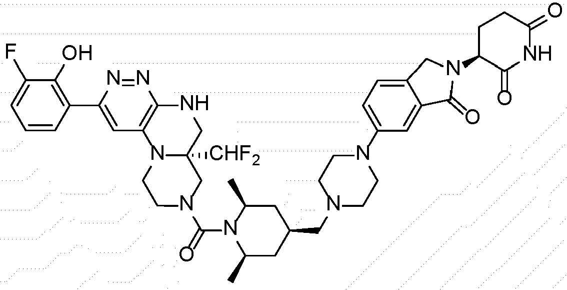

### Step 1. tert-butyl (2R,4r, 6S)-4-(hydroxymethyl)-2, 6-dimethylpiperidine-1-carboxylate

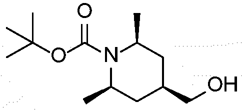

Borane tetrahydrofuran complex (1 M, 1.65 mL, 1.65 mmol) was added to (2*R*,4*r*,6*S*)-1-(tert-butoxycarbonyl)-2,6-dimethylpiperidine-4-carboxylic acid (170.0 mg, 0.66 mmol) in THF (3.3 mL) at 0 °C. The reaction mixture was warmed to room temperature overnight. The reaction mixture was cooled to 0 °C and quenched with MeOH (2 mL). The reaction mixture was directly condensed. The residue was dissolved in water (30 mL) and EtOAc (30 mL). The aqueous layer was extracted with EtOAc (2 x 30 mL) and the combined organic layers were dried over Na₂SO₄, filtered and concentrated under reduced pressure to obtain *tert*-butyl (2*R,*4*r*,6*S*)-4-(hydroxymethyl)-2,6-dimethylpiperidine-1-carboxylate (158 mg, 98%) as a clear oil. LCMS calcd for C₈H₁₈NO [M+H-C₅H₉O₂]⁺: m/z = 144.1; Found: 144.0

### Step 2. ((2R,4r,6S)-2,6-dimethylpiperidin-4-yl)methyl benzoate

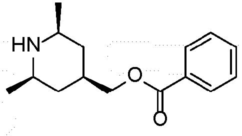

Benzoyl chloride was added to a stirring solution of *tert-*butyl (2*R*,4*r*,6*S*)-4-(hydroxymethyl)-2,6-dimethylpiperidine-1-carboxylate (188 mg, 0.77 mmol), triethylamine (118 µL, 0.85 mmol), and 4-(dimethylamino)pyridine (11 mg, 0.09 mmol) in DCM (2 mL). The reaction mixture was stirred at room temperature for 6 hours. The product mixture was diluted with EtOAc (40 mL) and washed with water (30 mL) and a saturated sodium chloride solution (2 x 30 mL). The organic layer was dried over Na₂SO₄, filtered, and concentrated under reduced pressure. The residue obtained was dissolved in DCM (6 mL). Hydrochloric acid (4 M in 1,4-dioxane, 1.9 mL, 7.7 mmol) was added to the reaction mixture. The reaction mixture was stirred overnight at room temperature. The product mixture was concentrated under reduced pressure to obtain the HCl salt of ((2R,4r,6S)-2,6-dimethylpiperidin-4-yl)methyl benzoate (220 mg, 100%) as a white powder. LCMS calcd for C₁₃H₂₂NO₂ [M+H]⁺: m/z = 248.2; Found: 248.1

### Step 3. ((2R, 4r, 6S)-1-(chlorocarbonyl)-2, 6-dimethylpiperidin-4-yl)methyl benzoate

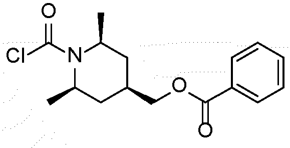

Triphosgene (132 mg, 0.45 mmol) was added to a stirring solution of ((2R,4r,6S)-2,6-dimethylpiperidin-4-yl)methyl benzoate (330 mg, 1.16 mmol) and pyridine (0.47 µL, 5.8 mmol) in DCM (5.8 mL) at 0 °C. The reaction mixture was warmed to room temperature and stirred for 2 hours. The product mixture was transferred to a separatory funnel containing 1 M aqueous HCl solution (30 mL). The diluted product mixture was extracted with DCM (2 x 30 mL). The combined organic layers were dried over Na₂SO₄, filtered and concentrated under reduced pressure to obtain ((2R,4r,6S)-1-(chlorocarbonyl)-2,6-dimethylpiperidin-4-yl)methyl benzoate as a yellow oil. The residue was used without further purification.

### Step 4. ((2R, 4S, 6S)-1-((S)-6a-(difluoromethyl)-2-(3-fluoro-2-hydroxyphenyl)-6,6a,7,8,9,10-hexahydro-5H-pyrazino[1',2":4,5]pyrazino[2,3-c]pyridazine-8-carbonyl)-2,6-dimethylpiperidin-4-yl)methyl benzoate

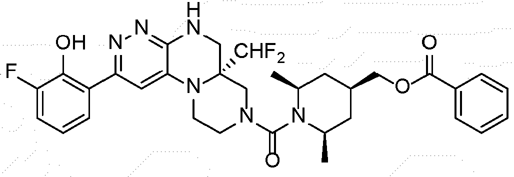

((2*R*,4*r*,6*S*)-1-(chlorocarbonyl)-2,6-dimethylpiperidin-4-yl)methyl benzoate (252 mg, 0.81 mmol) in a solution of dimethylacetamide (1 mL) was added to a stirring solution of (R)-2-(6a-(difluoromethyl)-6,6a,7,8,9,10-hexahydro-5*H*-pyrazino[1',2':4,5]pyrazino[2,3-*c*]pyridazin-2-yl)-6-fluorophenol (230.0 mg, 54 mmol), N,N-diisopropylethylamine (378 µL, 2.2 mmol), and 4-(dimethylamino)pyridine (20 mg, 0.16 mmol) in dimethylacetamide (4 mL) at room temperature. The reaction mixture was stirred at room temperature for 30 minutes. The product mixture was quenched with water (20 mL). The diluted product mixture was extracted with EtOAc (3 x 30 mL). The combined organic layers were dried over Na₂SO₄, filtered, and concentrated under reduced pressure. The residue obtained was purified by silica gel flash column chromatography with a gradient of 0-15% MeOH/DCM to obtain ((2*R*,4*S*,6*S*)-1-((S)-6a-(difluoromethyl)-2-(3-fluoro-2-hydroxyphenyl)-6,6a,7,8,9,10-hexahydro-5*H*-pyrazino[1',2':4,5]pyrazino[2,3-c]pyridazine-8-carbonyl)-2,6-dimethylpiperidin-4-yl)methyl benzoate (195 mg, 58%). LCMS calcd for C₃₂H₃₆F₃N₆O₄ [M+H]⁺: m/z = 625.3; Found: 625.2

### Step 5. ((S)-6a-(difluoromethyl)-2-(3-fluoro-2-hydroxyphenyl)-5,6, 6a, 7,9,10-hexahydro-8H-pyrazino[1',2':4,5]pyrazino[2,3-c]pyridazin-8-yl)((2R,4S,6S)-4-(hydroxymethyl)-2, 6-dimethylpiperidin-1-yl)methanone

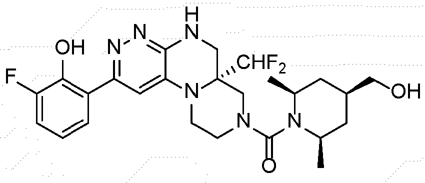

Potassium carbonate (431 mg, 3.1 mmol) was added to a stirring solution of *((2R,4S,6S)-*1-((*S*)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,6-dimethylpiperidin-4-yl)methyl benzoate (195 mg, 0.31 mmol) in methanol (4 mL) at room temperature. The reaction was stirred at room temperature for 3 hours. The product mixture was quenched with saturated sodium bicarbonate aqueous solution (30 mL). The diluted product mixture was extracted with EtOAc (3 x 30 mL). The combined organic layers were dried over Na₂SO₄, filtered, and concentrated under reduced pressure. The residue obtained was purified by silica gel flash column chromatography with a gradient of 0-20% MeOH/DCM to obtain ((S)-6a-(difluoromethyl)-2-(3-fluoro-2-hydroxyphenyl)-5,6,6a,7,9,10-hexahydro-8H-pyrazino[1',2':4,5]pyrazino[2,3-c]pyridazin-8-yl)((2*R*,4*S*,6*S*)-4-(hydroxymethyl)-2,6-dimethylpiperidin-1-yl)methanone (52 mg, 32%) as a yellow oil. LCMS calcd for C₂₅H₃₂F₃N₆O₃ [M+H]⁺: m/z = 521.3; Found: 521.1

### Step 6. (S)-3-(6-(4-(((2R, 4S, 6S)-1-((S)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,6-dimethylpiperidin-4-yl)methyl)piperazin-1-yl)-1-oxoisoindolin-2-yl)piperidine-2, 6-dione

Sulfur trioxide pyridine complex (77 mg, 0.48 mmol) in a solution of DMSO (500 µL) was added to a stirring solution of ((*S*)-6a-(difluoromethyl)-2-(3-fluoro-2-hydroxyphenyl)-5,6,6a,7,9,10-hexahydro-8*H*-pyrazino[1',2':4,5]pyrazino[2,3-c]pyridazin-8-yl)((2*R*,4*S*,6*S*)-4-(hydroxymethyl)-2,6-dimethylpiperidin-1-yl)methanone (42 mg, 0.081 mmol) and triethylamine (135 µL, 0.97 mmol) in DMSO (1.5 mL). The reaction mixture was stirred 1 hour. The product mixture was diluted with EtOAc (40 mL) and washed with saturated sodium bicarbonate aqueous solution (30 mL) and saturated sodium chloride aqueous solution. The organic layer was dried over Na₂SO₄, filtered, and concentrated under reduced pressure. The residue obtained was used directly in the next step. The residue obtained was dissolved in DMF (3 mL). (S)-3-(1-oxo-6-(piperazin-1-yl)isoindolin-2-yl)piperidine-2,6-dione (40 mg, 0.12 mmol) was added to the reaction mixture. The reaction mixture was stirred at room temperature for 15 minutes. The reaction mixture was cooled to 0 °C. Acetic acid (93 µL, 1.6 mmol) and sodium triacetoxyborohydride (86 mg, 41 mmol) were added sequentially to the reaction mixture at 0 °C. The reaction mixture was stirred for 1 hour at 0 °C. The product mixture was quenched with water (1 mL) and acetonitrile (1 mL) and filtered. The filtrate was purified by prep-HPLC (Waters CSH-Phenyl-Hexyl, 5 uM, 30x100mm, 5.9-23.9% MeCN/water (containing 0.1%TFA) over 5 min) to give (S)-3-(6-(4-(((2R,4S,6S)-1-((S)-6a-(difluoromethyl)-2-(3-fluoro-2-hydroxyphenyl)-6,6a,7,8,9,10-hexahydro-5*H*-pyrazino[1',2':4,5]pyrazino[2,3-*c*]pyridazine-8-carbonyl)-2,6-dimethylpiperidin-4-yl)methyl)piperazin-1-yl)-1-oxoisoindolin-2-yl)piperidine-2,6-dione as its TFA salt (21.8 mg, 21%). ¹H NMR (400 MHz, MeOD) δ 7.53 (d, *J* = 8.3 Hz, 1H), 7.43 - 7.26 (m, 5H), 7.12 - 6.95 (m, 1H), 6.22 (t, *J* = 54.6 Hz, 1H), 5.14 (dd, *J=* 13.4, 5.2 Hz, 1H), 4.95 - 4.87 (m, 2H), 4.58 - 4.34 (m, 2H), 4.10 (d, *J* = 13.0 Hz, 1H), 3.86 (d, *J= 12.8* Hz, 1H), 3.54 (t, *J= 11.6* Hz, 1H), 3.50 - 3.36 (m, 2H), 3.20 - 3.07 (m, 3H), 2.99 - 2.85 (m, 3H), 2.79 (ddd, *J=* 17.5, 4.5, 2.1 Hz, 1H), 2.51 (qd, *J=* 12.6, 4.8 Hz, 1H), 2.26 - 2.11 (m, 2H), 1.89 (d, *J=* 12.5 Hz, 2H), 1.18 *(d, J=* 12.8 Hz, 2H), 1.09 (dd, *J =* 13.4, 6.2 Hz, 6H). LCMS calcd for C₄₂H₅₀F₃N₁₀O₅ [M+H]⁺: m/z = 831.4; Found: 831.3.

### Examples 72 - 73

Examples 72-73 shown below in Table 13 were prepared as TFA salts by the method used in preparing Example 71 using the appropriate intermediates and starting materials.

**Table 13. Examples 72-73**

| **Ex.** | **Structure** | **Calcd. (M+H)⁺ m/z** | **Found (M+H)⁺ m/z** |
|---|---|---|---|
| 72 | 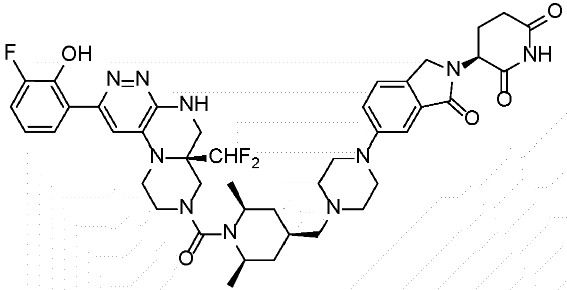 (*S*)-3-(6-(4-(((2*R*,4*R*,6*S*)-1-((*R*)-6a-(difluoromethyl)-2-(3-fluoro-2-hydroxyphenyl)-6,6a,7,8,9,10-hexahydro-5*H-*pyrazino[1',2':4,5]pyrazino[2,3-*c*]pyridazine-8-carbonyl)-2,6-dimethylpiperidin-4-yl)methyl)piperazin-1-yl)-1-oxoisoindolin-2-yl)piperidine-2,6-dione | 831.4 | 831.5 |
| 73 | 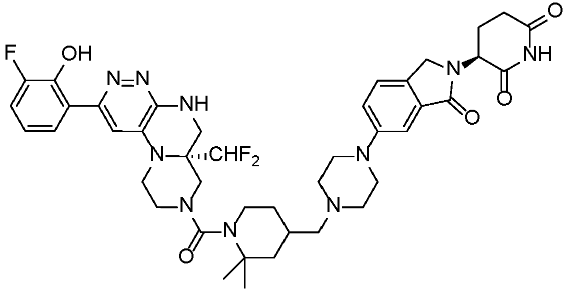 (3*S*)-3-(6-(4-((1-((*S*)-6a-(difluoromethyl)-2-(3-fluoro-2-hydroxyphenyl)-6,6a,7,8,9,10-hexahydro-5*H*-pyrazino [1',2':4,5]pyrazino[2,3-c]pyridazine-8-carbonyl)-2,2-dimethylpiperidin-4-yl)methyl)piperazin-1-yl)-1-oxoisoindolin-2-yl)piperidine-2,6-dione | 831.4 | 831.3 |

### Example 74. (3S)-3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione (Isomer 1)

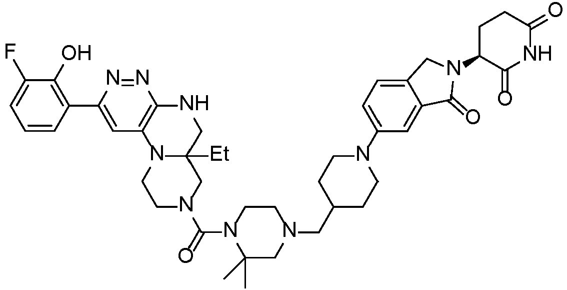

Sodium triacetoxyborohydride (179 mg, 0.84 mmol) was added to a stirring solution of acetic acid (0.12 mL, 2.11 mmol), Intermediate 40 (147 mg, 0.211 mmol), and Intermediate 37 (112 mg, 0.316 mmol) in DMF (6 mL) at 0 °C. The reaction mixture was stirred 1 hour at 0 °C. The product mixture was diluted with DMSO and purified by prep-HPLC (Waters CSH-C18, 5 uM, 30x100mm, 8.2-28.2% MeCN/water (containing 0.1%TFA) over 5 min) to give 3-(6-(4-((4-(6a-ethyl-2-(3-fluoro-2-hydroxyphenyl)-6,6a,7,8,9,10-hexahydro-5*H*-pyrazino[1',2':4,5] pyrazino[2,3-*c*]pyridazine-8-carbonyl)-3,3-dimethylpiperazin-1-yl)methyl)piperidin-1-yl)-1-oxoisoindolin-2-yl)piperidine-2,6-dione (Isomer 1) as its TFA salt (55.5 mg, 23%). ¹H NMR (400 MHz, MeOD) δ 7.37 (d, *J =* 8.3 Hz, 1H), 7.30 - 7.18 (m, 4H), 7.03 (s, 1H), 6.92 (td, J= 8.1, 4.8 Hz, 1H), 5.04 (dd, *J =* 13.3, 5.1 Hz, 1H), 4.42 - 4.22 (m, 2H), 4.06 (s, 1H), 3.82 (d, *J =* 12.4 Hz, 1H), 3.72 (d, *J =* 12.5 Hz, 2H), 3.56 (d, *J =* 12.5 Hz, 1H), 3.51 - 3.25 (m, 4H), 3.16 (d, *J*= 12.7 Hz, 2H), 3.09 - 3.00 (m, 3H), 2.90 - 2.75 (m, 4H), 2.68 (ddd, *J =* 17.7, 4.4, 2.3 Hz, 1H), 2.40 (qd, *J =* 13.3, 4.8 Hz, 1H), 2.11 - 1.98 (m, 2H), 1.82 (d, *J* = 12.8 Hz, 3H), 1.63 - 1.26 (m, 9H), 0.92 (s, 3H). LCMS calcd for C₄₃H₅₄FN₁₀O₅ [M+H]⁺: m/z = 809.4; Found: 809.2

### Examples 75 - 88

Examples 75-88 shown below in Table 14 were prepared as TFA salts by the method used in preparing Example 74 using the appropriate intermediates and starting materials

**Table 14. Examples 75-88**

| **Ex.** | **Structure** | **Calcd. (M+H)⁺ m/z** | **Found (M+H)⁺ m/z** |
|---|---|---|---|
| **75** | 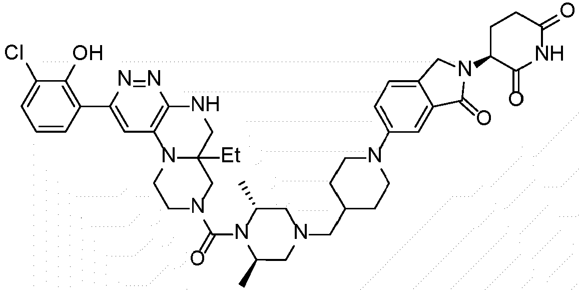 (35)-3-(6-(4-(((3R,5R)-4-(2-(3-chloro-2-hydroxyphenyl)-6a-ethyl-6,6a,7,8,9,10-hexahydro-5H-pyrazino[1',2':4,5] pyrazino[2,3-*c*]pyridazine-8-carbonyl)-3,5-dimethyl-piperazin-1-yl)methyl)piperidin-1-yl)-1-oxoisoindolin-2-yl)piperidine-2,6-dione (Isomer 2) | 825.4 | 825.2 |
| **76** | 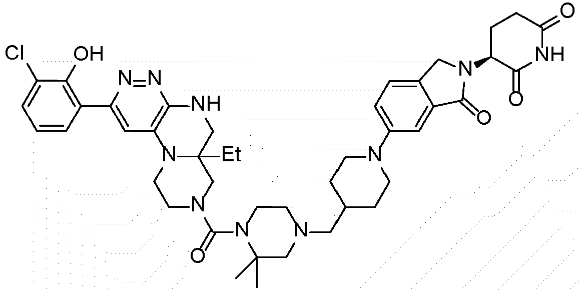 (3*S*)-3 -(6-(4-((4-(2-(3 -chloro-2-hydroxyphenyl)-6a-ethyl-6,6a,7,8,9,10-hexahydro-5*H*-pyrazino[1',2':4,5]pyrazino[2,3-*c*]pyridazine-8-carbonyl)-3,3-dimethylpiperazin-1-yl)methyl) piperidin-1-yl)-1-oxoisoindolin-2-yl)piperidine-2,6-dione (Isomer 1) | 825.4 | 825.2 |
| **77** | 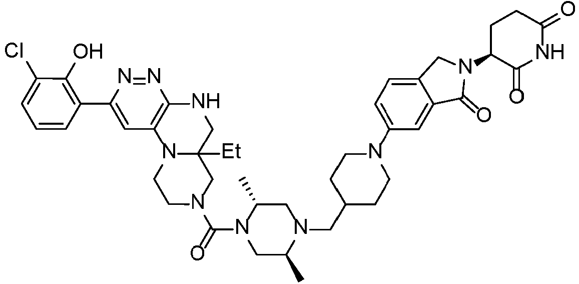 (3*S*)-3-(6-(4-(((2*S*,5*R*)-4-(2-(3-chloro-2-hydroxyphenyl)-6a-ethyl-6,6a,7,8,9,10-hexahydro-5H-pyrazino[1',2':4,5] pyrazino[2,3-*c*]pyridazine-8-carbonyl)-2,5-dimethyl-piperazin-1-yl)methyl)piperidin-1-yl)-1-oxoisoindolin-2-yl)piperidine-2,6-dione (Isomer 2) | 825.4 | 825.1 |
| **78** | 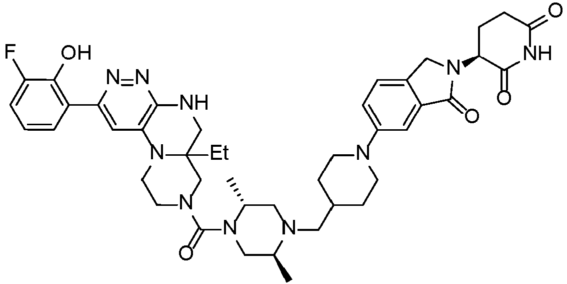 (3*S*)-3-(6-(4-(((2*S*,5*R*)-4-(6a-ethyl-2-(3-fluoro-2-hydroxyphenyl)-6,6a,7,8,9,10-hexahydro-5*H*-pyrazino [1',2':4,5]pyrazino[2,3-*c*]pyridazine-8-carbonyl)-2,5-dimethylpiperazin-1-yl)methyl)piperidin-1-yl)-1-oxoisoindolin-2-yl)piperidine-2,6-dione (Isomer 2) ¹H NMR (400 MHz, MeOD) δ 7.48 (d, *J =* 8.4 Hz, 1H), 7.42 - 7.27 (m, 4H), 7.13 (s, 1H), 7.02 (td, *J* = 8.1, 4.8 Hz, 1H), 5.14 (dd, *J* = 13.3, 5.1 Hz, 1H), 4.42 (dd, *J =* 17.0, 9.6 Hz, 2H), 4.26 (s, 1H), 4.00 - 3.89 (m, 1H), 3.82 (d, *J* = 12.4 Hz, 2H), 3.66 (d, *J* = 12.6 Hz, 2H), 3.56 - 3.33 (m, 5H), 3.26 (d, *J* = 12.6 Hz, 1H), 3.23 - 3.00 (m, 2H), 3.01 - 2.85 (m, 4H), 2.78 (ddd, *J* = 17.6, 4.7, 2.5 Hz, 1H), 2.50 (qd, *J* = 13.2, 4.7 Hz, 1H), 2.26 - 2.09 (m, 2H), 2.05 (d, *J* = 12.6 Hz, 2H), 1.91 (d, *J* = 12.9 Hz, 2H), 1.71 - 1.47 (m, 3H), 1.43 (d, J= 6.3 Hz, 3H), 1.33 (s, 3H), 1.03 (t, *J* = 7.4 Hz, 3H). | 809.4 | 809.2 |
| **79** | 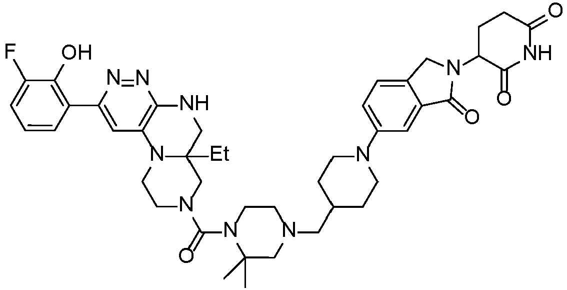 3-(6-(4-((4-(6a-ethyl-2-(3-fluoro-2-hydroxyphenyl)-6,6a,7,8,9,10-hexahydro-5*H*-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione (Isomer 2) | 809.4 | 809.2 |
| **80** | 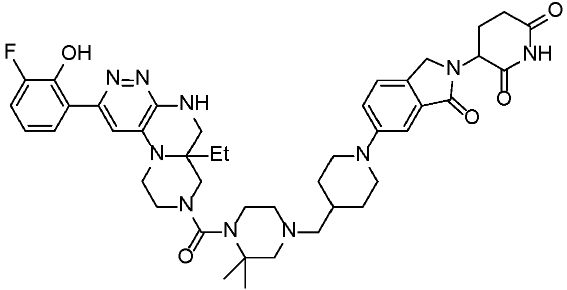 3-(6-(4-((4-(6a-ethyl-2-(3-fluoro-2-hydroxyphenyl)-6,6a,7,8,9,10-hexahydro-5*H*-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione (Isomer 1) | 809.4 | 809.2 |
| **81** | 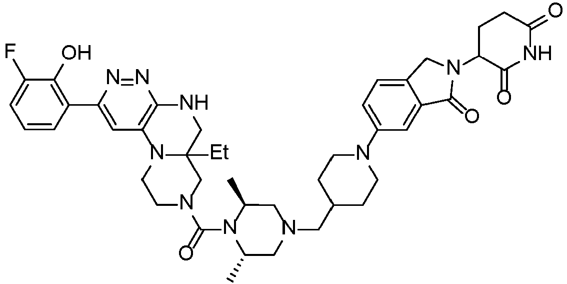 3-(6-(4-(((3*S*,5*S*)-4-(6a-ethyl-2-(3-fluoro-2-hydroxyphenyl)-6,6a,7,8,9,10-hexahydro-5*H*-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione (Isomer 2) | 809.4 | 809.2 |
| **82** | 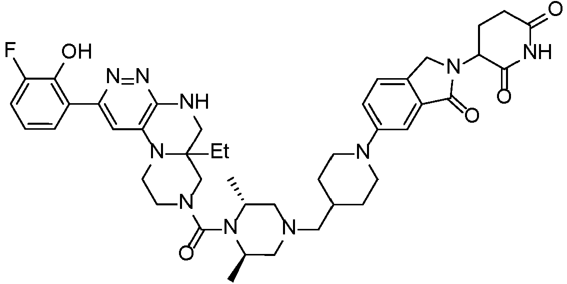 3 -(6-(4-(((3*R*, 5*R*)-4-(6a-ethyl-2-(3 -fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione (Isomer 2) | 809.4 | 809.2 |
| **83** | 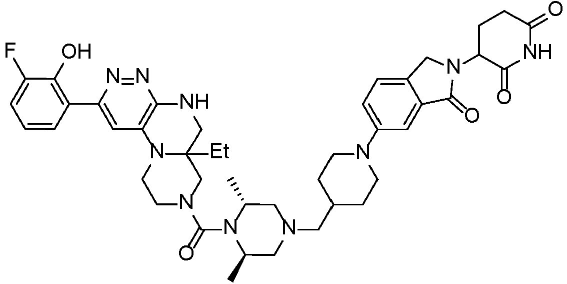 3-(6-(4-(((3*R*, 5*R*)-4-(6a-ethyl-2-(3 -fluoro-2-hydroxyphenyl)-6,6a,7,8,9,10-hexahydro-5*H*-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione (Isomer 1) | 809.4 | 809.2 |
| **84** | 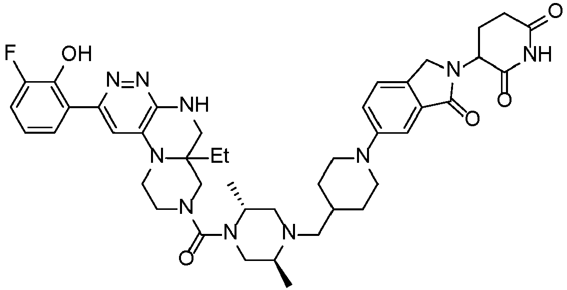 3 -(6-(4-(((2*S*, 5*R*)-4-(6a-ethyl-2-(3 -fluoro-2-hydroxyphenyl)-6,6a,7,8,9,10-hexahydro-5*H*-pyrazino[1',2':4,5]pyrazino[2,3-c]pyridazine-8-carbonyl)-2,5-dimethylpiperazin-1-yl)methyl) piperidin-1-yl)-1-oxoisoindolin-2-yl)piperidine-2,6-dione (Isomer 1) | 809.4 | 809.1 |
| **85** | 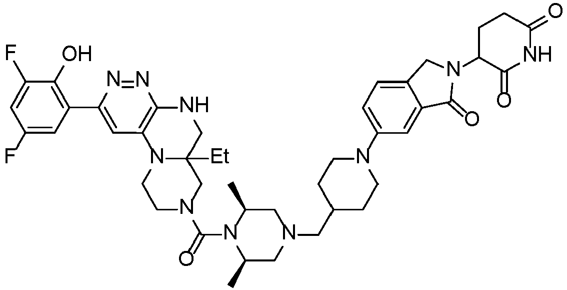 3-(6-(4-(((3*R*,5*S*)-4-(2-(3,5-difluoro-2-hydroxyphenyl)-6a-ethyl-6,6a,7,8,9,10-hexahydro-5H-pyrazino[1',2':4,5] pyrazino[2,3-*c*]pyridazine-8-carbonyl)-3,5-dimethyl-piperazin-1-yl)methyl)piperidin-1-yl)-1-oxoisoindolin-2-yl)piperidine-2,6-dione (Isomer 2) | 827.4 | 827.2 |
| **86** | 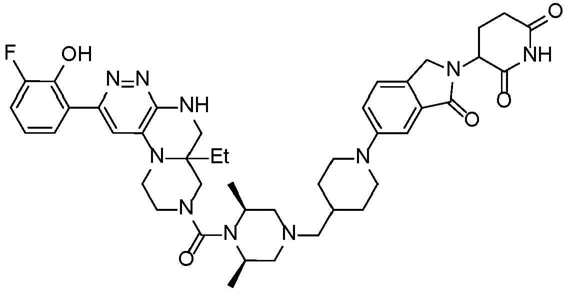 3 -(6-(4-(((3*R*,5*S*)-4-(6a-ethyl-2-(3 -fluoro-2-hydroxyphenyl)-6,6a,7,8,9,10-hexahydro-5*H*-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione (Isomer 2) | 809.4 | 809.2 |
| **87** | 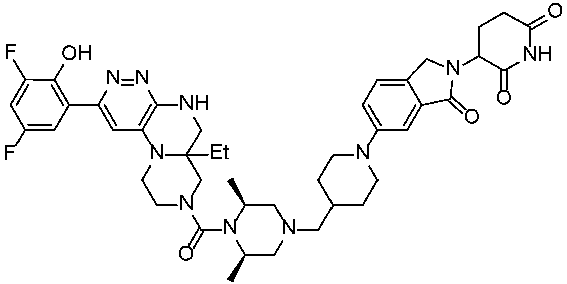 3-(6-(4-(((3*R*,5*S*)-4-(2-(3,5-difluoro-2-hydroxyphenyl)-6a-ethyl-6,6a,7,8,9,10-hexahydro-5*H*-pyrazino[1',2':4,5] pyrazino[2,3-*c*]pyridazine-8-carbonyl)-3,5-dimethyl-piperazin-1-yl)methyl)piperidin-1-yl)-1-oxoisoindolin-2-yl)piperidine-2,6-dione (Isomer 1) | 827.4 | 827.1 |
| **88** | 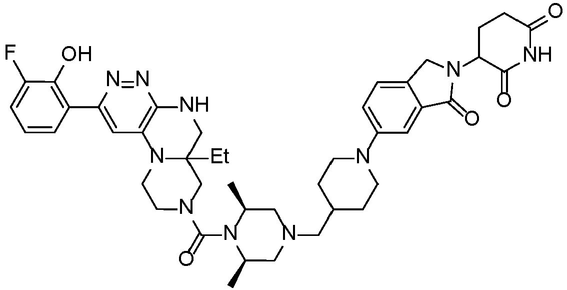 3 -(6-(4-(((3R, 5S)-4-(6a-ethyl-2-(3 -fluoro-2-hydroxyphenyl)-6,6a,7,8,9,10-hexahydro-5*H*-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione (Isomer 1) | 809.4 | 809.4 |

### Example 89. (3S)-3-(6-(4-((4-(2-(3-fluoro-2-hydroxyphenyl)-6a-(fluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione (Isomer 2)

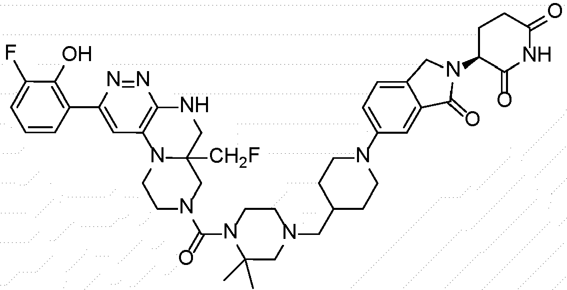

Sodium triacetoxyborohydride (89 mg, 0.42 mmol) was added to a stirring solution of acetic acid (80 µL, 1.39 mmol), (2,2-dimethylpiperazin-1-yl)(2-(3-fluoro-2-hydroxyphenyl)-6a-(fluoromethyl)-5,6,6a,7,9,10-hexahydro-8*H*-pyrazino[1',2':4,5]pyrazino[2,3-*c*]pyridazin-8-yl)methanone (Isomer 2) (Intermediate 54) (66 mg, 0.14 mmol), and Intermediate 37 (74 mg, 0.21 mmol) in DMF (3 mL) at 0 °C. The reaction mixture was stirred 1 hour at 0 °C. The product mixture was diluted with DMSO and purified by prep-HPLC (Waters CSH-C18, 5 uM, 30x100mm, 7.5-27.5% MeCN/water (containing 0.1%TFA) over 5 min) to give (3*S*)-3-(6-(4-((4-(2-(3-fluoro-2-hydroxyphenyl)-6a-(fluoromethyl)-6,6a,7,8,9,10-hexahydro-5*H*-pyrazino[1',2':4,5] pyrazino[2,3-c]pyridazine-8-carbonyl)-3,3-dimethylpiperazin-1-yl)methyl)piperidin-1-yl)-1-oxoisoindolin-2-yl)piperidine-2,6-dione as its TFA salt (84.4 mg, 52%). ¹H NMR (400 MHz, MeOD) δ 7.47 (d, *J* = 8.4 Hz, 1H), 7.39 - 7.28 (m, 4H), 7.20 (s, 1H), 7.03 (td, *J =* 8.1, 4.8 Hz, 1H), 5.14 (dd, *J =* 13.3, 5.1 Hz, 1H), 4.60 (d, *J =* 47.5 Hz, 2H), 4.42 (dd, *J* = 16.8, 8.6 Hz, 2H), 4.15 (s, 1H), 3.96 (d, *J=* 9.7 Hz, 1H), 3.82 (d, *J* = 12.6 Hz, 2H), 3.74 (d, *J =* 12.5 Hz, 1H), 3.63 - 3.42 (m, 4H), 3.35 (dd, *J* = 12.3, 2.4 Hz, 2H), 3.21 - 3.10 (m, 2H), 2.99 - 2.85 (m, 3H), 2.79 (ddd, *J=* 17.6, 4.7, 2.5 Hz, 1H), 2.50 (qd, *J =* 13.2, 4.7 Hz, 1H), 2.26 - 2.06 (m, 2H), 1.92 (d, *J =* 12.8 Hz, 2H), 1.64 - 1.46 (m, 8H). LCMS calcd for C₄₂H₅₁F₂N₁₀O₅ [M+H]⁺: m/z = 813.4; Found: 813.3

### Example 90

Example 90 shown below in Table 15 was prepared as the TFA salt by the method used in preparing Example 89 using the appropriate intermediates and starting materials.

**Table 15. Example 90**

| **Example** | **Structure** | **Calcd. (M+H)⁺ m/z** | **Found (M+H)⁺ m/z** |
|---|---|---|---|
| **90** | 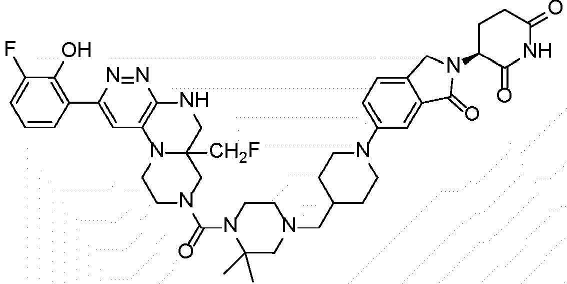 (3*S*)-3 -(6-(4-((4-(2-(3 -fluoro-2-hydroxyphenyl)-6a-(fluoromethyl)-6,6a,7,8,9,10-hexahydro-5*H*-pyrazino [1',2':4,5]pyrazino[2,3-*c*]pyridazine-8-carbonyl)-3,3-dimethylpiperazin-1-yl)methyl)piperidin-1-yl)-1-oxoisoindolin-2-yl)piperidine-2,6-dione (Isomer 1) | 813.4 | 813.2 |

### Example 91. 3-(6-(4-(((2S,5R)-4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione (Isomer 1)

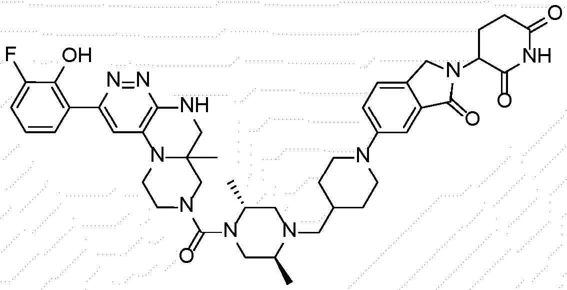

### Step 1. 2-(3-fluoro-2-hydroxyphenyl)-6a-methyl-7,8,9,10-tetrahydro-5H-pyrazino [1',2':4,5]pyrazino[2,3-c]pyridazin-6(6aH)-one

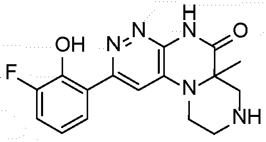

Hydrochloric acid (4 M in 1,4-dioxane, 186 µL, 0.745 mmol) was added to a stirring solution of *tert*-butyl 2-(3-fluoro-2-hydroxyphenyl)-6a-methyl-6-oxo-5,6,6a,7,9,10-hexahydro-8*H*-pyrazino[1',2':4,5]pyrazino[2,3-c]pyridazine-8-carboxylate (Isomer 1) (32 mg, 0.75 mmol). The reaction mixture was stirred overnight. The product mixture was concentrated under reduced pressure to obtain 2-(3-fluoro-2-hydroxyphenyl)-6a-methyl-7,8,9,10-tetrahydro-5H-pyrazino [1',2':4,5]pyrazino[2,3-c]pyridazin-6(6aH)-one (Isomer 1) (27 mg, 99%) as the HCl salt. LCMS calcd for C₁₆H₁₇FN₅O₂ [M+H]⁺: m/z = 330.1; Found: 329.9.

### Step 2. tert-butyl (2S, 5R)-4-(chlorocarbonyl)-2,5-dimethylpiperazine-1-carboxylate

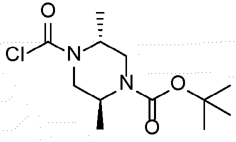

Triphosgene (41.5 mg, 0.14 mmol) was added to a stirring solution of (2*S*,5*R*)-*tert*-butyl 2,5-dimethylpiperazine-1-carboxylate (50 mg, 0.23 mmol) and pyridine (57 µL, 0.70 mmol) at 0 °C. The reaction was warmed to room temperature and stirred for 2 hours. The product mixture was washed with 1 M HCl aqueous solution (30 mL). The aqueous layer was extract with DCM (2 x 30 mL). The combined organic layers were dried over Na₂SO₄, filtered, and concentrated under reduced pressure. The residue obtained was used without further purification. *tert-butyl* (2S,5R)-4-(chlorocarbonyl)-2,5-dimethylpiperazine-1-carboxylate was obtained as a yellow oil (62 mg, 96%).

### Step 3. tert-butyl (2S,5R)-4-(2-(3-fluoro-2-hydroxyphenyl)-6a-methyl-6-oxo-6,6a,7,8,9,10-hexahydro-5H-pyrazino[1',2":4,5]pyrazino[2,3-c]pyridazine-8-carbonyl)-2,5-dimethylpiperazine-1-carboxylate (Isomer 1)

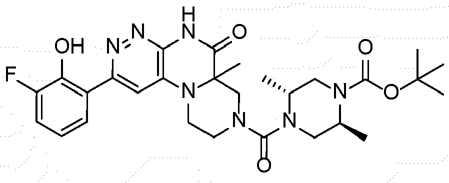

*tert-Butyl* (2S,5R)-4-(chlorocarbonyl)-2,5-dimethylpiperazine-1-carboxylate (45 mg, 0.16 mmol) in DCM (1 mL) was added to a stirring solution of 2-(3-fluoro-2-hydroxyphenyl)-6a-methyl-7,8,9,10-tetrahydro-5H-pyrazino[1',2':4,5]pyrazino[2,3-*c*]pyridazin-6(6aH)-one (Isomer 1) (0.075 mmol) and triethylamine (62 µL, 0.45 mmol) in DCM (1 mL) at room temperature. The reaction was stirred at room temperature for 2 hours. The product mixture was purified by silica gel flash column chromatography with a gradient of 0-10% MeOH/DCM to obtain *tert-butyl* (2S,5R)-4-(2-(3-fluoro-2-hydroxyphenyl)-6a-methyl-6-oxo-6,6a,7,8,9,10-hexahydro-5H-pyrazino[1',2':4,5]pyrazino[2,3-*c*]pyridazine-8-carbonyl)-2,5-dimethylpiperazine-1-carboxylate (Isomer 1) (28 mg, 66%). LCMS calcd for C₂₈H₃₇FN₇O₅ [M+H]⁺: m/z = 570.3; Found: 570.1

### Step 4. ((2R,5S)-2,5-dimethylpiperazin-1-yl)(2-(3-fluoro-2-hydroxyphenyl)-6a-methyl-5,6,6a,7,9,10-hexahydro-8H-pyrazino[1',2':4,5]pyrazino[2,3-c]pyridazin-8-yl)methanone (Isomer 1)

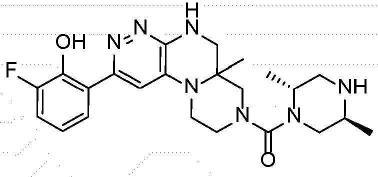

Borane tetrahydrofuran complex (1 M in THF, 0.43 mL, 0.43 mmol) was added to a stirring solution of *tert*-butyl (2S,5R)-4-(2-(3-fluoro-2-hydroxyphenyl)-6a-methyl-6-oxo-6,6a,7,8,9,10-hexahydro-5H-pyrazino[1',2':4,5]pyrazino[2,3-*c*]pyridazine-8-carbonyl)-2,5-dimethylpiperazine-1-carboxylate (Isomer 1) (40 mg, 0.070 mmol) in THF (1.5 mL) at room temperature. The reaction mixture was heated to 65 °C and stirred overnight at 65 °C. The product mixture was cooled to 0 °C and quenched with MeOH (3 mL). The quenched product mixture was heated to 80 °C and stirred 30 minutes and then was cooled to room temperature and concentrated under reduced pressure. The residue obtain was dissolve in DCM (2 mL). Trifluoroacetic acid (173 µL, 2.27 mmol) was added to the diluted residue at room temperature. The resulting mixture was stirred at room temperature for 1 hour. The product mixture was concentrated under reduced pressure and purified by prep-HPLC (Waters CSH-C18, 5 uM, 30x100mm, 6.4-26.4% MeCN/water (containing 0.1%TFA) over 5 min) to give ((2*R*,5*S*)-2,5-dimethylpiperazin-1-yl)(2-(3-fluoro-2-hydroxyphenyl)-6a-methyl-5,6,6a,7,9,10-hexahydro-8*H-*pyrazino[1',2':4,5]pyrazino[2,3-c]pyridazin-8-yl)methanone (Isomer 1) (8 mg, 25%) as its TFA salt. LCMS calcd for C₂₃H₃₁FN₇O₂ [M+H]⁺: m/z = 456.3; Found: 456.0

### Step 5. 3-(6-(4-(((2S,5R)-4-(2-(3-fluoro-2-hydroxyphenyl)-6a-methyl-6, 6a, 7,8,9,10-hexahydro-5H-pyrazino[1',2':4,5]pyrazino[2,3-c]pyridazine-8-carbonyl)-2,5-dimethyl-piperazin-1-yl)methyl)piperidin-1-yl)-1-oxoisoindolin-2-yl)piperidine-2,6-dione (Isomer 1)

Sodium triacetoxyborohydride (11.2 mg, 0.053 mmol) was added to a stirring solution of acetic acid (7.5 µL, 0.13 mmol), (((2*R*,5*S*)-2,5-dimethylpiperazin-1-yl)(2-(3-fluoro-2-hydroxyphenyl)-6a-methyl-5,6,6a,7,9,10-hexahydro-8*H*-pyrazino[1',2':4,5]pyrazino[2,3-*c*]pyridazin-8-yl)methanone (Isomer 1) (9 mg, 0.013 mmol), and 1-(2-(2,6-dioxopiperidin-3-yl)-3-oxoisoindolin-5-yl)piperidine-4-carbaldehyde (9 mg, 0.025 mmol) in DMF (1 mL) at 0 °C. The reaction mixture was stirred 1 hour at 0 °C. The product mixture was diluted with DMSO and purified by prep-HPLC (Waters CSH-C18, 5 uM, 30x100mm, 6.9-26.9% MeCN/water (containing 0.1%TFA) over 5 min) to give 3-(6-(4-(((2*S*,5*R*)-4-(2-(3-fluoro-2-hydroxyphenyl)-6a-methyl-6,6a,7,8,9,10-hexahydro-5*H*-pyrazino[1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione (Isomer 1) (10.6 mg, 71%) as its TFA salt. LCMS calcd for C₄₂H₅₂FN₁₀O₅ [M+H]+: m/z = 795.4; Found: 795.1.

### Example 92

Example 92 shown below in Table 16 was prepared as the TFA salt by the method used in preparing Example 91 using the appropriate intermediates and starting materials.

**Table 16. Example 92**

| **Ex.** | **Structure** | **Calcd. (M+H)⁺ m/z** | **Found (M+H)⁺ m/z** |
|---|---|---|---|
| **92** | 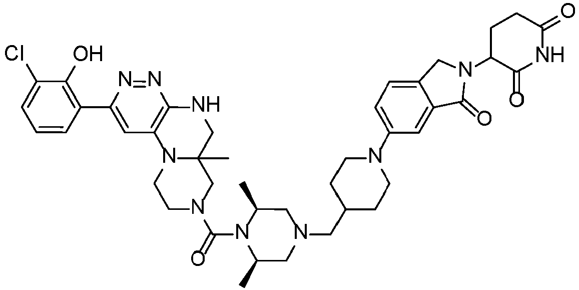 3-(6-(4-(((3*R*,5*S*)-4-(2-(3-chloro-2-hydroxyphenyl)-6a-methyl-6,6a,7,8,9,10-hexahydro-5*H*-pyrazino[1',2':4,5] pyrazino[2,3-*c*]pyridazine-8-carbonyl)-3,5-dimethyl-piperazin-1-yl)methyl)piperidin-1-yl)-1-oxoisoindolin-2-yl)piperidine-2,6-dione (Isomer 2) | 811.4 | 811.1 |

### Examples 93. ((2S,6R)-2,6-dimethylpiperazin-1-yl)(2-(3-fluoro-2-hydroxyphenyl)-6a-methyl-5,6,6a,7,9,10-hexahydro-8H-pyrazino[1',2':4,5]pyrazino[2,3-c]pyridazin-8-yl)methanone (Isomer 1)

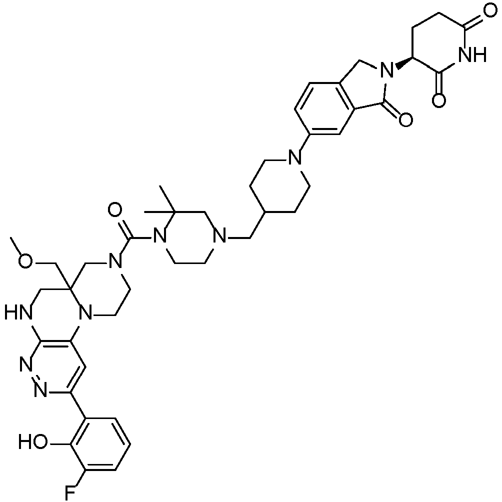

### Step 1: 1, 4-di-tert-butyl 2-methyl 2-(methoxymethyl)piperazine-1, 2, 4-tricarboxylate (racemic mixture)

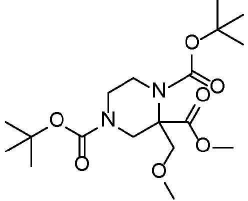

To a solution of 1-O,4-O-di*tert*-butyl 2-O-methyl piperazine-1,2,4-tricarboxylate (0.85 g, 2.47 mmol) in THF (6 mL) was added LiHMDS (4.94 mL, 4.94 mmol) dropwise at -78 °C. The mixture was stirred at -78 °C for 0.5h. To the mixture was added a solution of bromo(methoxy)-methane (0.34 mL, 4.94 mmol) in THF (2 mL) dropwise at -78 °C. The reaction was allowed to warm for 1 h. The reaction was quenched with sat. aq. NH₄Cl solution (10 mL) and water (10 mL). The mixture was extracted with ethyl acetate, concentrated and purified on silica gel column (0-70% EA/Hexanes) to give 1,4-di-tert-butyl 2-methyl 2-(methoxymethyl)piperazine-1,2,4-tricarboxylate (0.84 g, 2.16 mmol, 88% yield). LCMS calcd for C₁₃H₂₅N₂O₅ [M+H-C₅H₈O₂]+: m/z = 289.2; Found: 289.1.

### Step 2: 1,4-bis(tert-butoxycarbonyl)-2-(methoxymethyl)piperazine-2-carboxylic acid (racemic mixture)

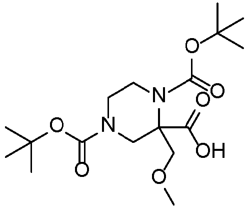

1,4-di-tert-butyl 2-methyl 2-(methoxymethyl)piperazine-1,2,4-tricarboxylate (0.84 g, 2.16 mmol) was treated with LiOH · H₂O (0.85 g, 20.28 mmol) in THF (6 mL), methanol (6 mL) and water (6 mL) at 60 °C overnight. The reaction was quenched with 4N aq. HCl solution, extracted with DCM, dried over MgSO₄ and concentrated to obtain 1,4-bis(tert-butoxycarbonyl)-2-(methoxymethyl)piperazine-2-carboxylic acid (0.8 g, 2.14 mmol, 98% yield). LCMS calcd for C₁₂H₂₃N₂O₅ [M+H- C₅H₈O₂]+: m/z = 275.2; Found: 275.1.

### Step 3: tert-butyl 2-chloro-6a-(methoxymethyl)-6-oxo-5, 6, 6a, 7,9,10-hexahydro-8H-pyrazino[1', 2':4,5]pyrazino[2,3-c]pyridazine-8-carboxylate (racemic mixture)

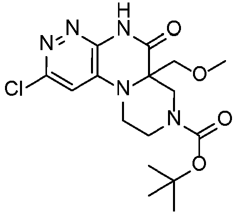

To a solution of 2-(methoxymethyl)-1,4-bis[(2-methylpropan-2-yl)oxycarbonyl] piperazine-2-carboxylic acid (0.86 g, 2.3 mmol) and pyridine (0.28 mL, 3.45 mmol) in DCM (1.5 mL) was carefully added DMF (436 µL) and oxalyl chloride (0.26 mL, 2.99 mmol). The mixture was stirred at room temperature for 30 minutes. The volatiles were removed under reduced pressure, then DMF (3 mL), N,N-Diisopropylethylamine (1.2 mL, 6.89 mmol) and 4-bromo-6-chloropyridazin-3-amine (0.48 g, 2.3 mmol) were added sequentially. The resulting mixture was stirred at 120 °C overnight. The reaction mixture was diluted with EtOAc (100 mL) and washed with a saturated brine solution (30 mL x 2). The combined organics were dried over MgSO₄, filtered and concentrated under reduced pressure. The crude material was purified by silica gel flash column chromatography, eluting with a gradient of 0-100% EtOAc/hexanes to give *tert-*butyl 2-chloro-6a-(methoxymethyl)-6-oxo-5,6,6a,7,9,10-hexahydro-8H-pyrazino[1',2':4,5] pyrazino[2,3-c]pyridazine-8-carboxylate (140 mg, 0.36 mmol, 72% yield). LCMS calcd for C₁₆H₂₃ClN₅O₄ [M+H]⁺: m/z = 384.1; Found: 384.1.

### Step 4: di-tert-butyl 2-chloro-6a-(methoxymethyl)-6-oxo-6a, 7,9,10-tetrahydro-5H-pyrazino[1',2':4, 5]pyrazino[2,3-c]pyridazine-5,8(6H)-dicarboxylate (racemic mixture)

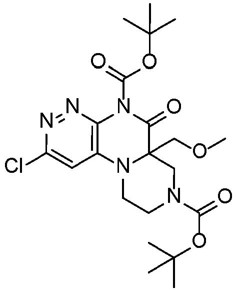

tert-butyl 4-chloro-10-(methoxymethyl)-9-oxo-1,5,6,8,12-pentazatricyclo[8.4.0.02,7] tetradeca-2(7),3,5-triene-12-carboxylate (0.14 g, 0.36 mmol) was dissolved in THF (3 mL). Di-*tert* butyl dicarbonate (0.25 mL, 1.09 mmol) and 4-(dimethylamino)pyridine (0.04 g, 0.36 mmol) were added. The mixture was stirred at RT overnight. The reaction was diluted with DCM, washed with water, concentrated and purified by column (0-80% EA/Hexanes) to obtain *di-tert-*butyl 4-chloro-10-(methoxymethyl)-9-oxo-1,5,6,8,12-pentazatricyclo[8.4.0.02,7]tetradeca-2(7),3,5-triene-8,12-dicarboxylate (140 mg, 0.29 mmol, 79% yield). LCMS calcd for C₂₁H₃₁ClN₅O₆ [M+H]⁺: m/z = 484.2; Found: 484.1.

### Step 5: Chiral separation of di-tert-butyl 2-chloro-6a-(methoxymethyl)-6-oxo-6a, 7,9,10-tetrahydro-5H-pyrazino[1',2':4,5]pyrazino[2,3-c]pyridazine-5,8(6H)-dicarboxylate (Isomer 1 and 2)

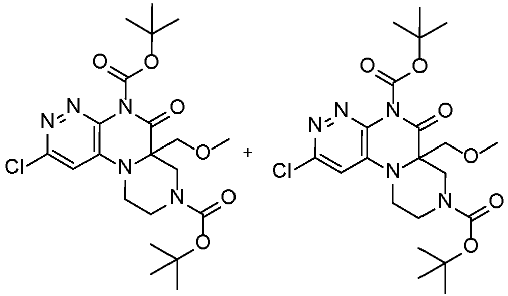

The racemic mixture of *di-tert-butyl 2-chloro-6a-(methoxymethyl)-6-oxo-6a, 7,9,10-tetrahydro-5H-pyrazino[1',2':4,5]pyrazino[2,3-c]pyridazine-5,8(6H)-dicarboxylate* (644 mg, 1.33 mmol) was subjected to chiral separation (Lux Cellulose-2 column, 20 mL/min of 10:90 Hexanes/IPA:MeOH (1:1)) to give di-tert-butyl 2-chloro-6a-(methoxymethyl)-6-oxo-6a,7,9,10-tetrahydro-5*H*-pyrazino[1',2':4,5]pyrazino[2,3-*c*]pyridazine-5,8(6H)-dicarboxylate (280 mg, 43.5% yield, peak A, Isomer 1) and di-tert-butyl 2-chloro-6a-(methoxymethyl)-6-oxo-6a,7,9,10-tetrahydro-5*H*-pyrazino[1',2':4,5]pyrazino[2,3-*c*]pyridazine-5,8(6H)-dicarboxylate (280 mg, 43.5% yield, peak B, Isomer 2). LCMS calcd for C₂₁H₃₁ClN₅O₆ [M+H]⁺: m/z = 484.2; Found: 484.1.

### Step 6: tert-butyl 2-(3-fluoro-2-hydroxyphenyl)-6a-(methoxymethyl)-6-oxo-5,6, 6a, 7,9,10-hexahydro-8H-pyrazino[1',2':4,5]pyrazino[2,3-c]pyridazine-8-carboxylate (Isomer 1)

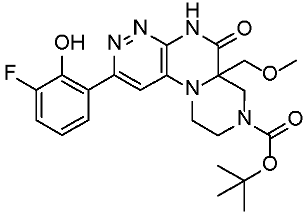

A mixture of di-tert-butyl 4-chloro-10-(methoxymethyl)-9-oxo-1,5,6,8,12-pentaza-tricyclo-[8.4.0.02,7]tetradeca-2(7),3,5-triene-8,12-dicarboxylate (isomer 1) (140.0 mg, 0.29 mmol), 3-fluoro-2-hydroxyphenylboronic acid (90.21 mg, 0.58 mmol) , XPhos Pd G2 (45.5 mg, 0.06 mmol) and cesium carbonate (188.5 mg, 0.58 mmol) in 1,4-dioxane (5 mL) and water (0.5 mL) was stirred at 85 °C for 3h. The reaction was diluted with DCM and washed with water. The organic layer was concentrated and purified on silica gel column (0-100% EA/Hex followed by 0-15% MeOH/DCM) to give tert-butyl 4-(3-fluoro-2-hydroxyphenyl)-10-(methoxymethyl)-9-oxo-1,5,6,8,12-pentazatricyclo[8.4.0.02,7]tetradeca-2(7),3,5-triene-12-carboxylate (120 mg, 0.26 mmol, 90% yield). LCMS calcd for C₂₂H₂₇FN₅O₅ [M+H]+: m/z = 460.2; Found: 460.2.

### Step 7: 2-fluoro-6-(6a-(methoxymethyl)-6, 6a, 7,8,9,10-hexahydro-5H-pyrazino[1',2': 4,5] pyrazino[2,3-c]pyridazin-2-yl)phenol (Isomer 1)

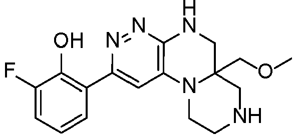

To a mixture of tert-butyl 4-(3-fluoro-2-hydroxyphenyl)-10-(methoxymethyl)-9-oxo-1,5,6,8,12-pentazatricyclo[8.4.0.02,7]tetradeca-2(7),3,5-triene-12-carboxylate (isomer 1) (120.0 mg, 0.26 mmol) in THF (3 mL) at room temperature was added borane;tetrahydrofuran (2.61 mL, 2.61 mmol). The mixture was stirred at 60 °C for 2 hours. The reaction was quenched with drops of MeOH, diluted with EA, washed with sat. aq. NaHCO₃ solution and water, dried over MgSO₄ and concentrated to give the intermediate, which was dissolved in THF (3 mL) and methanol (1.5 mL), to which acetic acid (0.45 mL, 7.83 mmol) and sodium cyanoborohydride (164.12 mg, 2.61 mmol) were added. The mixture was stirred at 80 °C overnight. The mixture was purified on prep-LCMS (Waters CSH-C18, 5 uM, 30x100mm, 23.5-43.5% MeCN/water (containing 0.1%TFA) over 5 min) to give tert-butyl 4-(3-fluoro-2-hydroxyphenyl)-10-(methoxymethyl)-1,5,6,8,12-pentazatricyclo[8.4.0.02,7]tetradeca-2(7),3,5-triene-12-carboxylate (32 mg, 0.07 mmol, 27% yield). The product was treated with 4 N Hydrochloric acid in dioxane (1.0 mL, 4.0 mmol) in DCM (1 mL) at rt for 1h and stripped to dryness to give 2-fluoro-6-[10-(methoxymethyl)-1,5,6,8,12-pentazatricyclo[8.4.0.02,7]tetradeca-2(7),3,5-trien-4-yl]phenol as HCl salt (30 mg, 0.07 mmol, 27% yield). LCMS calcd for C₁₇H₂₁FN₅O; [M+H]+: m/z = 346.2; Found: 346.1.

### Step 8: tert-butyl 4-(2-(3-fluoro-2-hydroxyphenyl)-6a-(methoxymethyl)-6,6a,7,8,9,10-hexahydro-5H-pyrazino[1',2':4,5]pyrazino[2, 3-c]pyridazine-8-carbonyl)-3,3-dimethylpiperazine-1-carboxylate (isomer 1)

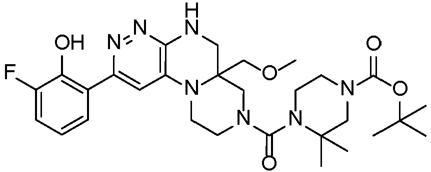

To a solution of 2-fluoro-6-[10-(methoxymethyl)-1,5,6,8,12-pentazatricyclo[8.4.0.02,7] tetradeca-2(7),3,5-trien-4-yl]phenol (isomer 1) (10.0 mg, 0.02 mmol) in DMA (1 mL) was added N,N-diisopropylethylamine (0.04 mL, 0.24 mmol) and 4-(dimethylamino)pyridine (0.88 mg, 0.01 mmol). The mixture was stirred for 5 min and then a solution of *tert-butyl* 4-carbono-chloridoyl-3,3-dimethylpiperazine-1-carboxylate (13.23 mg, 0.05 mmol) in DMA was added. The reaction was stirred at RT overnight. The reaction was quenched with water and purified on prep-CMS (Waters CSH-C18, 5 uM, 30x100mm, 27-47% MeCN/water (containing 0.1% TFA) over 5 min) to give *tert-butyl* 4-(2-(3-fluoro-2-hydroxyphenyl)-6a-(methoxymethyl)-6,6a,7,8,9,10-hexahydro-5H-pyrazino[1',2':4,5]pyrazino[2,3-c]pyridazine-8-carbonyl)-3,3-dimethylpiperazine-1-carboxylate (6 mg, 0.01 mmol, 43% yield). LCMS calcd for C₂₉H₄₁FN₇O₅ [M+H]+: m/z = 586.3; Found: 586.1.

### Step 9: (2,2-dimethylpiperazin-1-yl)(2-(3-fluoro-2-hydroxyphenyl)-6a-(methoxymethyl)-5,6,6a,7,9,10-hexahydro-8H-pyrazino[1',2':4,5]pyrazino[2,3-c]pyridazin-8-yl)methanone (isomer 1)

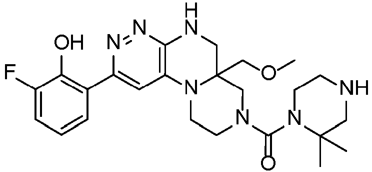

*tert*-butyl 4-(2-(3-fluoro-2-hydroxyphenyl)-6a-(methoxymethyl)-6,6a,7,8,9,10-hexahydro-5*H*-pyrazino[1',2':4,5]pyrazino[2,3-*c*]pyridazine-8-carbonyl)-3,3-dimethylpiperazine-1-carboxylate (isomer 1) (6.0 mg, 0.01 mmol) was treated with 4 N Hydrochloric acid in dioxane (0.69 mL, 2.78 mmol) in DCM (1 mL) at RT for 1 h. The reaction mixture was concentrated to afford (2,2-dimethylpiperazin-1-yl)(2-(3-fluoro-2-hydroxyphenyl)-6a-(methoxymethyl)-5,6,6a,7,9,10-hexahydro-8H-pyrazino[1',2':4,5]pyrazino[2,3-c]pyridazin-8-yl)methanone as HCl salt (5 mg, 0.009 mmol, 87% yield). LCMS calcd for C₂₄H₃₃FN₇O₃ [M+H]+: m/z = 486.3; Found: 486.1.

### Step 10: (3S)-3-(6-(4-((4-(2-(3-fluoro-2-hydroxyphenyl)-6a-(methoxymethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1yl)-1-oxoisoindolin-2-yl)piperidine-2, 6-dione (isomer 1)

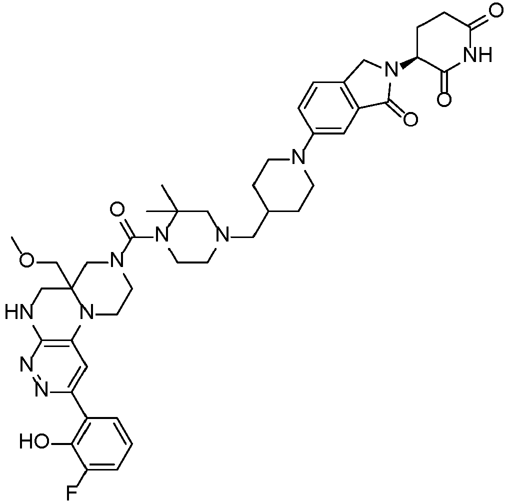

A mixture of (S)-1-(2-(2,6-dioxopiperidin-3-yl)-3-oxoisoindolin-5-yl)piperidine-4-carbaldehyde (6.4 mg, 0.02 mmol), (2,2-dimethylpiperazin-1-yl)(2-(3-fluoro-2-hydroxyphenyl)-6a-(methoxymethyl)-5,6,6a,7,9,10-hexahydro-8H-pyrazino[1',2':4,5]pyrazino[2,3-c]pyridazin-8-yl)methanone (isomer 1) (5.0 mg, 0.01 mmol), N,N-diisopropylethylamine (12.5 uL, 0.07 mmol) and acetic acid (10 uL, 0.18 mmol) in DMF (1.5 mL) was stirred at room temperature for 30 min, then sodium triacetoxyborohydride (7.6 mg, 0.04 mmol) was added. The mixture was stirred at room temperature for 2h and then purified on prep-LCMS (Waters CSH-C18, 5 uM, 30x100mm, 9.3-29.3% MeCN/water (containing 0.1%TFA) over 5 min) to give (3*S*)-3-(6-(4-((4-(2-(3-fluoro-2-hydroxyphenyl)-6a-(methoxymethyl)-6,6a,7,8,9,10-hexahydro-5*H*-pyrazino[1',2':4,5]pyrazino [2,3-c]pyridazine-8-carbonyl)-3,3-dimethylpiperazin-1-yl)methyl)piperidin-1-yl)-1-oxoisoindolin-2-yl)piperidine-2,6-dione as TFA salt (isomer 1) (2.6 mg, 0.003 mmol, 35% yield) . LCMS m/z calcd for C₄₃H₅₄FN₁₀O₆ [M+H]+: m/z = 825.4; Found: 825.1.

### Example 94. ((2S,6R)-2,6-dimethylpiperazin-1-yl)(2-(3-fluoro-2-hydroxyphenyl)-6a-methyl-5,6,6a,7,9,10-hexahydro-8H-pyrazino[1',2':4,5]pyrazino[2,3-c]pyridazin-8-yl)methanone (Isomer 2)

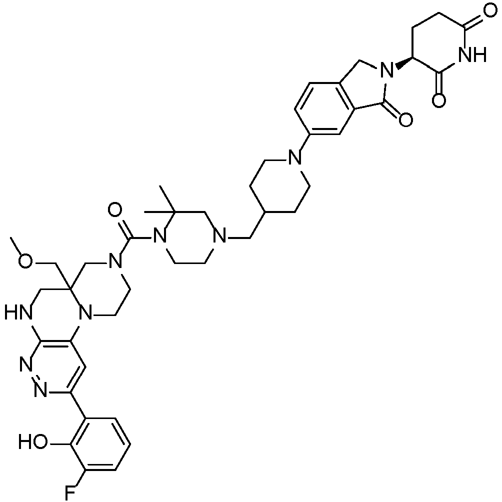

Example 94 was prepared as the TFA salt by the method described in preparing Example 93 using isomer 2 obtained in Step 5.

### Example 95. (3S)-3-(6-(4-(((3S,5R)-4-(2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione (Isomer 1)

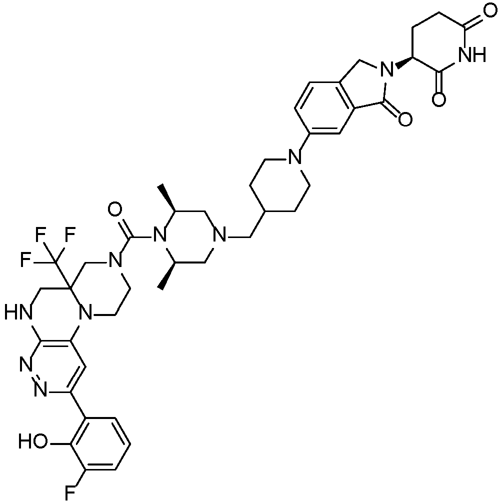

### Step 1: di-tert-butyl 2-chloro-6-oxo-6a-(trifluoromethyl)-6a, 7,9,10-tetrahydro-5H-pyrazino[1',2':4, 5]pyrazino[2,3-c]pyridazine-5, 8(6H)-dicarboxylate (racemic mixture)

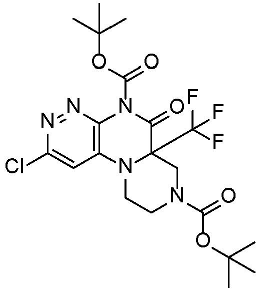

To a solution of di-*tert*-butyl 4-chloro-9-oxo-1,5,6,8,12-pentazatricyclo[8.4.0.02,7] tetradeca-2(7),3,5-triene-8,12-dicarboxylate (1.0 g, 2.27 mmol) in THF (12 mL) was added LiHMDS (4.55 mL, 4.55 mmol) at -78 °C dropwise. The mixture was stirred at -78 °C for 30 min. Then 1-(trifluoromethyl)-1,2-benziodoxol-3(1H)-one (1.08 g, 3.41 mmol) was added in one portion. The reaction was stirred at -78 °C for 1h. The reaction was diluted with EA, washed with brine and water, concentrated and purified on silica gel column (0-100% EA/Hex) to give *di-tert-*butyl 4-chloro-9-oxo-10-(trifluoromethyl)-1,5,6,8,12-pentazatricyclo [8.4.0.02,7]tetradeca-2(7), 3,5-triene-8,12-dicarboxylate (310 mg, 0.61 mmol, 27% yield). LCMS calcd for C₂₀H₂₆ClF₃N₅O₅ [M+H]+: m/z = 508.2; Found: 508.1.

### Step 2: Chiral separation of di-tert-butyl 2-chloro-6-oxo-6a-(trifluoromethyl)-6a,7,9,10-tetrahydro-5H-pyrazino[1',2':4,5]pyrazino[2,3-c]pyridazine-5,8(6H)-dicarboxylate (Isomer 1 and 2)

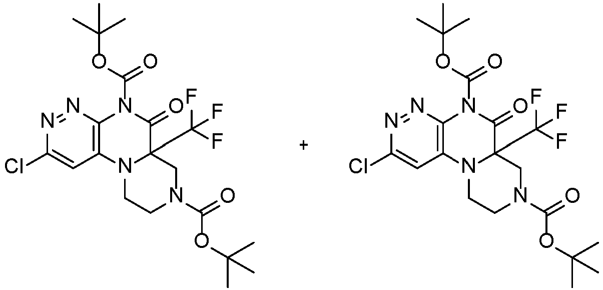

The racemic mixture of di-tert-butyl 4-chloro-9-oxo-10-(trifluoromethyl)-1,5,6,8,12-pentazatricyclo[8.4.0.02,7]tetradeca-2(7),3,5-triene-8,12-dicarboxylate (310 mg, 0.61 mmol) was subjected to chiral separation (Lux Cellulose-2 column, 20 mL/min of 10:90 Hexanes/IPA:MeOH (1:1)) to give di-tert-butyl 4-chloro-9-oxo-10-(trifluoromethyl)-1,5,6,8,12-pentazatricyclo[8.4.0.02,7]tetradeca-2(7),3,5-triene-8,12-dicarboxylate (150 mg, peak A, isomer 1) and di-tert-butyl 4-chloro-9-oxo-10-(trifluoromethyl)-1,5,6,8,12-pentazatricyclo[8.4.0.02,7] tetradeca-2(7),3,5-triene-8,12-dicarboxylate (160 mg, peak B, isomer 2). LCMS calcd for C₂₀H₂₆ClF₃N₅O₅ [M+H]+: m/z = 508.2; Found: 508.1.

### Step 3: tert-butyl 2-(3-fluoro-2-hydroxyphenyl)-6-oxo-6a-(trifluoromethyl)-5,6,6a, 7,9,10-hexahydro-8H-pyrazino[1',2':4,5]pyrazino[2,3-c]pyridazine-8-carboxylate (isomer 1)

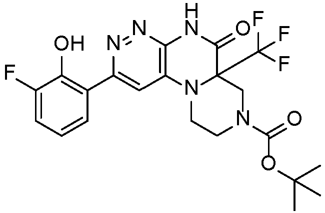

A mixture of di-tert-butyl 4-chloro-9-oxo-10-(trifluoromethyl)-1,5,6,8,12-pentazatricyclo [8.4.0.02,7]tetradeca-2(7),3,5-triene-8,12-dicarboxylate (isomer 1) (150.0 mg, 0.3 mmol), 3-fluoro-2-hydroxyphenylboronic acid (92.1 mg, 0.59 mmol), XPhos Pd G2 (46.5 mg, 0.06 mmol) and cesium carbonate (192.5 mg, 0.59 mmol) in 1,4-dioxane (3 mL) and water (0.3 mL) was stirred at 90 °C for 2h. The reaction was diluted with EA, washed with brine, concentrated and purified on silica gel column (0-30% Acetone/Hex followed by 0-10% MeOH/DCM) to give *tert-butyl* 2-(3-fluoro-2-hydroxyphenyl)-6-oxo-6a-(trifluoromethyl)-5,6,6a,7,9,10-hexahydro-8*H*-pyrazino[1',2':4,5]pyrazino[2,3-c]pyridazine-8-carboxylate (130 mg, 0.27 mmol, 91% yield). LCMS calcd for C₂₁H₂₂F₄N₅O₄ [M+H]+: m/z = 484.2; Found: 484.1.

### Step 4: 2-fluoro-6-(6a-(trifluoromethyl)-6,6a,7,8,9,10-hexahydro-5H-pyrazino[1',2':4,5] pyrazino[2,3-c]pyridazin-2-yl)phenol (isomer 1)

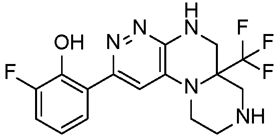

To a mixture of *tert-butyl* 2-(3-fluoro-2-hydroxyphenyl)-6-oxo-6a-(trifluoromethyl)-5,6,6a,7,9,10-hexahydro-8H-pyrazino[1',2':4,5]pyrazino[2,3-c]pyridazine-8-carboxylate (isomer 1) (138.0 mg, 0.29 mmol) in THF (3 mL) at room temperature was added borane;tetrahydrofuran (2.85 mL, 2.85 mmol). The mixture was stirred at 60 °C for 2 hours. The reaction was quenched with 3 drops of MeOH, diluted with EA, washed with sat. aq. NaHCO₃ solution and water, dried over MgSO₄ and concentrated to give the intermediate, which was dissolved in THF (4 mL) and methanol (4 mL), to which acetic acid (0.49 mL, 8.56 mmol) and sodium cyanoborohydride (179 mg, 2.85 mmol) were added. The mixture was stirred at 80 °C overnight. The mixture was purified on prep-LCMS (Waters CSH-C18 column, 25.1-45.1% MeCN/water (containing 0.1% TFA) over 5 min) to give *tert-butyl* 2-(3-fluoro-2-hydroxyphenyl)-6a-(trifluoromethyl)-5,6,6a,7,9,10-hexahydro-8H-pyrazino[1',2':4,5]pyrazino[2,3-c]pyridazine-8-carboxylate (41 mg, 0.087 mmol, 31% yield). The product was treated with 4 N hydrochloric acid in dioxane (0.97 mL, 3.9 mmol) in DCM (1 mL) at room temperature for 1h and stripped to dryness to give 2-fluoro-6-(6a-(trifluoromethyl)-6,6a,7,8,9,10-hexahydro-5*H*-pyrazino[1',2':4,5]pyrazino[2,3-c]pyridazin-2-yl)phenol as HCl salt (38 mg, 0.086 mmol, 30.1% yield). LCMS calcd for C₁₆H₁₆F₄N₅O [M+H]+: m/z = 370.2; Found: 370.2.

### Step 5: tert-butyl (3S,5R)-4-(2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,5-dimethylpiperazine-1-carboxylate (isomer 1)

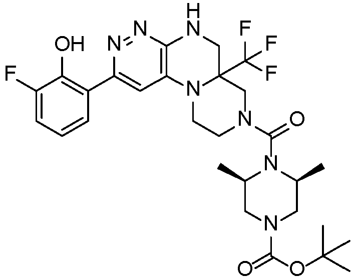

To a solution of 2-fluoro-6-(6a-(trifluoromethyl)-6,6a,7,8,9,10-hexahydro-5H-pyrazino [1',2':4,5]pyrazino[2,3-c]pyridazin-2-yl)phenol (isomer 1) (8.0 mg, 0.02 mmol) in DMA (1 mL) was added N,N-diisopropylethylamine (0.03 mL, 0.18 mmol) and 4-(dimethylamino) pyridine (0.66 mg, 0.01 mmol). The mixture was stirred for 5 min and then a solution of tert-butyl (3*R*,5*S*)-4-(chlorocarbonyl)-3,5-dimethylpiperazine-1-carboxylate (10.0 mg, 0.04 mmol) in DMA was added. The reaction was stirred at room temperature overnight. The reaction was quenched with water and purified on prep-LCMS (Waters CSH-C18 column, 28-48% MeCN/water (containing 0.1% TFA) over 5 min) to give *tert-butyl* (3S,SR)-4-(2-(3-fluoro-2-hydroxyphenyl)-6a-(trifluoromethyl)-6,6a,7,8,9,10-hexahydro-5*H*-pyrazino[1',2':4,5]pyrazino[2,3-*c*]pyridazine-8-carbonyl)-3,5-dimethylpiperazine-1-carboxylate (4.6 mg, 0.007 mmol, 41% yield). LCMS calcd for C₂₈H₃₆F₄N₇O₄ [M+H]+: m/z = 610.3; Found: 610.1.

### Step 6: ((2S,6R)-2,6-dimethylpiperazin-1-yl)(2-(3-fluoro-2-hydroxyphenyl)-6a-(trijluoromethyl)-5,6,6a,7,9,10-hexahydro-8H-pyrazino[1',2':4,5]pyrazino[2,3-c]pyridazin-8-yl)methanone

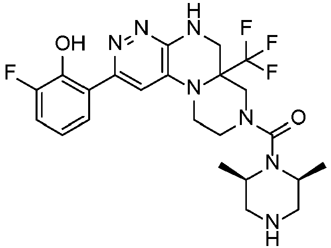

*tert-*butyl (3*S*,5*R*)-4-(2-(3-fluoro-2-hydroxyphenyl)-6a-(trifluoromethyl)-6,6a,7,8,9,10-hexahydro-5*H*-pyrazino[1',2':4,5]pyrazino[2,3-*c*]pyridazine-8-carbonyl)-3,5-dimethylpiperazine-1-carboxylate (isomer 1) (4.6 mg, 0.01 mmol) was treated with 4 N hydrochloric acid in dioxane (0.55 mL, 2.19 mmol) in DCM (1 mL) at RT for 1 h. The reaction mixture was concentrated to afford ((2S,6R)-2,6-dimethylpiperazin-1-yl)(2-(3-fluoro-2-hydroxyphenyl)-6a-(trifluoromethyl)-5,6,6a,7,9,10-hexahydro-8*H*-pyrazino[1',2':4,5]pyrazino[2,3-*c*]pyridazin-8-yl) methanone as HCl salt (4 mg, 0.007 mmol, 91% yield). LCMS calcd for C₂₃H₂₈F₄N₇O₂ [M+H]+: m/z = 510.2; Found: 510.1.

### Step 7: (3S)-3-(6-(4-(((3S,5R)-4-(2-(3-fluoro-2-hydroxyphenyl)-6a-(trifluoromethyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1yl)-1-oxoisoindolin-2-yl)piperidine-2, 6-dione (isomer 1)

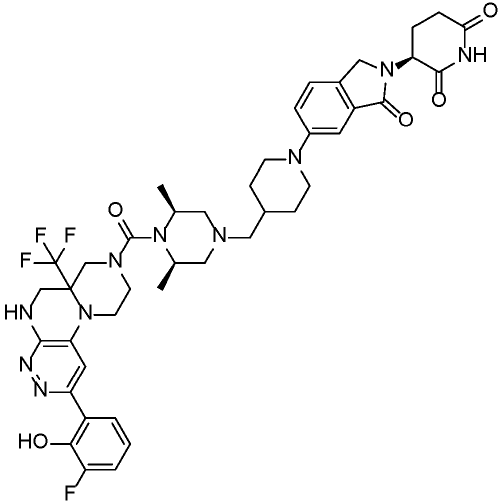

A mixture of (*S*)-1-[2-(2,6-dioxopiperidin-3-yl)-3-oxo-1H-isoindol-5-yl]piperidine-4-carbaldehyde (6.1 mg, 0.02 mmol), ((2S,6R)-2,6-dimethylpiperazin-1-yl)(2-(3-fluoro-2-hydroxyphenyl)-6a-(trifluoromethyl)-5,6,6a,7,9,10-hexahydro-8*H*-pyrazino[1',2':4,5]pyrazino [2,3-c]pyridazin-8-yl)methanone (5.0 mg, 0.01 mmol), N,N-diisopropylethylamine (12 uL, 0.07 mmol) and acetic acid (9.8 uL, 0.17 mmol) in DMF (1.5 mL) was stirred at rt for 30 min, then sodium triacetoxyborohydride (7.3 mg, 0.03 mmol) was added. The mixture was stirred at RT for 2h and then purified on prep-LCMS (Waters CSH-C18, 5 uM, 30x100mm, 8.7-28.7% MeCN/water (containing 0.1%TFA) over 5 min) to give (3S)-3-(6-(4-(((3*S*,5*R*)-4-(2-(3-fluoro-2-hydroxyphenyl)-6a-(trifluoromethyl)-6,6a,7,8,9,10-hexahydro-5*H*-pyrazino[1',2':4,5]pyrazino [2,3-c]pyridazine-8-carbonyl)-3,5-dimethylpiperazin-1-yl)methyl)piperidin-1-yl)-1-oxoisoindolin-2-yl)piperidine-2,6-dione as TFA salt (1 mg, 0.001 mmol, 14% yield). LCMS calcd for C₄₂H₄₉F₄N₁₀O₅ [M+H]+: m/z = 849.4; Found: 849.1.

### Examples 96 - 101

Examples 96-101 shown below in Table 17 were prepared as TFA salts by the method used in preparing Example 95 using the appropriate intermediates and starting materials.

**Table 17. Examples 96-101**

| **Ex.** | **Structure/Name** | **Calcd. (M+H)⁺ m/z** | **Found (M+H)⁺ m/z** |
|---|---|---|---|
| **96** | 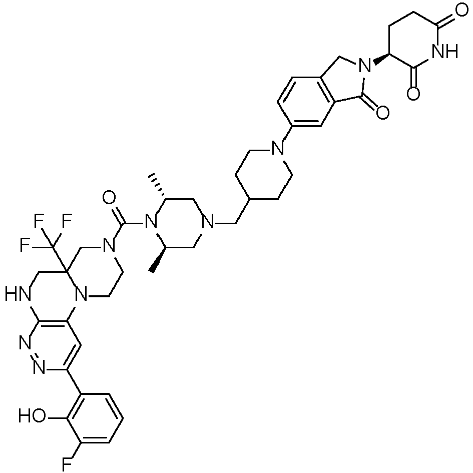 (3S)-3 -(6-(4-(((3*R*, 5*R*)-4-(2-(3 -fluoro-2-hydroxyphenyl)-6a-(trifluoromethyl)-6,6a,7,8,9,10-hexahydro-5*H*-pyrazino[1',2':4,5] pyrazino[2,3-*c*]pyridazine-8-carbonyl)-3,5-dimethylpiperazin-1-yl)methyl)piperidin-1-yl)-1-oxoisoindolin-2-yl)piperidine-2,6-dione (isomer 1) | 849.4 | 849.3 |
| **97** | 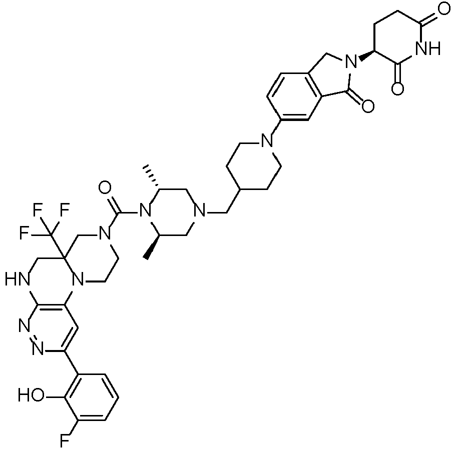 (3*S*)-3-(6-(4-(((3*R*,5*R*)-4-(2-(3-fluoro-2-hydroxyphenyl)-6a-(trifluoromethyl)-6,6a,7,8,9,10-hexahydro-5*H*-pyrazino[1',2':4,5] pyrazino[2,3-c]pyridazine-8-carbonyl)-3,5-dimethylpiperazin-1-yl)methyl)piperidin-1-yl)-1-oxoisoindolin-2-yl)piperidine-2,6-dione (isomer 2) | 849.4 | 849.1 |
| **98** | 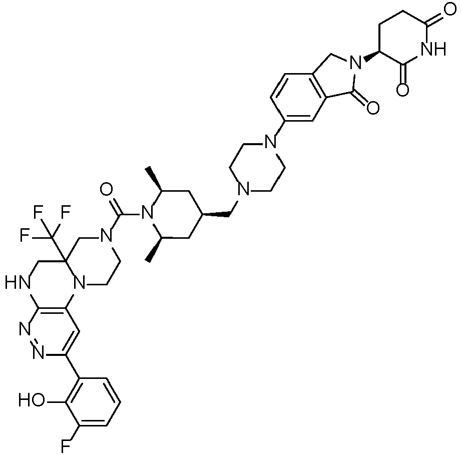 (3*S*)-3-(6-(4-(((2*R*,4*r*,6*S*)-1-(2-(3-fluoro-2-hydroxyphenyl)-6a-(trifluoromethyl)-6,6a,7,8,9,10-hexahydro-5*H*-pyrazino[1',2':4,5] pyrazino[2,3-*c*]pyridazine-8-carbonyl)-2,6-dimethylpiperidin-4-yl)methyl)piperazin-1-yl)-1-oxoisoindolin-2-yl)piperidine-2,6-dione (isomer 1) | 849.4 | 849.1 |
| **99** | 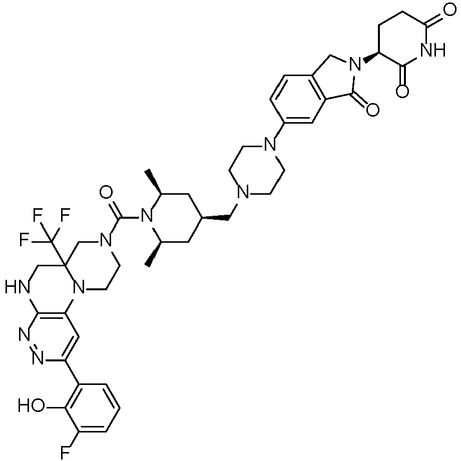 (3*S*)-3-(6-(4-(((2*R*,4*r*,6*S*)-1-(2-(3-fluoro-2-hydroxyphenyl)-6a-(trifluoromethyl)-6,6a,7,8,9,10-hexahydro-5*H*-pyrazino[1',2':4,5] pyrazino[2,3-c]pyridazine-8-carbonyl)-2,6-dimethylpiperidin-4-yl)methyl)piperazin-1-yl)-1-oxoisoindolin-2-yl)piperidine-2,6-dione (isomer 2) | 849.4 | 849.1 |
| **100** | 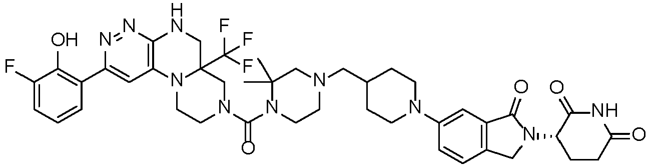 (3*S*)-3-(6-(4-((4-(2-(3-fluoro-2-hydroxyphenyl)-6a-(trifluoromethyl)-6,6a,7,8,9,10-hexahydro-5*H*-pyrazino[1',2':4,5]pyrazino [2,3-c]pyridazine-8-carbonyl)-3,3-dimethylpiperazin-1-yl)methyl) piperidin-1-yl)-1-oxoisoindolin-2-yl)piperidine-2,6-dione (isomer 1) ¹H NMR (400 MHz, DMSO) δ 11.00 (s, 1H), 9.34 (s, 1H), 8.33 (s, 1H), 7.50 - 7.39 (m, 2H), 7.32 (d, *J* = 8.1 Hz, 2H), 7.25 - 7.20 (m, 2H), 7.02 (td, *J* = 8.0, 4.9 Hz, 1H), 5.11 (dd, *J =* 13.2, 5.1 Hz, 1H), 4.37 (d, *J=* 16.9 Hz, 1H), 4.23 (d, *J* = 16.9 Hz, 1H), 4.15 - 4.10 (m, 1H), 3.84 - 3.81 (m, 3H), 3.68 (d, *J=* 10.1 Hz, 2H), 3.52 - 3.19 (m, 6H), 3.15 - 3.04 (m, 3H), 2.93 - 2.79 (m, 5H), 2.69 - 2.55 (m, 1H), 2.40 (ddd, *J* = 26.0, 13.1, 4.4 Hz, 2H), 2.09 - 1.99 (m 2H), 1.83 (d, *J= 11.9* Hz, 2H), 1.51-1.34 (m, 8H). | 849.4 | 849.1 |
| **101** | 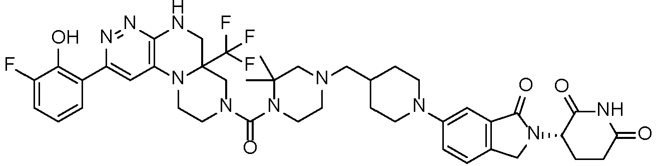 (3*S*)-3-(6-(4-((4-(2-(3-fluoro-2-hydroxyphenyl)-6a-(trifluoromethyl)-6,6a,7,8,9,10-hexahydro-5*H*-pyrazino[1',2':4,5]pyrazino [2,3-c]pyridazine-8-carbonyl)-3,3-dimethylpiperazin-1-yl)methyl) piperidin-1-yl)-1-oxoisoindolin-2-yl)piperidine-2,6-dione (isomer 2) | 849.4 | 849.1 |

### Example 102. (3S)-3-(6-(4-((4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluorocyclo-hexyl)methyl)piperazin-1-yl)-1-oxoisoindolin-2-yl)piperidine-2,6-dione (Isomer 2)

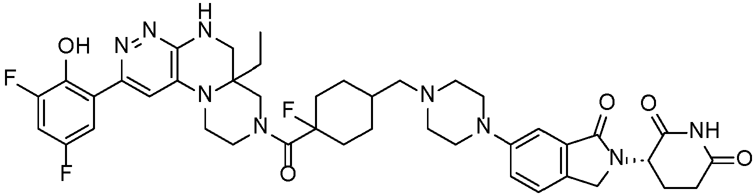

### Step 1: methyl 1-fluoro-4-formylcyclohexane-1-carboxylate

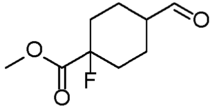

(Methoxymethyl)triphenylphosphonium chloride (433.0 mg, 1.26 mmol) was suspended in THF (5 mL) and cooled to -78 °C. A solution of LiHMDS (1.26 mL, 1.26 mmol) was added drop-wise. The reaction mixture was then stirred at -78 °C for another 2 hours followed by the addition of a solution of methyl 1-fluoro-4-oxocyclohexane-1-carboxylate (200.0 mg, 1.15 mmol) in THF (2 mL). The reaction was slowly warmed to room temperature and stirred overnight. Next, hydrochloric acid (2.0 mL, 4.0 mmol) was added and the reaction was stirred for 2 hrs. The reaction mixture was extracted with ethyl acetate. The organic layer was dried with Na₂SO₄, filtered, and concentrated to give crude methyl 1-fluoro-4-formylcyclohexane-1-carboxylate (216 mg, 1.15 mmol, 99% yield) which was used directly in the next step.

### Step 2: methyl (S)-4-((4-(2-(2,6-dioxopiperidin-3-yl)-3-oxoisoindolin-5-yl)piperazin-1-yl)methyl)-1-fluorocyclohexane-1-carboxylate (isomer 1)

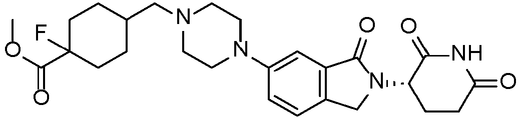

A mixtue of methyl 1-fluoro-4-formylcyclohexane-1-carboxylate (211 mg, 1.12 mmol), (*S*)-3-(1-oxo-6-(piperazin-1-yl)isoindolin-2-yl)piperidine-2,6-dione (97.0 mg, 0.3 mmol) and acetic acid (0.03 mL, 0.59 mmol) in DMF (5 mL) was stirred at rt for 20 min. Sodium triacetoxyborohydride (187 mg, 0.89 mmol) was then added and the mixture was stirred at rt for 1h. The mixture was purified by prep-LCMS (Waters CSH-C18, 5 uM, 30x100mm, 10.3-30.3% MeCN/water (containing 0.1%TFA) over 5 min) to obtained (S)-4-((4-(2-(2,6-dioxopiperidin-3-yl)-3-oxoisoindolin-5-yl)piperazin-1-yl)methyl)-1-fluorocyclohexane-1-carboxylate (peak 1) (35 mg, 0.07 mmol, 24% yield). LCMS calcd for C₂₆H₃₄FN₄O₅ [M+H]+: m/z = 501.2; Found: 501.2.

### Step 3: (S)-4-((4-(2-(2, 6-dioxopiperidin-3-yl)-3-oxoisoindolin-5yl)piperazin-1-yl)methyl)-1-fluorocyclohexane-1-carboxylic acid (isomer 1)

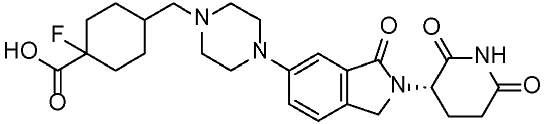

(S)-4-((4-(2-(2,6-dioxopiperidin-3-yl)-3-oxoisoindolin-5-yl)piperazin-1-yl)methyl)-1-fluorocyclohexane-1-carboxylate (35.0 mg, 0.07 mmol) was treated with HCl (1.0 mL, 2.0 mmol) in THF (1 mL) at 90 °C for 2h. The mixture was purified on prep-LCMS (Waters CSH-C18, 5 uM, 30x100mm, 5.4-25.4% MeCN/water (containing 0.1%TFA) over 5 min) to give (S)-4-((4-(2-(2,6-dioxopiperidin-3-yl)-3-oxoisoindolin-5-yl)piperazin-1-yl)methyl)-1-fluorocyclo-hexane-1-carboxylic acid (25 mg, 0.05 mmol, 86% yield). LCMS calcd for C₂₅H₃₂FN₄O₅ [M+H]+: m/z = 487.1; Found: 487.2.

### Step 4: (3S)-3-(6-(4-((4-(2-(3, 5-difluoro-2-hydroxyphenyl)-6a-ethyl-6, 6a, 7,8,9,10-hexahydro-5H-pyrazino[1',2':4,5]pyrazino[2,3-c]pyridazine-8-carbonyl)-4-fluorocyclo-hexyl)methyl)piperazin-1-yl)-1-oxoisoindolin-2-yl)piperidine-2,6-dione (isomer 2)

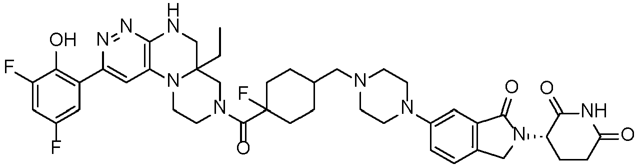

A mixture of *N*,*N-*diisopropylethylamine (27 uL, 0.16 mmol) , 1-[bis(dimethylamino)-methylene]-1*H*-1,2,3-triazolo[4,5-b]pyridinium 3-oxid hexafluorophosphate (11.9 mg, 0.03 mmol), (*S*)-4-((4-(2-(2,6-dioxopiperidin-3-yl)-3-oxoisoindolin-5-yl)piperazin-1-yl)methyl)-1-fluorocyclohexane-1-carboxylic acid (11.5 mg, 0.02 mmol) and 2,4-difluoro-6-[rac-10-ethyl-1,5,6,8,12-pentazatricyclo[8.4.0.02,7]-tetradeca-2(7),3,5-trien-4-yl]phenol dihydrochloride (6.6 mg, 0.02 mmol) in DMF (1 mL) was stirred at rt for 2h. The mixture was purified on prep-LCMS (Waters CSH-C18, 5 uM, 30x100mm, 5.4-25.4% MeCN/water (containing 0.1%TFA) over 5 min) to give (3S)-3-[5-[4-[[4-[(10S)-4-(3,5-difluoro-2-hydroxyphenyl)-10-ethyl-1,5,6,8,12-pentazatricyclo[8.4.0.02,7]tetradeca-2(7),3,5-triene-12-carbonyl]-4-fluorocyclohexyl]methyl] piperazin-1-yl]-3-oxo-1H-isoindol-2-yl]piperidine-2,6-dione (1 mg, 0.001 mmol, 7.8% yield). LCMS calcd for C₄₂H₄₉F₃N₉O₅ [M+H]+: m/z = 816.4; Found: 816.3.

### Examples 103. (3S)-3-(6-(4-((4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluorocyclo-hexyl)methyl)piperazin-1-yl)-1-oxoisoindolin-2-yl)piperidine-2,6-dione (Isomer 1)

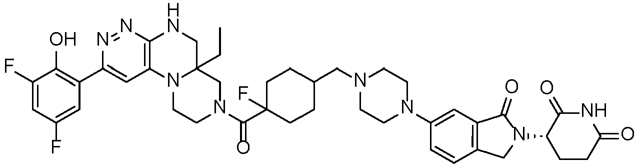

Example 103 was prepared as the TFA salt by the method described in preparing Example 102.

### Example 104. 3-(6-(4-(((1S,4r)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)cyclohexyl) methyl)piperazin-1-yl)-1-oxoisoindolin-2-yl)piperidine-2,6-dione

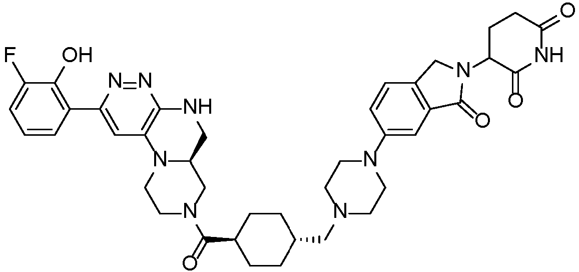

### Step 1: ((S)-2-(3-fluoro-2-hydroxyphenyl)-5,6,6a, 7,9,10-hexahydro-8H-pyrazino [1',2':4,5]pyrazino[2,3-c]pyridazin-8-yl)((1r,4S)-4-(hydroxymethyl)cyclohexyl)methanone

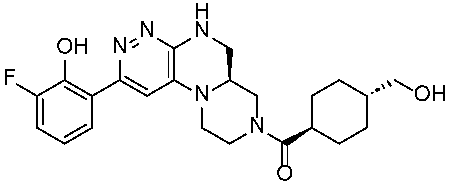

A mixture of 2-fluoro-6-[(10*R*)-1,5,6,8,12-pentazatricyclo[8.4.0.02,7]tetradeca-2(7),3,5-trien-4-yl]phenol;dihydrochloride (85.0 mg, 0.23 mmol), trans-4-(hydroxymethyl)cyclohexanecarboxylic acid (43.1 mg, 0.27 mmol), 1-[bis(dimethylamino)methylene]-1H-1,2,3-triazolo[4,5-b]pyridinium 3-oxid hexafluorophosphate (129 mg, 0.34 mmol) and N,N-diisopropylethylamine (0.2 mL, 1.14 mmol) in DMF (1 mL) was stirred at rt for 1h. The mixture was diluted with water, extracted with DCM, concentrated and purified on silica gel (0-100% EA/Hex followed by 0-10% MeOH/DCM) to give [(10*S*)-4-(3-fluoro-2-hydroxyphenyl)-1,5,6,8,12-pentazatricyclo [8.4.0.02,7]tetradeca-2(7),3,5-trien-12-yl]-[4-(hydroxymethyl)cyclohexyl]methanone (39 mg, 0.09 mmol, 39% yield). LCMS calcd for C₂₃H₂₉FN₅O₃ [M+H]+: m/z = 442.2; Found: 442.2.

### Step 2: 3-(6-(4-(((1S,4r)-4-((S)-2-(3-fluoro-2-hydroxyphenyl)-6,6a,7,8,9,10-hexahydro-5H-pyrazino-[1',2':4,5]pyrazino[2,3-c]pyridazine-8-carbonyl)cyclohexyl)methyl)piperazin-1-yl)-1-oxoisoindolin-2-yl)piperidine-2,6-dione

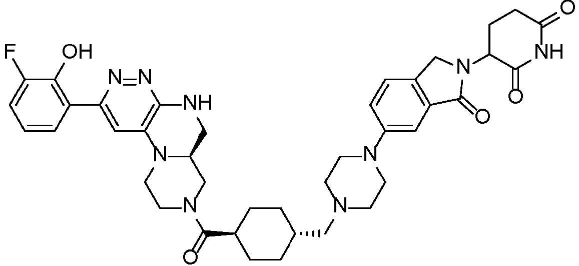

To a mixture of [(10*S*)-4-(3-fluoro-2-hydroxyphenyl)-1,5,6,8,12-pentazatricyclo-[8.4.0.02,7]tetradeca-2(7),3,5-trien-12-yl]-[4-(hydroxymethyl)cyclohexyl]methanone (19.5 mg, 0.04 mmol) and triethylamine (0.09 mL, 0.66 mmol) in DMSO (0.45 mL) was added sulfurtrioxide pyridine (40 mg, 0.25 mmol) in DMSO (0.45 mL) dropwise. Reaction mixture allowed to warm to rt and stirred 3.5 hours, then added 3-(3-oxo-5-piperazin-1-yl-1H-isoindol-2-yl)piperidine-2,6-dione (14.5 mg, 0.04 mmol) and MeCN (0.5 mL). Cooled to 0 °C and added acetic acid (0.1 mL, 1.77 mmol), then allowed to warm to rt and stirred 45 minutes. Added sodium triacetoxyborohydride (48 mg, 0.23 mmol) and stirred at rt 1.5 hours. Quenched with water, and purified on prep-LCMS (Waters CSH-C18, 5 uM, 30x100mm, 7.6-27.6% MeCN/water (containing 0.1%TFA) over 5 min) to give 3-[5-[4-[[4-[(10S)-4-(3-fluoro-2-hydroxyphenyl)-1,5,6,8,12-pentazatricyclo[8.4.0.02,7]tetradeca-2(7),3,5-triene-12-carbonyl] cyclohexyl]methyl]piperazin-1-yl]-3-oxo-1*H*-isoindol-2-yl]piperidine-2,6-dione (3.6 mg, 0.005 mmol, 11% yield). LCMS calcd for C₄₀H₄₇FN₉O₅ [M+H]+: m/z = 752.4; Found: 752.2.

### Examples 105 - 113

Examples 105-113 shown below in Table 18 were prepared as TFA salts by the method used in preparing Example 104 using the appropriate intermediates and starting materials.

**Table 18. Examples 105 - 113**

| **Example** | **Structure/Name** | **Calcd. (M+H) ⁺ m/z** | **Found (M+H) ⁺ m/z** |
|---|---|---|---|
| **105** | 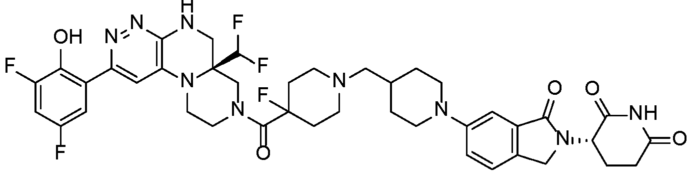 (*S*)-3-(6-(4-((4-((*R*)-2-(3,5-difluoro-2-hydroxyphenyl)-6a-(difluoromethyl)-6,6a,7,8,9,10-hexahydro-5*H*-pyrazino[1',2':4,5] pyrazino[2,3-*c*]pyridazine-8-carbonyl)-4-fluoropiperidin-1-yl)methyl)piperidin-1-yl)-1-oxoisoindolin-2-yl)piperidine-2,6-dione | 838.4 | 838.1 |
| **106** | 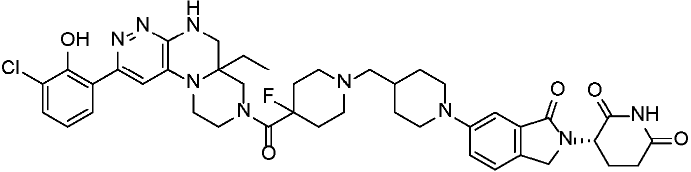 (3*S*)-3-(6-(4-((4-(2-(3-chloro-2-hydroxyphenyl)-6a-ethyl-6,6a,7,8,9,10-hexahydro-5*H*-pyrazino[1',2':4,5]pyrazino[2,3-*c*] pyridazine-8-carbonyl)-4-fluoropiperidin-1-yl)methyl)piperidin-1-yl)-1-oxoisoindolin-2-yl)piperidine-2,6-dione (isomer 1) | 814.4 | 814.1 |
| **107** | 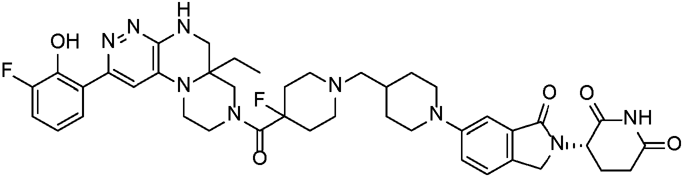 (3*S*)-3-(6-(4-((4-(6a-ethyl-2-(3-fluoro-2-hydroxyphenyl)-6,6a,7,8,9,10-hexahydro-5*H*-pyrazino[1',2':4,5]pyrazino[2,3-*c*] pyridazine-8-carbonyl)-4-fluoropiperidin-1-yl)methyl)piperidin-1-yl)-1-oxoisoindolin-2-yl)piperidine-2,6-dione (isomer 1) | 798.4 | 798.1 |
| **108** | 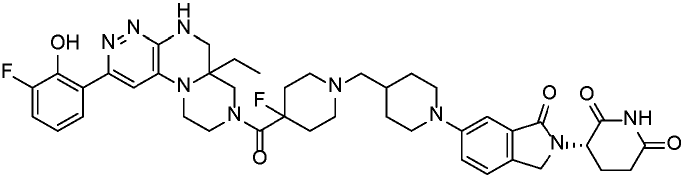 (3*S*)-3-(6-(4-((4-(6a-ethyl-2-(3-fluoro-2-hydroxyphenyl)-6,6a,7,8,9,10-hexahydro-5*H*-pyrazino[1',2':4,5]pyrazino[2,3-*c*] pyridazine-8-carbonyl)-4-fluoropiperidin-1-yl)methyl)piperidin-1-yl)-1-oxoisoindolin-2-yl)piperidine-2,6-dione (isomer 2) | 798.4 | 798.1 |
| **109** | 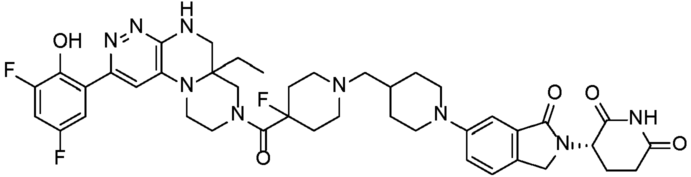 (3*S*)-3-(6-(4-((4-(2-(3,5-difluoro-2-hydroxyphenyl)-6a-ethyl-6,6a,7,8,9,10-hexahydro-5*H*-pyrazino[1',2':4,5]pyrazino[2,3-*c*] pyridazine-8-carbonyl)-4-fluoropiperidin-1-yl)methyl)piperidin-1-yl)-1-oxoisoindolin-2-yl)piperidine-2,6-dione (isomer 1) | 816.4 | 816.2 |
| **110** | 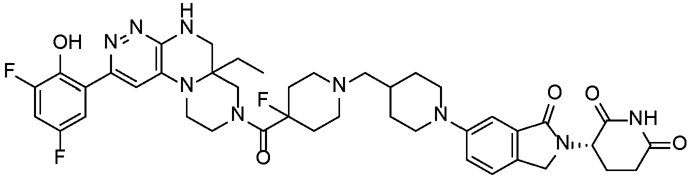 (3*S*)-3-(6-(4-((4-(2-(3,5-difluoro-2-hydroxyphenyl)-6a-ethyl-6,6a,7,8,9,10-hexahydro-5H-pyrazino[1',2':4,5]pyrazino[2,3-c] pyridazine-8-carbonyl)-4-fluoropiperidin-1-yl)methyl)piperidin-1-yl)-1-oxoisoindolin-2-yl)piperidine-2,6-dione (isomer 2) | 816.4 | 816.3 |
| **111** | 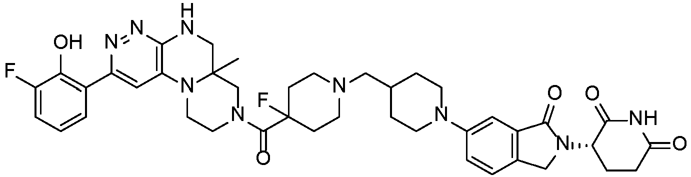 (3*S*)-3-(6-(4-((4-fluoro-4-(2-(3-fluoro-2-hydroxyphenyl)-6a-methyl-6,6a,7,8,9,10-hexahydro-5H-pyrazino[1',2':4,5]pyrazino[2,3-c] pyridazine-8-carbonyl)piperidin-1-yl)methyl)piperidin-1-yl)-1-oxoisoindolin-2-yl)piperidine-2,6-dione (isomer 1) | 784.4 | 784.2 |
| **112** | 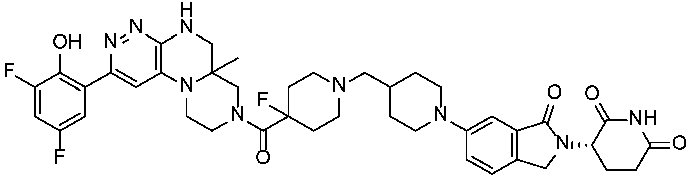 (3*S*)-3-(6-(4-((4-(2-(3,5-difluoro-2-hydroxyphenyl)-6a-methyl-6,6a,7,8,9,10-hexahydro-5H-pyrazino[1',2':4,5]pyrazino[2,3-c] pyridazine-8-carbonyl)-4-fluoropiperidin-1-yl)methyl)piperidin-1-yl)-1-oxoisoindolin-2-yl)piperidine-2,6-dione (Isomer 2) | 802.4 | 802.3 |
| **113** | 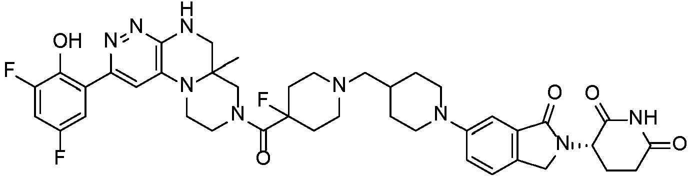 (3*S*)-3-(6-(4-((4-(2-(3,5-difluoro-2-hydroxyphenyl)-6a-methyl-6,6a,7,8,9,10-hexahydro-5*H*-pyrazino[1',2':4,5]pyrazino[2,3-c] pyridazine-8-carbonyl)-4-fluoropiperidin-1-yl)methyl)piperidin-1-yl)-1-oxoisoindolin-2-yl)piperidine-2,6-dione (Isomer 1) | 802.4 | 802.4 |

### Examples 114 - 118

Examples 114-118 shown below in Table 19 were prepared as TFA salts by the method described in the preparation of Example 54 using the appropriate intermediates and starting materials.

**Table 19. Examples 114-118**

| **Example** | **Structure/Name** | **Calcd. (M+H)⁺ m/z** | **Found (M+H)⁺ m/z** |
|---|---|---|---|
| **114** | 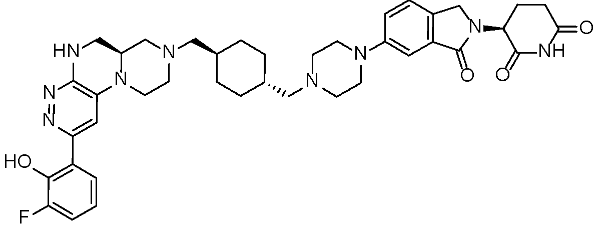 (*S*)-3-(6-(4-(((1*S*,4*S*)-4-(((*S*)-2-(3-fluoro-2-hydroxyphenyl)-5,6,6a,7,9,10-hexahydro-8H-pyrazino[1',2':4,5]pyrazino[2,3-*c*]pyridazin-8-yl)methyl)cyclohexyl)methyl)piperazin-1-yl)-1-oxoisoindolin-2-yl)piperidine-2,6-dione | 738.4 | 738.6 |
| | ¹H NMR (300 MHz, MeOD) δ 7.51 (d, *J* = 8.3 Hz, 1H), 7.39 - 7.30 (m, 5H), 7.02 (td, *J =* 8.1, 4.8 Hz, 1H), 5.13 (dd, *J =* 13.3, 5.1 Hz, 1H), 4.50 - 4.35 (m, 3H), 4.13 - 3.50 (m, 10H), 3.42 (dd, *J* = 12.5, 7.0 Hz, 1H), 3.26 - 2.65 (m, 11H), 2.49 (qd, *J =* 13.1, 4.8 Hz, 1H), 2.21 - 1.90 (m, 7H), 1.26 (dt, *J =* 19.0, 8.2 Hz, 4H). | | |
| **115** | 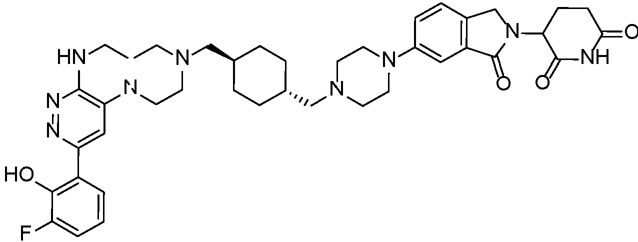 3-(6-(4-(((1*R*,4*r*)-4-(((*R*)-2-(3-fluoro-2-hydroxyphenyl)-6a-methyl-5,6,6a,7,9,10-hexahydro-8*H*-pyrazino[1',2':4,5] pyrazino[2,3-*c*]pyridazin-8-yl)methyl)cyclohexyl)methyl) piperazin-1-yl)-1-oxoisoindolin-2-yl)piperidine-2,6-dione (isomer 1) | 752.4 | 752.3 |
| **116** | 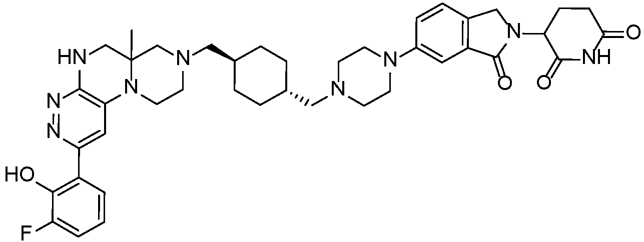 3-(6-(4-(((1*R*,4*r*)-4-(((*R*)-2-(3-fluoro-2-hydroxyphenyl)-6a-methyl-5,6,6a,7,9,10-hexahydro-8*H*-pyrazino[1',2':4,5]p yrazino[2,3-*c*]pyridazin-8-yl)methyl)cyclohexyl)methyl) piperazin-1-yl)-1-oxoisoindolin-2-yl)piperidine-2,6-dione (isomer 2) | 752.4 | 752.3 |
| **117** | 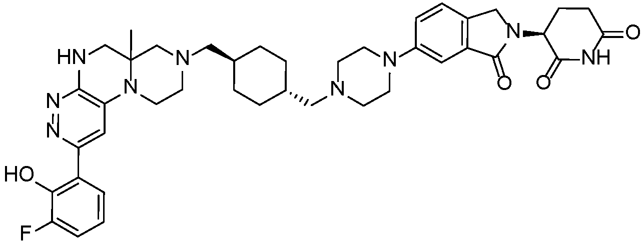 (*S*)-3-(6-(4-(((1*R*,4*r*)-4-(((*R*)-2-(3-fluoro-2-hydroxyphenyl)-6a-methyl-5,6,6a,7,9,10-hexahydro-8*H*-pyrazino[1',2':4,5] pyrazino[2,3-c]pyridazin-8-yl)methyl)cyclohexyl)methyl) piperazin-1-yl)-1-oxoisoindolin-2-yl)piperidine-2,6-dione (isomer 1) ¹H NMR (400 MHz, DMSO) δ 10.90 (s, 1H), 9.21 (s, 1H), 8.09 (s, 1H), 7.44 (d, *J =* 8.4 Hz, 1H), 7.38 - 7.18 (m, 4H), 7.03 (s, 1H), 6.90 (s, 1H), 5.03 (dd, *J =* 13.3, 5.1 Hz, 1H), 4.35 - 4.12 (m, 2H), 3.85 (d, *J =* 11.8 Hz, 2H), 3.48 (d, *J =* 29.0 Hz, 5H), 3.21 - 2.76 (m, 18H), 2.58 (d, *J =* 17.5 Hz, 2H), 2.37 - 2.23 (m, 2H), 1.95 - 1.89 (m, 1H), 1.77 (s, 5H), 1.50 (s, 1H), 1.28 (s, 3H), 0.93 (s, 4H) | 752.4 | 752.3 |
| **118** | 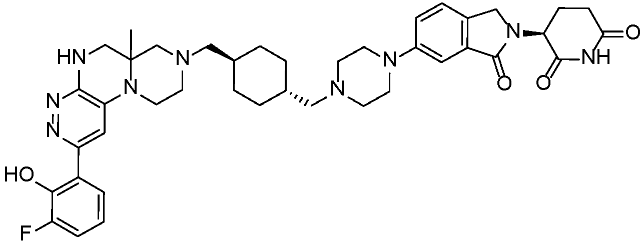 *(*S)-3-(6-(4-(1*R*,4*r*)-4-(*R*)-2-(3-fluoro-2-hydroxyphenyl)-6a-methyl-5,6,6a,7,9,10-hexahydro-8*H*-pyrazino[1',2':4,5] pyrazino[2,3-*c*]pyridazin-8-yl)methyl)cyclohexyl)methyl) piperazin-1-yl)-1-oxoisoindolin-2-yl)piperidine-2,6-dione (isomer 2) | 752.4 | 752.3 |

### Example 119

Example 119 shown in Table 20 was prepared as the TFA salt by the method described in the preparation of Example 55 using the appropriate intermediates and starting materials.

**Table 20. Example 119**

| **Example** | **Structure/Name** | **Calcd. (M+H)⁺ m/z** | **Found (M+H)⁺ m/z** |
|---|---|---|---|
| **119** | 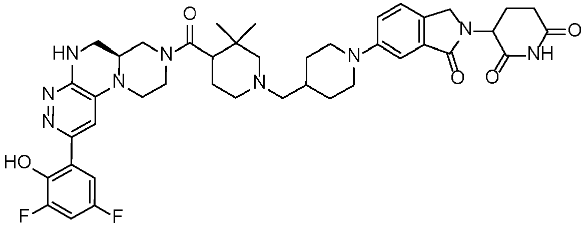 3-(6-(4-((4-((*S*)-2-(3,5-difluoro-2-hydroxyphenyl)-6,6a,7,8,9,10-hexahydro-5*H*-pyrazino[1',2':4,5]pyrazino [2,3-c]pyridazine-8-carbonyl)-3,3-dimethylpiperidin-1-yl)methyl) piperidin-1-yl)-1-oxoisoindolin-2-yl)piperidine-2,6-dione | 798.4 | 798.3 |

### Examples 120 - 124

Examples 120-124 shown below in Table 21 were prepared as TFA salts by the method described in the preparation of Example 71 using the appropriate intermediates and starting materials.

**Table 21. Examples 120-124**

| **Example** | **Structure/Name** | **Calcd. (M+H)⁺ m/z** | **Found (M+H)⁺ m/z** |
|---|---|---|---|
| **120** | 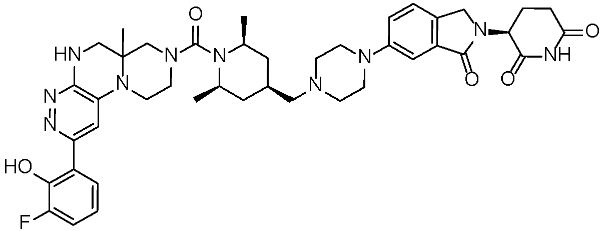 (*S*)-3-(6-(4-(((2*R*,4*R*,6*S*)-1-(2-(3-fluoro-2-hydroxyphenyl)-6a-methyl-6,6a,7,8,9,10-hexahydro-5H-pyrazino[1',2':4,5] pyrazino[2,3-*c*]pyridazine-8-carbonyl)-2,6-dimethylpiperidin-4-yl)methyl)piperazin-1-yl)-1-oxoisoindolin-2-yl)piperidine-2,6-dione (isomer 1) | 795.4 | 795.3 |
| **121** | 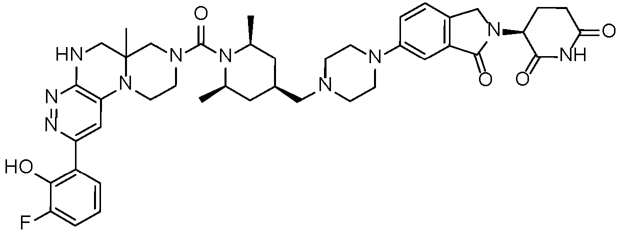 (*S*)-3-(6-(4-(((2*R*,4*R*,6*S*)-1-(2-(3-fluoro-2-hydroxyphenyl)-6a-methyl-6,6a,7,8,9,10-hexahydro-5H-pyrazino[1',2':4,5] pyrazino[2,3-*c*]pyridazine-8-carbonyl)-2,6-dimethylpiperidin-4-yl)methyl)piperazin-1-yl)-1-oxoisoindolin-2-yl)piperidine-2,6-dione (isomer 2) | 795.4 | 795.3 |
| **122** | 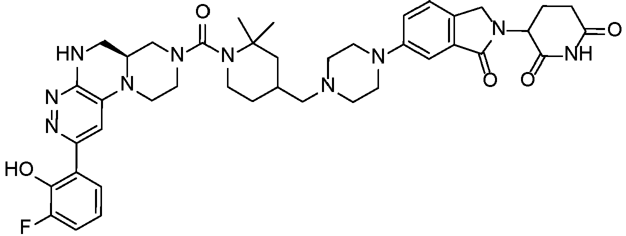 3-(6-(4-((1-((*S*)-2-(3-fluoro-2-hydroxyphenyl)-6,6a,7,8,9,10-hexahydro-5*H*-pyrazino[1',2':4,5]pyrazino[2,3-*c*]pyridazine-8-carbonyl)-2,2-dimethylpiperidin-4-yl)methyl)piperazin-1-yl)-1-oxoisoindolin-2-yl)piperidine-2,6-dione | 781.4 | 781.3 |
| **123** | 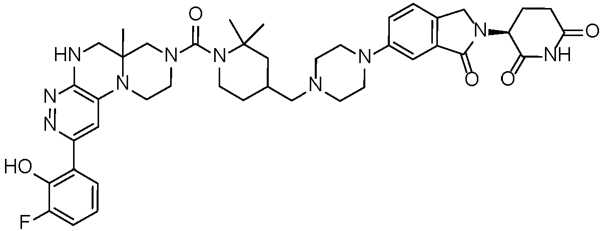 (3*S*)-3-(6-(4-((1-(2-(3-fluoro-2-hydroxyphenyl)-6a-methyl-6,6a,7,8,9,10-hexahydro-5*H*-pyrazino[1',2':4,5]pyrazino[2,3-c]pyridazine-8-carbonyl)-2,2-dimethylpiperidin-4-yl)methyl) piperazin-1-yl)-1-oxoisoindolin-2-yl)piperidine-2,6-dione (isomer 1) | 795.4 | 795.3 |
| **124** | 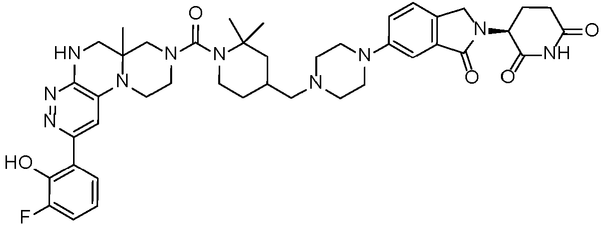 (3*S*)-3-(6-(4-((1-(2-(3-fluoro-2-hydroxyphenyl)-6a-methyl-6,6a,7,8,9,10-hexahydro-5*H*-pyrazino[1',2':4,5]pyrazino[2,3 - *c*]pyridazine-8-carbonyl)-2,2-dimethylpiperidin-4-yl)methyl) piperazin-1-yl)-1-oxoisoindolin-2-yl)piperidine-2,6-dione (isomer 2) | 795.4 | 795.3 |

### Example A. SMARCA2 HiBiT and SMARCA4 HiBiT Degradation Assay

### Preparation of SMARCA2/4-HiBiT knock-in cells

HiBiT peptide knock-in of SMARCA2 in LgBiT expressing HEK293T cells was performed by CRISPR-mediated tagging system as described Promega. The homozygous HiBiT knock-in on c-terminus SMARCA2 was confirmed by sanger sequence. SMARCA2-HiBiT knock-in Hela monoclonal cell (CS302366) and SMARCA4-HiBiT knock-in Hela monoclonal cell (CS3023226) were purchased from Promega. The heterozygous HiBiT-knock-in was confirmed by sanger sequence in both SMARCA2-HiBiT and SMARCA4-HiBiT monoclonal cells.

### SMARCA2 HiBiT and SMARCA4 HiBiT degradation assay in HeLa cells

Compounds were dissolved in DMSO to make 10 mM stock and 3-fold series dilutions were further conducted keeping the highest concentration 10 µM. NCIH1693 and NCIH520 cells were maintained in PRMI 1640 medium (Corning Cellgro, Catalog #:10-040-CV) supplemented with 10% v/v FBS (GE Healthcare, Catalog #: SH30910.03) by splitting 1:3 twice a week.

Dispense 10ul aliquot of prepared Hela-SMARCA2-HiBiT or Hela-SMARCA4-HiBiT cells (1:1 ratio of cells: Trypan Blue (#1450013, Bio-Rad)) onto cell counting slide (#145-0011, Bio-Rad) and obtain cell density and cell viability using cell counter (TC20, Bio-Rad). Remove appropriate volume of resuspended cells from culture flask to accommodate 2500 cells/well @ 20 µL/well. Transfer Hela-HiBiT cells to 50 mL conical (#430290, Corning). Spin down at 1000 rpm for 5 min using tabletop centrifuge (SPINCHRON 15, Beckman). Discard supernatant and resuspend cell pellet in modified EMEM (#30-2003, ATCC) cell culture media containing 10% FBS (F2422-500ML, Sigma), and 1X Penicillin/Streptomycin (200g/L) (30-002-CI, Corning) to a cell density of 125,000 cells/mL. Dispense 20 µLof resuspended Hela-HiBit cells per well in 384-well TC treated plate (#12-565-343, Thermo Scientific) using standard cassette (#50950372, Thermo Scientific) on Multidrop Combi (#5840310, Thermo Scientific) inside laminar flow cabinet. Dispense test compounds onto plates using digital liquid dispenser (D300E, Tecan). Incubate plates in humidified tissue culture incubator @37°°C for 18 hours. Add 20 µL of prepared Nano-Glo^{®} HiBiT Lytic detection buffer (N3050, Promega) to each well of 384-well plate using small tube cassette (#24073295, Thermo Scientific) on Multidrop Combi, incubate @ RT for 30-60 min. Read plates on microplate reader (Envision 2105, PerkinElmer) using 384 well Ultra-Sensitive luminescence mode. Raw data files and compound information reports are swept into centralized data lake and deconvoluted using automated scripts designed by TetraScience, Inc. Data analysis, curve-fitting and reporting done in Dotmatics Informatics Suite using Screening Ultra module.

**Table 22. Biological Data**

| **Example** | **SMARCA2 IC₅₀ (nM)** | **SMARCA2 D_{Max}%** | **SMARCA4 DC₅₀ (nM)** | **SMARCA4 D_{Max}%** |
|---|---|---|---|---|
| **1** | A | A | A | C |
| **2** | A | A | A | C |
| **3** | A | A | A | C |
| **4** | A | A | A | C |
| **5** | A | A | A | C |
| **6** | A | A | A | A |
| **7** | A | A | A | B |
| **8** | A | A | A | B |
| **9** | A | A | A | A |
| **10** | A | A | A | C |
| **11** | A | A | A | B |
| **12** | A | A | A | A |
| **13** | A | A | A | C |
| **14** | A | A | A | B |
| **15** | A | A | A | B |
| **16** | A | A | A | C |
| **17** | A | A | A | B |
| **18** | NT | NT | NT | NT |
| **19** | A | A | A | 51 |
| **20** | A | A | A | C |
| **21** | A | A | A | C |
| **22** | A | A | A | B |
| **23** | A | A | A | C |
| **24** | A | A | C | C |
| **25** | A | A | A | C |
| **26** | A | A | A | A |
| **27** | A | A | A | B |
| **28** | A | A | A | C |
| **29** | A | A | A | C |
| **30** | A | A | A | B |
| **31** | A | B | A | C |
| **32** | A | B | A | C |
| **33** | A | A | A | A |
| **34** | A | A | A | A |
| **35** | A | A | A | B |
| **36** | A | A | A | A |
| **37** | A | A | A | A |
| **37** | A | A | A | B |
| **39** | A | A | A | C |
| **40** | A | B | A | B |
| **41** | A | A | A | B |
| **42** | A | A | A | C |
| **42** | A | A | A | C |
| **44** | A | A | A | C |
| **45** | A | A | A | C |
| **46** | A | A | A | C |
| **47** | A | A | A | B |
| **48** | A | A | A | C |
| **49** | A | A | A | A |
| **50** | A | A | A | B |
| **51** | A | C | C | C |
| **52** | A | A | A | B |
| **53** | A | A | A | B |
| **54** | A | A | A | A |
| **55** | A | B | A | C |
| **56** | A | A | A | A |
| **57** | A | A | A | C |
| **58** | A | A | A | B |
| **59** | A | A | A | A |
| **60** | A | A | A | A |
| **61** | A | A | A | A |
| **62** | A | A | A | A |
| **63** | A | A | A | B |
| **64** | A | A | A | A |
| **65** | A | A | A | B |
| | | | | |
| **66** | A | A | A | A |
| **67** | A | B | A | B |
| **68** | A | A | A | B |
| **69** | A | A | C | C |
| **70** | A | A | A | B |
| **71** | A | A | B | C |
| **72** | A | A | A | A |
| **73** | A | A | B | C |
| **74** | A | A | A | B |
| **75** | A | A | A | B |
| **76** | A | A | A | B |
| **77** | A | A | A | B |
| **78** | A | A | A | B |
| **79** | A | A | C | C |
| **80** | A | A | A | B |
| **81** | A | A | B | C |
| **82** | A | A | A | A |
| **83** | A | A | A | B |
| **84** | A | A | A | B |
| **85** | A | A | A | A |
| **86** | A | A | A | A |
| **87** | A | A | A | A |
| **88** | A | A | A | A |
| **89** | A | A | B | C |
| **90** | A | A | C | C |
| **91** | A | A | B | C |
| **92** | A | A | C | C |
| **93** | A | A | C | C |
| **94** | A | B | C | C |
| **95** | A | A | A | A |
| **96** | A | A | A | A |
| **97** | A | A | B | C |
| **98** | A | A | A | A |
| **99** | A | A | A | B |
| **100** | A | A | A | B |
| **101** | A | B | C | C |
| **102** | A | A | A | A |
| **103** | A | A | A | B |
| **104** | A | A | B | C |
| **105** | A | A | A | B |
| **106** | A | A | A | A |
| **107** | A | A | B | C |
| **108** | A | A | A | A |
| **109** | A | A | A | A |
| **110** | A | A | A | A |
| **111** | A | B | C | C |
| **112** | A | A | A | A |
| **113** | A | A | C | C |
| **114** | A | A | A | B |
| **115** | A | A | A | B |
| **116** | A | A | A | B |
| **117** | A | A | A | B |
| **118** | A | A | A | B |
| **119** | A | A | B | C |
| **120** | A | A | A | A |
| **121** | A | A | A | A |
| **122** | A | A | A | B |
| **123** | A | A | A | A |
| **124** | A | A | A | B |

In Table 22, A = DC₅₀ < 0.1 µM and B = 0.1 µM ≤ DC₅₀ < 1 µM and C = DC₅₀ > 1 µM. In Table 22, A = Dₘₐₓ > 75% and B = 50% < Dₘₐₓ ≤ 75% and C = Dₘₐₓ < 50%. In Table 22, NT = not tested.

## Claims

1. A compound of Formula (I): 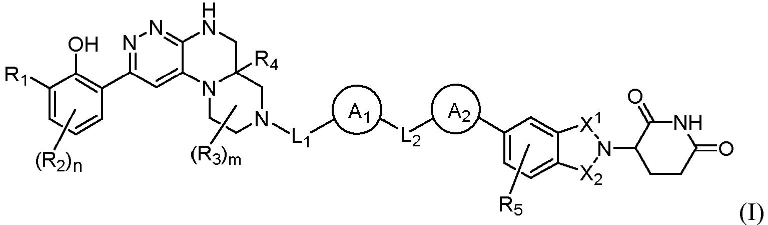 or a pharmaceutically acceptable salt thereof; wherein
R₁ is halo, C₁₋₆ alkyl, or haloalkyl;
each R₂ is independently H, D, or F;
each R₃ is independently H, D, C₁₋₆ alkyl, haloalkyl, or C₃₋₆ cycloalkyl;
n is 1, 2 or 3;
m is 1, 2, 3, 4, 5 or 6;
R₄ is H, D, C₁₋₆ alkyl, C₃₋₆ cycloalkyl, alkoxyalkyl, cyanoalkyl or haloalkyl;
R₅ is H, D, or F;
L₁ is a bond, C(R₃)₂, or CO;
L₂ is a bond, C(R₃)₂, or CO;
ring A₁ is a 3-7 membered cycloalkyl group, a 4-7-membered heterocycloalkyl group, an aryl group, or a heteroaryl group;
ring A₂ is a 3-7 membered cycloalkyl group, a 4-7-membered heterocycloalkyl group, an aryl group, or a heteroaryl group;
X₁ is CH₂, CO, CH=CH (when X₂ = CO), or N=CH (when X₂ = CO); and
X₂ is CH₂, CO, CH=CH (when X₁ = CO), or N=CH (when X₁ = CO).

2. The compound according to claim 1, wherein R₁ is halo, optionally F.

3. The compound according to any one of the preceding claims, wherein n is 1, or wherein n is 2, or wherein n is 3.

4. The compound according to any one of the preceding claims, wherein each R₂ is F, or wherein each R₂ is H.

5. The compound according to any one of the preceding claims, wherein m is 1, wherein m is 2, wherein m is 3, wherein m is 4, wherein m is 5, or wherein m is 6.

6. The compound according to any one of the preceding claims, wherein:
(a) each R₃ is H; and/or
(b) R₄ is H; R₄ is D; R₄ is C₁₋₆ alkyl; or R₄ is methyl; and/or
(c) R₅ is H.

7. The compound according to any one of the preceding claims, wherein:
(a) L₁ is CO; or L₁ is C(R₃)₂; or L₁ is a methylene group; and/or
(b) L₂ is CO; or L₂ is C(R₃)₂; or L₂ is a methylene group.

8. The compound according to any one of the preceding claims, wherein:
(a) ring A₁ is a 3-7 membered cycloalkyl group, optionally wherein ring A₁ is a cyclohexyl group; or
(b) ring A₁ is a 4-7-membered heterocycloalkyl group, optionally (i) wherein ring A₁ is a piperazine group, a morpholine group, a piperidine group, a pyrrolidine group, an azetidine group or an azabicyclo-hexane group; or (ii) wherein ring A₁ is a piperazine group.

9. The compound according to any one of the preceding claims, wherein:
(a) ring A₂ is a 3-7 membered cycloalkyl group, optionally wherein ring A₂ is a cyclohexyl group; or
(b) ring A₂ is a 4-7-membered heterocycloalkyl group, optionally (i) wherein ring A₂ is a piperazine group, a morpholine group, a piperidine group, a pyrrolidine group, an azetidine group or an azabicyclo-hexane group; or (ii) wherein ring A₂ is a piperidine group.

10. The compound according to any one of the preceding claims, wherein:
(a) X₁ is CH₂, or X₁ is CO; and/or
(b) X₂ is CH₂, or X₂ is CO.

11. The compound according to any one of the preceding claims that is a compound of formula II: 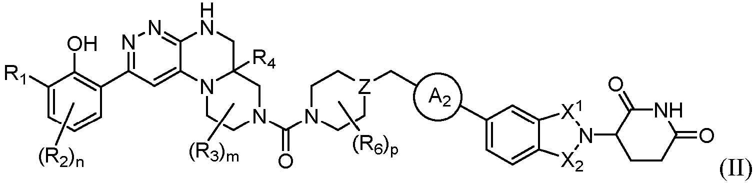
or a pharmaceutically acceptable salt thereof ; wherein
each R₆ is independently H, D, C₁₋₆ alkyl, C₃₋₆ cycloalkyl, or haloalkyl;
p is 1, 2, 3, 4, 5, 6, 7 or 8; and
Z is N or CR₆;
optionally wherein:
(a) Z is N, pis 1, p is 2, p is 3, p is 4, p is 5, p is 6, p is 7, or p is 8; and/or
(b) each R₆ is H, each R₆ is C₁₋₆ alkyl, or each R₆ is methyl.

12. The compound according to claim 11 that is a compound of formula III: 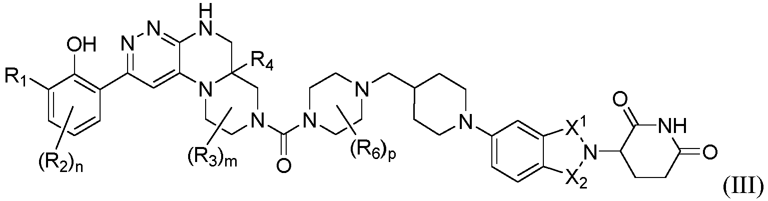
or a pharmaceutically acceptable salt thereof; or that is a compound of formula IV: 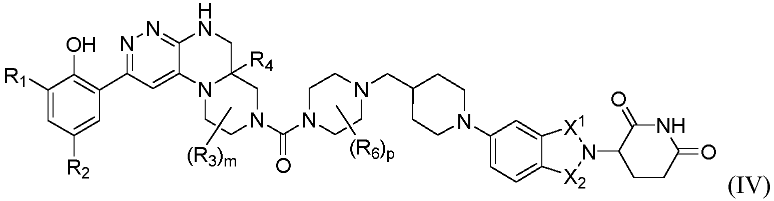
or a pharmaceutically acceptable salt thereof; or that is a compound of formula V: 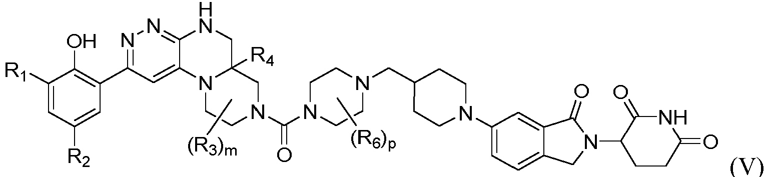
or a pharmaceutically acceptable salt thereof; or that is a compound of formula VI: 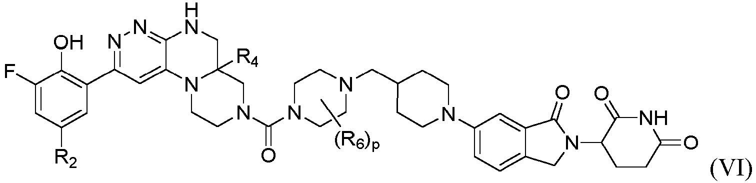
or a pharmaceutically acceptable salt thereof.

13. The compound according to claim 1 that is:
3-(6-(4-(3R,5S)-4-((S)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(3-(((3R,5S)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)azetidin-1-yl)-1-oxoisoindolin-2-yl)piperidine-2,6-dione;
3-(5-((S)-2-(((3R,5S)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)morpholino)-1-oxoisoindolin-2-yl)piperidine-2,6-dione;
3-(6-(4-((4-((6aS,9S)-2-(3-fluoro-2-hydroxyphenyl)-9-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-((4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
2-(2,6-dioxopiperidin-3-yl)-5-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)isoindoline-1,3-dione;
3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-((4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-(((3R,5S)-4-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-(((3R,5S)-4-2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-(((3R,5R)-4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-(((3R,5R)-4-(2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-(((3R,5S)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-(((3S,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3S,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(R)-3-(6-(4-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(3-(((3R,5S)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)azetidin-1-yl)-1-oxoisoindolin-2-yl)piperidine-2,6-dione;
3-(5-((S)-2-(((3R,5S)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)morpholino)-1-oxoisoindolin-2-yl)piperidine-2,6-dione;
3-(6-(4-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(3-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)azetidin-1-yl)-1-oxoisoindolin-2-yl)piperidine-2,6-dione;
3-(6-(3-(((3S,5R)-4-((6aS,9S)-2-(3,5-difluoro-2-hydroxyphenyl)-9-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)azetidin-1-yl)-1-oxoisoindolin-2-yl)piperidine-2,6-dione;
3-(6-(4-((4-((6aS,95)-2-(3,5-difluoro-2-hydroxyphenyl)-9-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-(((3S,5R)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-((4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-(((3R,5R)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(3-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3-ethylpiperazin-1-yl)methyl)azetidin-1-yl)-1-oxoisoindolin-2-yl)piperidine-2,6-dione;
3-(6-(4-(((3R,5S)-4-(2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-((4-(2-(3-chloro-2-hydroxyphenyl)-6a-methyl-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
2-(2,6-dioxopiperidin-3-yl)-5-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)isoindoline-1,3-dione;
3-(6-(4-(((3R,5R)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-(((3S,5R)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-((4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3-ethylpiperazin-1-yl)methyl)piperidin-1-yl)-1-oxoisoindolin-2-yl)piperidine-2,6-dione;
5-(4-(((3S,5R)-4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-2-(2,6-dioxopiperidin-3-yl)isoindoline-1,3-dione;
3-(6-((3R,4R)-4-(((1R,5S,6R)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)-3-fluoropiperidin-1-yl)-1-oxoisoindolin-2-yl)piperidine-2,6-dione;
3-(6-((3R,4R)-4-(((1R,5S,6R)-6-(((6aS,9S)-2-(3,5-difluoro-2-hydroxyphenyl)-9-methyl-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)-3-fluoropiperidin-1-yl)-1-oxoisoindolin-2-yl)piperidine-2,6-dione;
3-(6-(4-(((1R,5S,6r)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)-3,3-difluoropiperidin-1-yl)-1-oxoisoindolin-2-yl)piperidine-2,6-dione;
3-(6-(4-(((1R,5S,6r)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)-3,3-difluoropiperidin-1-yl)-1-oxoisoindolin-2-yl)piperidine-2,6-dione;
3-(6-((rac-3R,4R)-4-(((3S,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)-3-fluoropiperidin-1-yl)-1-oxoisoindolin-2-yl)piperidine-2,6-dione;
3-(6-((3R,4R)-3-fluoro-4-(((3S,5R)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-(((3R,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(5-((S)-2-(((3R,5R)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)morpholino)-1-oxoisoindolin-2-yl)piperidine-2,6-dione;
3-(6-(4-(((3S,5S)-4-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-((1-((S)-2-(3,5-difluoro-2-hydroxyphenyl)-6,6a,7,8,9,10-hexahydro-5H-pyrazino[1',2':4,5]pyrazino[2,3-c]pyridazine-8-carbonyl)piperidin-4-yl)methyl)piperazin-1-yl)-1-oxoisoindolin-2-yl)piperidine-2,6-dione;
3-(6-(2-(((1R,5S,6s)-6-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)morpholino)-1-oxoisoindolin-2-yl)piperidine-2,6-dione;
3-(6-(2-(((1R,5S,6r)-6-(((6aS,9S)-2-(3,5-difluoro-2-hydroxyphenyl)-9-methyl-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)morpholino)-1-oxoisoindolin-2-yl)piperidine-2,6-dione;
3-(5-((S)-2-(((3R,4R)-4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)-3-fluoropiperidin-1-yl)methyl)morpholino)-1-oxoisoindolin-2-yl)piperidine-2,6-dione;
3-(6-((S)-3-(((R)-3-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)pyrrolidin-1-yl)methyl)pyrrolidin-1-yl)-1-oxoisoindolin-2-yl)piperidine-2,6-dione;
3-(5-((S)-3-((4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)piperidin-1-yl)methyl)pyrrolidin-1-yl)-1-oxoisoindolin-2-yl)piperidine-2,6-dione;
3-(6-(4-(((1S,4r)-4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidine-2,6-dione;
3-(6-(4-((4-((S)-2-(3,5-difluoro-2-hydroxyphenyl)-6,6a,7,8,9,10-hexahydro-5H-pyrazino [1',2':4,5]pyrazino[2,3-c]pyridazine-8-carbonyl)-4-fluoropiperidin-1-yl)methyl)piperidin-1-yl)-1-oxoisoindolin-2-yl)piperidine-2,6-dione;
3-(6-(4-(((1R,4s)-4-(((S)-2-(3,5-difluoro-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidine-2,6-dione;
3-(6-(4-(((2*S*,5*R*)-4-(2-(3-fluoro-2-hydroxyphenyl)-6a-methyl-6,6a,7,8,9,10-hexahydro-5*H*-pyrazino[1',2':4,5]pyrazino[2,3-c]pyridazine-8-carbonyl)-2,5-dimethyl-piperazin-1-yl) methyl)piperidin-1-yl)-1-oxoisoindolin-2-yl)piperidine-2,6-dione;
3-(6-(4-(((1*S*,4*r*)-4-(((S)-2-(3-fluoro-2-hydroxyphenyl)-5,6,6a,7,9,10-hexahydro-8*H-*pyrazino[1',2':4,5]pyrazino[2,3-*c*]pyridazin-8-yl)methyl)cyclohexyl)methyl)piperazin-1-yl)-1-oxoisoindolin-2-yl)piperidine-2,6-dione;
3-(6-(4-(((3*R*,5*S*)-4-(2-(3,5-difluoro-2-hydroxyphenyl)-6a-ethyl-6,6a,7,8,9,10-hexahydro-5*H*-pyrazino[1',2':4,5]pyrazino[2,3-*c*]pyridazine-8-carbonyl)-3,5-dimethyl-piperazin-1-yl)methyl)piperidin-1-yl)-1-oxoisoindolin-2-yl)piperidine-2,6-dione;
3-(6-(4-(((3*R*,5*S*)-4-(6a-ethyl-2-(3-fluoro-2-hydroxyphenyl)-6,6a,7,8,9,10-hexahydro-5*H-*pyrazino[1',2':4,5]pyrazino[2,3-*c*]pyridazine-8-carbonyl)-3,5-dimethyl-piperazin-1-yl)methyl) piperidin-1-yl)-1-oxoisoindolin-2-yl)piperidine-2,6-dione;
3-(6-(4-(((3*R*,5*S*)-4-(2-(3,5-difluoro-2-hydroxyphenyl)-6a-ethyl-6,6a,7,8,9,10-hexahydro-5*H*-pyrazino[1',2':4,5]pyrazino[2,3-*c*]pyridazine-8-carbonyl)-3,5-dimethyl-piperazin-1-yl)methyl)piperidin-1-yl)-1-oxoisoindolin-2-yl)piperidine-2,6-dione;
3-(6-(4-(((3*R*,5*S*)-4-(6a-ethyl-2-(3-fluoro-2-hydroxyphenyl)-6,6a,7,8,9,10-hexahydro-5*H-*pyrazino[1',2':4,5]pyrazino[2,3-*c*]pyridazine-8-carbonyl)-3,5-dimethyl-piperazin-1-yl)methyl) piperidin-1-yl)-1-oxoisoindolin-2-yl)piperidine-2,6-dione;
3-(6-(4-(((3*R*,5*R*)-4-(2-(3-chloro-2-hydroxyphenyl)-6a-methyl-6,6a,7,8,9,10-hexahydro-5*H*-pyrazino[1',2':4,5]pyrazino[2,3-*c*]pyridazine-8-carbonyl)-3,5-dimethyl-piperazin-1-yl)methyl)piperidin-1-yl)-1-oxoisoindolin-2-yl)piperidine-2,6-dione;
3-(6-(4-(((3*R*,5*S*)-4-(2-(3,5-difluoro-2-hydroxyphenyl)-6a-methyl-6,6a,7,8,9,10-hexahydro-5*H*-pyrazino[1',2':4,5]pyrazino[2,3-*c*]pyridazine-8-carbonyl)-3,5-dimethyl-piperazin-1-yl)methyl)piperidin-1-yl)-1-oxoisoindolin-2-yl)piperidine-2,6-dione;
or a pharmaceutically acceptable salt thereof; or that is:
(S)-3-(6-(4-((4-((R)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(S)-3-(6-(4-(((3S,5S)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((3R,5R)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((2S,5R)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
3-(6-(4-(((3R,5S)-4-((S)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
3-(6-(4-(((3R,5S)-4-((R)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(S)-3-(6-(4-(((2R,4S,6S)-1-((S)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(S)-3-(6-(4-(((2R,4R,6S)-1-((R)-6a-(difluoromethyl)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(3S)-3-(6-(4-((1-((S)-6a-(difluoromethyl)-2-(3-fluoro-2-hydroxyphenyl)-6,6a,7,8,9,10-hexahydro-5H-pyrazino [1',2':4,5]pyrazino[2,3-c]pyridazine-8-carbonyl)-2,2-dimethylpiperidin-4-yl)methyl)piperazin-1-yl)-1-oxoisoindolin-2-yl)piperidine-2,6-dione;
(3S)-3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(3S)-3-(6-(4-(((3R,5R)-4-(2-(3-chloro-2-hydroxyphenyl)-6a-ethyl-6,6a,7,8,9,10-hexahydro-5H-pyrazino[1',2':4,5] pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
(3S)-3-(6-(4-((4-(2-(3-chloro-2-hydroxyphenyl)-6a-ethyl-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
(3S)-3-(6-(4-(((2S,5R)-4-(2-(3-chloro-2-hydroxyphenyl)-6a-ethyl-6,6a,7,8,9,10-hexahydro-5H-pyrazino[1',2':4,5] pyrazino[2,3-c]pyridazine-8-carbonyl)-2,5-dimethyl-piperazin-1-yl)methyl)piperidin-1-yl)-1-oxoisoindolin-2-yl)piperidine-2,6-dione;
(3S)-3-(6-(4-(((2S,5R)-4-(6a-ethyl-2-(3-fluoro-2-hydroxyphenyl)-6,6a,7,8,9,10-hexahydro-5H-pyrazino [1',2':4,5]pyrazino[2,3-c]pyridazine-8-carbonyl)-2,5-dimethylpiperazin-1-yl)methyl)piperidin-1-yl)-1-oxoisoindolin-2-yl)piperidine-2,6-dione;
3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,3-dimethylpiperazin-1-yl)methyl) piperidin-1-yl)-1-oxoisoindolin-2-yl)piperidine-2,6-dione;
3-(6-(4-(((3S,5S)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((3R,5R)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((3R,5R)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((2S,5R)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-2,5-dimethylpiperazin-1-yl)methyl) piperidin-1-yl)-1-oxoisoindolin-2-yl)piperidine-2,6-dione;
3-(6-(4-(((3R,5S)-4-(2-(3,5-difluoro-2-hydroxyphenyl)-6a-ethyl-6,6a,7,8,9,10-hexahydro-5H-pyrazino[1',2':4,5] pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
3-(6-(4-(((3R,5S)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
3-(6-(4-(((3R,5S)-4-(2-(3,5-difluoro-2-hydroxyphenyl)-6a-ethyl-6,6a,7,8,9,10-hexahydro-5H-pyrazino[1',2':4,5] pyrazino[2,3-c]pyridazine-8-carbonyl)-3,5-dimethyl-piperazin-1-yl)methyl)piperidin-1-yl)-1-oxoisoindolin-2-yl)piperidine-2,6-dione;
3-(6-(4-(((3R,5S)-4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-3,5-dimethylpiperazin-1-yl)methyl) piperidin-1-yl)-1-oxoisoindolin-2-yl)piperidine-2,6-dione;
(3S)-3-(6-(4-((4-(2-(3-fluoro-2-hydroxyphenyl)-6a-(fluoromethyl)-6,6a,7,8,9,10-hexahydro-5H-pyrazino[1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
(3*S*)-3-(6-(4-((4-(2-(3-fluoro-2-hydroxyphenyl)-6a-(fluoromethyl)-6,6a,7,8,9,10-hexahydro-5H-pyrazino [1',2':4,5]pyrazino[2,3-c]pyridazine-8-carbonyl)-3,3-dimethylpiperazin-1-yl)methyl)piperidin-1-yl)-1-oxoisoindolin-2-yl)piperidine-2,6-dione;
3-(6-(4-(((2*S*,5*R*)-4-(2-(3-fluoro-2-hydroxyphenyl)-6a-methyl-6,6a,7,8,9,10-hexahydro-5*H*-pyrazino[1',2':4,5]pyrazino[2,3-*c*]pyridazine-8-carbonyl)-2,5-dimethylpiperazin-1-yl)methyl)piperidin-1-yl)-1-oxoisoindolin-2-yl)piperidine-2,6-dione;
3-(6-(4-(((2*S*,5*R*)-4-(2-(3-fluoro-2-hydroxyphenyl)-6a-methyl-6,6a,7,8,9,10-hexahydro-5*H*-pyrazino[1',2':4,5]pyrazino[2,3-*c*]pyridazine-8-carbonyl)-2,5-dimethylpiperazin-1-yl)methyl)piperidin-1-yl)-1-oxoisoindolin-2-yl)piperidine-2,6-dione;
3-(6-(4-(((3*R*,5*S*)-4-(2-(3-chloro-2-hydroxyphenyl)-6a-methyl-6,6a,7,8,9,10-hexahydro-5*H*-pyrazino[1',2':4,5] pyrazino[2,3-*c*]pyridazine-8-carbonyl)-3,5-dimethyl-piperazin-1-yl)methyl)piperidin-1-yl)-1-oxoisoindolin-2-yl)piperidine-2,6-dione;
((2*S*,6*R*)-2,6-dimethylpiperazin-1-yl)(2-(3-fluoro-2-hydroxyphenyl)-6a-methyl-5,6,6a,7,9,10-hexahydro-8*H*-pyrazino[1',2':4,5]pyrazino[2,3-*c*]pyridazin-8-yl)methanone;
((2*S*,6*R*)-2,6-dimethylpiperazin-1-yl)(2-(3-fluoro-2-hydroxyphenyl)-6a-methyl-5,6,6a,7,9,10-hexahydro-8*H*-pyrazino[1',2':4,5]pyrazino[2,3-*c*]pyridazin-8-yl)methanone;
(3*S*)-3-(6-(4-(((3*S*,5*R*)-4-(2-(3-fluoro-2-hydroxyphenyl)-6a-(trifluoromethyl)-6,6a,7,8,9,10-hexahydro-5*H*-pyrazino[1',2':4,5]pyrazino[2,3-*c*]pyridazine-8-carbonyl)-3,5-dimethylpiperazin-1-yl)methyl)piperidin-1-yl)-1-oxoisoindolin-2-yl)piperidine-2,6-dione;
(3S)-3-(6-(4-(((3R,5R)-4-(2-(3-fluoro-2-hydroxyphenyl)-6a-(trifluoromethyl)-6,6a,7,8,9,10-hexahydro-5H-pyrazino[1',2':4,5] pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(3S)-3-(6-(4-(((3R,5R)-4-(2-(3-fluoro-2-hydroxyphenyl)-6a-(trifluoromethyl)-6,6a,7,8,9,10-hexahydro-5H-pyrazino[1',2':4,5] pyrazino[2,3-c]pyridazine-8-carbonyl)-3,5-dimethylpiperazin-1-yl)methyl)piperidin-1-yl)-1-oxoisoindolin-2-yl)piperidine-2,6-dione;
(3S)-3-(6-(4-(((2R,4r,6S)-1-(2-(3-fluoro-2-hydroxyphenyl)-6a-(trifluoromethyl)-6,6a,7,8,9,10-hexahydro-5H-pyrazino[1',2':4,5] pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(3S)-3-(6-(4-(((2R,4r,6S)-1-(2-(3-fluoro-2-hydroxyphenyl)-6a-(trifluoromethyl)-6,6a,7,8,9,10-hexahydro-5H-pyrazino[1',2':4,5] pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(3S)-3-(6-(4-((4-(2-(3-fluoro-2-hydroxyphenyl)-6a-(trifluoro-methyl)-6,6a,7,8,9,10-hexahydro-5H-pyrazino[1',2':4,5]pyrazino [2,3-c]pyridazine-8-carbonyl)-3,3-dimethylpiperazin-1-yl)methyl) piperidin-1-yl)-1-oxoisoindolin-2-yl)piperidine-2,6-dione;
(3S)-3-(6-(4-((4-(2-(3-fluoro-2-hydroxyphenyl)-6a-(trifluoro-methyl)-6,6a,7,8,9,10-hexahydro-5H-pyrazino[1',2':4,5]pyrazino [2,3-c]pyridazine-8-carbonyl)-3,3-dimethylpiperazin-1-yl)methyl) piperidin-1-yl)-1-oxoisoindolin-2-yl)piperidine-2,6-dione;
(3S)-3-(6-(4-(((1s,4R)-4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluorocyclohexyl) methyl)piperazin-1-yl)-1-oxoisoindolin-2-yl)piperidine-2,6-dione;
(S)-3-(6-(4-(((1R,4R)-4-((S)-2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluorocyclohexyl) methyl)piperazin-1-yl)-1-oxoisoindolin-2-yl)pi peridine-2,6-dione;
(S)-3-(6-(4-((4-((R)-2-(3,5-difluoro-2-hydroxyphenyl)-6a-(difluoromethyl)-6,6a,7,8,9,10-hexahydro-5H-pyrazino[1',2':4,5] pyrazino[2,3-c]pyridazine-8-carbonyl)-4-fluoropiperidin-1-yl)methyl)piperidin-1-yl)-1-oxoisoindolin-2-yl)piperidine-2,6-dione;
(3S)-3-(6-(4-((4-(2-(3-chloro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-(6a-ethyl-2-(3-fluoro-2-hydroxyphenyl)-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-(2-(3,5-difluoro-2-hydroxyphenyl)-6a-ethyl-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-fluoro-4-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)piperidin-1-yl)methyl) piperidin-1-yl)-1-oxoisoindolin-2-yl)piperidine-2,6-dione;
(3S)-3-(6-(4-((4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
(3S)-3-(6-(4-((4-(2-(3,5-difluoro-2-hydroxyphenyl)-6a-methyl-6,6a,7,8,9,10-hexahydro-5H-pyrazino[1',2':4,5]pyrazino[2,3-c]pyridazine-8-carbonyl)-4-fluoropiperidin-1-yl)methyl) piperidin-1-yl)-1-oxoisoindolin-2-yl)piperidine-2,6-dione;
3-(6-(4-(((15,4r)-4-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)cyclohexyl)methyl)piperazin-1-yl)-1-oxoisoindolin-2-yl)piperidine-2,6-dione;
(S)-3-(6-(4-(((1S,4S)-4-(((S)-2-(3-fluoro-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidine-2,6-dione;
3-(6-(4-(((1R,4r)-4-(((R)-2-(3-fluoro-2-hydroxyphenyl)-6a-methyl-5,6,6a,7,9,10-hexahydro-8H-pyrazino[1',2':4,5] pyrazino[2,3-c]pyridazin-8-yl)methyl)cyclohexyl)methyl) piperazin-1-yl)-1-oxoisoindolin-2-yl)piperidine-2,6-dione;
3-(6-(4-(((1R,4r)-4-(((R)-2-(3-fluoro-2-hydroxyphenyl)-6a-methyl-5,6,6a,7,9,10-hexahydro-8H-pyrazino[1',2':4,5]p yrazino[2,3-c]pyridazin-8-yl)methyl)cyclohexyl)methyl) piperazin-1-yl)-1-oxoisoindolin-2-yl)piperidine-2,6-dione;
(S)-3-(6-(4-(((1R,4r)-4-(((R)-2-(3-fluoro-2-hydroxyphenyl)-6a-methyl-5,6,6a,7,9,10-hexahydro-8H-pyrazino[1',2':4,5] pyrazino[2,3-c]pyridazin-8-yl)methyl)cyclohexyl)methyl) piperazin-1-yl)-1-oxoisoindolin-2-yl)piperidine-2,6-dione;
(S)-3-(6-(4-(((1R,4r)-4-(((R)-2-(3-fluoro-2-hydroxyphenyl)-6a-methyl-5,6,6a,7,9,10-hexahydro-8H-pyrazino[1',2':4,5] pyrazino[2,3-c]pyridazin-8-yl)methyl)cyclohexyl)methyl) piperazin-1-yl)-1-oxoisoindolin-2-yl)piperidine-2,6-dione;
3-(6-(4-((4-((S)-2-(3,5-difluoro-2-hydroxyphenyl)-6,6a,7,8,9,10-hexahydro-5H-pyrazino[1',2':4,5]pyrazino [2,3-c]pyridazine-8-carbonyl)-3,3-dimethylpiperidin-1-yl)methyl) piperidin-1-yl)-1-oxoisoindolin-2-yl)piperidine-2,6-dione;
(S)-3-(6-(4-(((2R,4R,6S)-1-(2-(3-fluoro-2-hydroxyphenyl)-6a-methyl-6,6a,7,8,9,10-hexahydro-5H-pyrazino[1',2':4,5] pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
(S)-3-(6-(4-(((2R,4R,6S)-1-(2-(3-fluoro-2-hydroxyphenyl)-6a-methyl-6,6a,7,8,9,10-hexahydro-5H-pyrazino[1',2':4,5] pyrazino[2,3-c]pyridazine-8-carbonyl)-2,6-dimethylpiperidin-4-yl)methyl)piperazin-1-yl)-1-oxoisoindolin-2-yl)piperidine-2,6-dione;
3-(6-(4-((1-((S)-2-(3-fluoro-2-hydroxyphenyl)-6,6a,7,8,9,10-hexahydro-5H-pyrazino[1',2':4,5]pyrazino[2,3-c]pyridazine-8-carbonyl)-2,2-dimethylpiperidin-4-yl)methyl) piperazin-1-yl)-1-oxoisoindolin-2-yl)piperidine-2,6-dione;
(3S)-3-(6-(4-((1-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,2-dimethylpiperidin-4-yl)methyl) piperazin-1-yl)-1-oxoisoindolin-2-yl)piperidine-2,6-dione;
(3S)-3-(6-(4-((1-(2-(3-fluoro-2-hydroxyphenyl)-6a-methyl-6,6a,7,8,9,10-hexahydro-5H-pyrazino[1',2':4,5]pyrazino[2,3-c]pyridazine-8-carbonyl)-2,2-dimethylpiperidin-4-yl)methyl) piperazin-1-yl)-1-oxoisoindolin-2-yl)piperidine-2,6-dione;
or a pharmaceutically acceptable salt thereof.

14. A pharmaceutical composition comprising a compound according to any one of the preceding claims, or a pharmaceutically acceptable salt thereof, and a pharmaceutically acceptable excipient.

15. A compound, or a pharmaceutically acceptable salt thereof, of any one of claims 1-13, or a pharmaceutical composition of claim 14, for use in a method of treating cancer in a subject in need thereof, wherein the method comprises administering to the subject a compound of any one of claims 1-13, or a pharmaceutically acceptable salt thereof, or a pharmaceutical composition of claim 14, optionally wherein:
(a) the cancer is SMARCA4 deleted cancer; or
(b) the cancer is squamous-cell carcinoma, basal cell carcinoma, adenocarcinoma, hepatocellular carcinomas, and renal cell carcinomas, cancer of the bladder, bowel, breast, cervix, colon, esophagus, head, kidney, liver, lung, neck, ovary, pancreas, prostate, and stomach; leukemias; benign and malignant lymphomas, particularly Burkitt's lymphoma and Non-Hodgkin's lymphoma; benign and malignant melanomas; myeloproliferative diseases; sarcomas, including Ewing's sarcoma, hemangiosarcoma, Kaposi's sarcoma, liposarcoma, myosarcomas, peripheral neuroepithelioma, synovial sarcoma, gliomas, astrocytomas, oligodendrogliomas, ependymomas, gliobastomas, neuroblastomas, ganglioneuromas, gangliogliomas, medulloblastomas, pineal cell tumors, meningiomas, meningeal sarcomas, neurofibromas, and Schwannomas; bowel cancer, breast cancer, prostate cancer, cervical cancer, uterine cancer, lung cancer, ovarian cancer, testicular cancer, thyroid cancer, astrocytoma, esophageal cancer, pancreatic cancer, stomach cancer, liver cancer, colon cancer, melanoma; carcinosarcoma, Hodgkin's disease, Wilms' tumor and teratocarcinomas,
optionally wherein the lung cancer is SMARCA4 deficient non-small cell lung cancer; or
(c) the cancer is T-lineage Acute lymphoblastic Leukemia (T-ALL), T-lineage lymphoblastic Lymphoma (T-LL), Peripheral T-cell lymphoma, Adult T-cell Leukemia, Pre-B ALL, Pre-B Lymphomas, Large B-cell Lymphoma, Burkitts Lymphoma, B-cell ALL, Philadelphia chromosome positive ALL and Philadelphia chromosome positive CML.

## Patentansprüche

1. Verbindung der Formel (I): 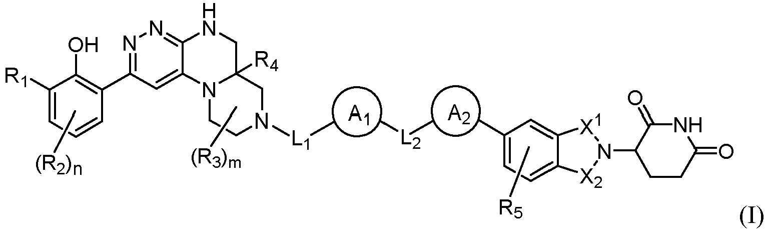
oder ein pharmazeutisch unbedenkliches Salz davon; wobei
R₁ für Halogen, C₁₋₆-Alkyl oder Halogenalkyl steht;
R₂ jeweils unabhängig für H, D oder F steht;
R₃ jeweils unabhängig für H, D, C₁₋₆-Alkyl, Halogenalkyl oder C₃₋₆-Cycloalkyl steht;
n für 1, 2 oder 3 steht;
m für 1, 2, 3, 4, 5 oder 6 steht;
R₄ für H, D, C₁₋₆-Alkyl, C₃₋₆-Cycloalkyl, Alkoxyalkyl, Cyanoalkyl oder Halogenalkyl steht;
R₅ für H, D oder F steht;
L₁ für eine Bindung, C(R₃)₂ oder CO steht;
L₂ für eine Bindung, C(R₃)₂ oder CO steht;
Ring A₁ für eine 3-7-gliedrige Cycloalkylgruppe, eine 4-7-gliedrige Heterocycloalkylgruppe, eine Arylgruppe oder eine Heteroarylgruppe steht;
Ring A₂ für eine 3-7-gliedrige Cycloalkylgruppe, eine 4-7-gliedrige Heterocycloalkylgruppe, eine Arylgruppe oder eine Heteroarylgruppe steht;
X₁ für CH₂, CO, CH=CH (wenn X₂ = CO) oder N=CH (wenn X₂ = CO) steht und
X₂ für CH₂, CO, CH=CH (wenn X₁ = CO) oder N=CH (wenn X₁ = CO) steht.

2. Verbindung nach Anspruch 1, wobei R₁ für Halogen, gegebenenfalls F, steht.

3. Verbindung nach einem der vorhergehenden Ansprüche, wobei n für 1 steht oder wobei n für 2 steht oder wobei n für 3 steht.

4. Verbindung nach einem der vorhergehenden Ansprüche, wobei R₂ jeweils für F steht oder wobei R₂ jeweils für H steht.

5. Verbindung nach einem der vorhergehenden Ansprüche, wobei m für 1 steht, wobei m für 2 steht, wobei m für 3 steht, wobei m für 4 steht, wobei m für 5 steht oder wobei m für 6 steht.

6. Verbindung nach einem der vorhergehenden Ansprüche, wobei:
(a) R₃ jeweils für H steht und/oder
(b) R₄ für H steht; R₄ für D steht; R₄ für C₁₋₆-Alkyl steht oder R₄ für Methyl steht und/oder
(c) R₅ für H steht.

7. Verbindung nach einem der vorhergehenden Ansprüche, wobei:
(a) L₁ für CO steht oder L₁ für C(R₃)₂ steht oder L₁ für eine Methylengruppe steht und/oder
(b) L₂ für CO steht oder L₂ für C(R₃)₂ steht oder L₂ für eine Methylengruppe steht.

8. Verbindung nach einem der vorhergehenden Ansprüche, wobei:
(a) Ring A₁ für eine 3-7-gliedrige Cycloalkylgruppe steht, gegebenenfalls wobei Ring A₁ für eine Cyclohexylgruppe steht; oder
(b) Ring A₁ für eine 4-7-gliedrige Heterocycloalkylgruppe steht, gegebenenfalls (i) wobei Ring A₁ für eine Piperazingruppe, eine Morpholingruppe, eine Piperidingruppe, eine Pyrrolidingruppe, eine Azetidingruppe oder eine Azabicyclohexangruppe steht oder (ii) wobei Ring A₁ für eine Piperazingruppe steht.

9. Verbindung nach einem der vorhergehenden Ansprüche, wobei:
(a) Ring A₂ für eine 3-7-gliedrige Cycloalkylgruppe steht, gegebenenfalls wobei Ring A₂ für eine Cyclohexylgruppe steht; oder
(b) Ring A₂ für eine 4-7-gliedrige Heterocycloalkylgruppe steht, gegebenenfalls (i) wobei Ring A₂ für eine Piperazingruppe, eine Morpholingruppe, eine Piperidingruppe, eine Pyrrolidingruppe, eine Azetidingruppe oder eine Azabicyclohexangruppe steht oder (ii) wobei Ring A₂ für eine Piperidingruppe steht.

10. Verbindung nach einem der vorhergehenden Ansprüche, wobei:
(a) X₁ für CH₂ steht oder X₁ für CO steht und/oder
(b) X₂ für CH₂ steht oder X₂ für CO steht.

11. Verbindung nach einem der vorhergehenden Ansprüche, bei der es sich um eine Verbindung der Formel II handelt: 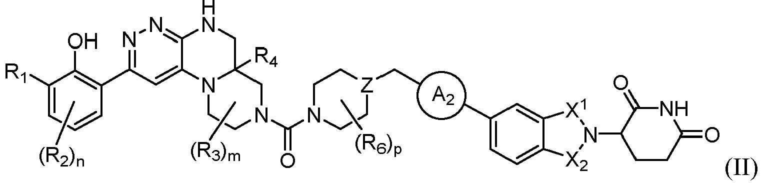
oder ein pharmazeutisch unbedenkliches Salz davon; wobei
R₆ jeweils unabhängig für H, D, C₁₋₆-Alkyl, C₃₋₆-Cycloalkyl oder Halogenalkyl steht;
p für 1, 2, 3, 4, 5, 6, 7 oder 8 steht und
Z für N oder CR₆ steht;
gegebenenfalls wobei:
(a) Z für N steht, p für 1 steht, p für 2 steht, p für 3 steht, p für 4 steht, p für 5 steht, p für 6 steht, p für 7 steht oder p für 8 steht und/oder
(b) R₆ jeweils für H steht, R₆ jeweils für C₁₋₆-Alkyl steht oder R₆ jeweils für Methyl steht.

12. Verbindung nach Anspruch 11, bei der es sich um eine Verbindung der Formel III handelt: 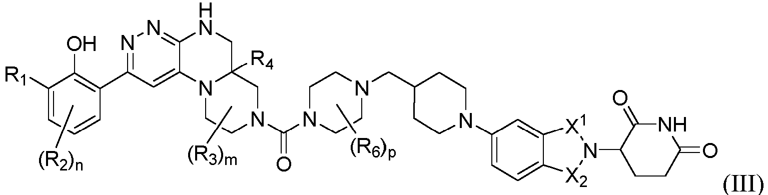
oder ein pharmazeutisch unbedenkliches Salz davon; oder bei der es sich um eine Verbindung der Formel IV handelt: 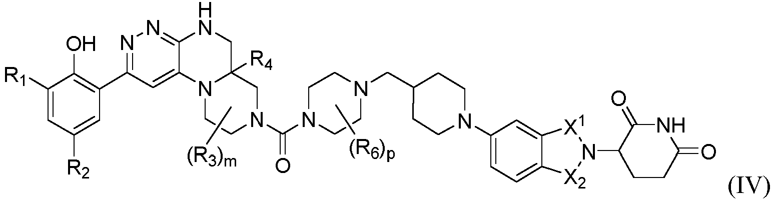
oder ein pharmazeutisch unbedenkliches Salz davon; oder bei der es sich um eine Verbindung der Formel V handelt: 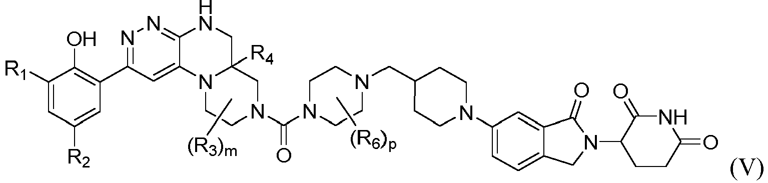
oder ein pharmazeutisch unbedenkliches Salz davon; oder bei der es sich um eine Verbindung der Formel VI handelt: 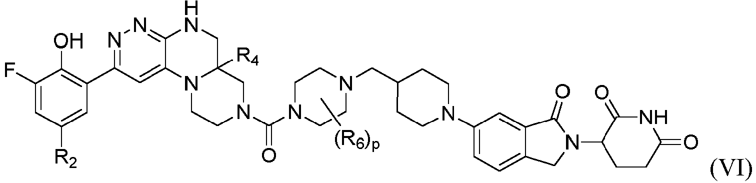
oder ein pharmazeutisch unbedenkliches Salz davon.

13. Verbindung gemäß Anspruch 1, bei der es sich um Folgendes handelt:
3-(6-(4-(((3R,5S)-4-((S)-2-(3-Fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(3-(((3R,5S)-4-((S)-2-(3-Fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)azetidin-1-yl)-1-oxoisoindolin-2-yl)piperidin-2,6-dion;
3-(5-((S)-2-(((3R,5S)-4-((S)-2-(3-Fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)morpholino)-1-oxoisoindolin-2-yl)piperidin-2,6-dion;
3-(6-(4-((4-((6aS,9S)-2-(3-Fluor-2-hydroxyphenyl)-9-methyl-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(4-((4-((S)-2-(3-Fluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(4-((4-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
2-(2,6-Dioxopiperidin-3-yl)-5-(4-((4-(2-(3-fluor-2-hydroxyphenyl)-6a-methyl-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)isoindolin-1,3-dion;
3-(6-(4-((4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(4-((4-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(4-((4-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(4-(((3R,5S)-4-2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((3R,5S)-4-2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((3R,5R)-4-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((3R,5R)-4-(2-(3-Chlor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((3R,5S)-4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
(R)-3-(6-(4-(((3S,5R)-4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
(S)-3-(6-(4-(((3S,5R)-4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
(R)-3-(6-(4-((4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
(S)-3-(6-(4-((4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(3-(((3R,5S)-4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)azetidin-1-yl)-1-oxoisoindolin-2-yl)piperidin-2,6-dion;
3-(5-((S)-2-(((3R,5S)-4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)morpholino)-1-oxoisoindolin-2-yl)piperidin-2,6-dion;
3-(6-(4-((4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(3-((4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)azetidin-1-yl)-1-oxoisoindolin-2-yl)piperidin-2,6-dion;
3-(6-(3-(((3S,5R)-4-((6aS,9S)-2-(3,5-Difluor-2-hydroxyphenyl)-9-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)azetidin-1-yl)-1-oxoisoindolin-2-yl)piperidin-2,6-dion;
3-(6-(4-((4-((6aS,9S)-2-(3,5-Difluor-2-hydroxyphenyl)-9-methyl-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(4-(((3S,5R)-4-(2-(3,5-Difluor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-((4-(2-(3,5-Difluor-2-hydroxyphenyl)-6a-ethyl-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(4-(((3R,5R)-4-(2-(3,5-Difluor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(3-((4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3-ethylpiperazin-1-yl)methyl)azetidin-1-yl)-1-oxoisoindolin-2-yl)piperidin-2,6-dion;
3-(6-(4-(((3R,5S)-4-(2-(3-Chlor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-((4-(2-(3-Chlor-2-hydroxyphenyl)-6a-methyl-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
2-(2,6-Dioxopiperidin-3-yl)-5-(4-((4-(6a-ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)isoindolin-1,3-dion;
3-(6-(4-(((3R,5R)-4-(2-(3,5-Difluor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((3S,5R)-4-(2-(3,5-Difluor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-((4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3-ethylpiperazin-1-yl)methyl)piperidin-1-yl)-1-oxoisoindolin-2-yl)piperidin-2,6-dion;
5-(4-(((3S,5R)-4-(2-(3,5-Difluor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-2-(2,6-dioxopiperidin-3-yl)isoindolin-1,3-dion;
3-(6-((3R,4R)-4-(((1R,5S,6R)-6-(((S)-2-(3,5-Difluor-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)-3-fluorpiperidin-1-yl)-1-oxoisoindolin-2-yl)piperidin-2,6-dion;
3-(6-((3R,4R)-4-(((1R,5S,6R)-6-(((6aS,9S)-2-(3,5-Difluor-2-hydroxyphenyl)-9-methyl-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)-3-fluorpiperidin-1-yl)-1-oxoisoindolin-2-yl)piperidin-2,6-dion;
3-(6-(4-(((1R,5S,6r)-6-(((S)-2-(3,5-Difluor-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)-3,3-difluorpiperidin-1-yl)-1-oxoisoindolin-2-yl)piperidin-2,6-dion;
3-(6-(4-(((1R,5S,6r)-6-(((S)-2-(3,5-Difluor-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)-3,3-difluorpiperidin-1-yl)-1-oxoisoindolin-2-yl)piperidin-2,6-dion;
3-(6-((rac-3R,4R)-4-(((3S,5R)-4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)-3-fluorpiperidin-1-yl)-1-oxoisoindolin-2-yl)piperidin-2,6-dion;
3-(6-((3R,4R)-3-Fluor-4-(((3S,5R)-4-((S)-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((3R,5R)-4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(5-((S)-2-(((3R,5R)-4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)morpholino)-1-oxoisoindolin-2-yl)piperidin-2,6-dion;
3-(6-(4-(((3S,5S)-4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-((1-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)piperidin-4-yl)methyl)piperazin-1-yl)-1-oxoisoindolin-2-yl)piperidin-2,6-dion;
3-(6-(2-(((1R,5S,6s)-6-(((S)-2-(3,5-Difluor-2-hydroxyphenyl)-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)morpholino)-1-oxoisoindolin-2-yl)piperidin-2,6-dion;
3-(6-(2-(((1R,5S,6r)-6-(((6aS,9S)-2-(3,5-Difluor-2-hydroxyphenyl)-9-methyl-5,6,6a,7,9,10-hexahydro-8H-pyrazino[1',2':4,5]pyrazino[2,3-c]pyridazin-8-yl)methyl)-3-azabicyclo[3.1.0]hexan-3-yl)methyl)morpholino)-1-oxoisoindolin-2-yl)piperidin-2,6-dion;
3-(5-((S)-2-(((3R,4R)-4-(((S)-2-(3,5-Difluor-2-hydroxyphenyl)-5,6,6a,7,9,10-hexahydro-8H-pyrazino[1',2':4,5]pyrazino[2,3-c]pyridazin-8-yl)methyl)-3-fluorpiperidin-1-yl)methyl)morpholino)-1-oxoisoindolin-2-yl)piperidin-2,6-dion;
3-(6-((S)-3-(((R)-3-(((S)-2-(3,5-Difluor-2-hydroxyphenyl)-5,6,6a,7,9,10-hexahydro-8H-pyrazino[1',2':4,5]pyrazino[2,3-c]pyridazin-8-yl)methyl)pyrrolidin-1-yl)methyl)pyrrolidin-1-yl)-1-oxoisoindolin-2-yl)piperidin-2,6-dion;
3-(5-((S)-3-((4-(((S)-2-(3,5-Difluor-2-hydroxyphenyl)-5,6,6a,7,9,10-hexahydro-8H-pyrazino[1',2':4,5]pyrazino[2,3-c]pyridazin-8-yl)methyl)piperidin-1-yl)methyl)pyrrolidin-1-yl)-1-oxoisoindolin-2-yl)piperidin-2,6-dion;
3-(6-(4-(((1S,4r)-4-(((S)-2-(3,5-Difluor-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidin-2,6-dion;
3-(6-(4-((4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-4-fluorpiperidin-1-yl)methyl)piperidin-1-yl)-1-oxoisoindolin-2-yl)piperidin-2,6-dion;
3-(6-(4-(((1R,4s)-4-(((S)-2-(3,5-Difluor-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidin-2,6-dion;
3-(6-(4-(((2S,5R)-4-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-2,5-dimethyl-piperazin-1-yl)methyl)piperidin-1-yl)-1-oxoisoindolin-2-yl)piperidin-2,6-dion;
3-(6-(4-(((1S,4r)-4-(((S)-2-(3-Fluor-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidin-2,6-dion;
3-(6-(4-(((3R,5S)-4-(2-(3,5-Difluor-2-hydroxyphenyl)-6a-ethyl-6,6a,7,8,9,10-hexahydro-5H-pyrazino[1',2':4,5]pyrazino[2,3-c]pyridazin-8-carbonyl)-3,5-dimethyl-piperazin-1-yl)methyl)piperidin-1-yl)-1-oxoisoindolin-2-yl)piperidin-2,6-dion;
3-(6-(4-(((3R,5S)-4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethyl-piperazin-1-yl)methyl)piperidin-1-yl)-1-oxoisoindolin-2-yl)piperidin-2,6-dion;
3-(6-(4-(((3R,5S)-4-(2-(3,5-Difluor-2-hydroxyphenyl)-6a-ethyl-6,6a,7,8,9,10-hexahydro-5H-pyrazino[1',2':4,5]pyrazino[2,3-c]pyridazin-8-carbonyl)-3,5-dimethyl-piperazin-1-yl)methyl)piperidin-1-yl)-1-oxoisoindolin-2-yl)piperidin-2,6-dion;
3-(6-(4-(((3R,5S)-4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethyl-piperazin-1-yl)methyl)piperidin-1-yl)-1-oxoisoindolin-2-yl)piperidin-2,6-dion;
3-(6-(4-(((3R,5R)-4-(2-(3-Chlor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethyl-piperazin-1-yl)methyl)piperidin-1-yl)-1-oxoisoindolin-2-yl)piperidin-2,6-dion;
3-(6-(4-(((3R,5S)-4-(2-(3,5-Difluor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethyl-piperazin-1-yl)methyl)piperidin-1-yl)-1-oxoisoindolin-2-yl)piperidin-2,6-dion;
oder ein pharmazeutisch unbedenkliches Salz davon; oder bei der sich um Folgendes handelt:
(S)-3-(6-(4-((4-((R)-6a-(Difluormethyl)-2-(3-fluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
(S)-3-(6-(4-(((3S,5S)-4-((R)-6a-(Difluormethyl)-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
(S)-3-(6-(4-(((3R,5R)-4-((R)-6a-(Difluormethyl)-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
(S)-3-(6-(4-(((2S,5R)-4-((R)-6a-(Difluormethyl)-2-(3-fluor-2-hydroxyphenyl)-6,6a,7,8,9,10-hexahydro-5H-pyrazino[1',2':4,5]pyrazino[2,3-c]pyridazin-8-carbonyl)-2,5-dimethylpiperazin-1-yl)methyl)piperidin-1-yl)-1-oxoisoindolin-2-yl)piperidin-2,6-dion;
3-(6-(4-(((3R,5S)-4-((S)-6a-(Difluormethyl)-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((3R,5S)-4-((R)-6a-(Difluormethyl)-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
(S)-3-(6-(4-(((2R,4S,6S)-1-((S)-6a-(Difluormethyl)-2-(3-fluor-2-hydroxyphenyl)-6,6a,7,8,9,10-hexahydro-5H-pyrazino[1',2':4,5]pyrazino[2,3-c]pyridazin-8-carbonyl)-2,6-dimethylpiperidin-4-yl)methyl)piperazin-1-yl)-1-oxoisoindolin-2-yl)piperidin-2,6-dion;
(S)-3-(6-(4-(((2R,4R,6S)-1-((R)-6a-(Difluormethyl)-2-(3-fluor-2-hydroxyphenyl)-6,6a,7,8,9,10-hexahydro-5H-pyrazino[1',2':4,5]pyrazino[2,3-c]pyridazin-8-carbonyl)-2,6-dimethylpiperidin-4-yl)methyl)piperazin-1-yl)-1-oxoisoindolin-2-yl)piperidin-2,6-dion;
(3S)-3-(6-(4-((1-((S)-6a-(Difluormethyl)-2-(3-fluor-2-hydroxyphenyl)-6,6a,7,8,9,10-hexahydro-5H-pyrazino[1',2':4,5]pyrazino[2,3-c]pyridazin-8-carbonyl)-2,2-dimethylpiperidin-4-yl)methyl)piperazin-1-yl)-1-oxoisoindolin-2-yl)piperidin-2,6-dion;
(3S)-3-(6-(4-((4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
(3S)-3-(6-(4-(((3R,5R)-4-(2-(3-Chlor-2-hydroxyphenyl)-6a-ethyl-6,6a,7,8,9,10-hexahydro-5H-pyrazino[1',2':4,5]pyrazino[2,3-c]pyridazin-8-carbonyl)-3,5-dimethyl-piperazin-1-yl)methyl)piperidin-1-yl)-1-oxoisoindolin-2-yl)piperidin-2,6-dion;
(3S)-3-(6-(4-((4-(2-(3-Chlor-2-hydroxyphenyl)-6a-ethyl-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
(3S)-3-(6-(4-(((2S,5R)-4-(2-(3-Chlor-2-hydroxyphenyl)-6a-ethyl-6,6a,7,8,9,10-hexahydro-5H-pyrazino[1',2':4,5]pyrazino[2,3-c]pyridazin-8-carbonyl)-2,5-dimethyl-piperazin-1-yl)methyl)piperidin-1-yl)-1-oxoisoindolin-2-yl)piperidin-2,6-dion;
(3S)-3-(6-(4-(((2S,5R)-4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-2,5-dimethylpiperazin-1-yl)methyl)piperidin-1-yl)-1-oxoisoindolin-2-yl)piperidin-2,6-dion;
3-(6-(4-((4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(4-((4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(4-(((3S,5S)-4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((3R,5R)-4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((3R,5R)-4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((2S,5R)-4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-2,5-dimethylpiperazin-1-yl)methyl)piperidin-1-yl)-1-oxoisoindolin-2-yl)piperidin-2,6-dion;
3-(6-(4-(((3R,5S)-4-(2-(3,5-Difluor-2-hydroxyphenyl)-6a-ethyl-6,6a,7,8,9,10-hexahydro-5H-pyrazino[1',2':4,5]pyrazino[2,3-c]pyridazin-8-carbonyl)-3,5-dimethyl-piperazin-1-yl)methyl)piperidin-1-yl)-1-oxoisoindolin-2-yl)piperidin-2,6-dion;
3-(6-(4-(((3R,5S)-4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
3-(6-(4-(((3R,5S)-4-(2-(3,5-Difluor-2-hydroxyphenyl)-6a-ethyl-6,6a,7,8,9,10-hexahydro-5H-pyrazino[1',2':4,5]pyrazino[2,3-c]pyridazin-8-carbonyl)-3,5-dimethyl-piperazin-1-yl)methyl)piperidin-1-yl)-1-oxoisoindolin-2-yl)piperidin-2,6-dion;
3-(6-(4-(((3R,5S)-4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
(3S)-3-(6-(4-((4-(2-(3-Fluor-2-hydroxyphenyl)-6a-(fluormethyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
(3S)-3-(6-(4-((4-(2-(3-Fluor-2-hydroxyphenyl)-6a-(fluormethyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
3-(6-(4-(((2S,5R)-4-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-2,5-dimethylpiperazin-1-yl)methyl)piperidin-1-yl)-1-oxoisoindolin-2-yl)piperidin-2,6-dion;
3-(6-(4-(((2S,5R)-4-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-2,5-dimethylpiperazin-1-yl)methyl)piperidin-1-yl)-1-oxoisoindolin-2-yl)piperidin-2,6-dion;
3-(6-(4-(((3R,5S)-4-(2-(3-Chlor-2-hydroxyphenyl)-6a-methyl-6,6a,7,8,9,10-hexahydro-5H-pyrazino[1',2':4,5]pyrazino[2,3-c]pyridazin-8-carbonyl)-3,5-dimethyl-piperazin-1-yl)methyl)piperidin-1-yl)-1-oxoisoindolin-2-yl)piperidin-2,6-dion;
((2S,6R)-2,6-Dimethylpiperazin-1-yl)(2-(3-fluor-2-hydroxyphenyl)-6a-methyl-5,6,6a,7,9,10-hexahydro-8H-pyrazino[1',2':4,5]pyrazino[2,3-c]pyridazin-8-yl)methanon;
((2S,6R)-2,6-Dimethylpiperazin-1-yl)(2-(3-fluor-2-hydroxyphenyl)-6a-methyl-5,6,6a,7,9,10-hexahydro-8H-pyrazino[1',2':4,5]pyrazino[2,3-c]pyridazin-8-yl)methanon;
(3S)-3-(6-(4-(((3S,5R)-4-(2-(3-Fluor-2-hydroxyphenyl)-6a-(trifluormethyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
(3S)-3-(6-(4-(((3R,5R)-4-(2-(3-Fluor-2-hydroxyphenyl)-6a-(trifluormethyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
(3S)-3-(6-(4-(((3R,5R)-4-(2-(3-Fluor-2-hydroxyphenyl)-6a-(trifluormethyl)-6,6a,7,8,9,10-hexahydro-5H-pyrazino[1',2':4,5]pyrazino[2,3-c]pyridazin-8-carbonyl)-3,5-dimethylpiperazin-1-yl)methyl)piperidin-1-yl)-1-oxoisoindolin-2-yl)piperidin-2,6-dion;
(3S)-3-(6-(4-(((2R,4r,6S)-1-(2-(3-Fluor-2-hydroxyphenyl)-6a-(trifluormethyl)-6,6a,7,8,9,10-hexahydro-5H-pyrazino[1',2':4,5]pyrazino[2,3-c]pyridazin-8-carbonyl)-2,6-dimethylpiperidin-4-yl)methyl)piperazin-1-yl)-1-oxoisoindolin-2-yl)piperidin-2,6-dion;
(3S)-3-(6-(4-(((2R,4r,6S)-1-(2-(3-Fluor-2-hydroxyphenyl)-6a-(trifluormethyl)-6,6a,7,8,9,10-hexahydro-5H-pyrazino[1',2':4,5]pyrazino[2,3-c]pyridazin-8-carbonyl)-2,6-dimethylpiperidin-4-yl)methyl)piperazin-1-yl)-1-oxoisoindolin-2-yl)piperidin-2,6-dion;
(3S)-3-(6-(4-((4-(2-(3-Fluor-2-hydroxyphenyl)-6a-(trifluormethyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
(3S)-3-(6-(4-((4-(2-(3-Fluor-2-hydroxyphenyl)-6a-(trifluormethyl)-6,6a,7,8,9,10-hexahydro-5H-pyrazino[1',2':4,5]pyrazino[2,3-c]pyridazin-8-carbonyl)-3,3-dimethylpiperazin-1-yl)methyl)piperidin-1-yl)-1-oxoisoindolin-2-yl)piperidin-2,6-dion;
(3S)-3-(6-(4-(((1s,4R)-4-(2-(3,5-Difluor-2-hydroxyphenyl)-6a-ethyl-6,6a,7,8,9,10-hexahydro-5H-pyrazino[1',2':4,5]pyrazino[2,3-c]pyridazin-8-carbonyl)-4-fluorcyclohexyl)methyl)piperazin-1-yl)-1-oxoisoindolin-2-yl)piperidin-2,6-dion;
(S)-3-(6-(4-(((1R,4R)-4-((S)-2-(3,5-Difluor-2-hydroxyphenyl)-6a-ethyl-6,6a,7,8,9,10-hexahydro-5H-pyrazino[1',2':4,5]pyrazino[2,3-c]pyridazin-8-carbonyl)-4-fluorcyclohexyl)methyl)piperazin-1-yl)-1-oxoisoindolin-2-yl)piperidin-2,6-dion;
(S)-3-(6-(4-((4-((R)-2-(3,5-Difluor-2-hydroxyphenyl)-6a-(difluormethyl)-6,6a,7,8,9,10-hexahydro-5H-pyrazino[1',2':4,5]pyrazino[2,3-c]pyridazin-8-carbonyl)-4-fluorpiperidin-1-yl)methyl)piperidin-1-yl)-1-oxoisoindolin-2-yl)piperidin-2,6-dion;
(3S)-3-(6-(4-((4-(2-(3-Chlor-2-hydroxyphenyl)-6a-ethyl-6,6a,7,8,9,10-hexahydro-5H-pyrazino[1',2':4,5]pyrazino[2,3-c]pyridazin-8-carbonyl)-4-fluorpiperidin-1-yl)methyl)piperidin-1-yl)-1-oxoisoindolin-2-yl)piperidin-2,6-dion;
(3S)-3-(6-(4-((4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-4-fluorpiperidin-1-yl)methyl)piperidin-1-yl)-1-oxoisoindolin-2-yl)piperidin-2,6-dion;
(3S)-3-(6-(4-((4-(6a-Ethyl-2-(3-fluor-2-hydroxyphenyl)-6,6a,7,8,9,10-hexahydro-5H-pyrazino[1',2':4,5]pyrazino[2,3-c]pyridazin-8-carbonyl)-4-fluorpiperidin-1-yl)methyl)piperidin-1-yl)-1-oxoisoindolin-2-yl)piperidin-2,6-dion;
(3S)-3-(6-(4-((4-(2-(3,5-Difluor-2-hydroxyphenyl)-6a-ethyl-6,6a,7,8,9,10-hexahydro-5H-
pyrazino[1',2':4,5]pyrazino[2,3-c]pyridazin-8- carbonyl)-4-fluorpiperidin-1-yl)methyl)piperidin-1-yl)-1-oxoisoindolin-2-yl)piperidin-2,6-dion;
(3S)-3-(6-(4-((4-(2-(3,5-Difluor-2-hydroxyphenyl)-6a- ethyl-6,6a,7,8,9,10-hexahydro-5H-
pyrazino[1',2':4,5]pyrazino[2,3-c]pyridazin-8-carbonyl)-4-fluorpiperidin-1-yl)methyl)piperidin-1-yl)-1-oxoisoindolin-2-yl)piperidin-2,6-dion;
(3S)-3-(6-(4-((4-Fluor-4-(2-(3-fluor-2-hydroxyphenyl)- 6a-methyl-6,6a,7,8,9,10-hexahydro-5H-
pyrazino[1',2':4,5]pyrazino[2,3-c]pyridazin-8-carbonyl)piperidin-1-yl)methyl)piperidin-1-yl)-1-oxoisoindolin-2-yl)piperidin-2,6-dion;
(3S)-3-(6-(4-((4-(2-(3,5-Difluor-2-hydroxyphenyl)-6a- methyl-6,6a,7,8,9,10-hexahydro-5H-
pyrazino[1',2':4,5]pyrazino[2,3-c]pyridazin-8-carbonyl)-4-fluorpiperidin-1-yl)methyl)piperidin-1-yl)- 1-oxoisoindolin-2-yl)piperidin-2,6-dion;
(3S)-3-(6-(4-((4-(2-(3,5-Difluor-2-hydroxyphenyl)-6a- methyl-6,6a,7,8,9,10-hexahydro-5H-
pyrazino[1',2':4,5]pyrazino[2,3-c]pyridazin-8-carbonyl)-4-fluorpiperidin-1-yl)methyl)piperidin-1-yl)-1-oxoisoindolin-2-yl)piperidin-2,6-dion;
3-(6-(4-(((1S,4r)-4-((S)-2-(3-Fluor-2-hydroxyphenyl)-6,6a,7,8,9,10-hexahydro-5H-
pyrazino[1',2':4,5]pyrazino[2,3-c]pyridazin-8-carbonyl)cyclohexyl)methyl)piperazin-1-yl)-1-oxoisoindolin-2-yl)piperidin-2,6-dion;
(S)-3-(6-(4-(((1S,4S)-4-(((S)-2-(3-Fluor-2-hydroxyphenyl)-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidin-2,6-dion;
3-(6-(4-(((1R,4r)-4-(((R)-2-(3-Fluor-2-hydroxyphenyl)-6a-methyl-5,6,6a,7,9,10-hexahydro-8H- pyrazino[1',2':4,5]pyrazino[2,3-c]pyridazin-8-yl)methyl)cyclohexyl)methyl)piperazin-1-yl)-1-oxoisoindolin-2-yl)piperidin-2,6-dion;
3-(6-(4-(((1R,4r)-4-(((R)-2-(3-Fluor-2-hydroxyphenyl)-6a-methyl-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidin-2,6-dion;
(S)-3-(6-(4-(((1R,4r)-4-(((R)-2-(3-Fluor-2-hydroxyphenyl)-6a-methyl-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidin-2,6-dion;
(S)-3-(6-(4-(((1R,4r)-4-(((R)-2-(3-Fluor-2-hydroxyphenyl)-6a-methyl-5,6,6a,7,9,10-hexahydro-8H-pyrazino[1',2':4,5]pyrazino[2,3-c]pyridazin-8-yl)methyl)cyclohexyl)methyl)piperazin-1-yl)-1-oxoisoindolin-2-yl)piperidin-2,6-dion;
3-(6-(4-((4-((S)-2-(3,5-Difluor-2-hydroxyphenyl)-6,6a,7,8,9,10-hexahydro-5H-pyrazino[1',2':4,5]pyrazino[2,3-c]pyridazin-8-carbonyl)-3,3-dimethylpiperidin-1-yl)methyl)piperidin-1-yl)-1-oxoisoindolin-2-yl)piperidin-2,6-dion;
(S)-3-(6-(4-(((2R,4R,6S)-1-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-2,6-dimethylpiperidin-4-yl)methyl)piperazin-1-yl)-1-oxoisoindolin-2-yl)piperidin-2,6-dion;
(S)-3-(6-(4-(((2R,4R,6S)-1-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-2,6-dimethylpiperidin-4-yl)methyl)piperazin-1-yl)-1-oxoisoindolin-2-yl)piperidin-2,6-dion;
3-(6-(4-((1-((S)-2-(3-Fluor-2-hydroxyphenyl)-6,6a,7,8,9,10-hexahydro-5H-pyrazino[1',2':4,5]pyrazino[2,3-c]pyridazin-8-carbonyl)-2,2-dimethylpiperidin-4-yl)methyl)piperazin-1-yl)-1-oxoisoindolin-2-yl)piperidin-2,6-dion;
(3S)-3-(6-(4-((1-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-2,2-dimethylpiperidin-4-yl)methyl)piperazin-1-yl)-1-oxoisoindolin-2-yl)piperidin-2,6-dion;
(3S)-3-(6-(4-((1-(2-(3-Fluor-2-hydroxyphenyl)-6a-methyl-6,6a,7,8,9,10-hexahydro-5H-pyrazino[1',2':4,5]pyrazino[2,3-c]pyridazin-8-carbonyl)-2,2-dimethylpiperidin-4-yl)methyl)piperazin-1-yl)-1-oxoisoindolin-2-yl)piperidin-2,6-dion;
oder ein pharmazeutisch unbedenkliches Salz davon.

14. Pharmazeutische Zusammensetzung, umfassend eine Verbindung nach einem der vorhergehenden Ansprüche oder ein pharmazeutisch unbedenkliches Salz davon und einen pharmazeutisch unbedenklichen Hilfsstoff.

15. Verbindung oder pharmazeutisch unbedenkliches Salz davon nach einem der Ansprüche 1-13 oder pharmazeutische Zusammensetzung nach Anspruch 14 zur Verwendung bei einem Verfahren zur Behandlung von Krebs bei einem Individuum, bei dem diesbezüglicher Bedarf besteht, wobei das Verfahren das Verabreichen einer Verbindung nach einem der Ansprüche 1-13 oder eines pharmazeutisch unbedenklichen Salzes davon oder einer pharmazeutischen Zusammensetzung nach Anspruch 14 an das Individuum umfasst, gegebenenfalls wobei:
(a) es sich bei dem Krebs um Krebs mit SMARCA4-Deletion handelt oder
(b) es sich bei dem Krebs um Plattenepithelkarzinom, Basalzellkarzinom, Adenokarzinom, Leberzellkarzinome und Nierenzellkarzinome, Blasen-, Darm-, Brust-, Gebärmutterhals-, Dickdarm-, Speiseröhren-, Kopf-, Nieren-, Leber-, Lungen-, Hals-, Eierstock-, Bauchspeicheldrüsen-, Prostata- und Magenkrebs; Leukämien; benigne und maligne Lymphome, insbesondere Burkitt-Lymphom und Non-Hodgkin-Lymphom; benigne und maligne Melanome; myeloproliferative Erkrankungen; Sarkome einschließlich Ewing-Sarkom, Hämangiosarkom, Kaposi-Sarkom, Liposarkom, Myosarkome, peripheres Neuroepitheliom, Synovialsarkom, Gliome, Astrozytome, Oligodendrogliome, Ependymome, Gliobastome, Neuroblastome, Ganglioneurome, Gangliogliome, Medulloblastome, Pinealzelltumore, Meningiome, Meningealsarkome, Neurofibrome und Schwannome; Darmkrebs, Brustkrebs, Prostatakrebs, Gebärmutterhalskrebs, Gebärmutterkrebs, Lungenkrebs, Eierstockkrebs, Hodenkrebs, Schilddrüsenkrebs, Astrozytom, Speiseröhrenkrebs, Bauchspeicheldrüsenkrebs, Magenkrebs, Leberkrebs, Dickdarmkrebs, Melanom; Karzinosarkom, Morbus Hodgkin, Wilms-Tumor und Teratokarzinome handelt,
gegebenenfalls wobei es sich bei dem Lungenkrebs um SMARCA4-defizienten nichtkleinzelligen Lungenkrebs handelt; oder
(c) es sich bei dem Krebs um akute lymphoblastische Leukämie der T-Zelllinie (T-ALL), lymphoblastisches Lymphom der T-Zelllinie (T-LL), peripheres T-Zell-Lymphom, adulte T-Zell-Leukämie, Prä-B-ALL, Prä-B-Lymphome, großzelliges B-Zell-Lymphom, Burkitt-Lymphom, B-Zell-ALL, Philadelphia-Chromosom-positives ALL und Philadelphia-Chromosom-positives CML handelt.

## Revendications

1. Composé de formule (I) : 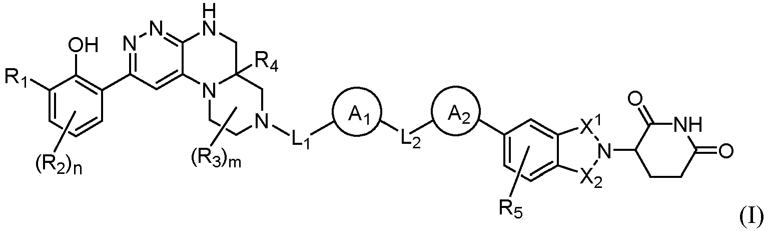
ou sel pharmaceutiquement acceptable de celui-ci ; formule dans laquelle
R₁ est halogéno, alkyle en C₁₋₆, ou halogénoalkyle ;
chaque R₂ est indépendamment H, D, ou F ;
chaque R₃ est indépendamment H, D, alkyle en C₁₋₆ , halogénoalkyle, ou cycloalkyle en C₃₋₆ ;
n est 1, 2 ou 3 ;
m est 1, 2, 3, 4, 5 ou 6 ;
R₄ est H, D, alkyle en C₁₋₆ , cycloalkyle en C₃₋₆, alcoxyalkyle, cyanoalkyle ou halogénoalkyle ;
R₅ est H, D, ou F ;
L₁ est une liaison, C(R₃)₂, ou CO ;
L₂ est une liaison, C(R₃)₂, ou CO ;
le cycle A₁ est un groupe cycloalkyle à 3-7 chaînons, un groupe hétérocycloalkyle à 4-7 chaînons, un groupe aryle, ou un groupe hétéroaryle ;
le cycle A₂ est un groupe cycloalkyle à 3-7 chaînons, un groupe hétérocycloalkyle à 4-7 chaînons, un groupe aryle, ou un groupe hétéroaryle ;
X₁ est CH₂, CO, CH=CH (lorsque X₂ = CO), ou N=CH (lorsque X₂ = CO) ; et
X₂ est CH₂, CO, CH=CH (lorsque X₁ = CO), ou N=CH (lorsque X₁ = CO).

2. Composé selon la revendication 1, dans lequel R₁ est halogéno, éventuellement F.

3. Composé selon l'une quelconque des revendications précédentes, dans lequel n est 1, ou, dans lequel n est 2, ou dans lequel n est 3.

4. Composé selon l'une quelconque des revendications précédentes, dans lequel chaque R₂ est F, ou dans lequel chaque R₂ est H.

5. Composé selon l'une quelconque des revendications précédentes, dans lequel m est 1, dans lequel m est 2, dans lequel m est 3, dans lequel m est 4, dans lequel m est 5, ou, dans lequel m est 6.

6. Composé selon l'une quelconque des revendications précédentes, dans lequel :
(a) chaque R₃ est H ; et/ou
(b) R₄ est H ; R₄ est D ; R₄ est alkyle en C₁₋₆ ; ou R₄ est méthyle ; et/ou
(c) R₅ est H.

7. Composé selon l'une quelconque des revendications précédentes, dans lequel :
(a) L₁ est CO ; ou L₁ est C(R₃)₂ ; ou L₁ est un groupe méthylène ; et/ou
(b) L₂ est CO ; ou L₂ est C(R₃)₂ ; ou L₂ est un groupe méthylène.

8. Composé selon l'une quelconque des revendications précédentes, dans lequel :
(a) le cycle A₁ est un groupe cycloalkyle à 3 à 7 chaînons, éventuellement dans lequel le cycle A₁ est un groupe cyclohexyle ; ou
(b) le cycle A₁ est un groupe hétérocycloalkyle à 4 à 7 chaînons, éventuellement (i) le cycle A₁ est un groupe pipérazine, un groupe morpholine, un groupe pipéridine, un groupe pyrrolidine, un groupe azétidine ou un groupe azabicyclohexane ; ou (ii) le cycle A₁ est un groupe pipérazine.

9. Composé selon l'une quelconque des revendications précédentes, dans lequel :
(a) le cycle A₂ est un groupe cycloalkyle à 3 à 7 chaînons, éventuellement dans lequel le cycle A₂ est un groupe cyclohexyle ; ou
(b) le cycle A₂ est un groupe hétérocycloalkyle à 4 à 7 chaînons, éventuellement (i) dans lequel le cycle A₂ est un groupe pipérazine, un groupe morpholine, un groupe pipéridine, un groupe pyrrolidine, un groupe azétidine ou un groupe azabicyclohexane ; ou (ii) dans lequel le cycle A₂ est un groupe pipéridine.

10. Composé selon l'une quelconque des revendications précédentes, dans lequel :
(a) X₁ est CH₂, ou X₁ est CO ; et/ou
(b) X₂ est CH₂, ou X₂ est CO.

11. Composé selon l'une quelconque des revendications précédentes qui est un composé de formule II : 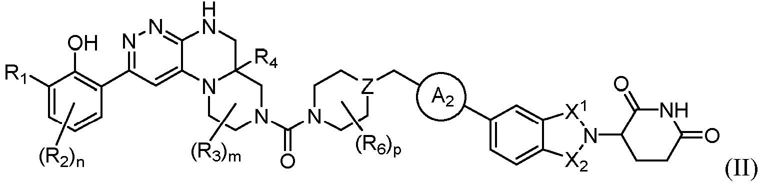
ou un sel pharmaceutiquement acceptable de celui-ci ; formule dans laquelle
chaque R₆ est indépendamment H, D, alkyle en C₁₋₆ , cycloalkyle en C₃₋₆ , ou halogénoalkyle ;
p est 1, 2, 3, 4, 5, 6, 7 ou 8 ; et
Z est N ou CR₆ ;
éventuellement dans laquelle,
(a) Z est N, p est 1, p est 2, p est 3, p est 4, p est 5, p est 6, p est 7, ou p est 8 ; et/ou
(b) chaque R₆ est H, chaque R₆ est alkyle en C₁₋₆ , ou chaque R₆ est méthyle.

12. Composé selon la revendication 11 qui est un composé de formule III: 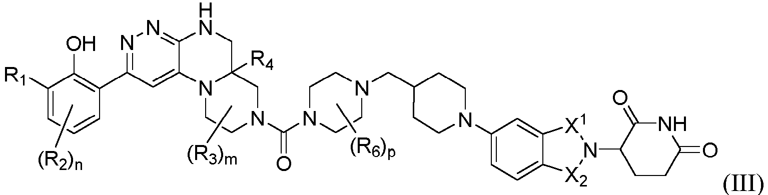
ou un sel pharmaceutiquement acceptable de celui-ci ; ou qui est un composé de formule IV : 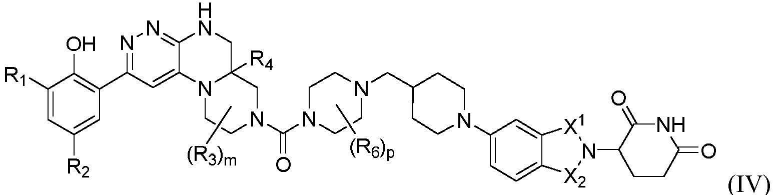
ou un sel pharmaceutiquement acceptable de celui-ci ; ou qui est un composé de formule V : 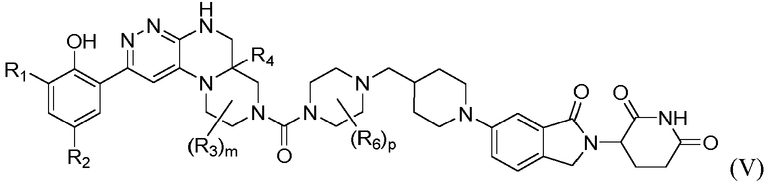
ou un sel pharmaceutiquement acceptable de celui-ci ; ou qui est un composé de formule VI : 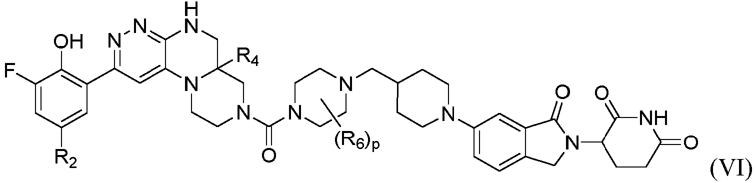
ou un sel pharmaceutiquement acceptable de celui-ci.

13. Composé selon la revendication 1 qui est :
3-(6-(4-(((3R,5S)-4-((S)-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(3-(((3R,5S)-4-((S)-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)azétidin-1-yl)-1-oxoisoindolin-2-yl)pipéridine-2,6-dione ;
3-(5-((S)-2-(((3R,5S)-4-((S)-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)morpholino)-1-oxoisoindolin-2-yl)pipéridine-2,6-dione ;
3-(6-(4-((4-((6aS,9S)-2-(3-fluoro-2-hydroxyphényl)-9-méthyl-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(4-((4-((S)-2-(3-fluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(4-((4-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
2-(2,6-dioxopipéridin-3-yl)-5-(4-((4-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)isoindoline-1,3-dione ;
3-(6-(4-((4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(4-((4-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(4-((4-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(4-(((3R,5S)-4-2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((3R,5S)-4-2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((3R,5R)-4-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((3R,5R)-4-(2-(3-chloro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((3R,5S)-4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
(R)-3-(6-(4-(((3S,5R)-4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
(S)-3-(6-(4-(((3S,5R)-4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
(R)-3-(6-(4-((4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
(S)-3-(6-(4-((4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(3-(((3R,5S)-4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)azétidin-1-yl)-1-oxoisoindolin-2-yl)pipéridine-2,6-dione ;
3-(5-((S)-2-(((3R,5S)-4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)morpholino)-1-oxoisoindolin-2-yl)pipéridine-2,6-dione ;
3-(6-(4-((4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(3-((4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)azétidin-1-yl)-1-oxoisoindolin-2-yl)pipéridine-2,6-dione ;
3-(6-(3-(((3S,5R)-4-((6aS,9S)-2-(3,5-difluoro-2-hydroxyphényl)-9-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)azétidin-1-yl)-1-oxoisoindolin-2-yl)pipéridine-2,6-dione ;
3-(6-(4-((4-((6aS,9S)-2-(3,5-difluoro-2-hydroxyphényl)-9-méthyl-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(4-(((3S,5R)-4-(2-(3,5-difluoro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-((4-(2-(3,5-difluoro-2-hydroxyphényl)-6a-éthyl-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(4-(((3R,5R)-4-(2-(3,5-difluoro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(3-((4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3-éthylpipérazin-1-yl)méthyl)azétidin-1-yl)-1-oxoisoindolin-2-yl)pipéridine-2,6-dione ;
3-(6-(4-(((3R,5S)-4-(2-(3-chloro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-((4-(2-(3-chloro-2-hydroxyphényl)-6a-méthyl-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
2-(2,6-dioxopipéridin-3-yl)-5-(4-((4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)isoindoline-1,3-dione ;
3-(6-(4-(((3R,5R)-4-(2-(3,5-difluoro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((3S,5R)-4-(2-(3,5-difluoro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-((4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3-éthylpipérazin-1-yl)méthyl)pipéridin-1-yl)-1-oxoisoindolin-2-yl)pipéridine-2,6-dione ;
5-(4-(((3S,5R)-4-(2-(3,5-difluoro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-2-(2,6-dioxopipéridin-3-yl)isoindoline-1,3-dione ;
3-(6-((3R,4R)-4-(((1R,5S,6R)-6-(((S)-2-(3,5-difluoro-2-hydroxyphényl)-5,6,6a,7,9,10-hexahydro-8H-pyrazino[1',2':4,5]pyrazino[2,3-c]pyridazin-8-yl)méthyl)-3-azabicyclo[3.1.0]hexan-3-yl)méthyl)-3-fluoropipéridin-1-yl)-1-oxoisoindolin-2-yl)pipéridine-2,6-dione ;
3-(6-((3R,4R)-4-(((1R,5S,6R)-6-(((6aS,9S)-2-(3,5-difluoro-2-hydroxyphényl)-9-méthyl-5,6,6a,7,9,10-hexahydro-8H-pyrazino[1',2':4,5]pyrazino[2,3-c]pyridazin-8-yl)méthyl)-3-azabicyclo[3.1.0]hexan-3-yl)méthyl)-3-fluoropipéridin-1-yl)-1-oxoisoindolin-2-yl)pipéridine-2,6-dione ;
3-(6-(4-(((1R,5S,6r)-6-(((S)-2-(3,5-difluoro-2-hydroxyphényl)-5,6,6a,7,9,10-hexahydro-8H-pyrazino[1',2':4,5]pyrazino[2,3-c]pyridazin-8-yl)méthyl)-3-azabicyclo[3.1.0]hexan-3-yl)méthyl)-3,3-difluoropipéridin-1-yl)-1-oxoisoindolin-2-yl)pipéridine-2,6-dione ;
3-(6-(4-(((1R,5S,6r)-6-(((S)-2-(3,5-difluoro-2-hydroxyphényl)-5,6,6a,7,9,10-hexahydro-8H-pyrazino[1',2':4,5]pyrazino[2,3-c]pyridazin-8-yl)méthyl)-3-azabicyclo[3.1.0]hexan-3-yl)méthyl)-3,3-difluoropipéridin-1-yl)-1-oxoisoindolin-2-yl)pipéridine-2,6-dione ;
3-(6-((rac-3R,4R)-4-(((3S,5R)-4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)-3-fluoropipéridin-1-yl)-1-oxoisoindolin-2-yl)pipéridine-2,6-dione ;
3-(6-((3R,4R)-3-fluoro-4-(((3S,5R)-4-((S)-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((3R,5R)-4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(5-((S)-2-(((3R,5R)-4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)morpholino)-1-oxoisoindolin-2-yl)pipéridine-2,6-dione ;
3-(6-(4-(((3S,5S)-4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-((1-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)pipéridin-4-yl)méthyl)pipérazin-1-yl)-1-oxoisoindolin-2-yl)pipéridine-2,6-dione ;
3-(6-(2-(((1R,5S,6s)-6-(((S)-2-(3,5-difluoro-2-hydroxyphényl)-5,6,6a,7,9,10-hexahydro-8H-pyrazino[1',2':4,5]pyrazino[2,3-c]pyridazin-8-yl)méthyl)-3-azabicyclo[3.1.0]hexan-3-yl)méthyl)morpholino)-1-oxoisoindolin-2-yl)pipéridine-2,6-dione ;
3-(6-(2-(((1R,5S,6r)-6-(((6aS,9S)-2-(3,5-difluoro-2-hydroxyphényl)-9-méthyl-5,6,6a,7,9,10-hexahydro-8H-pyrazino[1',2':4,5]pyrazino[2,3-c]pyridazin-8-yl)méthyl)-3-azabicyclo[3.1.0]hexan-3-yl)méthyl)morpholino)-1-oxoisoindolin-2-yl)pipéridine-2,6-dione ;
3-(5-((S)-2-(((3R,4R)-4-(((S)-2-(3,5-difluoro-2-hydroxyphényl)-5,6,6a,7,9,10-hexahydro-8H-pyrazino[1',2':4,5]pyrazino[2,3-c]pyridazin-8-yl)méthyl)-3-fluoropipéridin-1-yl)méthyl)morpholino)-1-oxoisoindolin-2-yl)pipéridine-2,6-dione ;
3-(6-((S)-3-(((R)-3-(((S)-2-(3,5-difluoro-2-hydroxyphényl)-5,6,6a,7,9,10-hexahydro-8H-pyrazino[1',2':4,5]pyrazino[2,3-c]pyridazin-8-yl)méthyl)pyrrolidin-1-yl)méthyl)pyrrolidin-1-yl)-1-oxoisoindolin-2-yl)pipéridine-2,6-dione ;
3-(5-((S)-3-((4-(((S)-2-(3,5-difluoro-2-hydroxyphényl)-5,6,6a,7,9,10-hexahydro-8H-pyrazino[1',2':4,5]pyrazino[2,3-c]pyridazin-8-yl)méthyl)pipéridin-1-yl)méthyl)pyrrolidin-1-yl)-1-oxoisoindolin-2-yl)pipéridine-2,6-dione ;
3-(6-(4-(((1S,4r)-4-(((S)-2-(3,5-difluoro-2-hydroxyphényl)-5,6,6a,7,9,10-hexahydro-8H-pyrazino[1',2':4,5]pyrazino[2,3-c]pyridazin-8-yl)méthyl)cyclohexyl)méthyl)pipérazin-1-yl)-1-oxoisoindolin-2-yl)pipéridine-2,6-dione ;
3-(6-(4-((4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-4-fluoropipéridin-1-yl)méthyl)pipéridin-1-yl)-1-oxoisoindolin-2-yl)pipéridine-2,6-dione ;
3-(6-(4-(((1R,4s)-4-(((S)-2-(3,5-difluoro-2-hydroxyphényl)-5,6,6a,7,9,10-hexahydro-8H-pyrazino[1',2':4,5]pyrazino[2,3-c]pyridazin-8-yl)méthyl)cyclohexyl)méthyl)pipérazin-1-yl)-1-oxoisoindolin-2-yl)pipéridine-2,6-dione ;
3-(6-(4-(((2S,5R)-4-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-2,5-diméthyl-pipérazin-1-yl)méthyl)pipéridin-1-yl)-1-oxoisoindolin-2-yl)pipéridine-2,6-dione ;
3-(6-(4-(((1S,4r)-4-(((S)-2-(3-fluoro-2-hydroxyphényl)-5,6,6a,7,9,10-hexahydro-8H-pyrazino[1',2':4,5]pyrazino[2,3-c]pyridazin-8-yl)méthyl)cyclohexyl)méthyl)pipérazin-1-yl)-1-oxoisoindolin-2-yl)pipéridine-2,6-dione ;
3-(6-(4-(((3R,5S)-4-(2-(3,5-difluoro-2-hydroxyphényl)-6a-éthyl-6,6a,7,8,9,10-hexahydro-5H-pyrazino[1',2':4,5]pyrazino[2,3-c]pyridazine-8-carbonyl)-3,5-diméthyl-pipérazin-1-yl)méthyl)pipéridin-1-yl)-1-oxoisoindolin-2-yl)pipéridine-2,6-dione ;
3-(6-(4-(((3R,5S)-4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthyl-pipérazin-1-yl)méthyl)pipéridin-1-yl)-1-oxoisoindolin-2-yl)pipéridine-2,6-dione ;
3-(6-(4-(((3R,5S)-4-(2-(3,5-difluoro-2-hydroxyphényl)-6a-éthyl-6,6a,7,8,9,10-hexahydro-5H-pyrazino[1',2':4,5]pyrazino[2,3-c]pyridazine-8-carbonyl)-3,5-diméthyl-pipérazin-1-yl)méthyl)pipéridin-1-yl)-1-oxoisoindolin-2-yl)pipéridine-2,6-dione ;
3-(6-(4-(((3R,5S)-4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthyl-pipérazin-1-yl)méthyl)pipéridin-1-yl)-1-oxoisoindolin-2-yl)pipéridine-2,6-dione ;
3-(6-(4-(((3R,5R)-4-(2-(3-chloro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthyl-pipérazin-1-yl)méthyl)pipéridin-1-yl)-1-oxoisoindolin-2-yl)pipéridine-2,6-dione ;
3-(6-(4-(((3R,5S)-4-(2-(3,5-difluoro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthyl-pipérazin-1-yl)méthyl)pipéridin-1-yl)-1-oxoisoindolin-2-yl)pipéridine-2,6-dione ;
ou un sel pharmaceutiquement acceptable de l'un quelconque de ces composés ; ou qui est :
(S)-3-(6-(4-((4-((R)-6a-(difluorométhyl)-2-(3-fluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
(S)-3-(6-(4-(((3S,5S)-4-((R)-6a-(difluorométhyl)-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
(S)-3-(6-(4-(((3R,5R)-4-((R)-6a-(difluorométhyl)-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
(S)-3-(6-(4-(((2S,5R)-4-((R)-6a-(difluorométhyl)-2-(3-fluoro-2-hydroxyphényl)-6,6a,7,8,9,10-hexahydro-5H-pyrazino[1',2':4,5]pyrazino[2,3-c]pyridazine-8-carbonyl)-2,5-diméthylpipérazin-1-yl)méthyl)pipéridin-1-yl)-1-oxoisoindolin-2-yl)pipéridine-2,6-dione ;
3-(6-(4-(((3R,5S)-4-((S)-6a-(difluorométhyl)-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((3R,5S)-4-((R)-6a-(difluorométhyl)-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
(S)-3-(6-(4-(((2R,4S,6S)-1-((S)-6a-(difluorométhyl)-2-(3-fluoro-2-hydroxyphényl)-6,6a,7,8,9,10-hexahydro-5H-pyrazino[1',2':4,5]pyrazino[2,3-c]pyridazine-8-carbonyl)-2,6-diméthylpipéridin-4-yl)méthyl)pipérazin-1-yl)-1-oxoisoindolin-2-yl)pipéridine-2,6-dione ;
(S)-3-(6-(4-(((2R,4R,6S)-1-((R)-6a-(difluorométhyl)-2-(3-fluoro-2-hydroxyphényl)-6,6a,7,8,9,10-hexahydro-5H-pyrazino[1',2':4,5]pyrazino[2,3-c]pyridazine-8-carbonyl)-2,6-diméthylpipéridin-4-yl)méthyl)pipérazin-1-yl)-1-oxoisoindolin-2-yl)pipéridine-2,6-dione ;
(3S)-3-(6-(4-((1-((S)-6a-(difluorométhyl)-2-(3-fluoro-2-hydroxyphényl)-6,6a,7,8,9,10-hexahydro-5H-pyrazino[1',2':4,5]pyrazino[2,3-c]pyridazine-8-carbonyl)-2,2-diméthylpipéridin-4-yl)méthyl)pipérazin-1-yl)-1-oxoisoindolin-2-yl)pipéridine-2,6-dione ;
(3S)-3-(6-(4-((4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
(3S)-3-(6-(4-(((3R,5R)-4-(2-(3-chloro-2-hydroxyphényl)-6a-éthyl-6,6a,7,8,9,10-hexahydro-5H-pyrazino[1',2':4,5]pyrazino[2,3-c]pyridazine-8-carbonyl)-3,5-diméthyl-pipérazin-1-yl)méthyl)pipéridin-1-yl)-1-oxoisoindolin-2-yl)pipéridine-2,6-dione ;
(3S)-3-(6-(4-((4-(2-(3-chloro-2-hydroxyphényl)-6a-éthyl-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
(3S)-3-(6-(4-(((2S,5R)-4-(2-(3-chloro-2-hydroxyphényl)-6a-éthyl-6,6a,7,8,9,10-hexahydro-5H-pyrazino[1',2':4,5]pyrazino[2,3-c]pyridazine-8-carbonyl)-2,5-diméthyl-pipérazin-1-yl)méthyl)pipéridin-1-yl)-1-oxoisoindolin-2-yl)pipéridine-2,6-dione ;
(3S)-3-(6-(4-(((2S,5R)-4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-2,5-diméthylpipérazin-1-yl)méthyl)pipéridin-1-yl)-1-oxoisoindolin-2-yl)pipéridine-2,6-dione ;
3-(6-(4-((4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(4-((4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(4-(((3S,5S)-4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((3R,5R)-4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((3R,5R)-4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((2S,5R)-4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-2,5-diméthylpipérazin-1-yl)méthyl)pipéridin-1-yl)-1-oxoisoindolin-2-yl)pipéridine-2,6-dione ;
3-(6-(4-(((3R,5S)-4-(2-(3,5-difluoro-2-hydroxyphényl)-6a-éthyl-6,6a,7,8,9,10-hexahydro-5H-pyrazino[1',2':4,5]pyrazino[2,3-c]pyridazine-8-carbonyl)-3,5-diméthyl-pipérazin-1-yl)méthyl)pipéridin-1-yl)-1-oxoisoindolin-2-yl)pipéridine-2,6-dione ;
3-(6-(4-(((3R,5S)-4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
3-(6-(4-(((3R,5S)-4-(2-(3,5-difluoro-2-hydroxyphényl)-6a-éthyl-6,6a,7,8,9,10-hexahydro-5H-pyrazino[1',2':4,5]pyrazino[2,3-c]pyridazine-8-carbonyl)-3,5-diméthyl-pipérazin-1-yl)méthyl)pipéridin-1-yl)-1-oxoisoindolin-2-yl)pipéridine-2,6-dione ;
3-(6-(4-(((3R,5S)-4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
(3S)-3-(6-(4-((4-(2-(3-fluoro-2-hydroxyphényl)-6a-(fluorométhyl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
(3S)-3-(6-(4-((4-(2-(3-fluoro-2-hydroxyphényl)-6a-(fluorométhyl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
3-(6-(4-(((2S,5R)-4-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-2,5-diméthylpipérazin-1-yl)méthyl)pipéridin-1-yl)-1-oxoisoindolin-2-yl)pipéridine-2,6-dione ;
3-(6-(4-(((2S,5R)-4-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-2,5-diméthylpipérazin-1-yl)méthyl)pipéridin-1-yl)-1-oxoisoindolin-2-yl)pipéridine-2,6-dione ;
3-(6-(4-(((3R,5S)-4-(2-(3-chloro-2-hydroxyphényl)-6a-méthyl-6,6a,7,8,9,10-hexahydro-5H-pyrazino[1',2':4,5]pyrazino[2,3-c]pyridazine-8-carbonyl)-3,5-diméthyl-pipérazin-1-yl)méthyl)pipéridin-1-yl)-1-oxoisoindolin-2-yl)pipéridine-2,6-dione ;
((2S,6R)-2,6-diméthylpipérazin-1-yl)(2-(3-fluoro-2-hydroxyphényl)-6a-méthyl-5,6,6a,7,9,10-hexahydro-8H-pyrazino[1',2':4,5]pyrazino[2,3-c]pyridazin-8-yl)méthanone ;
((2S,6R)-2,6-diméthylpipérazin-1-yl)(2-(3-fluoro-2-hydroxyphényl)-6a-méthyl-5,6,6a,7,9,10-hexahydro-8H-pyrazino[1',2':4,5]pyrazino[2,3-c]pyridazin-8-yl)méthanone ;
(3S)-3-(6-(4-(((3S,5R)-4-(2-(3-fluoro-2-hydroxyphényl)-6a-(trifluorométhyl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
(3S)-3-(6-(4-(((3R,5R)-4-(2-(3-fluoro-2-hydroxyphényl)-6a-(trifluorométhyl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
(3S)-3-(6-(4-(((3R,5R)-4-(2-(3-fluoro-2-hydroxyphényl)-6a-(trifluorométhyl)-6,6a,7,8,9,10-hexahydro-5H-pyrazino[1',2':4,5]pyrazino[2,3-c]pyridazine-8-carbonyl)-3,5-diméthylpipérazin-1-yl)méthyl)pipéridin-1-yl)-1-oxoisoindolin-2-yl)pipéridine-2,6-dione ;
(3S)-3-(6-(4-(((2R,4r,6S)-1-(2-(3-fluoro-2-hydroxyphényl)-6a-(trifluorométhyl)-6,6a,7,8,9,10-hexahydro-5H-pyrazino[1',2':4,5]pyrazino[2,3-c]pyridazine-8-carbonyl)-2,6-diméthylpipéridin-4-yl)méthyl)pipérazin-1-yl)-1-oxoisoindolin-2-yl)pipéridine-2,6-dione ;
(3S)-3-(6-(4-(((2R,4r,6S)-1-(2-(3-fluoro-2-hydroxyphényl)-6a-(trifluorométhyl)-6,6a,7,8,9,10-hexahydro-5H-pyrazino[1',2':4,5]pyrazino[2,3-c]pyridazine-8-carbonyl)-2,6-diméthylpipéridin-4-yl)méthyl)pipérazin-1-yl)-1-oxoisoindolin-2-yl)pipéridine-2,6-dione ;
(3S)-3-(6-(4-((4-(2-(3-fluoro-2-hydroxyphényl)-6a-(trifluoro-méthyl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
(3S)-3-(6-(4-((4-(2-(3-fluoro-2-hydroxyphényl)-6a-(trifluoro-méthyl)-6,6a,7,8,9,10-hexahydro-5H-pyrazino[1',2':4,5]pyrazino[2,3-c]pyridazine-8-carbonyl)-3,3-diméthylpipérazin-1-yl)méthyl)pipéridin-1-yl)-1-oxoisoindolin-2-yl)pipéridine-2,6-dione ;
(3S)-3-(6-(4-(((1s,4R)-4-(2-(3,5-difluoro-2-hydroxyphényl)-6a-éthyl-6,6a,7,8,9,10-hexahydro-5H-pyrazino[1',2':4,5]pyrazino[2,3-c]pyridazine-8-carbonyl)-4-fluorocyclohexyl)méthyl)pipérazin-1-yl)-1-oxoisoindolin-2-yl)pipéridine-2,6-dione ;
(S)-3-(6-(4-(((1R,4R)-4-((S)-2-(3,5-difluoro-2-hydroxyphényl)-6a-éthyl-6,6a,7,8,9,10-hexahydro-5H-pyrazino[1',2':4,5]pyrazino[2,3-c]pyridazine-8-carbonyl)-4-fluorocyclohexyl)méthyl)pipérazin-1-yl)-1-oxoisoindolin-2-yl)pipéridine-2,6-dione ;
(S)-3-(6-(4-((4-((R)-2-(3,5-difluoro-2-hydroxyphényl)-6a-(difluorométhyl)-6,6a,7,8,9,10-hexahydro-5H-pyrazino[1',2':4,5]pyrazino[2,3-c]pyridazine-8-carbonyl)-4-fluoropipéridin-1-yl)méthyl)pipéridin-1-yl)-1-oxoisoindolin-2-yl)pipéridine-2,6-dione ;
(3S)-3-(6-(4-((4-(2-(3-chloro-2-hydroxyphényl)-6a-éthyl-6,6a,7,8,9,10-hexahydro-5H-pyrazino[1',2':4,5]pyrazino[2,3-c]pyridazine-8-carbonyl)-4-fluoropipéridin-1-yl)méthyl)pipéridin-1-yl)-1-oxoisoindolin-2-yl)pipéridine-2,6-dione ;
(3S)-3-(6-(4-((4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-4-fluoropipéridin-1-yl)méthyl)pipéridin-1-yl)-1-oxoisoindolin-2-yl)pipéridine-2,6-dione ;
(3S)-3-(6-(4-((4-(6a-éthyl-2-(3-fluoro-2-hydroxyphényl)-6,6a,7,8,9,10-hexahydro-5H-pyrazino[1',2':4,5]pyrazino[2,3-c]pyridazine-8-carbonyl)-4-fluoropipéridin-1-yl)méthyl)pipéridin-1-yl)-1-oxoisoindolin-2-yl)pipéridine-2,6-dione ;
(3S)-3-(6-(4-((4-(2-(3,5-difluoro-2-hydroxyphényl)-6a-éthyl-6,6a,7,8,9,10-hexahydro-5H-pyrazino[1',2':4,5]pyrazino[2,3-c]pyridazine-8-carbonyl)-4-fluoropipéridin-1-yl)méthyl)pipéridin-1-yl)-1-oxoisoindolin-2-yl)pipéridine-2,6-dione ;
(3S)-3-(6-(4-((4-(2-(3,5-difluoro-2-hydroxyphényl)-6a-éthyl-6,6a,7,8,9,10-hexahydro-5H-pyrazino[1',2':4,5]pyrazino[2,3-c]pyridazine-8-carbonyl)-4-fluoropipéridin-1-yl)méthyl)pipéridin-1-yl)-1-oxoisoindolin-2-yl)pipéridine-2,6-dione ;
(3S)-3-(6-(4-((4-fluoro-4-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)pipéridin-1-yl)méthyl)pipéridin-1-yl)-1-oxoisoindolin-2-yl)pipéridine-2,6-dione ;
(3S)-3-(6-(4-((4-(2-(3,5-difluoro-2-hydroxyphényl)-6a-méthyl-6,6a,7,8,9,10-hexahydro-5H-pyrazino[1',2':4,5]pyrazino[2,3-c]pyridazine-8-carbonyl)-4-fluoropipéridin-1-yl)méthyl)pipéridin-1-yl)-1-oxoisoindolin-2-yl)pipéridine-2,6-dione ;
(3S)-3-(6-(4-((4-(2-(3,5-difluoro-2-hydroxyphényl)-6a-méthyl-6,6a,7,8,9,10-hexahydro-5H-pyrazino[1',2':4,5]pyrazino[2,3-c]pyridazine-8-carbonyl)-4-fluoropipéridin-1-yl)méthyl)pipéridin-1-yl)-1-oxoisoindolin-2-yl)pipéridine-2,6-dione ;
3-(6-(4-(((1S,4r)-4-((S)-2-(3-fluoro-2-hydroxyphényl)-6,6a,7,8,9,10-hexahydro-5H-pyrazino[1',2':4,5]pyrazino[2,3-c]pyridazine-8-carbonyl)cyclohexyl)méthyl)pipérazin-1-yl)-1-oxoisoindolin-2-yl)pipéridine-2,6-dione ;
(S)-3-(6-(4-(((1S,4S)-4-(((S)-2-(3-fluoro-2-hydroxyphényl)-5,6,6a,7,9,10-hexahydro-8H-pyrazino[1',2':4,5]pyrazino[2,3-c]pyridazin-8-yl)méthyl)cyclohexyl)méthyl)pipérazin-1-yl)-1-oxoisoindolin-2-yl)pipéridine-2,6-dione ;
3-(6-(4-(((1R,4r)-4-(((R)-2-(3-fluoro-2-hydroxyphényl)-6a-méthyl-5,6,6a,7,9,10-hexahydro-8H-pyrazino[1',2':4,5]pyrazino[2,3-c]pyridazin-8-yl)méthyl)cyclohexyl)méthyl)pipérazin-1-yl)-1-oxoisoindolin-2-yl)pipéridine-2,6-dione ;
3-(6-(4-(((1R,4r)-4-(((R)-2-(3-fluoro-2-hydroxyphényl)-6a-méthyl-5,6,6a,7,9,10-hexahydro-8H-pyrazino[1',2':4,5]pyrazino[2,3-c]pyridazin-8-yl)méthyl)cyclohexyl)méthyl)pipérazin-1-yl)-1-oxoisoindolin-2-yl)pipéridine-2,6-dione ;
(S)-3-(6-(4-(((1R,4r)-4-(((R)-2-(3-fluoro-2-hydroxyphényl)-6a-méthyl-5,6,6a,7,9,10-hexahydro-8H-pyrazino[1',2':4,5]pyrazino[2,3-c]pyridazin-8-yl)méthyl)cyclohexyl)méthyl)pipérazin-1-yl)-1-oxoisoindolin-2-yl)pipéridine-2,6-dione ;
(S)-3-(6-(4-(((1R,4r)-4-(((R)-2-(3-fluoro-2-hydroxyphényl)-6a-méthyl-5,6,6a,7,9,10-hexahydro-8H-pyrazino[1',2':4,5]pyrazino[2,3-c]pyridazin-8-yl)méthyl)cyclohexyl)méthyl)pipérazin-1-yl)-1-oxoisoindolin-2-yl)pipéridine-2,6-dione ;
3-(6-(4-((4-((S)-2-(3,5-difluoro-2-hydroxyphényl)-6,6a,7,8,9,10-hexahydro-5H-pyrazino[1',2':4,5]pyrazino[2,3-c]pyridazine-8-carbonyl)-3,3-diméthylpipéridin-1-yl)méthyl)pipéridin-1-yl)-1-oxoisoindolin-2-yl)pipéridine-2,6-dione ;
(S)-3-(6-(4-(((2R,4R,6S)-1-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-2,6-diméthylpipéridin-4-yl)méthyl)pipérazin-1-yl)-1-oxoisoindolin-2-yl)pipéridine-2,6-dione ;
(S)-3-(6-(4-(((2R,4R,6S)-1-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-2,6-diméthylpipéridin-4-yl)méthyl)pipérazin-1-yl)-1-oxoisoindolin-2-yl)pipéridine-2,6-dione ;
3-(6-(4-((1-((S)-2-(3-fluoro-2-hydroxyphényl)-6,6a,7,8,9,10-hexahydro-5H-pyrazino[1',2':4,5]pyrazino[2,3-c]pyridazine-8-carbonyl)-2,2-diméthylpipéridin-4-yl)méthyl)pipérazin-1-yl)-1-oxoisoindolin-2-yl)pipéridine-2,6-dione ;
(3S)-3-(6-(4-((1-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-2,2-diméthylpipéridin-4-yl)méthyl)pipérazin-1-yl)-1-oxoisoindolin-2-yl)pipéridine-2,6-dione ;
(3S)-3-(6-(4-((1-(2-(3-fluoro-2-hydroxyphényl)-6a-méthyl-6,6a,7,8,9,10-hexahydro-5H-pyrazino[1',2':4,5]pyrazino[2,3-c]pyridazine-8-carbonyl)-2,2-diméthylpipéridin-4-yl)méthyl)pipérazin-1-yl)-1-oxoisoindolin-2-yl)pipéridine-2,6-dione ;
ou un sel pharmaceutiquement acceptable de l'un quelconque de ces composés.

14. Composition pharmaceutique comprenant un composé selon l'une quelconque des revendications 1 à 12, ou un sel pharmaceutiquement acceptable de celui-ci, et un excipient pharmaceutiquement acceptable.

15. Composé, ou sel pharmaceutiquement acceptable de celui-ci, selon l'une quelconque des revendications 1 à 13, ou composition pharmaceutique selon la revendication 14, pour utilisation dans un procédé de traitement d'un cancer chez un sujet en ayant besoin, où le procédé comprend l'administration au sujet d'un composé selon l'une quelconque des revendications 1 à 13, ou d'un sel pharmaceutiquement acceptable de celui-ci, ou d'une composition pharmaceutique selon la revendication 14, facultativement où :
(a) le cancer est un cancer avec délétion de SMARCA4 ; ou
(b) le cancer est choisi parmi un carcinome épidermoïde, un carcinome basocellulaire, un adénocarcinome, les carcinomes hépatocellulaires, et les carcinomes à cellules rénales, un cancer de la vessie, de l'intestin, du sein, du col de l'utérus, du côlon, de l'œsophage, de la tête, du rein, du foie, du poumon, du cou, de l'ovaire, du pancréas, de la prostate, et de l'estomac ; les leucémies ; les lymphomes bénins et les lymphomes malins, en particulier le lymphome de Burkitt et un lymphome non hodgkinien ; les mélanomes bénins et les mélanomes malins ; les maladies myéloprolifératives ; les sarcomes, incluant le sarcome d'Ewing, l'hémangiosarcome, le sarcome de Kaposi, le liposarcome, les myosarcomes, le neuroépithéliome périphérique, le sarcome synovial, les gliomes, les astrocytomes, les oligodendrogliomes, les épendymomes, les glioblastomes, les neuroblastomes, les ganglioneuromes, les gangliogliomes, les médulloblastomes, les tumeurs à cellules pinéales, les méningiomes, les sarcomes méningés, les neurofibromes et les Schwannomes ; un cancer de l'intestin, un cancer du sein, un cancer de la prostate, un cancer du col de l'utérus, un cancer de l'utérus, un cancer du poumon, un cancer de l'ovaire, un cancer du testicule, un cancer de la thyroïde, un astrocytome, un cancer de l'œsophage, un cancer du pancréas, un cancer de l'estomac, un cancer du foie, un cancer du côlon, un mélanome ; un carcinosarcome, la maladie de Hodgkin, la tumeur de Wilms et les tératocarcinomes,
éventuellement où le cancer du poumon est un cancer du poumon non à petites cellules déficient en SMARCA4 ; ou
(c) le cancer est choisi parmi une leucémie lymphoblastique aiguë de lignée T (LLA-T), un lymphome lymphoblastique de lignée T (LL-T), un lymphome à cellules T périphériques, une leucémie à cellules T de l'adulte, une LLA pré-B, les lymphomes pré-B, un lymphome à grandes cellules B, un lymphome de Burkitt, une LLA à cellules B, une LLA à chromosome Philadelphie positif et une LMC à chromosome Philadelphie positif.
